# EUROPÄISCHE PATENTSCHRIFT

(11) **EP 3 677 659 B1**
(45) Veröffentlichungstag und Bekanntmachung des Hinweises auf die Patenterteilung: **06.04.2022**
(21) Anmeldenummer: 20155207.2
(22) Anmeldetag: 12.03.2015
(51) Int. Cl.: C09K 19/12, C09K 19/34, C09K 19/04, C07D 307/91, C09K 19/30

(54) **FLÜSSIGKRISTALLINES MEDIUM**
LIQUID CRYSTALLINE MEDIUM
MILIEU À CRISTAUX LIQUIDES

(30) Priorität: 17.03.2014 DE 102014003613; 17.09.2014 US 201462051483 P
(43) Veröffentlichungstag der Anmeldung: 08.07.2020
(62) Teilanmeldung aus: 15710433.2
(73) Patentinhaber: Merck Patent GmbH, 64293 Darmstadt (DE)
(72) Erfinder: Hirschmann, Harald, 64291 DARMSTADT (DE); Bauer, Monika, 63500 Seligenstadt (DE); Windhorst, Martina, 64839 MUENSTER (DE); Reuter, Marcus, 64295 Darmstadt (DE); Bremer, Matthias, 64295 DARMSTADT (DE); Fortte, Rocco, 65933 FRANKFURT AM MAIN (DE); Reiffenrath, Volker, 64380 ROSSDORF (DE); Engel, Martin, 64291 DARMSTADT (DE); John, Nico, 64850 SCHAAFHEIM (DE); Marten, Christoph, 64354 REINHEIM (DE)

(56) Entgegenhaltungen:
- WO-A1-02/055463
- WO-A1-2009/091884
- DE-A1-102006 036 184
- US-A1- 2005 258 399

## Beschreibung

Die Erfindung betrifft ein flüssigkristallines Medium, welches mindestens eine Verbindung der Formel I, 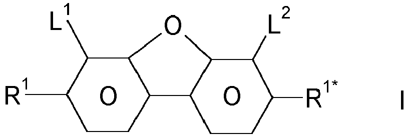 worin
- R¹ und R^{1*}: jeweils unabhängig voneinander einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -OCF₂-, -CH=CH-, 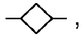 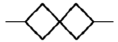 , -O-, -CO-O-, -O-CO- so ersetzt sein können, dass O-Atome nicht direkt miteinander verknüpft sind, und worin auch ein oder mehrere H-Atome durch Halogen ersetzt sein können,
- L¹ und L²: jeweils unabhängig voneinander F, Cl, CF₃ oder CHF₂, bedeuten,
enthält.

Derartige Medien sind insbesondere für elektrooptische Anzeigen mit einer Aktivmatrix-Adressierung basierend auf dem ECB-Effekt sowie für IPS-Anzeigen (In plane switching) oder FFS-Anzeigen (Fringe Field Switching) zu verwenden.

Das Prinzip der elektrisch kontrollierten Doppelbrechung, der ECB-Effekt (electrically controlled birefringence) oder auch DAP-Effekt (Deformation aufgerichteter Phasen) wurde erstmals 1971 beschrieben (M.F. Schieckel und K. Fahrenschon, "Deformation of nematic liquid crystals with vertical orientation in electrical fields", Appl. Phys. Lett. 19 (1971), 3912). Es folgten Arbeiten von J.F. Kahn (Appl. Phys. Lett. 20 (1972), 1193) und G. Labrunie und J. Robert (J. Appl. Phys. 44 (1973), 4869).

Die Arbeiten von J. Robert und F. Clerc (SID 80 Digest Techn. Papers (1980), 30), J. Duchene (Displays 7 (1986), 3) und H. Schad (SID 82 Digest Techn. Papers (1982), 244) haben gezeigt, dass flüssigkristalline Phasen hohe Werte für das Verhältnis der elastischen Konstanten K₃/K₁, hohe Werte für die optische Anisotropie Δn und Werte für die dielektrische Anisotropie von Δε ≤ -0,5 aufweisen müssen, um für hochinformative Anzeigeelemente basierend auf dem ECB-Effekt eingesetzt werden zu können. Auf dem ECB-Effekt basierende elektrooptische Anzeigeelemente weisen eine homeotrope Randorientierung auf (VA-Technologie = Vertical Aligned). Auch bei Anzeigen, die den sogenannten IPS- oder FFS-Effekt verwenden, können dielektrisch negative Flüssigkristallmedien zum Einsatz kommen.

Anzeigen, die den ECB-Effekt verwenden, haben sich als sogenannte VAN- (Vertically Aligned Nematic) Anzeigen beispielsweise in den Bauformen MVA (Multi-Domain Vertical Alignment, z.B.: Yoshide, H. et al., Vortrag 3.1: "MVA LCD for Notebook or Mobile PCs ...", SID 2004 International Symposium, Digest of Technical Papers, XXXV, Buch I, S. 6 bis 9 und Liu, C.T. et al., Vortrag 15.1: "A 46-inch TFT-LCD HDTV Technnology ...", SID 2004 International Symposium, Digest of Technical Papers, XXXV, Buch II, S. 750 bis 753), PVA (Patterned Vertical Alignment, z.B.: Kim, Sang Soo, Vortrag 15.4: "Super PVA Sets New State-of-the-Art for LCD-TV", SID 2004 International Symposium, Digest of Technical Papers, XXXV, Buch II, S. 760 bis 763), ASV- (Advanced Super View, z.B.: Shigeta, Mitzuhiro und Fukuoka, Hirofumi, Vortrag 15.2: "Development of High Quality LCDTV", SID 2004 International Symposium, Digest of Technical Papers, XXXV, Buch II, S. 754 bis 757) Anzeigen, neben IPS (In Plane Switching) (z.B.: Yeo, S.D., Vortrag 15.3: "A LC Display for the TV Application", SID 2004 International Symposium, Digest of Technical Papers, XXXV, Buch II, S. 758 & 759) den lange bekannten TN- (Twisted Nematic) Anzeigen, als eine der drei zur Zeit wichtigsten neueren Typen von Flüssigkristallanzeigen, insbesondere für Fernsehanwendungen, etabliert. In allgemeiner Form werden die Technologien z.B. in Souk, Jun, SIDSeminar 2004, Seminar M-6: "Recent Advances in LCD Technology", Seminar Lecture Notes, M-6/1 bis M-6/26 und Miller, Ian, SIDSeminar 2004, Seminar M-7: "LCD-Television", Seminar Lecture Notes, M-7/1 bis M-7/32, verglichen. Obwohl die Schaltzeiten moderner ECB-Anzeigen durch Ansteuerungsmethoden mit Übersteuerung (overdrive) bereits deutlich verbessert wurden, z.B.: Kim, Hyeon Kyeong et al., Vortrag 9.1: "A 57-in. Wide UXGA TFT-LCD for HDTV Application", SID 2004 International Symposium, Digest of Technical Papers, XXXV, Buch I, S. 106 bis 109, ist die Erzielung von videotauglichen Schaltzeiten, insbesondere beim Schalten von Graustufen, immer noch ein noch nicht zufriedenstellend gelöstes Problem.

Für die technische Anwendung dieses Effektes in elektrooptischen Anzeigeelementen werden FK-Phasen benötigt, die einer Vielzahl von Anforderungen genügen müssen. Besonders wichtig sind hier die chemische Beständigkeit gegenüber Feuchtigkeit, Luft und physikalischen Einflüssen wie Wärme, Strahlung im infraroten, sichtbaren und ultravioletten Bereich und elektrische Gleich- und Wechselfelder.

Ferner wird von technisch verwendbaren FK-Phasen eine flüssigkristalline Mesophase in einem geeigneten Temperaturbereich und eine niedrige Viskosität gefordert.

In keiner der bisher bekannten Reihen von Verbindungen mit flüssigkristalliner Mesophase gibt es eine Einzelverbindung, die allen diesen Erfordernissen entspricht. Es werden daher in der Regel Mischungen von zwei bis 25, vorzugsweise drei bis 18, Verbindungen hergestellt, um als FK-Phasen verwendbare Substanzen zu erhalten. Optimale Phasen konnten jedoch auf diese Weise nicht leicht hergestellt werden, da bisher keine Flüssigkristallmaterialien mit deutlich negativer dielektrischer Anisotropie und ausreichender Langzeitstabilität zur Verfügung standen.

Matrix-Flüssigkristallanzeigen (MFK-Anzeigen) sind bekannt. Als nichtlineare Elemente zur individuellen Schaltung der einzelnen Bildpunkte können beispielsweise aktive Elemente (d.h. Transistoren) verwendet werden. Man spricht dann von einer "aktiven Matrix", wobei man zwei Typen unterscheiden kann:
1. MOS (Metal Oxide Semiconductor)-Transistoren auf Silizium-Wafer als Substrat.
2. Dünnfilm-Transistoren (TFT) auf einer Glasplatte als Substrat.

Bei Typ 1 wird als elektrooptischer Effekt üblicherweise die dynamische Streuung oder der Guest-Host-Effekt verwendet. Die Verwendung von einkristallinem Silizium als Substratmaterial beschränkt die Displaygröße, da auch die modulartige Zusammensetzung verschiedener Teildisplays an den Stößen zu Problemen führt.

Bei dem aussichtsreicheren Typ 2, welcher bevorzugt ist, wird als elektrooptischer Effekt üblicherweise der TN-Effekt verwendet.

Man unterscheidet zwei Technologien: TFT's aus Verbindungshalbleitern wie z.B. CdSe oder TFT's auf der Basis von polykristallinem oder amorphem Silizium. An letzterer Technologie wird weltweit mit großer Intensität gearbeitet.

Die TFT-Matrix ist auf der Innenseite der einen Glasplatte der Anzeige aufgebracht, während die andere Glasplatte auf der Innenseite die transparente Gegenelektrode trägt. Im Vergleich zu der Größe der Bildpunkt-Elektrode ist der TFT sehr klein und stört das Bild praktisch nicht. Diese Technologie kann auch für voll farbtaugliche Bilddarstellungen erweitert werden, wobei ein Mosaik von roten, grünen und blauen Filtern derart angeordnet ist, dass je ein Filterelement einem schaltbaren Bildelement gegenüber liegt.

Der Begriff MFK-Anzeigen umfasst hier jedes Matrix-Display mit integrierten nichtlinearen Elementen, d.h. neben der aktiven Matrix auch Anzeigen mit passiven Elementen wie Varistoren oder Dioden (MIM = Metall-Isolator-Metall).

Derartige MFK-Anzeigen eignen sich insbesondere für TV-Anwendungen (z.B. Taschenfernseher) oder für hochinformative Displays in Automobil- oder Flugzeugbau. Neben Problemen hinsichtlich der Winkelabhängigkeit des Kontrastes und der Schaltzeiten resultieren bei MFK-Anzeigen Schwierigkeiten bedingt durch einen nicht ausreichend hohen spezifischen Widerstand der Flüssigkristallmischungen [TOGASHI, S., SEKIGUCHI, K., TANABE, H., YAMAMOTO, E., SORIMACHI, K., TAJIMA, E., WATANABE, H., SHIMIZU, H., Proc. Eurodisplay 84, Sept. 1984: A 210-288 Matrix LCD Controlled by Double Stage Diode Rings, p. 141 ff, Paris; STROMER, M., Proc. Eurodisplay 84, Sept. 1984: Design of Thin Film Transistors for Matrix Adressing of Television Liquid Crystal Displays, p. 145 ff, Paris]. Mit abnehmendem Widerstand verschlechtert sich der Kontrast einer MFK-Anzeige. Da der spezifische Widerstand der Flüssig-kristallmischung durch Wechselwirkung mit den inneren Oberflächen der Anzeige im allgemeinen über die Lebenszeit einer MFK-Anzeige abnimmt, ist ein hoher (Anfangs)-Widerstand sehr wichtig für Anzeigen die akzep-table Widerstandswerte über eine lange Betriebsdauer aufweisen müssen.

Es besteht somit immer noch ein großer Bedarf nach MFK-Anzeigen mit sehr hohem spezifischen Widerstand bei gleichzeitig großem Arbeitstemperaturbereich, kurzen Schaltzeiten und niedriger Schwellenspannung, mit deren Hilfe verschiedene Graustufen erzeugt werden können.

Der Nachteil der häufig verwendeten MFK-TN-Anzeigen beruht in ihrem vergleichsweise niedrigen Kontrast, der relativ hohen Blickwinkelabhängigkeit und der Schwierigkeit in diesen Anzeigen Graustufen zu erzeugen.

Wesentlich bessere Blickwinkelabhängigkeiten weisen VA-Displays auf und werden daher hauptsächlich für Fernseher und Monitore verwendet. Hier besteht jedoch weiterhin der Bedarf die Schaltzeiten zu verbessern, insbesondere im Hinblick auf die Verwendung für Fernseher mit "Frame Rates" (Bildwechselfrequenz/Wiederholraten) von mehr als 60 Hz. Dabei dürfen allerdings die Eigenschaften, wie beispielsweise die Tieftemperaturstabilität nicht verschlechtert werden.

Der Erfindung liegt die Aufgabe zugrunde, Flüssigkristall-Mischungen, insbesondere für Monitor- und TV-Anwendungen, welche auf dem ECB- oder auf dem IPS- oder FFS-Effekt beruhen, bereitzustellen, die die oben angegebenen Nachteile nicht oder nur in geringerem Maße aufweisen. Insbesondere muss für Monitore und Fernseher gewährleistet sein, dass diese auch bei extrem hohen und extrem niedrigen Temperaturen arbeiten und gleichzeitig sehr niedrige Schaltzeiten aufweisen und gleichzeitig ein verbessertes Reliability-Verhalten, insbesondere kein oder ein deutlich verringertes Image-Sticking nach langen Laufzeiten aufweisen. Überraschenderweise ist es möglich die Rotationsviskositäten und damit die Schaltzeiten zu verbessern, wenn man polare Verbindungen der allgemeinen Formel I in Flüssigkristallmischungen verwendet, insbesondere in LC-Mischungen mit negativer dielektrischer Anisotropie, vorzugsweise für VA- und FFS-Displays. Gegenstand der Erfindung ist somit ein flüssigkristallines Medium, welches mindestens eine Verbindung der Formel I enthält. Verbindungen der Formel I sind ebenfalls Gegenstand der vorliegenden Erfindung.

Die Verbindungen der Formel I werden von der generischen Formel (I) in der WO 02/055463 A1 umfasst. Weitere strukturähnliche Verbindungen sind in der DE 10 2006 036184 A1 offenbart.

Die erfindungsgemäßen Mischungen zeigen vorzugsweise sehr breite nematische Phasenbereiche mit Klärpunkten ≥ 70 °C, vorzugsweise ≥ 75 °C, insbesondere ≥ 80 °C, sehr günstige Werte für die kapazitive Schwelle, relativ hohe Werte für die Holding Ratio und gleichzeitig sehr gute Tieftemperaturstabilitäten bei -20 °C und -30 °C sowie sehr geringe Rotationsviskositäten und niedrige Schaltzeiten. Die erfindungsgemäßen Mischungen zeichnen sich weiterhin dadurch aus, dass zusätzlich zur Verbesserung der Rotationsviskosität γ₁, relativ hohe Werte der elastischen Konstanten K₃₃ zur Verbesserung der Schaltzeiten zu beobachten sind. Durch den Einsatz der Verbindungen der Formel I in LC-Mischungen, vorzugsweise mit negativer dielektrischer Anisotropie wird das Verhältnis von Rotationsviskosität γ₁ und elastischen Konstanten Kᵢ verringert.

Einige bevorzugte Ausführungsformen der erfindungsgemäßen Mischungen werden im Folgenden genannt.

In den Verbindungen der Formel I bedeuten R¹ und R^{1*} jeweils unabhängig voneinander vorzugsweise geradkettiges Alkoxy, insbesondere OCH₃, n-C₂H₅O, n-OC₃H₇, n-OC₄H₉, n-OC₅H₁₁, n-OC₆H₁₃, weiterhin Alkenyl, insbesondere CH₂₌CH₂, CH₂CH=CH₂, CH₂CH=CHCH₃, CH₂CH=CHC₂H₅, verzweigtes Alkoxy, insbesondere OC₃H₆CH(CH₃)₂, sowie Alkenyloxy, insbesondere OCH=CH₂, OCH₂CH=CH₂, OCH₂CH=CHCH₃, OCH₂CH=CHC₂H₅.

Besonders bevorzugt bedeuten R¹ und R^{1*} jeweils unabhängig voneinander geradkettiges Alkoxy mit 1-6 C-Atomen, insbesondere Methoxy, Ethoxy, Propoxy, Butoxy, Pentoxy, Hexoxy.

L¹ und L² bedeuten vorzugsweise beide F.

Bevorzugte Verbindungen der Formel I sind die Verbindungen der Formeln I-1 bis I-10, 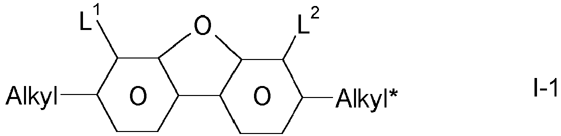 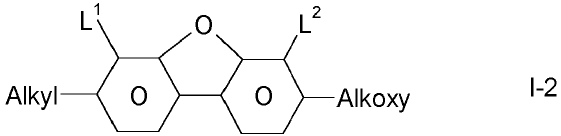 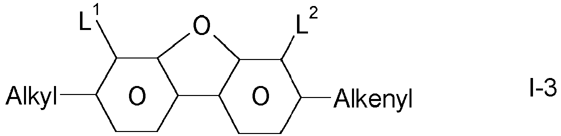 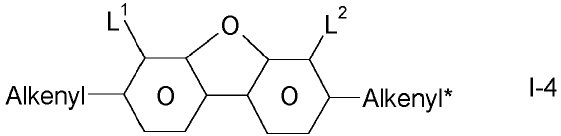 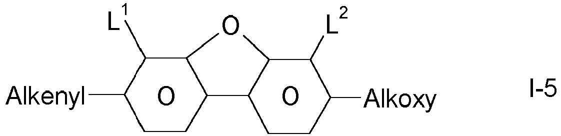 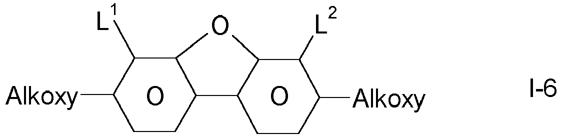 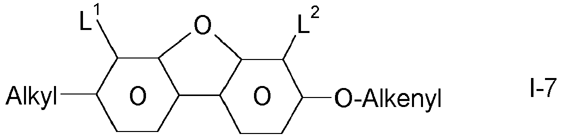 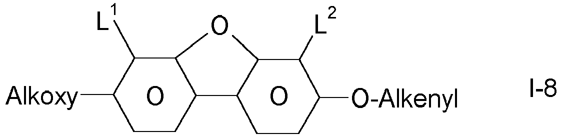 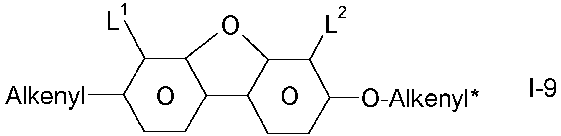 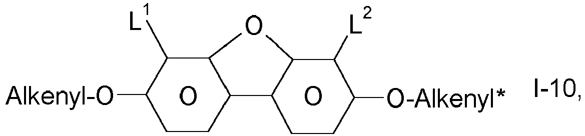 worin
Alkyl und Alkyl* jeweils unabhängig voneinander einen geradkettigen Alkylrest mit 1-6 C-Atomen, Alkenyl und Alkenyl* jeweils unabhängig voneinander einen geradkettigen Alkenylrest mit 2-6 C-Atomen, Alkoxy und Alkoxy* jeweils unabhängig voneinander einen geradkettigen Alkoxyrest mit 1-6 C-Atomen, und L¹ und L² jeweils unabhängig voneinander F oder Cl bedeuten.

In den Verbindungen der Formeln I-1 bis I-10 bedeuten L¹ und L² jeweils unabhängig voneinander vorzugsweise F oder Cl, insbesondere L¹ = L² = F. Besonders bevorzugt sind die Verbindungen der Formelnl-2 und I-6. Vorzugsweise bedeutet in den Verbindungen der Formeln I-2 und I-6 L¹ = L² = F.

Ganz besonders bevorzugt enthält die erfindungsgemäße Mischung mindestens eine Verbindung der Formeln I-1A, I-2A, I-4A, I-6A und/oder I-6B.

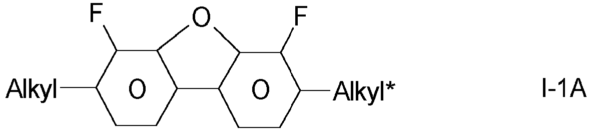

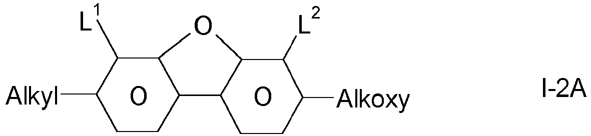

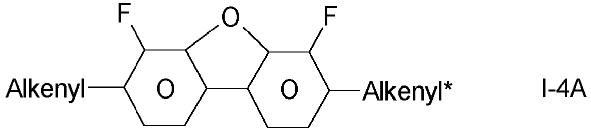

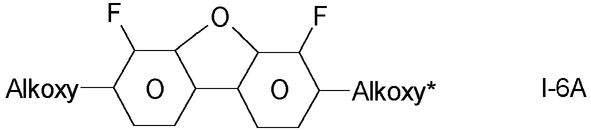

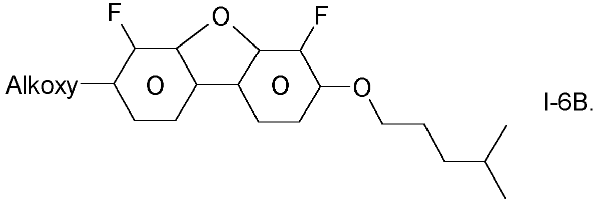

Ganz besonders bevorzugte Mischungen enthalten mindestens eine Verbindung der Formeln I-2.1 bis I-2.49 und I-6.1 bis I-6.28,

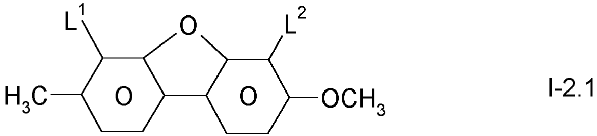

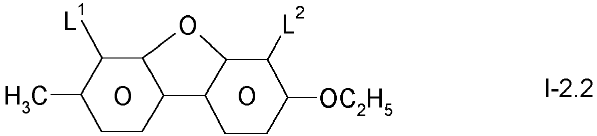

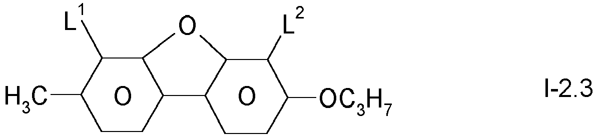

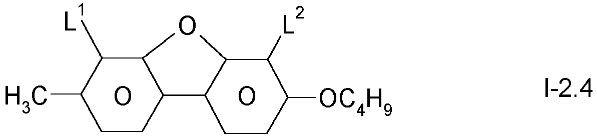

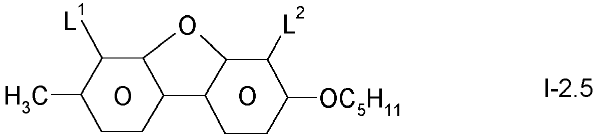

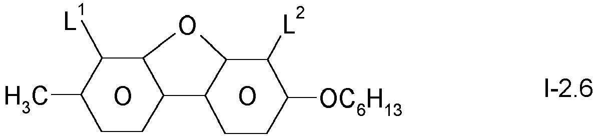

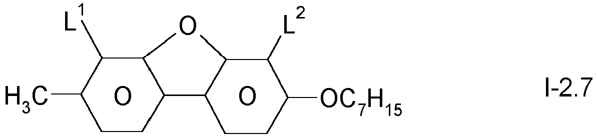

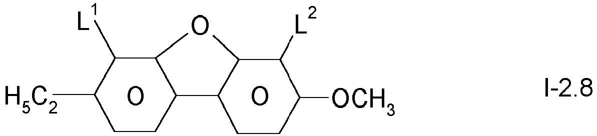

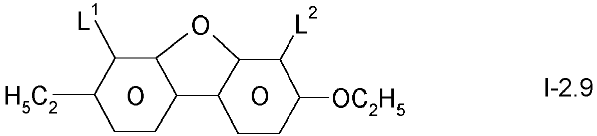

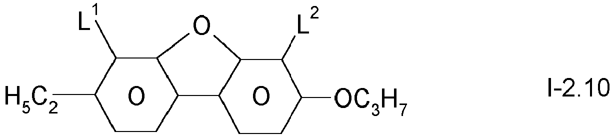

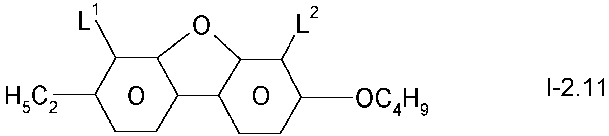

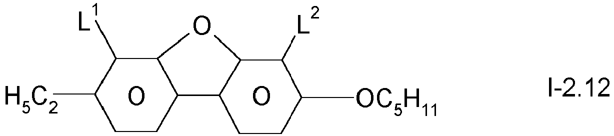

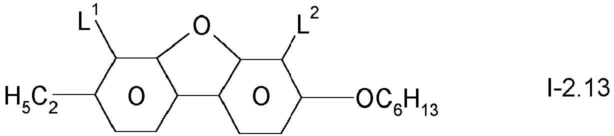

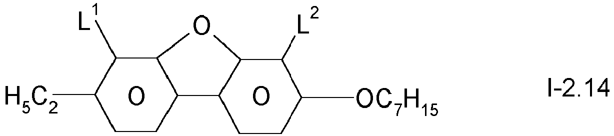

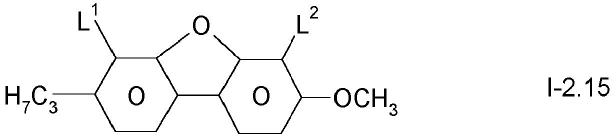

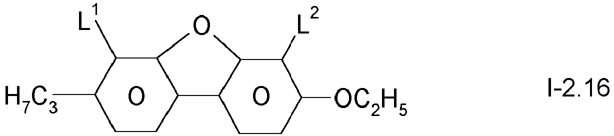

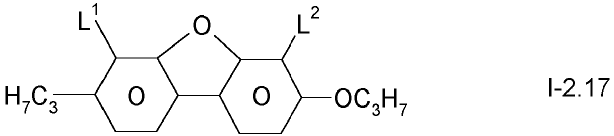

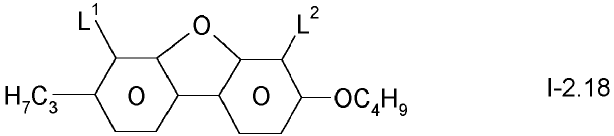

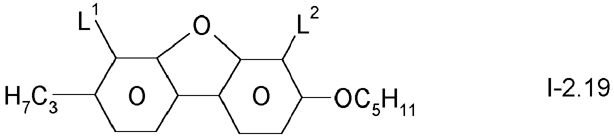

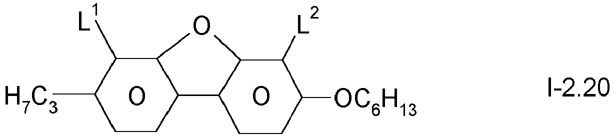

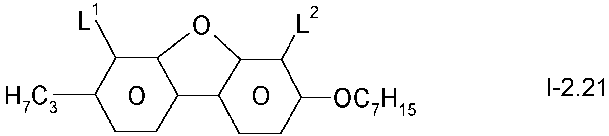

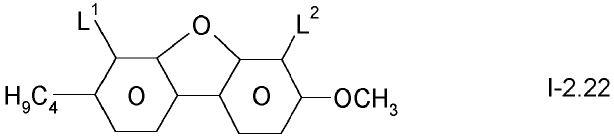

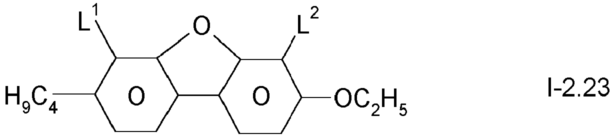

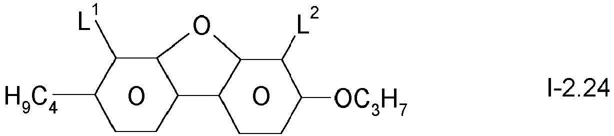

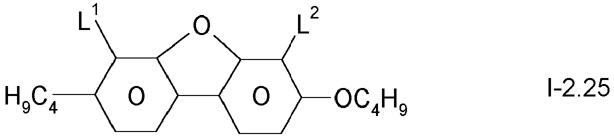

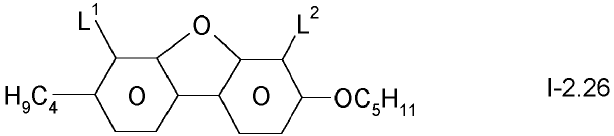

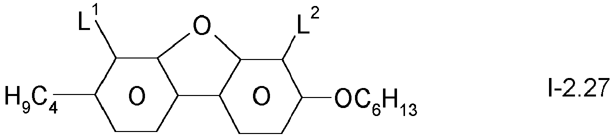

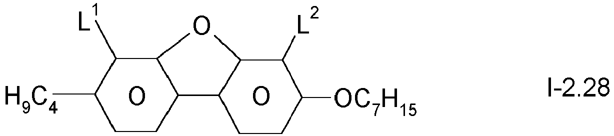

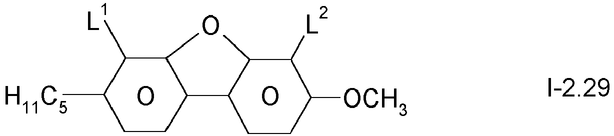

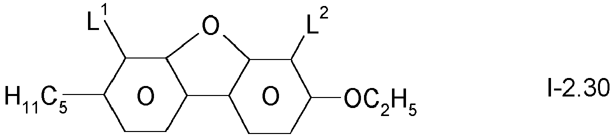

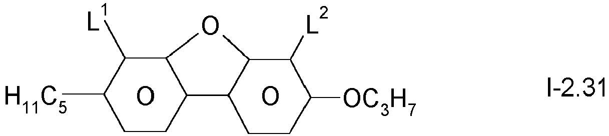

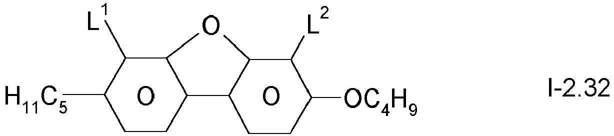

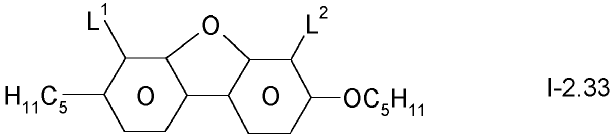

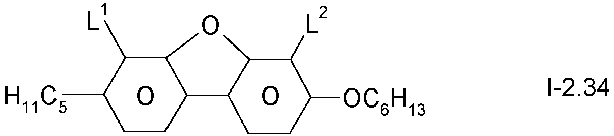

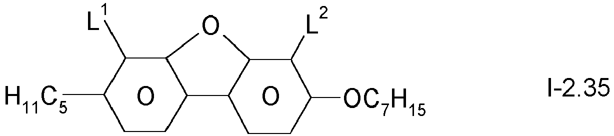

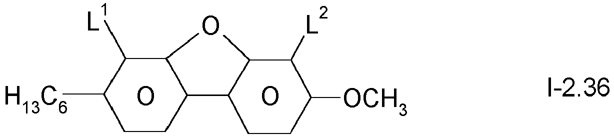

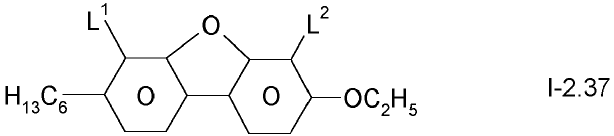

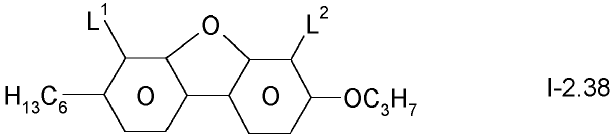

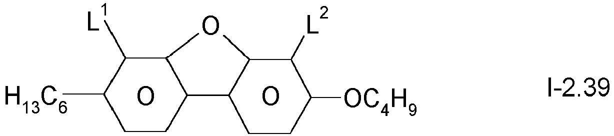

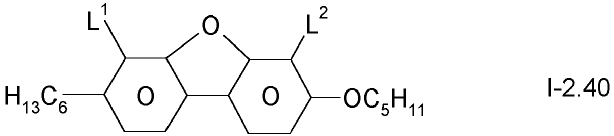

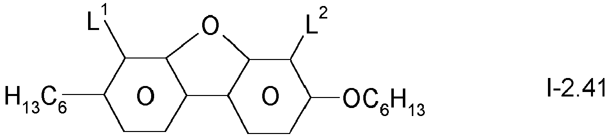

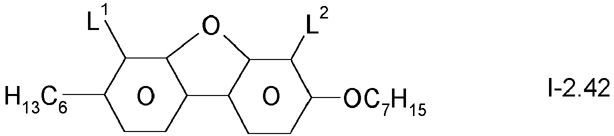

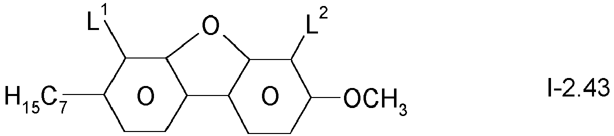

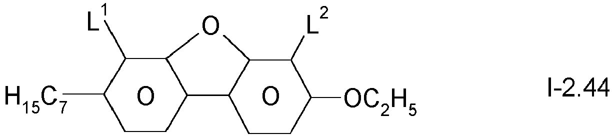

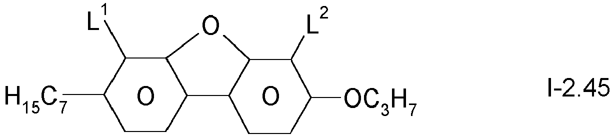

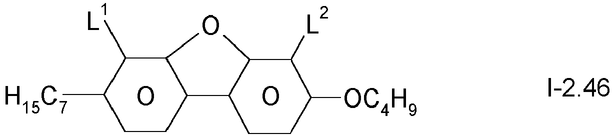

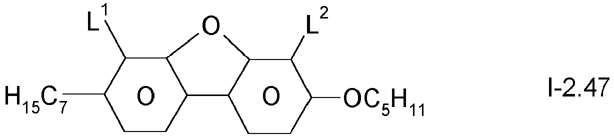

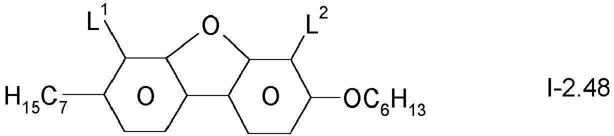

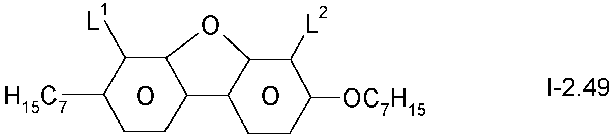

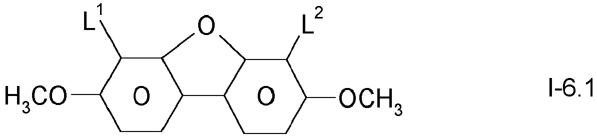

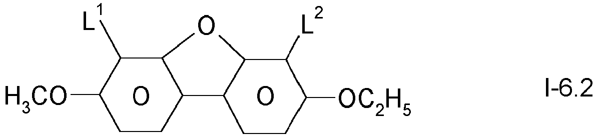

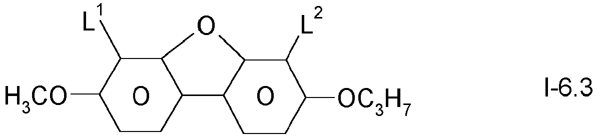

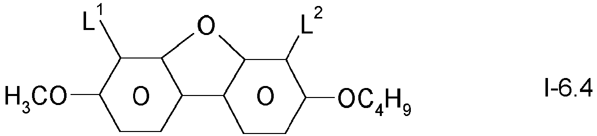

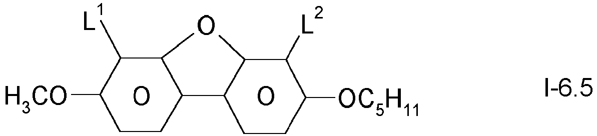

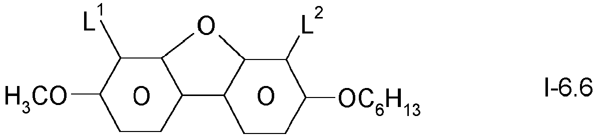

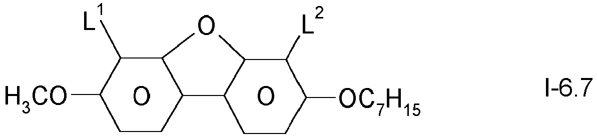

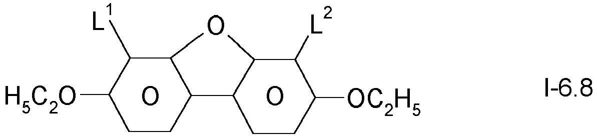

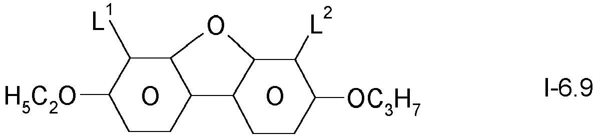

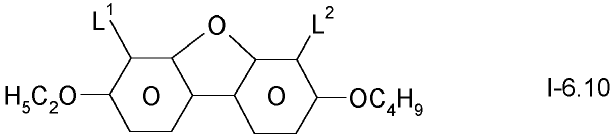

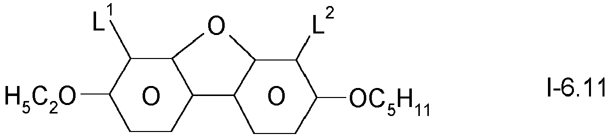

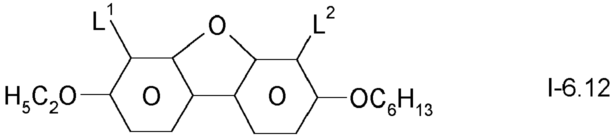

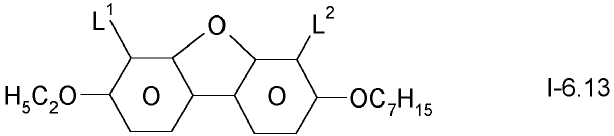

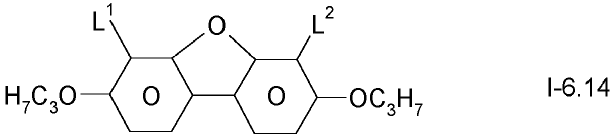

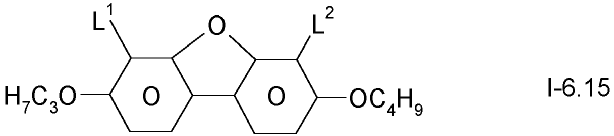

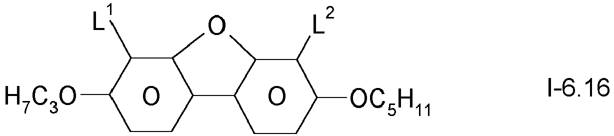

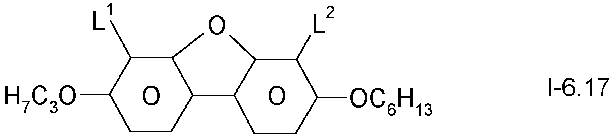

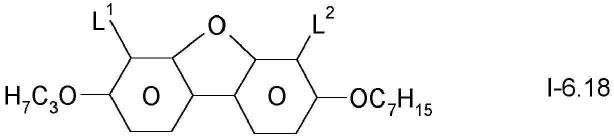

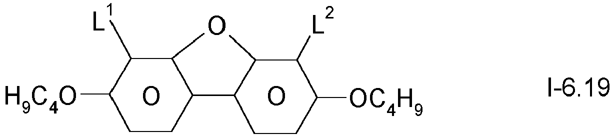

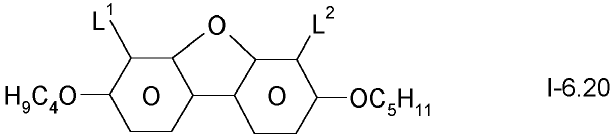

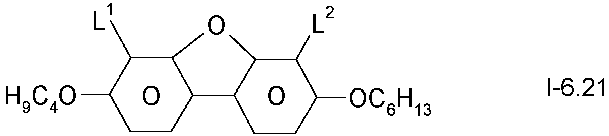

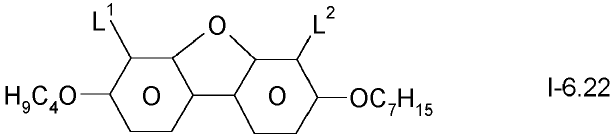

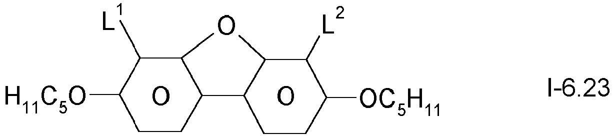

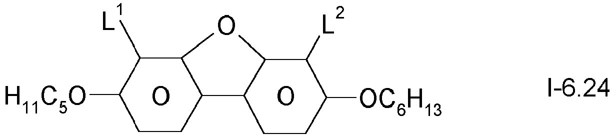

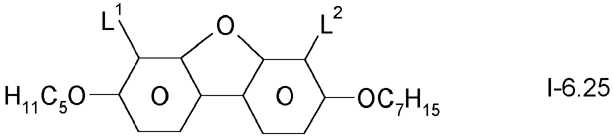

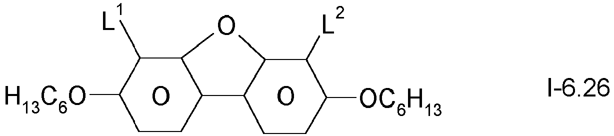

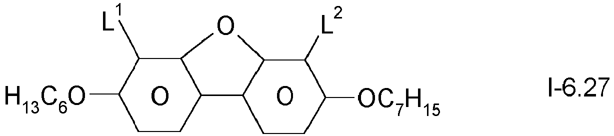

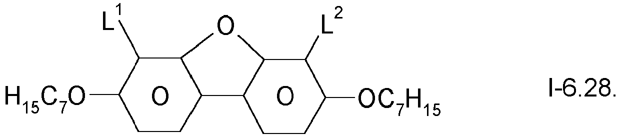

In den Verbindungen I-2.1 bis I-2.49 und I-6-1 bis I-6-28 bedeuten L¹ und L² vorzugsweise beide Fluor.

Ferner bevorzugt sind flüssigkristalline Mischungen, die mindestens eine Vebindung ausgewählt aus der Gruppe der Verbindungen der Formeln I-1.1 bis I-1.28 und I-6B.1 bis I-6B.3 enthalten: 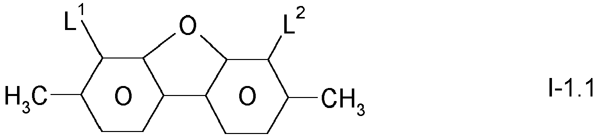 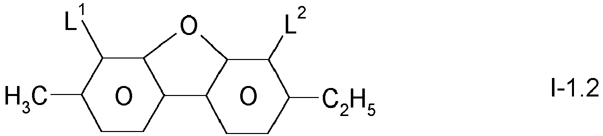 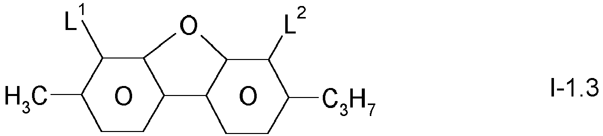 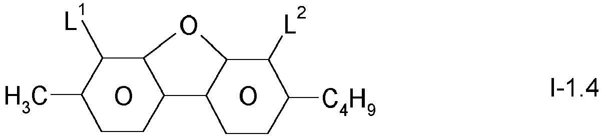 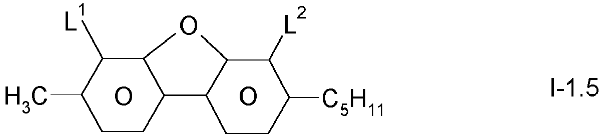 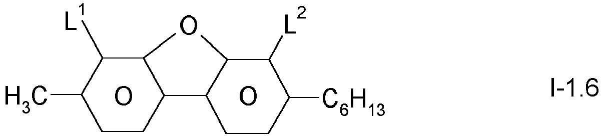 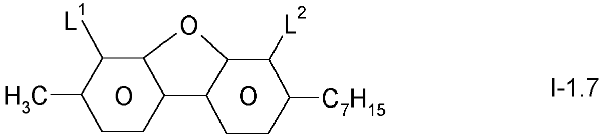 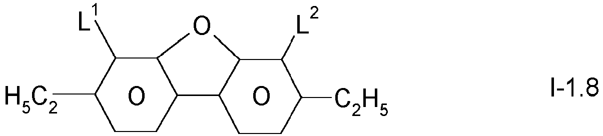 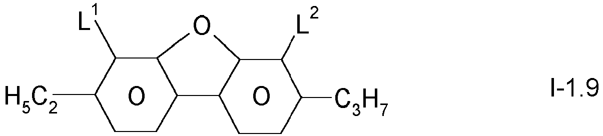 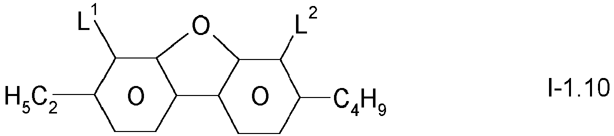 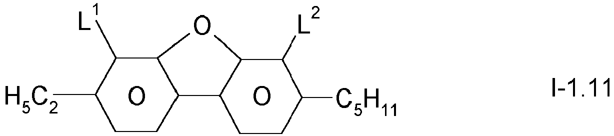 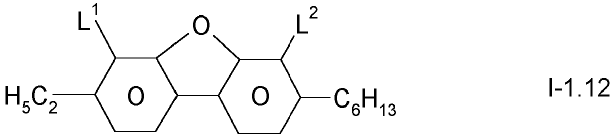 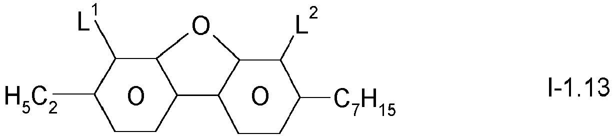 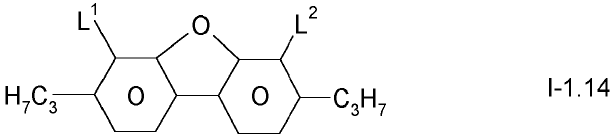 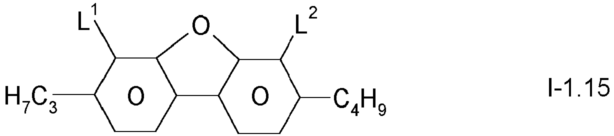 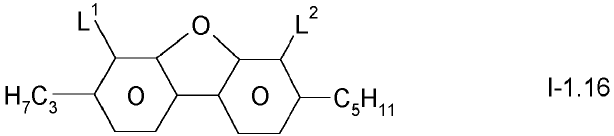 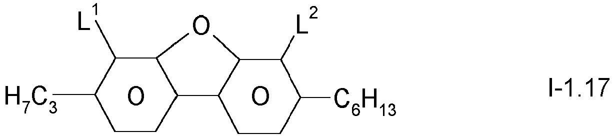 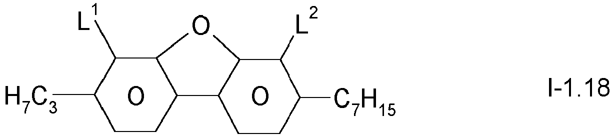 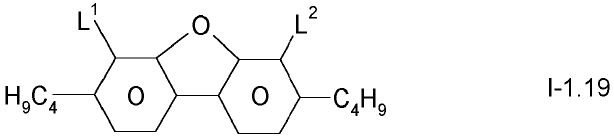 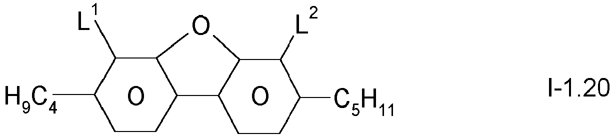 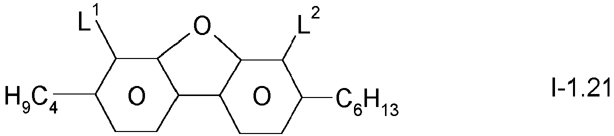 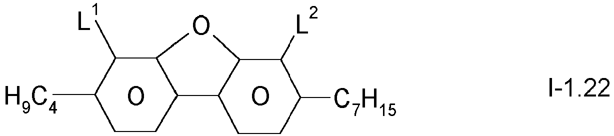 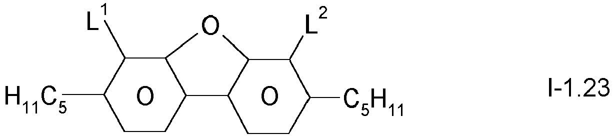 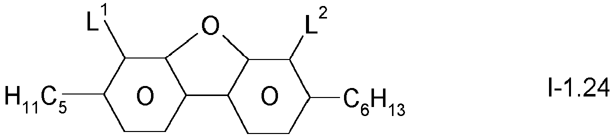 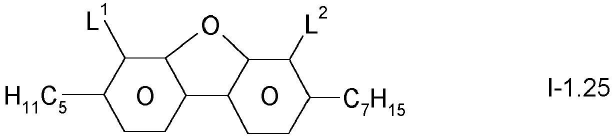 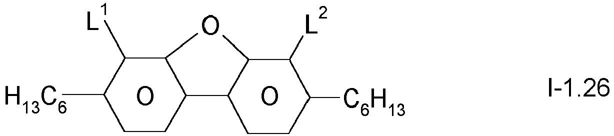 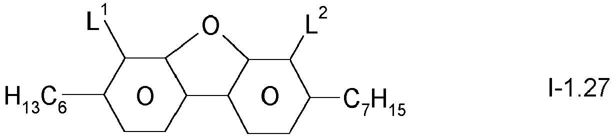 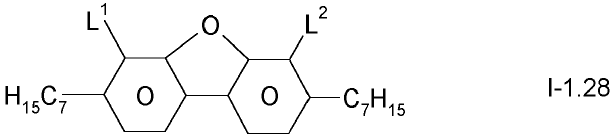 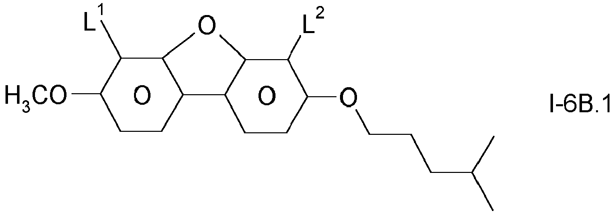 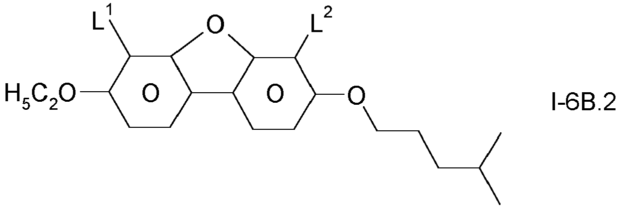 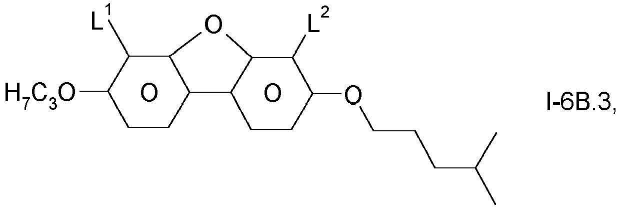 worin L¹ und L² jeweils unabhängig voneinander die in Anspruch 1 genannten Bedeutungen haben. Vorzugsweise bedeutet in den Verbindungen der Formeln I-1.1 bis I-1.28 und I-6B.1 bis I-6B.3 L¹ = L² = F.

Die Verbindungen der Formel I können beispielsweise wie in der

WO 02/055463 A1 beschrieben hergestellt werden. Vorzugsweise werden die Verbindungen der Formel I wie folgt hergestellt:

Ganz besonders bevorzugte Mischungen enthalten mindestens eine der nachfolgend genannten Verbindungen:

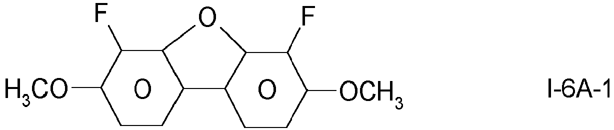

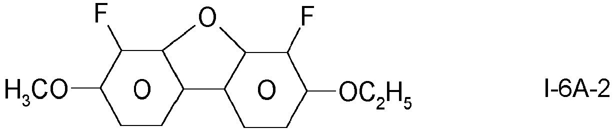

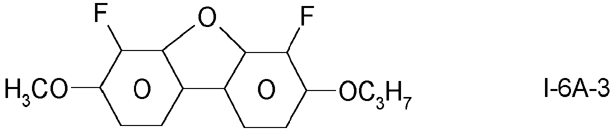

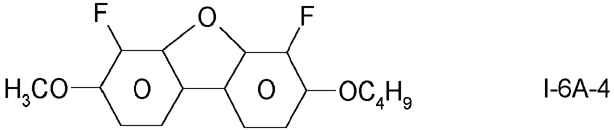

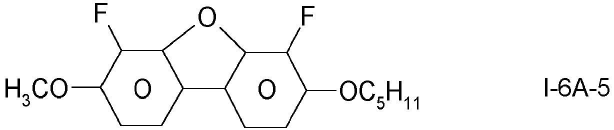

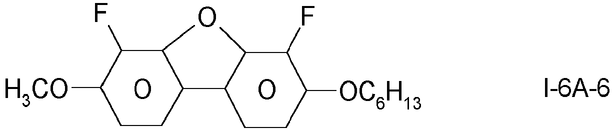

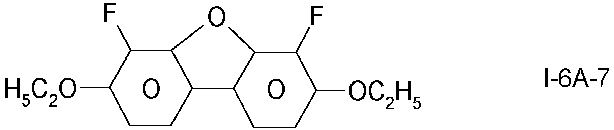

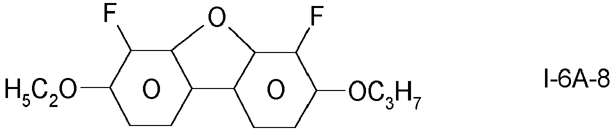

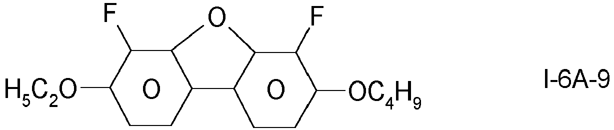

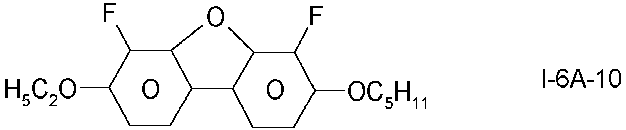

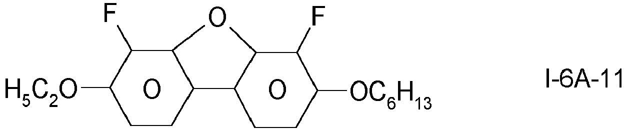

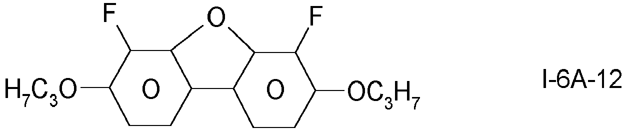

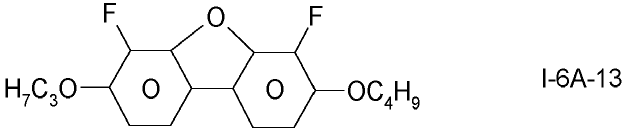

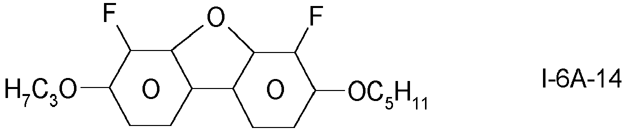

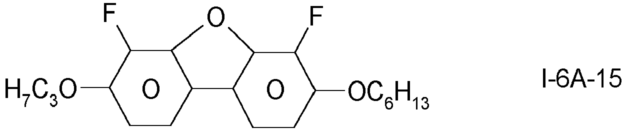

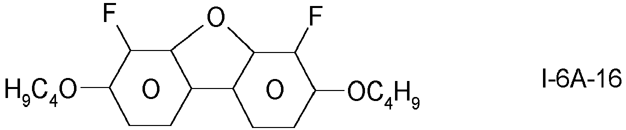

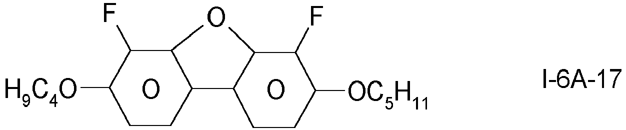

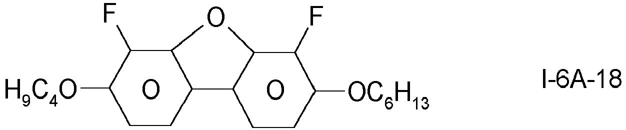

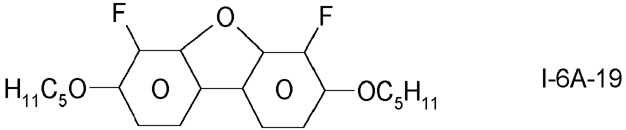

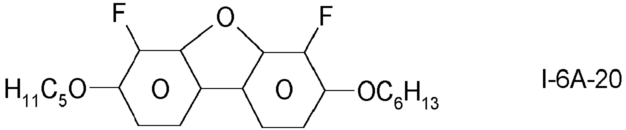

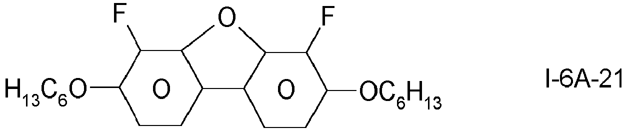

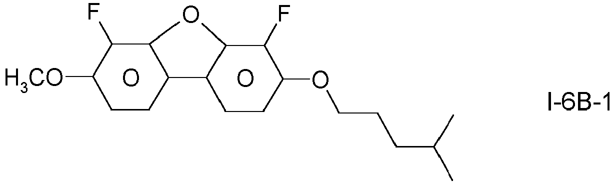

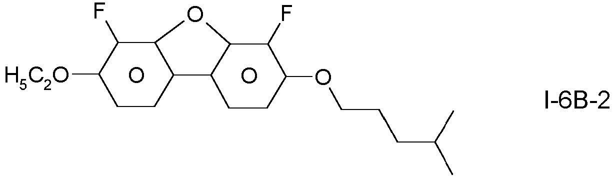

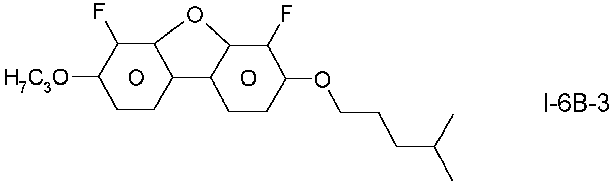

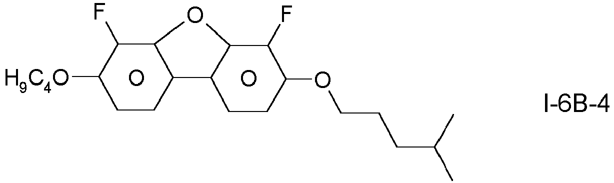

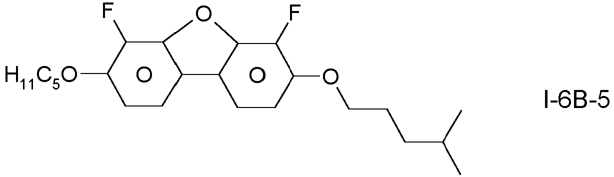

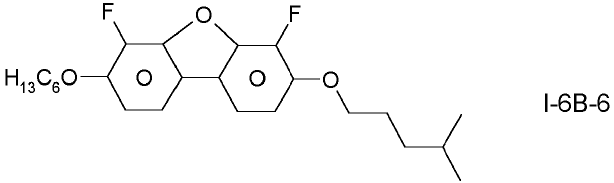

Die Verbindungen der Formel I können beispielsweise wie in der US 2005/0258399 oder WO 02/055463 A1 beschrieben hergestellt werden.

Vorzugsweise werden die Verbindungen der Formel I wie folgt hergestellt:

### Schema 1:

- R und R': jeweils unabhänging voneinander geradkettiges oder verzweigtes Alkyl oder Alkenyl

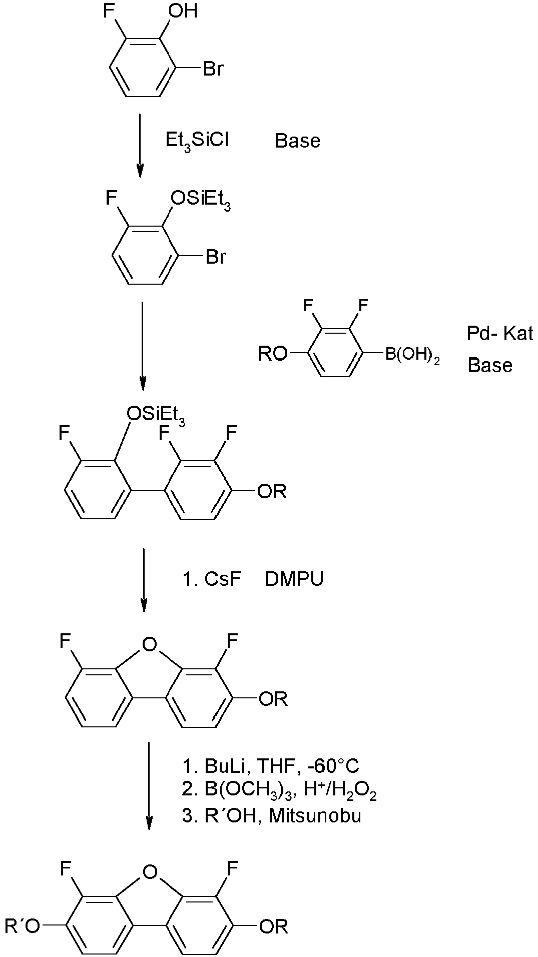

### Schema 2:

- R¹ und R²: jeweils unabhänging voneinander geradkettiges oder verzweigtes Alkyl oder Alkenyl

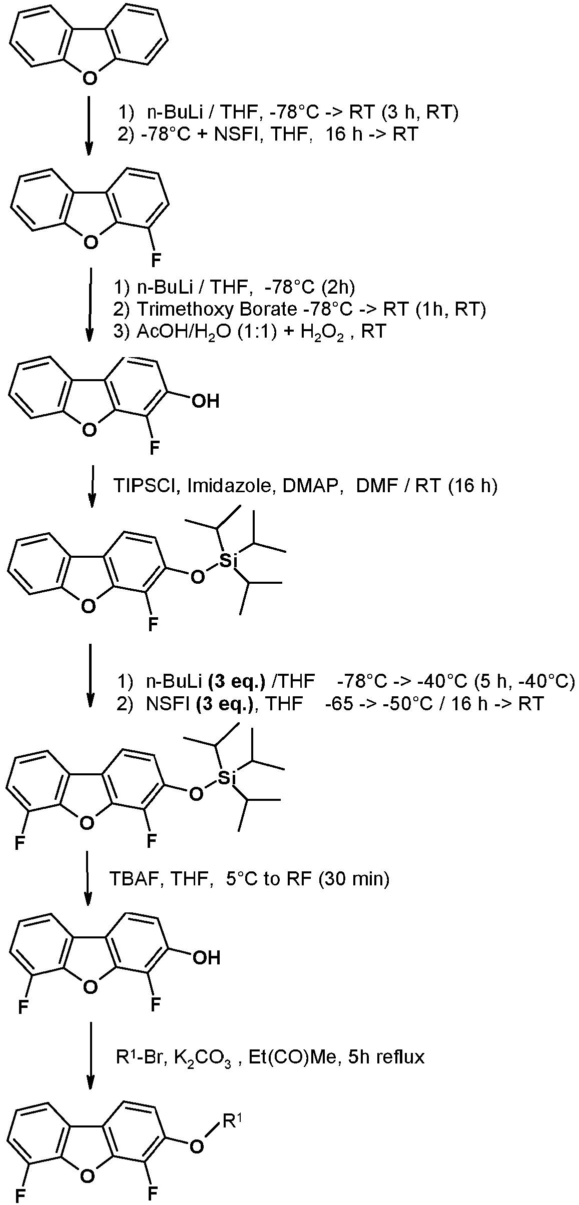

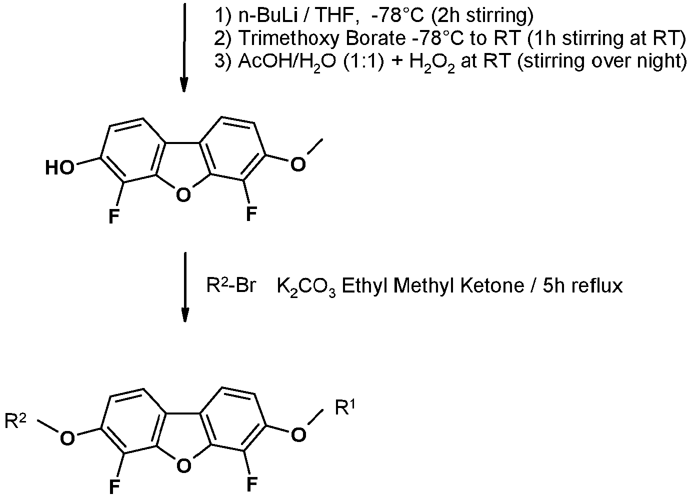

### Schema 3:

- R¹ und R²: jeweils unabhänging voneinander geradkettiges oder verzweigtes Alkyl oder Alkenyl

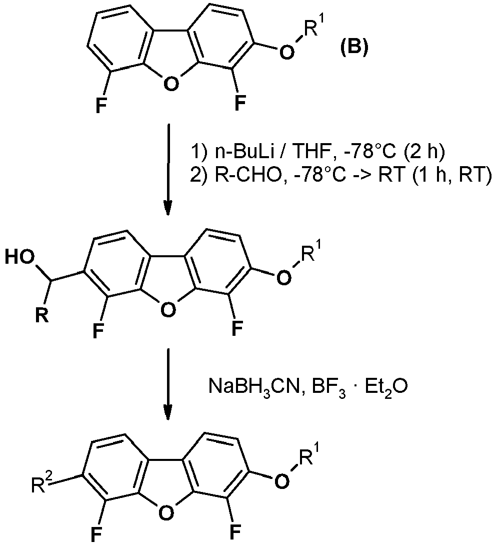

### Schema 4:

- R¹ und R²: jeweils unabhänging voneinander geradkettiges oder verzweigtes Alkyl, Alkoxy oder Alkenyl

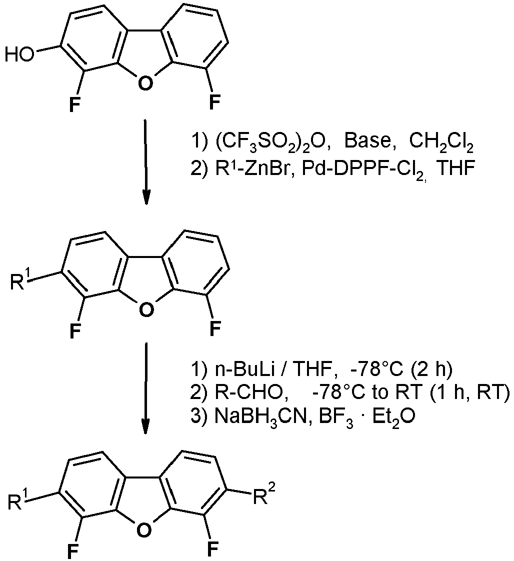

Gegenstand der vorliegenden Erfindung sind ebenfalls die Verbindungen der Formeln I-6B.

Die erfindungsgemäßen Medien enthalten vorzugsweise ein, zwei, drei, vier oder mehr, vorzugsweise ein, zwei oder drei, Verbindungen der Formel I.

Die Verbindungen der Formel I werden vorzugsweise im flüssigkristallinen Medium in Mengen von ≥ 1, vorzugsweise ≥ 3 Gew.%, bezogen auf das Gesamtgemisch, eingesetzt. Insbesondere bevorzugt sind flüssigkristalline Medien, die 1 - 40 Gew.%, ganz besonders bevorzugt 2 - 30 Gew%. einer oder mehrerer Verbindungen der Formel I enthalten.

Im Folgenden werden bevorzugte Ausführungsformen für das erfindungsgemäße flüssigkristalline Medium angeführt:
a) Flüssigkristallines Medium, welches zusätzlich eine oder mehrere Verbindungen ausgewählt aus der Gruppe der Verbindungen der Formeln IIA, IIB und IIC enthält, 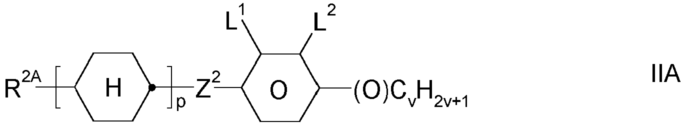 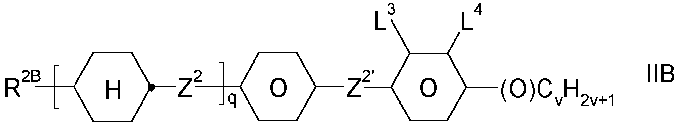 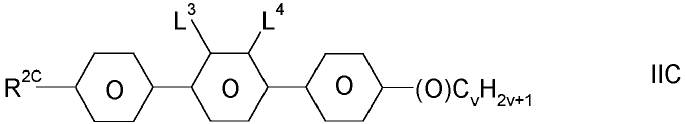 worin
   - R^{2A}, R^{2B} und R^{2C}: jeweils unabhängig voneinander H, einen unsubstituierten, einen einfach durch CN oder CF₃ oder mindestens einfach durch Halogen substituierten Alkyl- oder Alkenylrest mit bis zu 15 C-Atomen, wobei in diesen Resten auch eine oder mehrere CH₂-Gruppen durch -O-, -S-, 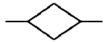 , -C≡C-, -CF₂O-, -OCF₂-, -OC-O- oder-O-CO- so ersetzt sein können, dass O-Atome nicht direkt miteinander verknüpft sind,
   - L¹⁻⁴: jeweils unabhängig voneinander F, Cl, CF₃ oder CHF₂,
   - Z² und Z^{2'}: jeweils unabhängig voneinander Einfachbindung, -CH₂CH₂-, -CH=CH-, -CF₂O-, -OCF₂-, -CH₂O-, -OCH₂-, -COO-, -OCO-, -C₂F₄-, -CF=CF-, -CH=CHCH₂O-,
   - p: 0, 1 oder 2,
   - q: 0 oder 1, und
   - v: 1 bis 6
   bedeuten.

In den Verbindungen der Formeln IIA und IIB können Z² gleiche oder unterschiedliche Bedeutungen haben. In den Verbindungen der Formel IIB können Z² und Z^{2'} gleiche oder verschiedene Bedeutungen aufweisen.

In den Verbindungen der Formeln IIA, IIB und IIC bedeuten R^{2A}, R^{2B} und R^{2C} jeweils vorzugsweise Alkyl mit 1-6 C-Atomen, insbesondere CH₃, C₂H₅, n-C₃H₇, n-C₄H₉, n-C₅H₁₁.

In den Verbindungen der Formeln IIA und IIB bedeuten L¹, L², L³ und L⁴ vorzugsweise L¹ = L² = F und L³ = L⁴ = F, ferner L¹ = F und L² = Cl, L¹ = Cl und L² = F, L³ = F und L⁴ = Cl, L³ = Cl und L⁴ = F. Z² und Z^{2'} bedeuten in den Formeln IIA und IIB vorzugsweise jeweils unabhängig voneinander eine Einfachbindung, ferner eine -C₂H₄-Brücke.

Sofern in der Formel IIB Z² = -C₂H₄- oder -CH₂O- ist, ist Z^{2'} vorzugsweise eine Einfachbindung bzw. falls Z^{2'} = -C₂H₄- oder -CH₂O- bedeutet, ist Z² vorzugsweise eine Einfachbindung. In den Verbindungen der Formeln IIA und IIB bedeutet (O)CᵥH₂ᵥ₊₁ vorzugsweise OCᵥH₂ᵥ₊₁, ferner CᵥH₂ᵥ₊₁. In den Verbindungen der Formel IIC bedeutet (O)CᵥH₂ᵥ₊₁ vorzugsweise CᵥH₂ᵥ₊₁. In den Verbindungen der Formel IIC bedeuten L³ und L⁴ vorzugsweise jeweils F.

Bevorzugte Verbindungen der Formeln IIA, IIB und IIC werden nachfolgend genannt: 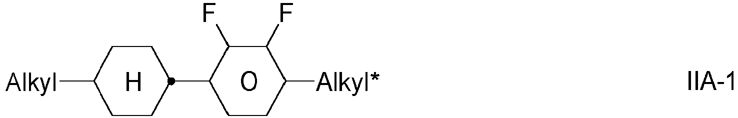 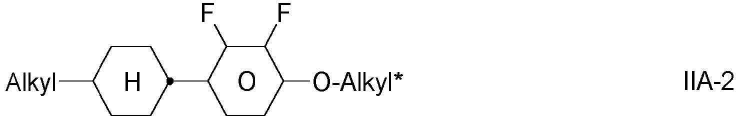 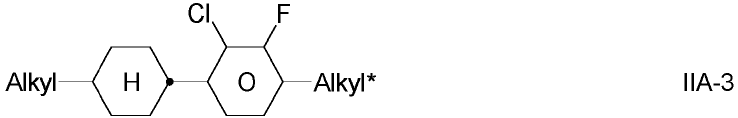 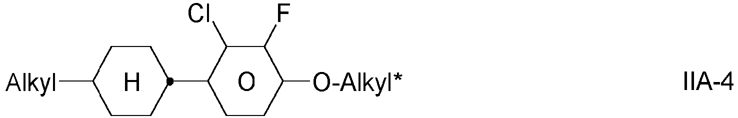 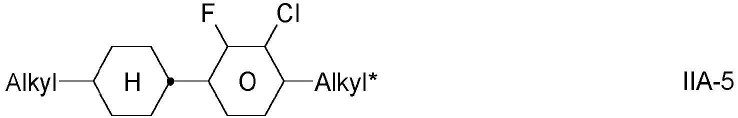 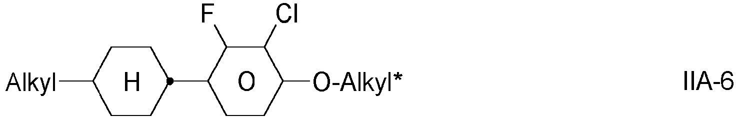 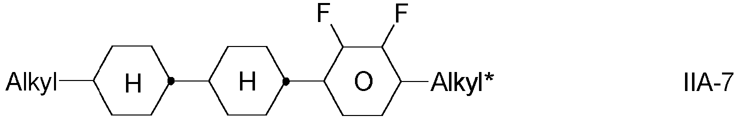 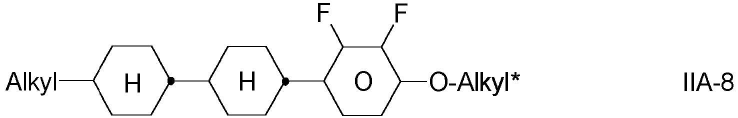 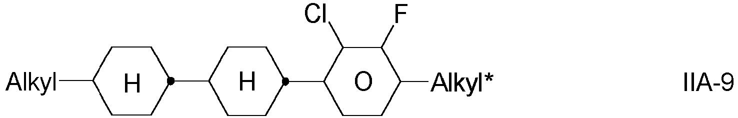 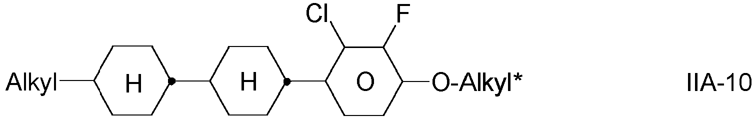 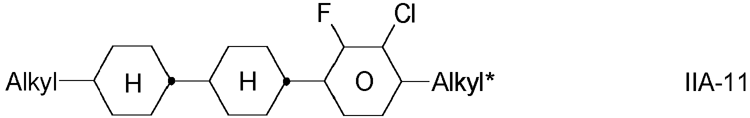 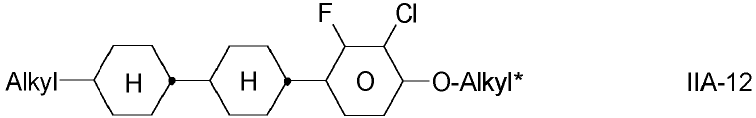 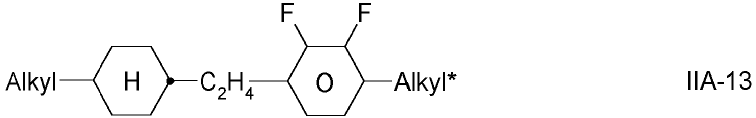 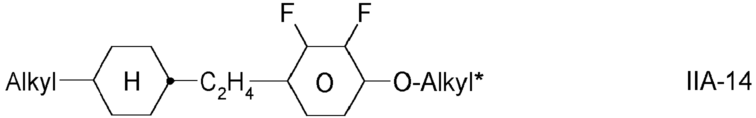 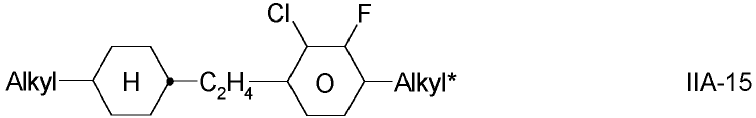 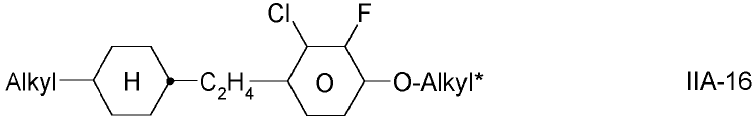 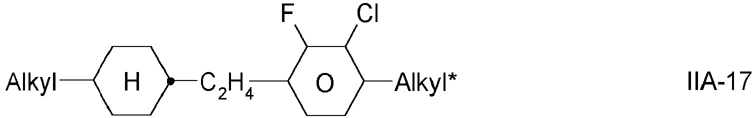 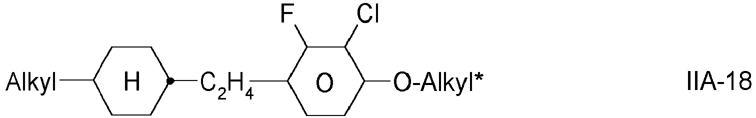 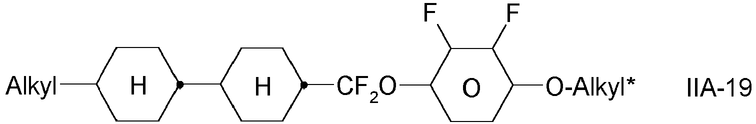 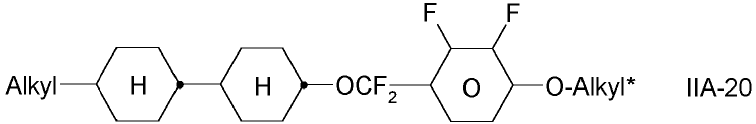 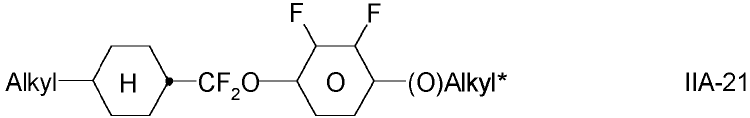 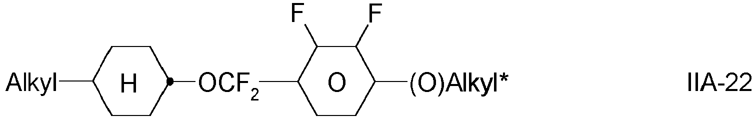 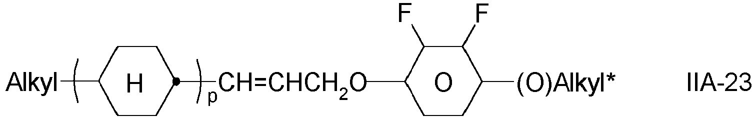 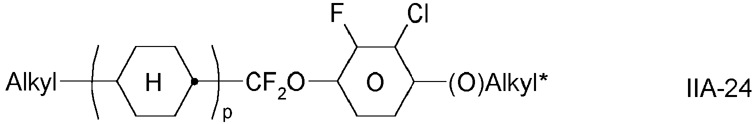 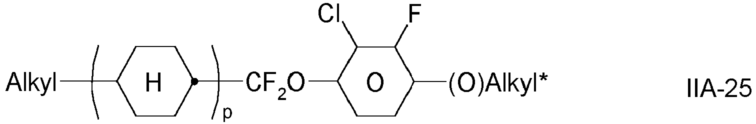 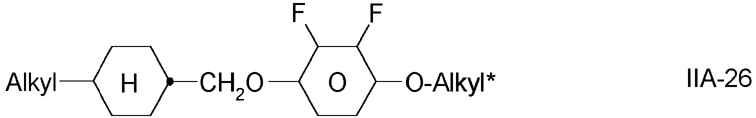 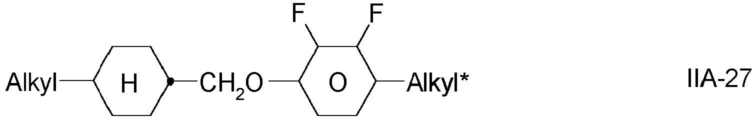 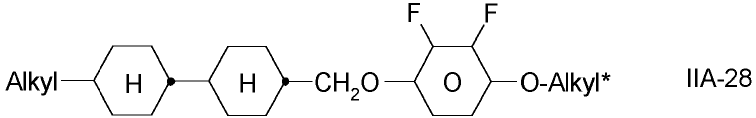 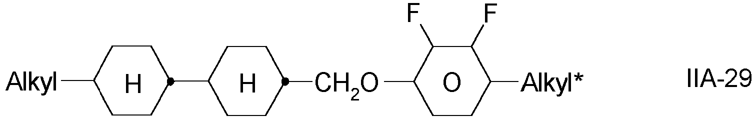 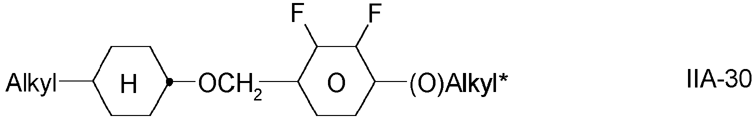 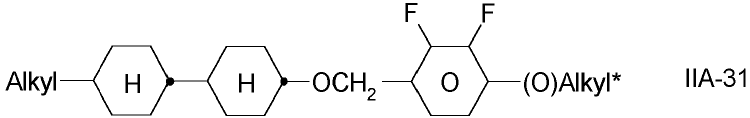 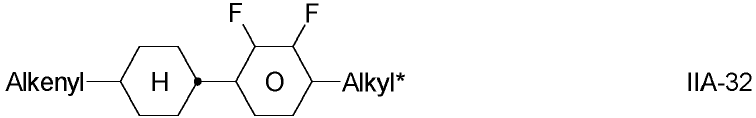 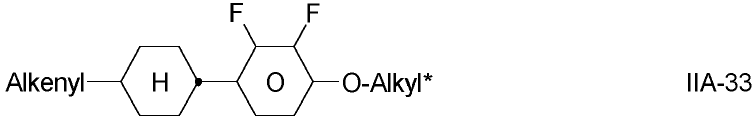 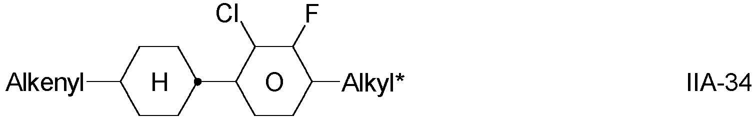 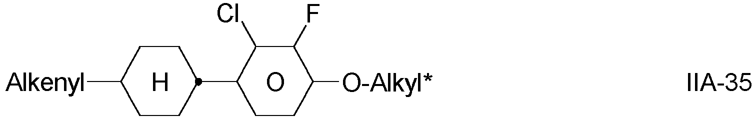 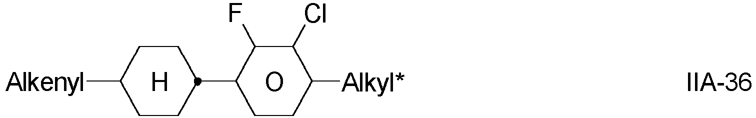 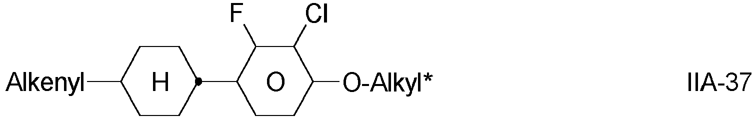 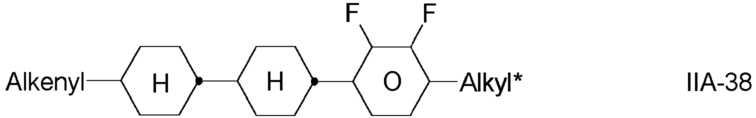 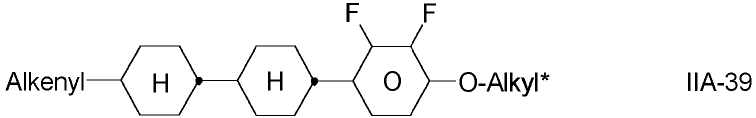 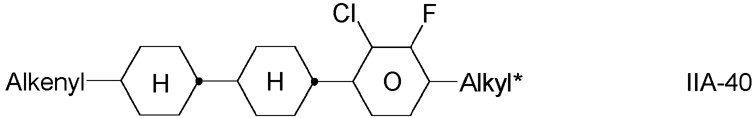 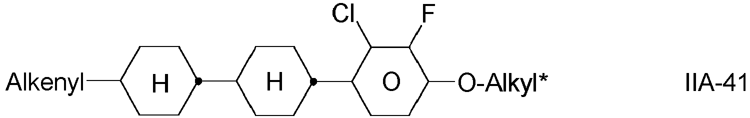 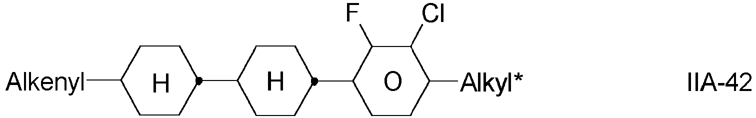 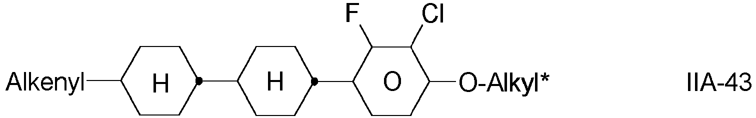 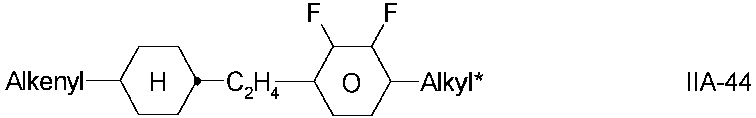 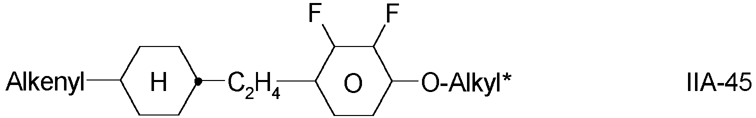 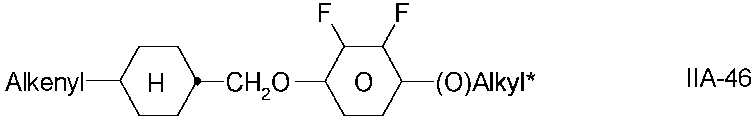 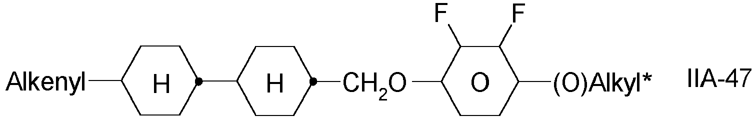 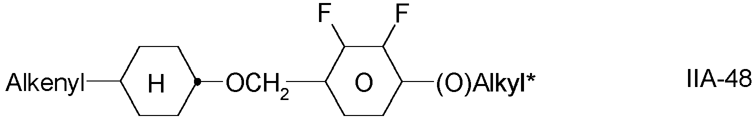 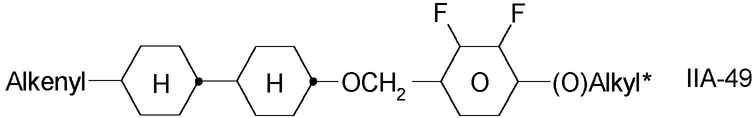 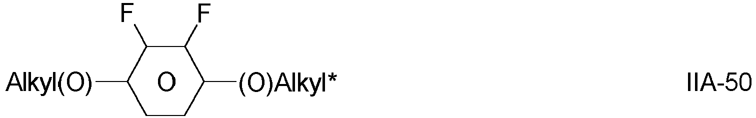 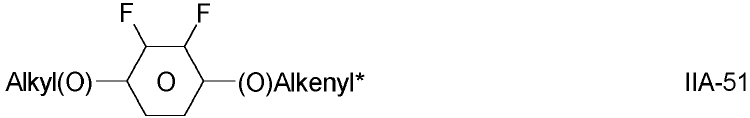 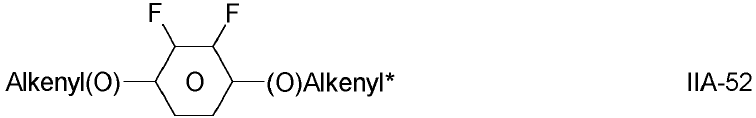 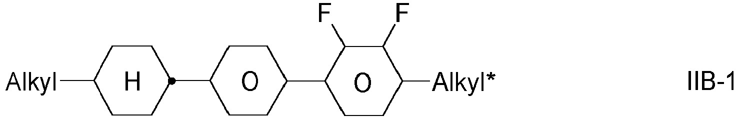 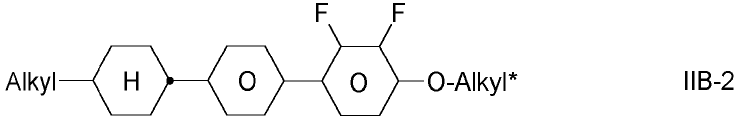 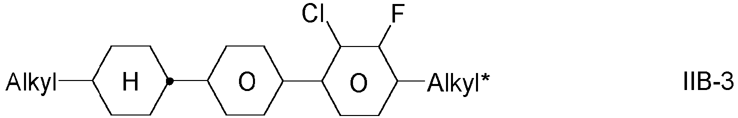 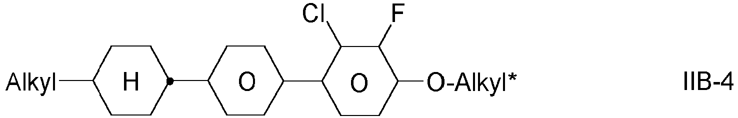 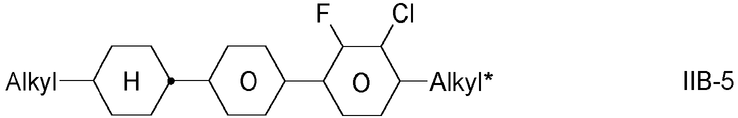 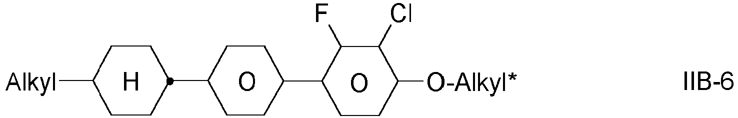 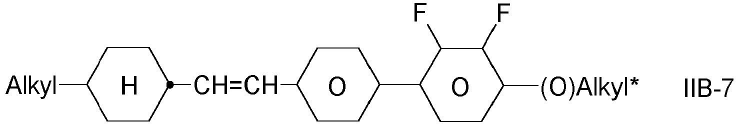 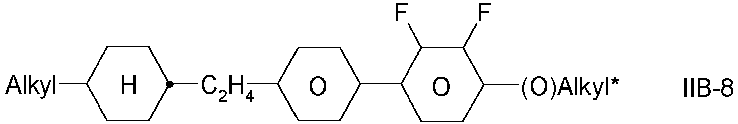 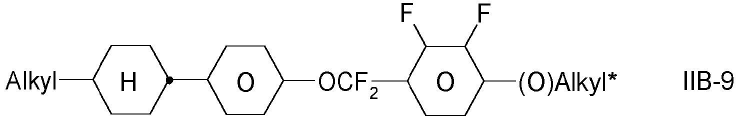 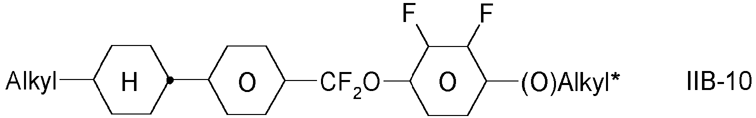 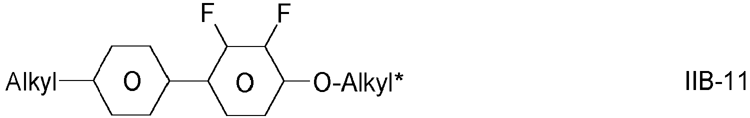 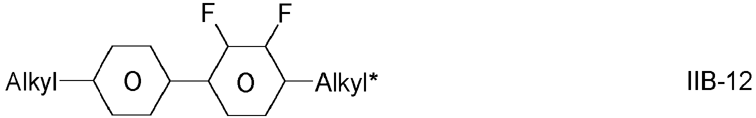 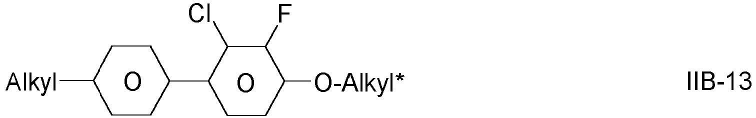 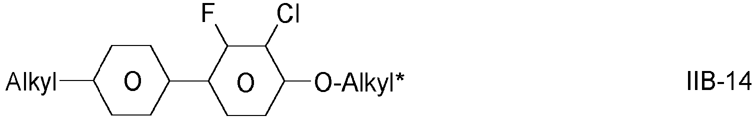 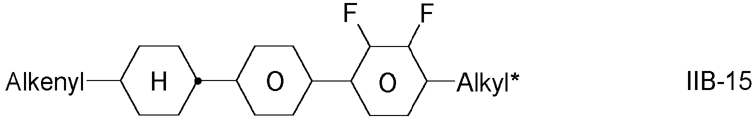 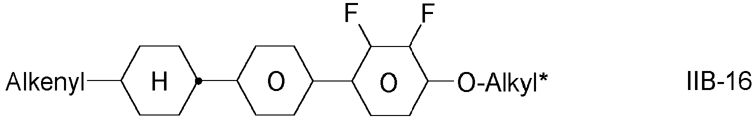 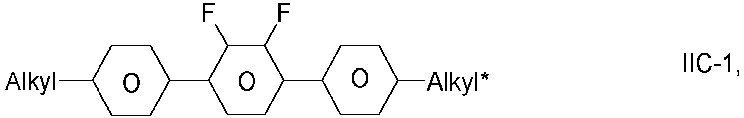 worin Alkyl und Alkyl* jeweils unabhängig voneinander einen geradkettigen Alkylrest mit 1-6 C-Atomen bedeuten.

Besonders bevorzugte erfindungsgemäße Mischungen enthalten eine oder mehrere Verbindungen der Formeln IIA-2, IIA-8, IIA-14, IIA-26, II-28, IIA-33, IIA-39, IIA-45, IIA-46, IIA-47, IIA-50, IIB-2, IIB-11, IIB-16 und IIC-1.

Vorzugsweise beträgt der Anteil an Verbindungen der Formeln IIA und/oder IIB im Gesamtgemisch mindestens 20 Gew.%.

Besonders bevorzugte erfindungsgemäße Medien enthalten mindestens eine Verbindung der Formel IIC-1, 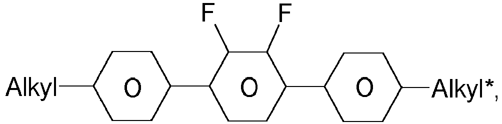 worin Alkyl und Alkyl*' die oben angegebenen Bedeutungen haben, vorzugsweise in Mengen von > 3 Gew.%, insbesondere > 5 Gew.% und besonders bevorzugt von 5-25 Gew.%.
b) Flüssigkristallines Medium, welches zusätzlich eine oder mehrere Verbindungen der Formel III 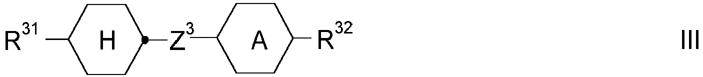 enthält,
worin
- R³¹ und R³²: jeweils unabhängig voneinander einen geradkettigen Alkyl-, Alkoxy, Alkenly-, Alkoxyalkyl- oder Alkoxyrest mit bis zu 12 C-Atomen, und 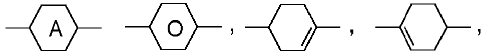 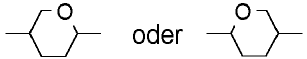
- Z³: Einfachbindung, -CH₂CH₂-, -CH=CH-, -CF₂O-, -OCF₂-,-CH₂O-, -OCH₂-, -COO-, -OCO-, -C₂F₄-, -C₄H₈-, -CF=CF-
bedeuten.

Bevorzugte Verbindungen der Formel III werden nachfolgend genannt, 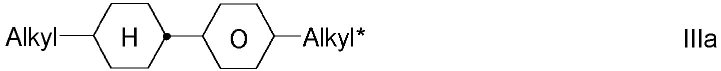 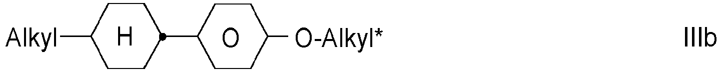 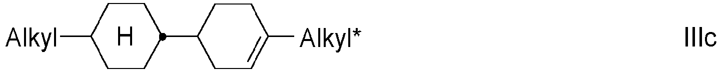 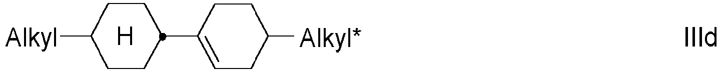 worin
Alkyl und
- Alkyl*: jeweils unabhängig voneinander einen geradkettigen Alkylrest mit 1-6 C-Atomen
bedeuten.

Vorzugsweise enthält das erfindungsgemäße Medium mindestens eine Verbindung der Formel IIIa und/oder Formel Illb.

Der Anteil an Verbindungen der Formel III im Gesamtgemisch beträgt vorzugsweise mindestens 5 Gew.%.
c) Flüssigkristallines Medium enthaltend zusätzlich eine Verbindung der Formel 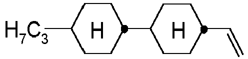 und / oder 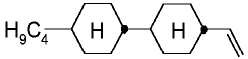 und / oder 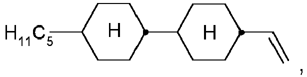 vorzugsweise in Gesamtmengen von ≥ 5 Gew.%, insbesondere von ≥ 10 Gew.%.

Weiterhin bevorzugt sind erfindungsgemäße Mischungen enthaltend die Verbindung (Acronym: CC-3-V1) 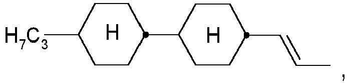 vorzugsweise in Mengen von 2-15 %.

Bevorzugte Mischungen enthalten 5-60 %, vorzugsweise 10-55 %, insbesondere 20-50 Gew.% der Verbindung der Formel (Acronym: CC-3-V)

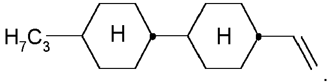

Weiterhin bevorzugt sind Mischungen, die eine Verbindung der Formel (Acronym: CC-3-V) 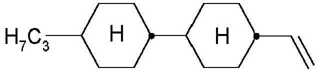 und eine Verbindung der Formel (Acronym: CC-3-V1) 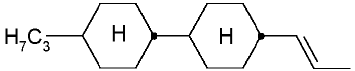 enthalten, vorzugsweise in Mengen von 10 - 60 %.
d) Flüssigkristallines Medium, welches zusätzlich eine oder mehrere Vierkernverbindungen der Formeln, 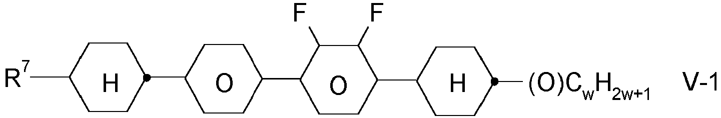 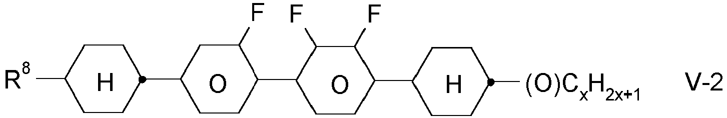 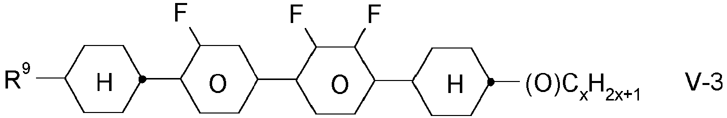 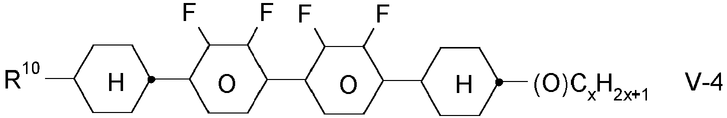 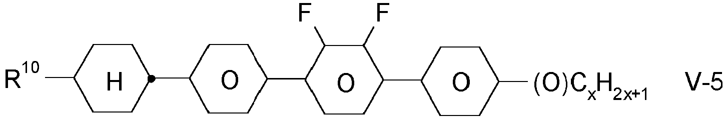 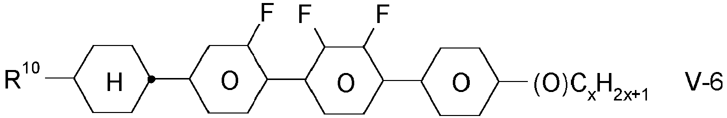 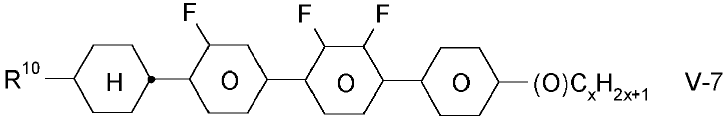 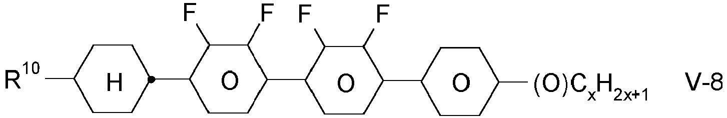 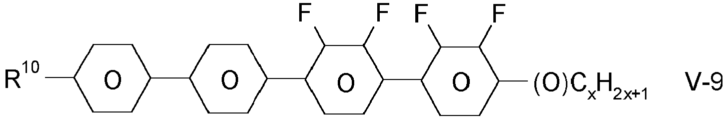 worin
- R⁷⁻¹⁰: jeweils unabhängig voneinander eine der in Anspruch 3 für R^{2A} angegebenen Bedeutung haben, und
- w und x: jeweils unabhängig voneinander 1 bis 6,
bedeuten,
enthält.

Insbesondere bevorzugt sind Mischungen enthaltend mindestens eine Verbindung der Formel V-9.
e) Flüssigkristallines Medium, welches zusätzlich eine oder mehrere Verbindungen der Formeln Y-1 bis Y-6, 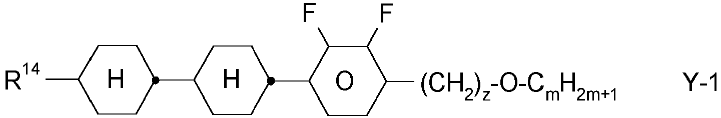 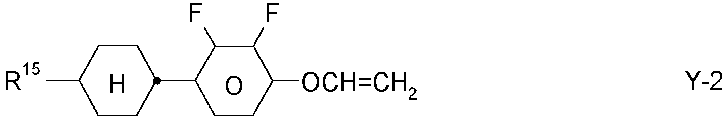 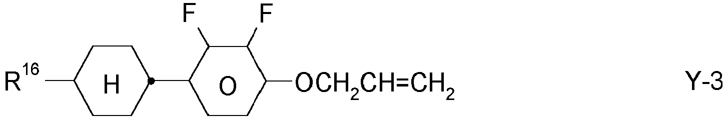 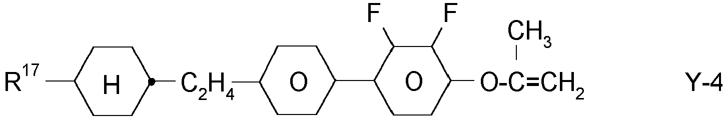 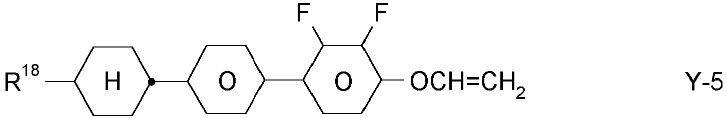 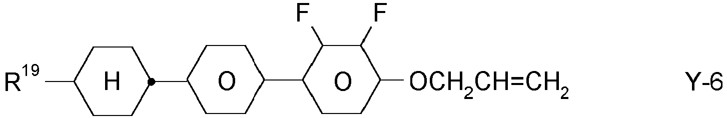 enthält,
worin R¹⁴-R¹⁹ jeweils unabhängig voneinander einen Alkyl- oder Alkoxyrest mit 1-6 C-Atomen bedeuten; z und m bedeuten jeweils unabhängig voneinander 1-6; x bedeutet 0, 1, 2 oder 3.

Insbesondere bevorzugt enthält das erfindungsgemäße Medium eine oder mehrere Verbindungen der Formel Y-1 bis Y-6, vorzugsweise in Mengen von ≥ 5 Gew.%.
f) Flüssigkristallines Medium enthaltend zusätzlich ein oder mehrere fluorierte Terphenyle der Formeln T-1 bis T-21, 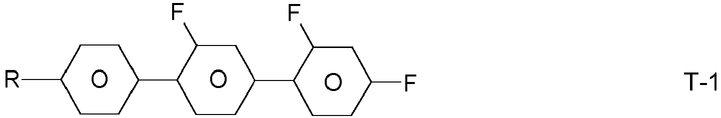 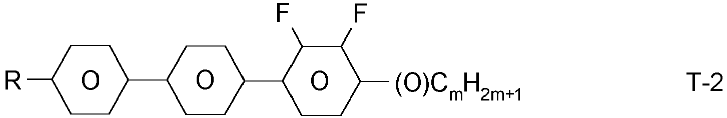 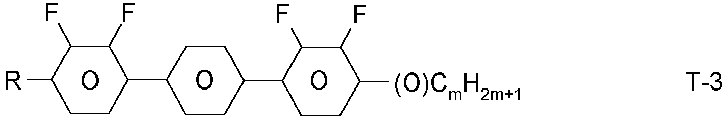 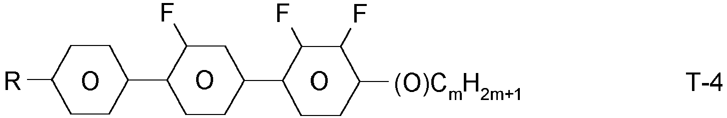 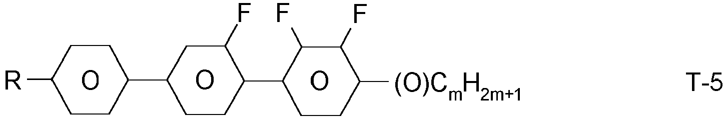 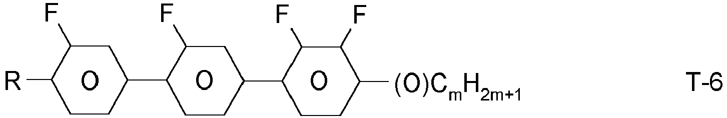 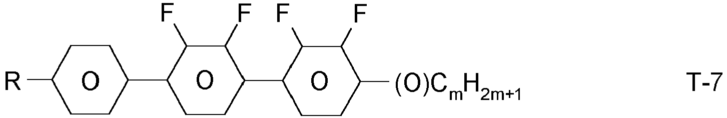 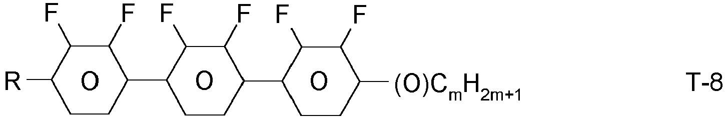 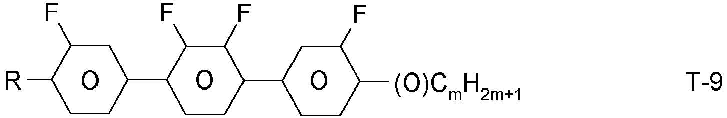 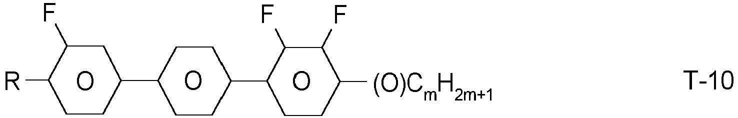 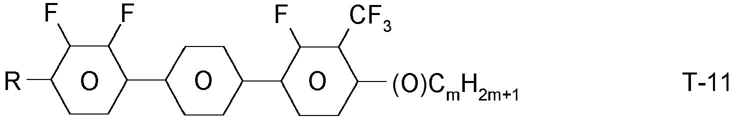 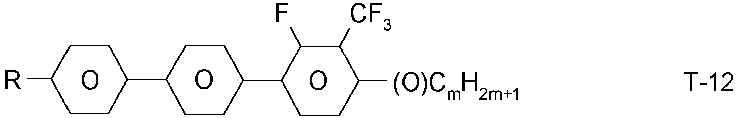 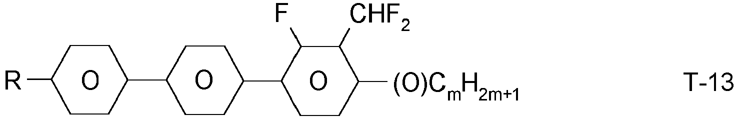 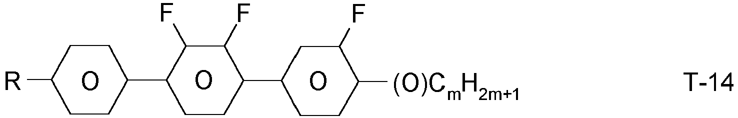 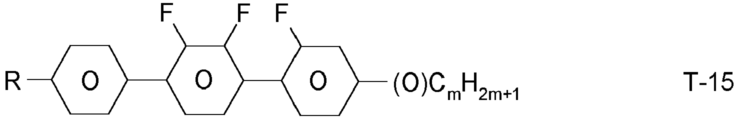 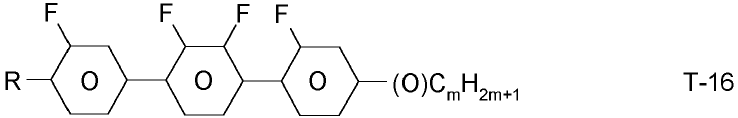 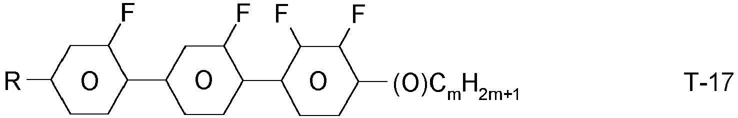 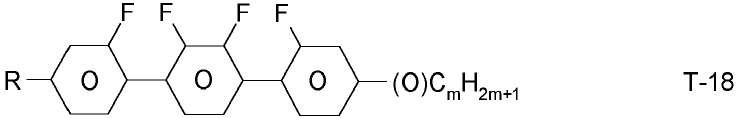 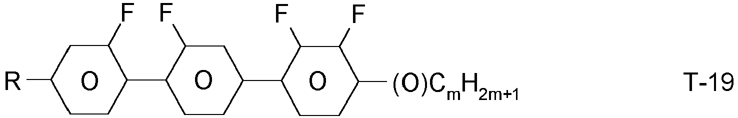 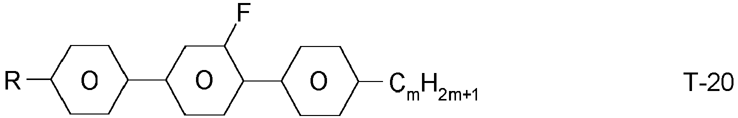 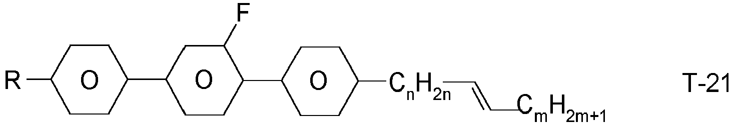 worin
R geradkettiger Alkyl- oder Alkoxyrest mit 1-7 C-Atomen bedeutet, und m = 0, 1, 2, 3, 4, 5 oder 6 und n 0, 1, 2, 3 oder 4 bedeuten,
enthält.
Vorzugsweise bedeutet R Methyl, Ethyl, Propyl, Butyl, Pentyl, Hexyl Methoxy, Ethoxy, Propoxy, Butoxy, Pentoxy.

Das erfindungsgemäße Medium enthält die Terphenyle der Formeln T-1 bis T-21 vorzugsweise in Mengen von 2-30 Gew.%, insbesondere von 5-20 Gew.%.

Besonders bevorzugt sind Verbindungen der Formeln T-1, T-2, T-4, T-20 und T-21. In diesen Verbindungen bedeutet R vorzugsweise Alkyl, ferner Alkoxy jeweils mit 1-5 C-Atomen. In den Verbindungen der Formel T-20 bedeutet R vorzugsweise Alkyl oder Alkenyl, insbesondere Alkyl. In der Verbindung der Formel T-21 bedeutet R vorzugsweise Alkyl.

Vorzugsweise werden die Terphenyle in den erfindungsgemäßen Mischungen eingesetzt, wenn der Δn-Wert der Mischung ≥ 0,1 sein soll. Bevorzugte Mischungen enthalten 2-20 Gew.% einer oder mehrerer Terphenyl-Verbindungen ausgewählt aus der Gruppe der Verbindungen T-1 bis T-21.
g) Flüssigkristallines Medium enthaltend zusätzlich ein oder mehrere Biphenyle der Formeln B-1 bis B-3, 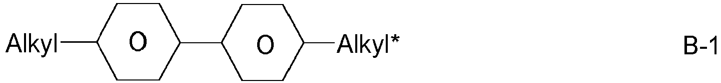 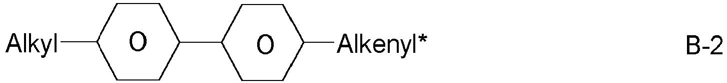 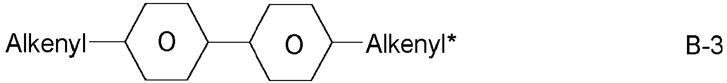 worin
- Alkyl und Alkyl*: jeweils unabhängig voneinander einen geradkettigen Alkylrest mit 1-6 C-Atomen, und
- Alkenyl und Alkenyl*: jeweils unabhängig voneinander einen geradkettigen Alkenylrest mit 2-6 C-Atomen
bedeuten.

Der Anteil der Biphenyle der Formeln B-1 bis B-3 in der Gesamtmischung beträgt vorzugsweise mindestens 3 Gew.%, insbesondere ≥ 5 Gew.%.

Von den Verbindungen der Formeln B-1 bis B-3 sind die Verbindungen der Formel B-2 insbesondere bevorzugt.

Besonders bevorzugte Biphenyle sind 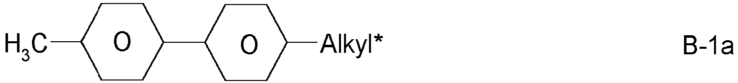 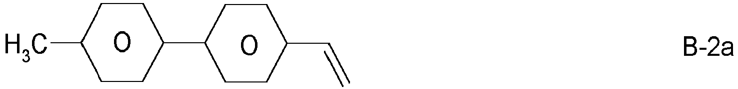 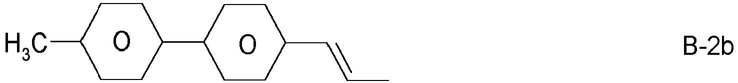 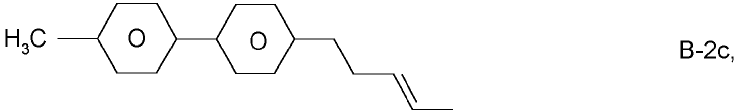 worin Alkyl* einen Alkylrest mit 1-6 C-Atomen bedeutet. Insbesondere bevorzugt enthält das erfindungsgemäße Medium eine oder mehrere Verbindungen der Formeln B-1a und/oder B-2c.
h) Flüssigkristallines Medium enthaltend mindestens eine Verbindung der Formeln Z-1 bis Z-7, 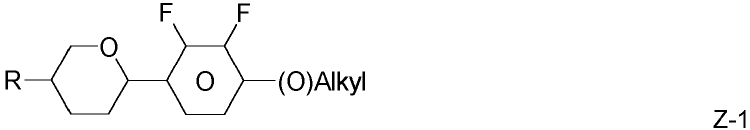 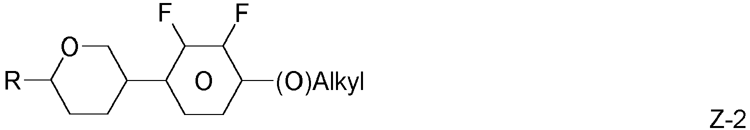 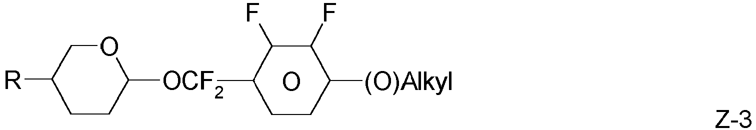 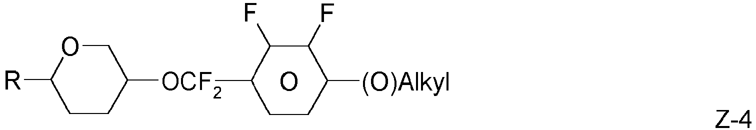 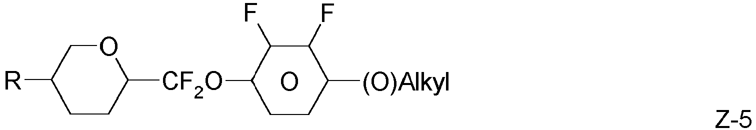 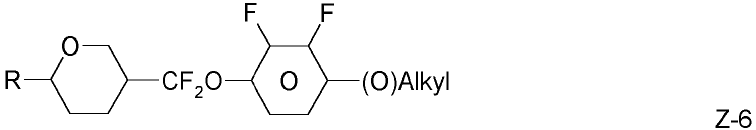 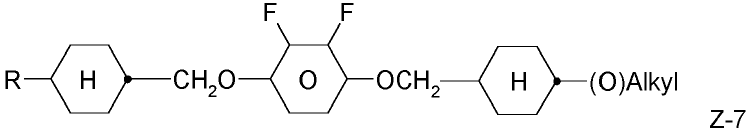 worin R und Alkyl die oben angegebenen Bedeutungen haben.
i) Flüssigkristallines Medium enthaltend mindestens eine Verbindung der Formeln O-1 bis O-18, 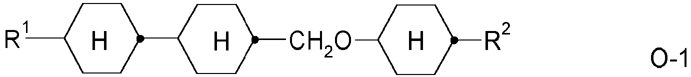 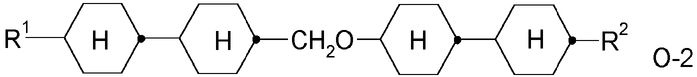 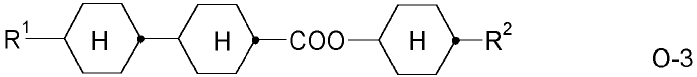 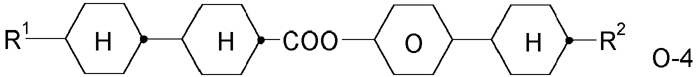 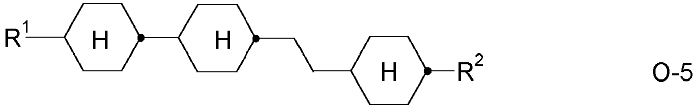 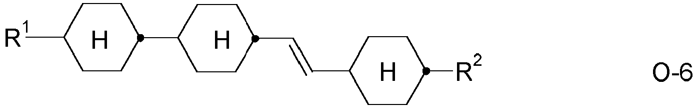 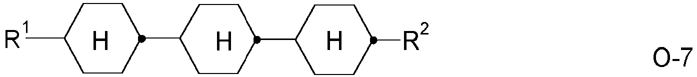 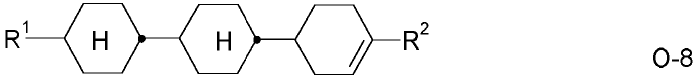 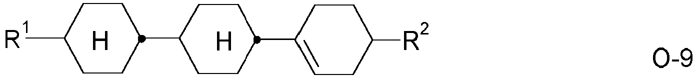 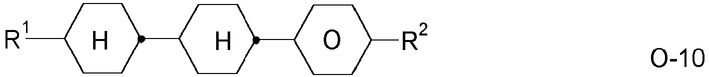 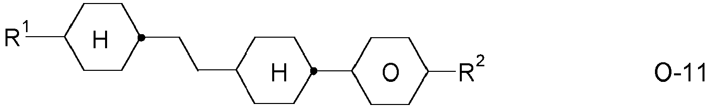 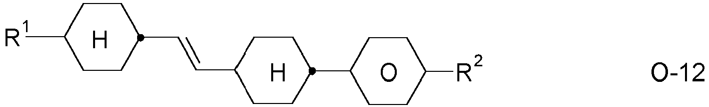 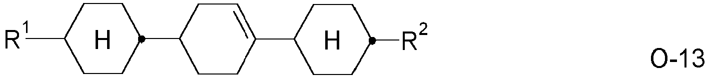 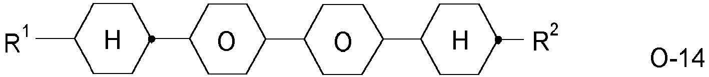 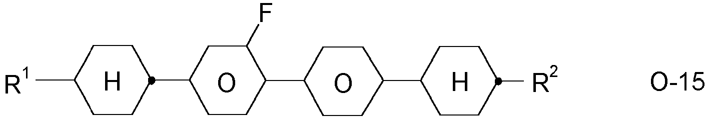 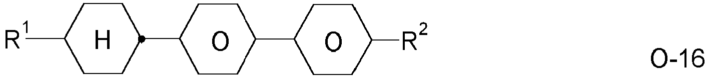 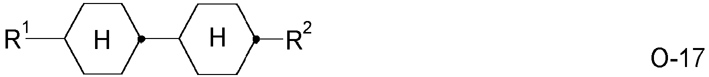 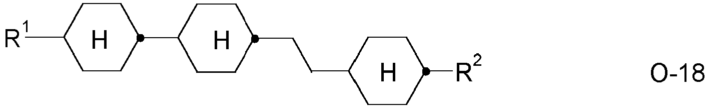 worin R¹ und R² die für R^{2A} angegebenen Bedeutungen haben. Vorzugsweise bedeuten R¹ und R² jeweils unabhängig voneinander geradkettiges Alkyl oder Alkenyl.

Bevorzugte Medien enthalten eine oder mehrere Verbindungen der Formeln O-1, O-3, O-4, O-6, O-7, O-10, O-11, O-12, O-14, O-15, O-16 und/oder O-17.

Erfindungsgemäße Mischungen enthalten ganz besonders bevorzugt die Verbindungen der Formel O-10, O-12, O-16 und/oder O-17, inbesondere in Mengen von 5-30 %.

Bevorzugte Verbindungen der Formeln O-10 und O-17 werden nachfolgend genannt:

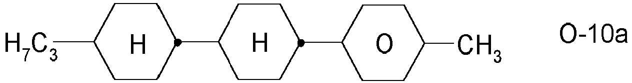

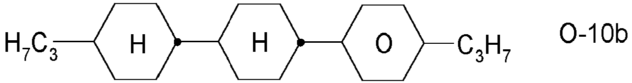

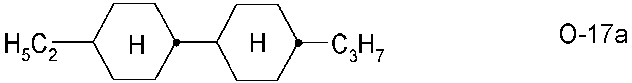

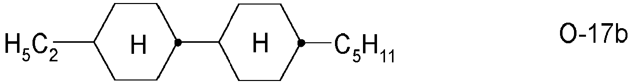

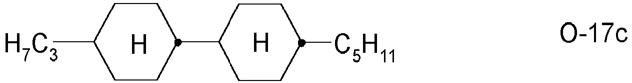

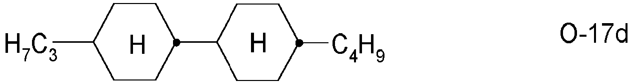

Besonders bevorzugt enthält das erfindungsgemäße Medium die Dreikern-Verbindungen der Formel O-10a und/oder der Formel O-10b in Kombination mit einer oder mehreren Zweikern-Verbindungen der Formeln O-17a bis 0-17d. Vorzugsweise beträgt der Gesamtanteil der Verbindungen der Formel O-10a und/oder O-10b in Kombination mit einer oder mehreren Verbindungen ausgewählt aus den Zweikern-verbindungen der Formeln O-17a bis 0-17d 5-40 %, ganz besonders bevorzugt 15-35 %.

Ganz besonders bevorzugte Mischungen enthalten die Verbindungen O-10a und O-17a:

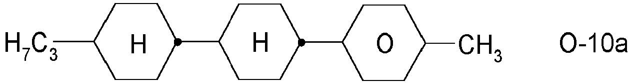

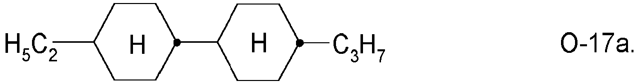

Vorzugsweise sind die Verbindungen O-10a und O-17a in der Mischung in einer Konzentration von 15-35 %, besonders bevorzugt von 15-25 % und insbesondere bevorzugt von 18-22 % bezogen auf die Gesamtmischung, enthalten.

Ganz besonders bevorzugte Mischungen enthalten die Verbindungen O-10b und O-17a:

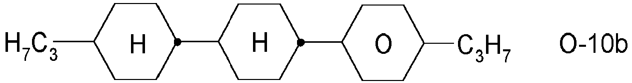

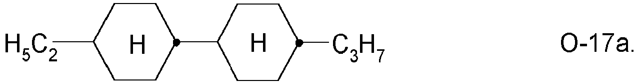

Vorzugsweise sind die Verbindungen O-10b und O-17a in der Mischung in einer Konzentration von 15-35 %, besonders bevorzugt von 15-25 % und insbesondere bevorzugt von 18-22 % bezogen auf die Gesamtmischung, enthalten.

Ganz besonders bevorzugte Mischungen enthalten folgende drei Verbindungen:

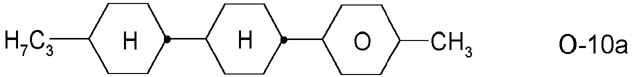

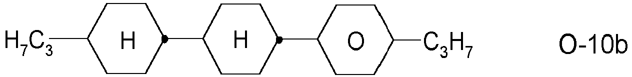

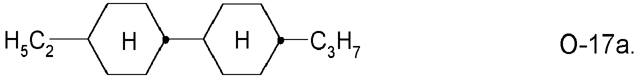

Vorzugsweise sind die Verbindungen O-10a, O-10b und O-17a in der Mischung in einer Konzentration von 15-35 %, besonders bevorzugt von 15-25 % und insbesondere bevorzugt von 18-22 % bezogen auf die Gesamtmischung, enthalten.

Bevorzugte Mischungen enthalten mindestens eine Verbindung ausgewählt aus der Gruppe der Verbindungen 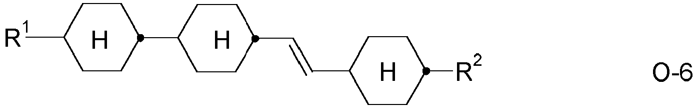 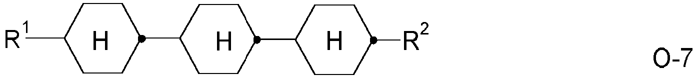 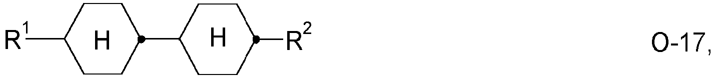 worin R¹ und R² die oben angegegebenen Bedeutungen haben. Vorzugsweise bedeutet in den Verbindungen O-6, O-7 und O-17 R¹ Alkyl oder Alkenyl mit 1-6 bzw. 2-6 C-Atomen und R² Alkenyl mit 2-6 C-Atomen.

Bevorzugte Mischungen enthalten mindestens eine Verbindung der Formeln O-6a, O-6b, O-7a, O-7b, 0-17e, O-17f, O-17g und O-17h: 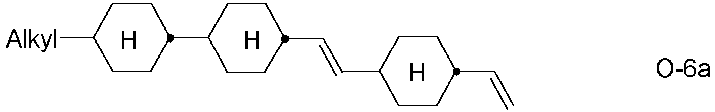 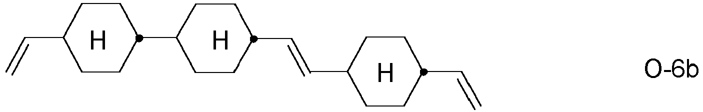 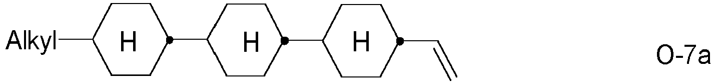 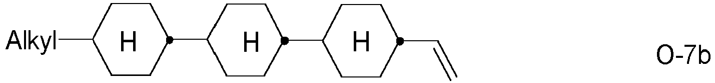 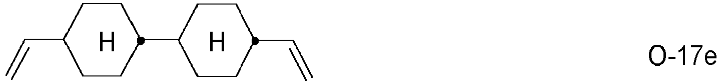 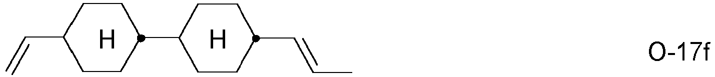 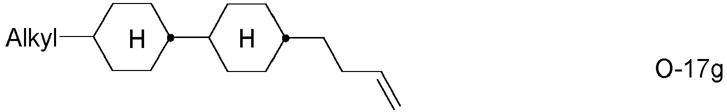 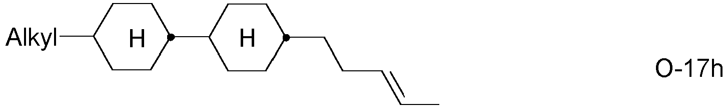 worin Alkyl einen Alkylrest mit 1-6 C-Atomen bedeutet.

Vorzugsweise sind die Verbindungen der Formel O-6, O-7 und O-17e-h in den erfindungsgemäßen Mischungen mit 1 - 40 Gew.%, vorzugsweise 2 - 35 Gew.% und ganz besonders bevorzugt 2 - 30 Gew.% enthalten.
j) Bevorzugte erfindungsgemäße flüssigkristalline Medien enthalten eine oder mehrere Substanzen, die eine Tetrahydronaphthyl- oder Naphthyl-Einheit aufweisen, wie z.B. die Verbindungen der Formeln N-1 bis N-5, 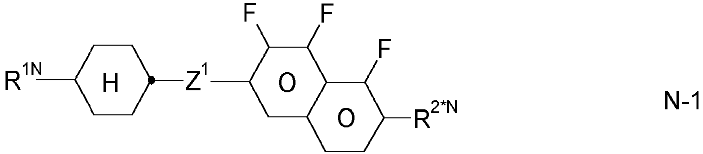 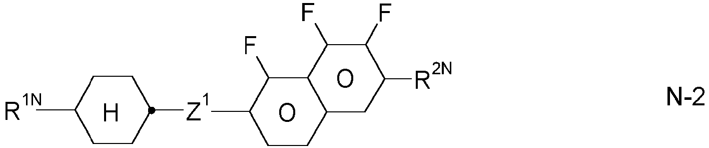 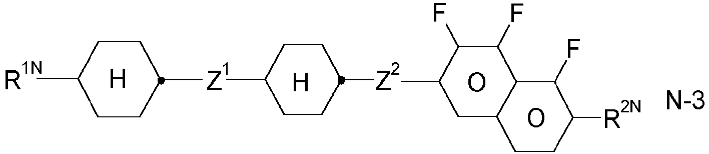 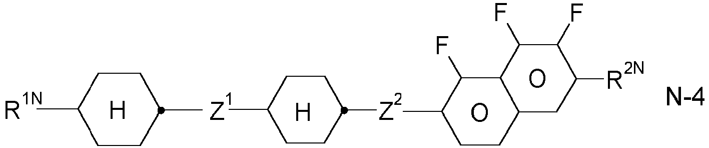 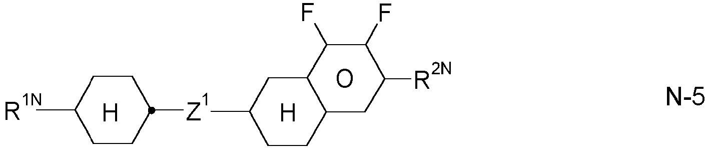 worin R^{1N} und R^{2N} jeweils unabhängig voneinander die für R^{2A} angegebenen Bedeutungen haben, vorzugsweise geradkettiges Alkyl, geradkettiges Alkoxy oder geradkettiges Alkenyl bedeuten, und
   Z¹ und Z² jeweils unabhängig voneinander -C₂H₄-, -CH=CH-, -(CH₂)₄-, -(CH₂)₃O-, -O(CH₂)₃-, -CH=CHCH₂CH₂-, -CH₂CH₂CH=CH-, -CH₂O-, -OCH₂-, -COO-, -OCO-, -C₂F₄-, -CF=CF-, -CF=CH-, -CH=CF-, -CF₂O-, -OCF₂-, -CH₂- oder eine Einfachbindung
   bedeuten.
k) Bevorzugte Mischungen enthalten eine oder mehrere Verbindungen ausgewählt aus der Gruppe der Difluordibenzochroman-Verbindungen der Formel BC, Chromane der Formeln CR, fluorierte Phenanthrene der Formeln PH-1 und PH-2, fluorierte Dibenzofurane der Formeln BF1 und BF-2, 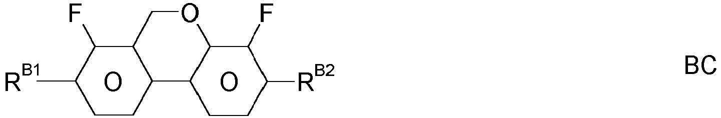 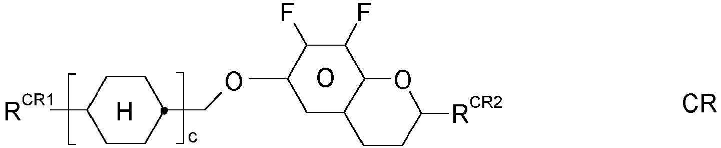 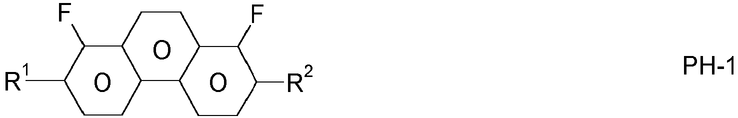 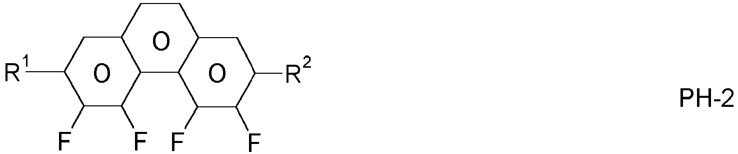 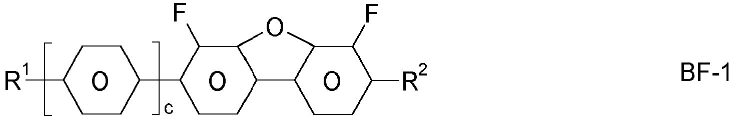 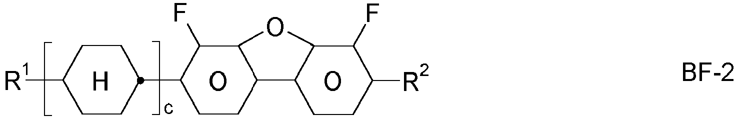 worin
   R^{B1}, R^{B2}, R^{CR1}, R^{CR2}, R¹, R² jeweils unabhängig voneinander die Bedeutung von R^{2A} aufweisen, c ist 1 oder 2. R¹ und R² bedeuten vorzugsweise unabhängig voneinander Alkyl oder Alkoxy mit 1 bis 6 C-Atomen.

Die erfindungsgemäßen Mischungen enthalten die Verbindungen der Formeln BC, CR, PH-1, PH-2 und/oder BF vorzugsweise in Mengen von 3 bis 20 Gew.%, insbesondere in Mengen von 3 bis 15 Gew.%.

Besonders bevorzugte Verbindungen der Formeln BC und CR sind die Verbindungen BC-1 bis BC-7 und CR-1 bis CR-5, 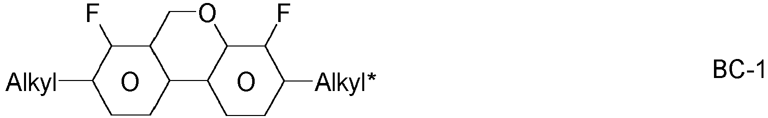 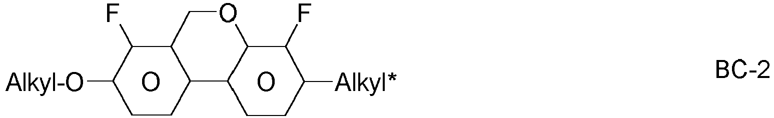 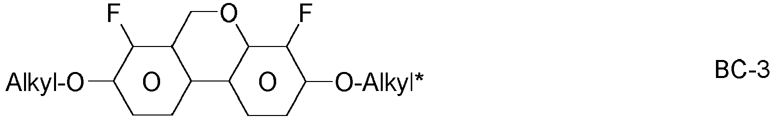 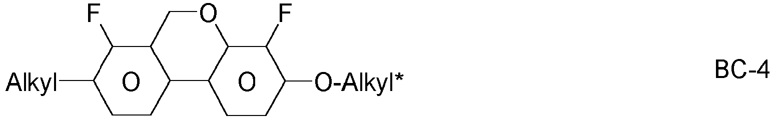 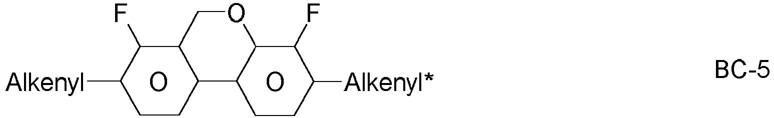 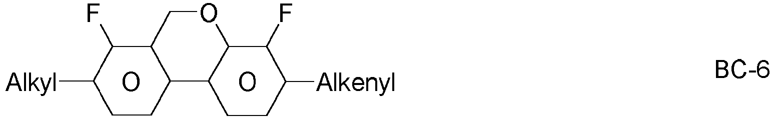 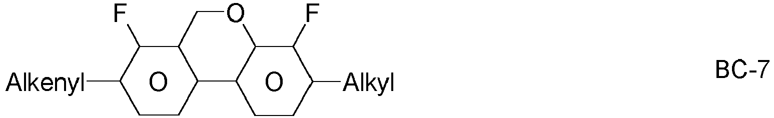 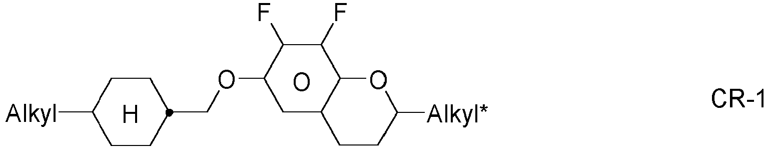 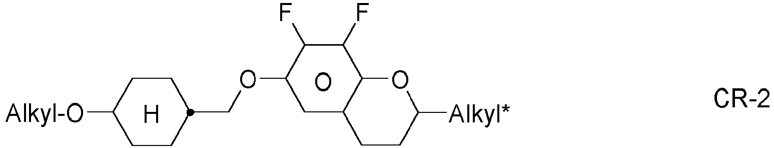 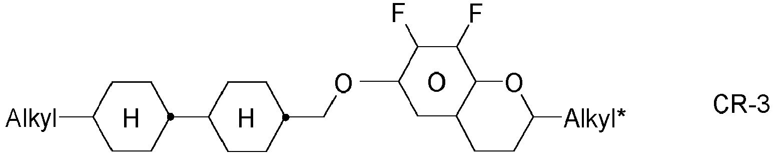 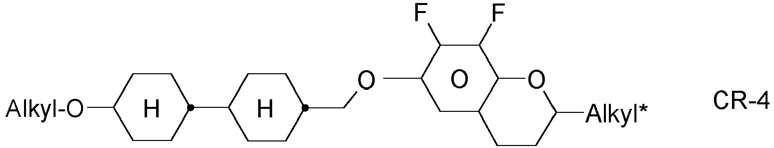 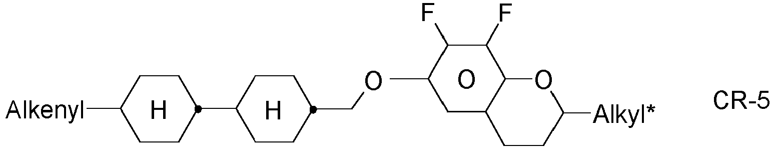 worin
- Alkyl und Alkyl*: jeweils unabhängig voneinander einen geradkettigen Alkylrest mit 1-6 C-Atomen, und
- Alkenyl und Alkenyl*: jeweils unabhängig voneinander einen geradkettigen Alkenylrest mit 2-6 C-Atomen,
bedeuten.

Ganz besonders bevorzugt sind Mischungen enthaltend eine, zwei oder drei Verbindungen der Formel BC-2, BF-1 und/oder BF-2.
I) Bevorzugte Mischungen enthalten eine oder mehrere Indan-Verbindungen der Formeln In, 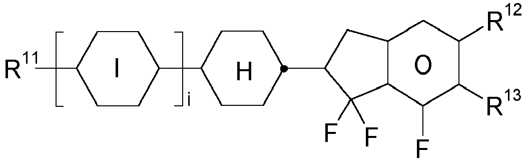 In worin
R¹¹ , R¹²,
- R¹³: jeweils unabhängig voneinander einen geradkettigen Alkyl-, oder Alkoxy-, Alkoxyalkyl-, Alkenylrest mit 1-6 C-Atomen,
- R¹² und R¹³: zusätzlich Halogen, vorzugsweise F,

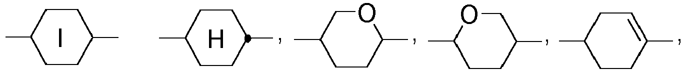

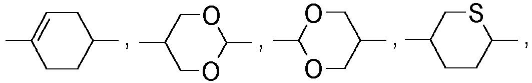

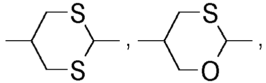

- i: 0, 1 oder 2
bedeuten.

Bevorzugte Verbindungen der Formel In sind die nachfolgend genannten Verbindungen der Formeln In-1 bis In-16,

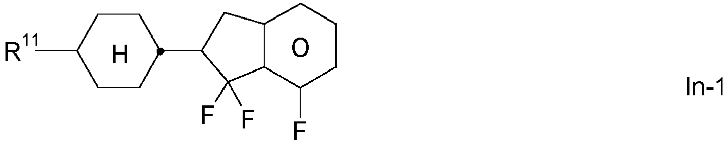

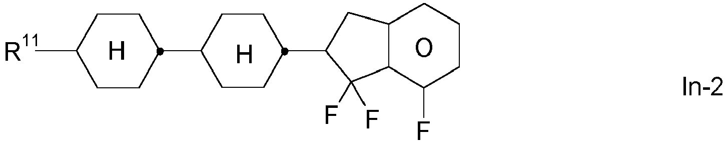

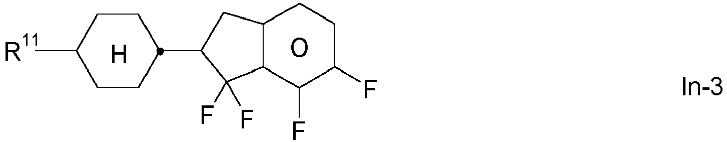

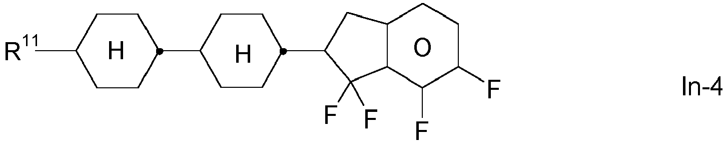

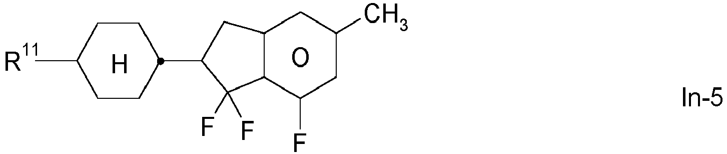

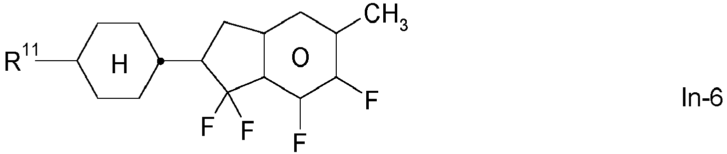

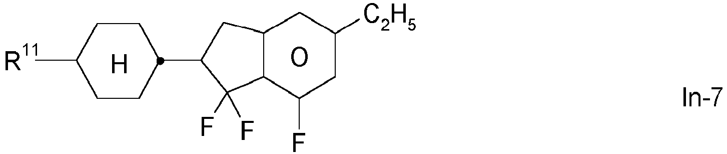

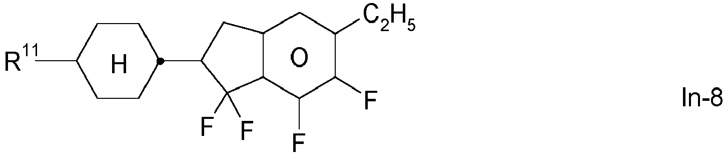

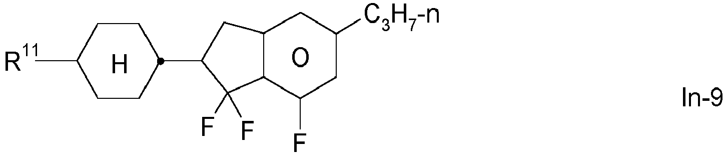

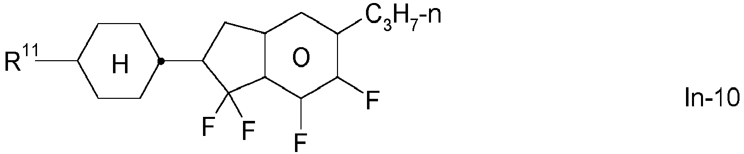

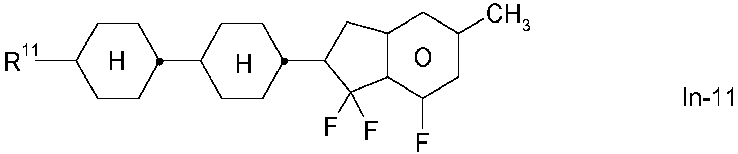

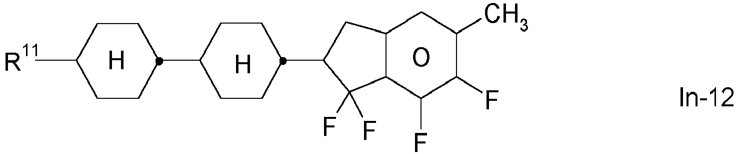

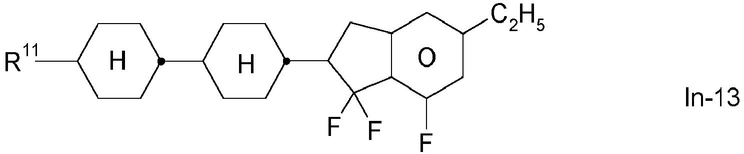

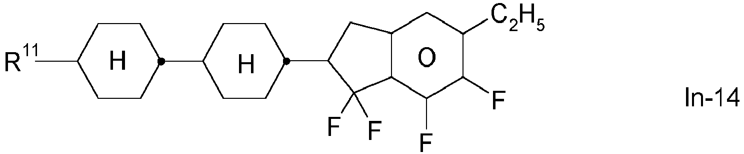

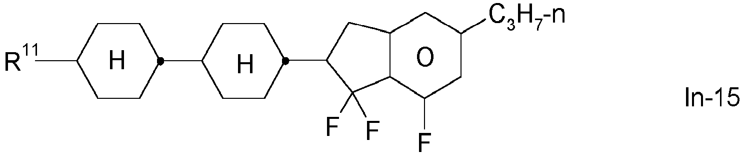

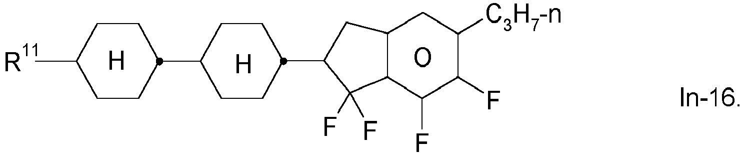

Besonders bevorzugt sind die Verbindungen der Formeln In-1, In-2, In-3 und In-4.

Die Verbindungen der Formeln In und der Unterformeln In-1 bis In-16 werden vorzugsweise in Konzentrationen ≥ 5 Gew.%, insbesondere 5 - 30 Gew.% und ganz besonders bevorzugt 5 - 25 Gew.% in den erfindungsgemäßen Mischungen eingesetzt.
m) Bevorzugte Mischungen enthalten zusätzlich eine oder mehrere Verbindungen der Formeln L-1 bis L-11, 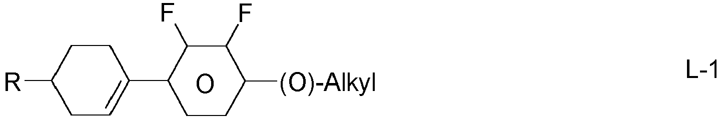 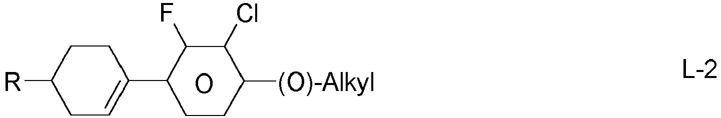 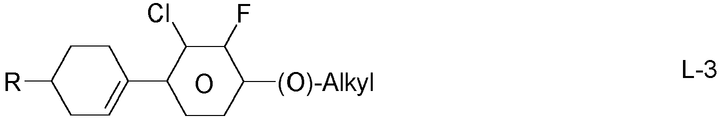 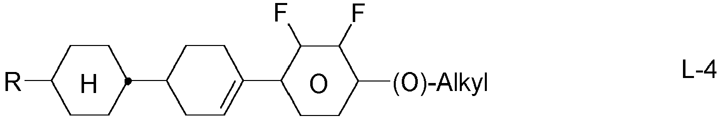 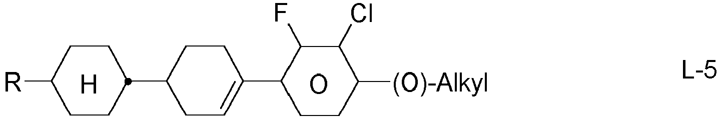 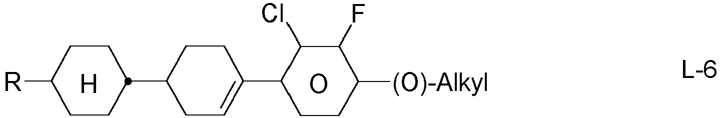 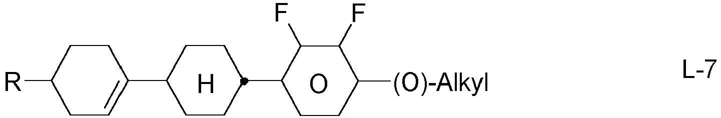 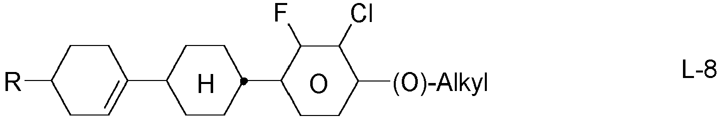 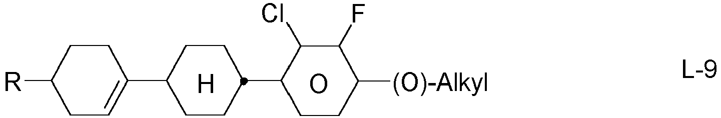 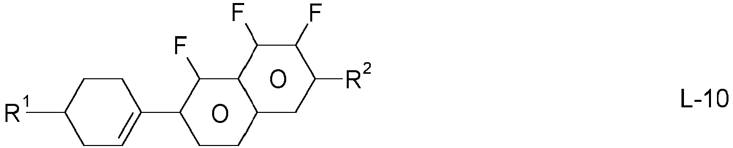 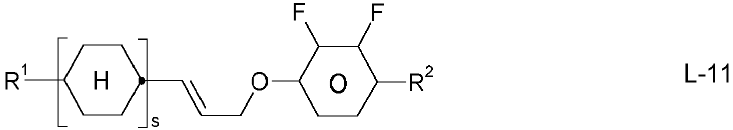 worin
R, R¹ und R² jeweils unabhängig voneinander die für R^{2A} in Anspruch 5 angegebenen Bedeutungen haben und Alkyl ein Alkylrest mit 1-6 C-Atomen bedeutet. s bedeutet 1 oder 2.

Besonders bevorzugt sind die Verbindungen der Formeln L-1 und L-4, insbesondere L-4.

Die Verbindungen der Formeln L-1 bis L-11 werden vorzugsweise in Konzentrationen von 5 - 50 Gew.%, insbesondere 5 - 40 Gew.% und ganz besonders bevorzugt 10 - 40 Gew.% eingesetzt.

Besonders bevorzugte Mischungskonzepte werden nachfolgend genannt: (Die verwendeten Acronyme sind in Tabelle A erklärt. n und m bedeuten hier jeweils unabhängig voneinander 1-15, vorzugsweise 1-6.)

Die erfindungsgemäßen Mischungen enthalten vorzugsweise
- eine oder mehrere Verbindungen der Formel I, worin L¹ = L² =F und R¹ = R^{1*} = Alkoxy bedeuten,
- CPY-n-Om, insbesondere CPY-2-O2, CPY-3-O2 und/oder CPY-5-O2, vorzugsweise in Konzentrationen > 5 %, insbesondere 10-30 %, bezogen auf die Gesamtmischung,
   und/oder
- CY-n-Om, vorzugsweise CY-3-O2, CY-3-O4, CY-5-O2 und/oder CY-5-O4, vorzugsweise in Konzentrationen > 5 %, insbesondere 15-50 %, bezogen auf die Gesamtmischung,
   und/oder
- CCY-n-Om, vorzugsweise CCY-4-O2, CCY-3-O2, CCY-3-O3, CCY-3-O1 und/oder CCY-5-O2, vorzugsweise in Konzentrationen > 5 %, insbesondere 10-30 %, bezogen auf die Gesamtmischung,
   und/oder
- CLY-n-Om, vorzugsweise CLY-2-O4, CLY-3-O2 und/oder CLY-3-O3, vorzugsweise in Konzentrationen > 5 %, insbesondere 10-30 %, bezogen auf die Gesamtmischung,
   und/oder
- CK-n-F, vorzugsweise CK-3-F, CK-4-F und/oder CK-5-F, vorzugsweise > 5 %, insbesondere 5-25 %, bezogen auf die Gesamtmischung.

Weiterhin bevorzugt sind erfindungsgemäße Mischungen, die folgende Mischungskonzepte enthalten:
(n und m bedeuten jeweils unabhängig voneinander 1-6.)
- CPY-n-Om und CY-n-Om, vorzugsweise in Konzentrationen von 10-80 % bezogen auf die Gesamtmischung,
   und/oder
- CPY-n-Om und CK-n-F, vorzugsweise in Konzentrationen von 10-70 % bezogen auf die Gesamtmischung,
   und/oder
- CPY-n-Om und PY-n-Om, vorzugsweise CPY-2-O2 und/oder CPY-3-O2 und PY-3-O2, vorzugsweise in Konzentrationen von 10 - 45 % bezogen auf die Gesamtmischung,
   und/oder
- CPY-n-Om und CLY-n-Om, vorzugsweise in Konzentrationen von 10-80 % bezogen auf die Gesamtmischung.
   und/oder
- CCVC-n-V, vorzugsweise CCVC-3-V, vorzugsweise in Konzentrationen von 2 - 10 % bezogen auf die Gesamtmischung,
   und/oder
- CCC-n-V, vorzugsweise CCC-2-V und/oder CCC-3-V, vorzugsweise in Konzentrationen von 2 - 10 % bezogen auf die Gesamtmischung,
   und/oder
- CC-V-V, vorzugsweise in Konzentrationen von 5 - 50 % bezogen auf die Gesamtmischung.

Ein weiterer Gegenstand der Erfindung ist eine elektrooptische Anzeige mit einer Aktivmatrix-Adressierung basierend auf dem ECB-, VA-, PS-VA-, PA-VA-, IPS-, PS-IPS-, FFS- oder PS-FFS-Effekt, dadurch gekennzeichnet, dass sie als Dielektrikum ein flüssigkristallines Medium nach einem oder mehreren der Ansprüche 1 bis 15 enthält.

Das erfindungsgemäße flüssigkristalline Medium weist bevorzugt eine nematische Phase von ≤ -20 °C bis ≥ 70 °C, besonders bevorzugt von ≤ -30 °C bis ≥ 80 °C, ganz besonders bevorzugt von ≤ -40 °C bis ≥ 90 °C auf.

Hierbei bedeutet der Begriff "eine nematische Phase aufweisen" einerseits, dass bei tiefen Temperaturen bei der entsprechenden Temperatur keine smektische Phase und keine Kristallisation beobachtet wird und andererseits, dass beim Aufheizen aus der nematischen Phase noch keine Klärung auftritt. Die Untersuchung bei tiefen Temperaturen wird in einem Fließviskosimeter bei der entsprechenden Temperatur durchgeführt sowie durch Lagerung in Testzellen einer der elektrooptischen Anwendung entsprechenden Schichtdicke für mindestens 100 Stunden überprüft. Wenn die Lagerstabilität bei einer Temperatur von -20 °C in einer entsprechenden Testzelle 1.000 h oder mehr beträgt, wird das Medium als bei dieser Temperatur stabil bezeichnet. Bei Temperaturen von -30 °C bzw. -40 °C betragen die entsprechenden Zeiten 500 h bzw. 250 h. Bei hohen Temperaturen wird der Klärpunkt nach üblichen Methoden in Kapillaren gemessen.

Vorzugsweise weist die Flüssigkristallmischung einen nematischen Phasenbereich von mindestens 60 K und eine Fließviskosität *ν*₂₀ von maximal 30 mm² · s⁻¹ bei 20 °C auf.

Die Werte der Doppelbrechung Δn in der Flüssigkristallmischung liegen in der Regel zwischen 0,07 und 0,16, vorzugsweise zwischen 0,08 und 0,13.

Die erfindungsgemäße Flüssigkristallmischung weist ein Δε von -0,5 bis -8,0, insbesondere von -2,5 bis -6,0 auf, wobei Δε die dielektrische Anisotropie bedeutet. Die Rotationsviskosität γ₁ bei 20 °C ist vorzugsweise ≤ 150 mPa·s, insbesondere ≤ 120 mPa·s.

Die erfindungsgemäßen Flüssigkristallmedien weisen relativ kleine Werte für die Schwellenspannung (V₀) auf. Vorzugsweise sind sie im Bereich von 1,7 V bis 3,0 V, besonders bevorzugt ≤ 2,5 V und ganz besonders bevorzugt ≤ 2,3 V.

Der Begriff "Schwellenspannung" bezieht sich für die vorliegende Erfindung auf die kapazitive Schwelle (V₀), auch Freedericksz-Schwelle genannt, sofern nicht explizit anders angegeben.

Außerdem weisen die erfindungsgemäßen Flüssigkristallmedien hohe Werte für die Voltage Holding Ratio in Flüssigkristallzellen auf.

In der Regel zeigen dabei Flüssigkristallmedien mit einer geringen Ansteuerspannung bzw. Schwellenspannung eine geringere Voltage Holding Ratio als solche mit einer größeren Ansteuerspannung bzw. Schwellenspannung und umgekehrt.

Für die vorliegende Erfindung bedeuten die Begriffe "dielektrisch positive Verbindungen" solche Verbindungen mit einem Δε > 1,5, "dielektrisch neutrale Verbindungen" solche mit -1,5 ≤ Δε ≤ 1,5 und "dielektrisch negative" Verbindungen solche mit Δε < -1,5. Hierbei wird die dielektrische Anisotropie der Verbindungen bestimmt, indem 10 % der Verbindungen in einem flüssigkristallinen Host gelöst werden und von der resultierenden Mischung die Kapazität in mindestens jeweils einer Testzelle mit 20 µm Schichtdicke mit homeotroper und mit homogener Oberflächenorientierung bei 1 kHz bestimmt wird. Die Messspannung beträgt typischerweise 0,5 V bis 1,0 V, sie ist jedoch stets niedriger als die kapazitive Schwelle der jeweiligen untersuchten Flüssigkristallmischung.

Alle angegebenen Werte für Temperaturen für die vorliegende Erfindung sind in °C.

Die erfindungsgemäßen Mischungen sind für alle VA-TFT-Anwendungen geeignet, wie z.B. VAN, MVA, (S)-PVA, ASV, PSA (polymer sustained VA) und PS-VA (polymer stabilized VA). Weiterhin sind sie für IPS (In plane switching) und FFS (Fringe field switching) mit negativem Δε geeignet. Die nematischen Flüssigkristallmischungen in den erfindungsgemäßen Anzeigen enthalten in der Regel zwei Komponenten A und B, die ihrerseits aus einer oder mehreren Einzelverbindungen bestehen.

Die Komponente A weist eine deutlich negative dielektrische Anisotropie auf und verleiht der nematischen Phase eine dielektrische Anisotropie von ≤ -0,5. Sie enthält bevorzugt neben einer oder mehreren Verbindungen der Formel I, die Verbindungen der Formeln IIA, IIB und/oder IIC, ferner eine oder mehrere Verbindungen der Formel O-17.

Der Anteil der Komponente A liegt vorzugsweise zwischen 45 und 100 %, insbesondere zwischen 60 und 100 %.

Für Komponente A wird vorzugsweise eine (oder mehrere) Einzelverbindung(en) gewählt, die einen Wert von Δε ≤ -0,8 haben. Dieser Wert muss umso negativer sein, je kleiner der Anteil A an der Gesamtmischung ist. Die Komponente B weist eine ausgeprägte Nematogenität und eine Fließviskosität von nicht mehr als 30 mm^{2·}s⁻¹, vorzugsweise nicht mehr als 25 mm^{2·}s⁻¹, bei 20 °C auf.

Dem Fachmann sind aus der Literatur eine Vielzahl geeigneter Materialien bekannt. Besonders bevorzugt sind Verbindungen der Formel O-17.

Besonders bevorzugte Einzelverbindungen der Komponente B sind extrem niedrig viskose nematische Flüssigkristalle mit einer Fließviskosität von nicht mehr als 18, vorzugsweise nicht mehr als 12 mm^{2·}s⁻¹, bei 20 °C.

Komponente B ist monotrop oder enantiotrop nematisch, weist keine smektischen Phasen auf und kann in Flüssigkristallmischungen das Auftreten von smektischen Phasen bis zu sehr tiefen Temperaturen verhindern. Versetzt man beispielsweise eine smektische Flüssigkristallmischung mit jeweils verschiedenen Materialien mit hoher Nematogenität, so kann durch den erzielten Grad der Unterdrückung smektischer Phasen die Nematogenität dieser Materialien verglichen werden.

Optional kann die Mischung auch eine Komponente C enthalten, wobei es sich um Verbindungen mit einer dielektrischen Anisotropie von Δε ≥1,5 handelt. Diese sogenannten positiven Verbindungen sind in der Regel in einer Mischung mit negativer dielektrischer Anisotropie in Mengen von ≤ 20 Gew.% bezogen auf die Gesamtmischung enthalten.

Sofern die erfindungsgemäße Mischung eine oder mehrere Verbindungen mit einer dielektrischen Anisotropie von Δε ≥1,5 enthält, handelt es sich vorzugsweise um eine oder mehrere Verbindungen ausgewählt aus der Gruppe der Verbindungen der Formeln P-1 bis P-4, 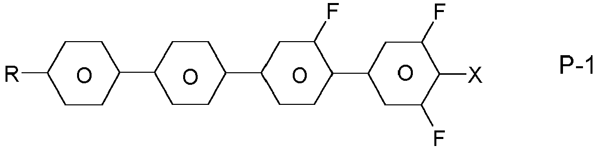 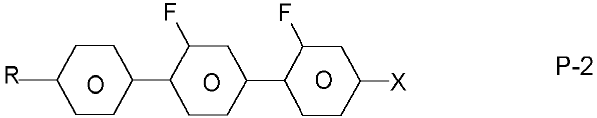 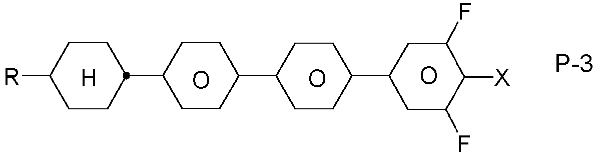 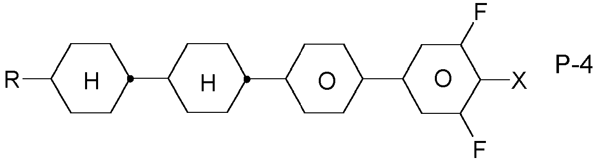 worin
- R: geradkettiges Alkyl, Alkoxy oder Alkenyl mit jeweils 1 bzw. 2 bis 6 C-Atomen, und
- X: F, Cl, CF₃, OCF₃, OCHFCF₃ oder CCF₂CHFCF₃, vorzugsweise F oder OCF₃
bedeuten.

Vorzugsweise werden die Verbindungen der Formeln P-1 bis P-4 in Konzentrationen von 2 - 15 %, inbesondere 2 - 10 % in den erfindungsgemäßen Mischungen eingesetzt.

Besonders bevorzugt ist die Verbindung der Formel, 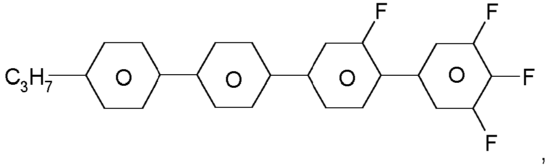 die vorzugsweise in Mengen von 2 - 15 % in den erfindungsgemäßen Mischungen eingesetzt wird.

Daneben können diese Flüssigkristallphasen auch mehr als 18 Komponenten, vorzugsweise 18 bis 25 Komponenten, enthalten.

Vorzugsweise enthalten die Phasen neben einer oder mehreren Verbindungen der Formel I, 4 bis 15, insbesondere 5 bis 12, und besonders bevorzugt < 10, Verbindungen der Formeln IIA, IIB und/oder IIC und optional eine oder mehrere Verbindungen der Formel O-17.

Neben Verbindungen der Formeln I und den Verbindungen der Formeln IIA, IIB und/oder IIC und optional O-17 können auch noch andere Bestandteile zugegen sein, z. B. in einer Menge von bis zu 45 % der Gesamtmischung, vorzugsweise jedoch bis zu 35 %, insbesondere bis zu 10 %.

Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexylester, Phenylcyclohexane, Cyclohexylbiphenyle, Cyclohexylcyclohexane, Cyclohexylnaphthaline, 1,4-Bis-cyclohexylbiphenyle oder Cylohexylpyrimidine, Phenyl- oder Cyclohexyldioxane, gegebenenfalls halogenierten Stilbenen, Benzylphenylether, Tolane und substituierten Zimtsäureestern.

Die wichtigsten als Bestandteile derartiger Flüssigkristallphasen in Frage kommenden Verbindungen lassen sich durch die Formel IV charakterisieren,

R²⁰-L-G-E-R²¹ IV

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituierten Naphthalin, Di- und Tetrahydronaphthalin, Chinazolin und Tetrahydrochinazolin gebildeten Gruppe,

| | | |
|---|---|---|
| G | -CH=CH- | -N(O)=N- |
| | -CH=CQ- | -CH=N(O)- |
| | -C≡C- | -CH₂-CH₂- |
| | -CO-O- | -CH₂-O- |
| | -CO-S- | -CH₂-S- |
| | -CH=N- | -COO-Phe-COO- |
| | -CF₂O- | -CF=CF- |
| | -OCF₂- | -OCH₂- |
| | -(CH₂)₄- | -(CH₂)₃O- |

oder eine C-C-Einfachbindung, Q Halogen, vorzugsweise Chlor oder -CN, und R²⁰ und R²¹ jeweils Alkyl, Alkenyl, Alkoxy, Alkoxyalkyl oder Alkoxycarbonyloxy mit bis zu 18, vorzugsweise bis zu 8 Kohlenstoffatomen, oder einer dieser Reste auch CN, NC, NO₂, NCS, CF₃, SF₅, OCF₃, F, Cl oder Br bedeuten.

Bei den meisten dieser Verbindungen sind R²⁰ und R²¹ voneinander verschieden, wobei einer dieser Reste meist eine Alkyl- oder Alkoxygruppe ist. Auch andere Varianten der vorgesehenen Substituenten sind gebräuchlich. Viele solcher Substanzen oder auch Gemische davon sind im Handel erhältlich. Alle diese Substanzen sind nach literaturbekannten Methoden herstellbar.

Es versteht sich für den Fachmann von selbst, dass die erfindungsgemäße VA-, IPS- oder FFS- Mischung auch Verbindungen enthalten kann, worin beispielsweise H, N, O, Cl, F durch die entsprechenden Isotope ersetzt sind.

Den erfindungsgemäßen Mischungen können weiterhin polymerisierbare Verbindungen, sogenannte reaktive Mesogene (RMs), beispielsweise wie in U.S. 6,861,107 offenbart, in Konzentrationen von bevorzugt 0,01 - 5 Gew.%, besonders bevorzugt 0,2 - 2 % bezogen auf die Mischung, zugesetzt werden. Optional können diese Mischungen auch einen Initiator enthalten, wie beispielsweise in der U.S 6,781,665 beschrieben. Der Initiator, z.B. Irganox-1076 der Fa. BASF, wird vorzugsweise der Mischung enthaltend polymerisierbare Verbindungen in Mengen von 0-1 % zugesetzt. Derartige Mischungen können für sogenannte Polymer Stabilized VA-Modes (PS-VA) oder PSA (Polymer sustained VA), bei denen eine Polymerisierung der reaktiven Mesogene in der flüssigkristallinen Mischung erfolgen soll, verwendet werden. Voraussetzung hierfür ist, dass die Flüssigkristallmischung selbst keine polymerisierbaren Komponenten enthält.

In einer bevorzugten Ausführungsform der Erfindung sind die polymerisierbaren Verbindungen ausgewählt aus den Verbindungen der Formel M

R^{Ma}-A^{M1}-(Z^{M1}-A^{M2})ₘ₁-R^{Mb} M

worin die einzelnen Reste folgende Bedeutung haben:
- R^{Ma} und R^{Mb}: jeweils unabhängig voneinander P, P-Sp-, H, Halogen, SF₅, NO₂, eine Alkyl-, Alkenyl- oder Alkinylgruppe, wobei bevorzugt mindestens einer der Reste R^{Ma} und R^{Mb} eine Gruppe P oder P-Sp- bedeutet oder enthält,
- P: eine polymerisierbare Gruppe,
- Sp: eine Abstandsgruppe oder eine Einfachbindung,
- A^{M1} und A^{M2}: jeweils unabhängig voneinander eine aromatische, heteroaromatische, alicyclische oder heterocyclische Gruppe, vorzugsweise mit 4 bis 25 Ringatomen, bevorzugt C-Atomen, welche auch anellierte Ringe umfasst oder enthalten kann, und die optional ein- oder mehrfach durch L substitutiert sein kann,
- L: P, P-Sp-, OH, CH₂OH, F, Cl, Br, I, -CN, -NO₂, -NCO, -NCS, -OCN, -SCN, -C(=O)N(R^{x})₂, -C(=O)Y¹, -C(=O)R^{x}, -N(R^{x})₂, optional substituiertes Silyl, optional substituiertes Aryl mit 6 bis 20 C Atomen, oder geradkettiges oder verzweigtes Alkyl, Alkoxy, Alkylcarbonyl, Alkoxycarbonyl, Alkylcarbonlyoxy oder Alkoxycarbonyloxy mit 1 bis 25 C-Atomen, worin auch ein oder mehrere H-Atome durch F, Cl, P oder P-Sp- ersetzt sein können, bevorzugt P, P-Sp-, H, OH, CH₂OH, Halogen, SF₅, NO₂, eine Alkyl-, Alkenyl- oder Alkinylgruppe,
- Y¹: Halogen,
- Z^{M1}: -O-, -S-, -CO-, -CO-O-, -OCO-, -O-CO-O-, -OCH₂-, -CH₂O- , -SCH₂-, -CH₂S-, -CF₂O-, -OCF₂-, -CF₂S-, -SCF₂-, -(CH₂)ₙ₁- , -CF₂CH₂-, -CH₂CF₂-, -(CF₂)ₙ₁-, -CH=CH-, -CF=CF-, -C≡C- , -CH=CH-, -COO-, -OCO-CH=CH-, CR⁰R⁰⁰ oder eine Einfachbindung,
- R⁰ und R⁰⁰: jeweils unabhängig voneinander H oder Alkyl mit 1 bis 12 C-Atomen,
- R^{x}: P, P-Sp-, H, Halogen, geradkettiges, verzweigtes oder cyclisches Alkyl mit 1 bis 25 C-Atomen, worin auch eine oder mehrere nicht benachbarte CH₂-Gruppen durch -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, P oder P-Sp- ersetzt sein können, eine optional substituierte Aryl- oder Aryloxygruppe mit 6 bis 40 C-Atomen, oder eine optional substituierte Heteroaryl- oder Heteroaryloxygruppe mit 2 bis 40 C-Atomen,
- m1: 0, 1, 2, 3 oder 4 und
- n1: 1, 2, 3 oder 4, wobei mindestens einer, bevorzugt einer, zwei oder drei, besonders bevorzugt einer oder zwei, aus der Gruppe R^{Ma}, R^{Mb} und der vorhandenen Substituenten L eine Gruppe P oder P-Sp- bedeutet oder mindestens eine Gruppe P oder P-Sp- enthält.

Besonders bevorzugte Verbindungen der Formel M sind solche, worin
- R^{Ma} und R^{Mb}: jeweils unabhängig voneinander P, P-Sp-, H, F, Cl, Br, I, -CN, -NO₂, -NCO, -NCS, -OCN, -SCN, SF₅ oder geradkettiges oder verzweigtes Alkyl mit 1 bis 25 C-Atomen, worin auch eine oder mehrere nicht benachbarte CH₂-Gruppen jeweils unabhängig voneinander durch -C(R⁰)=C(R⁰⁰)-, -C≡C- , -N(R⁰⁰)-, -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, Br, I, CN, P oder P-Sp- ersetzt sein können, wobei bevorzugt mindestens einer der Reste R^{Ma} und R^{Mb} eine Gruppe P oder P-Sp- bedeutet oder enthält,
- A^{M1} und A^{M2}: jeweils unabhängig voneinander 1,4-Phenylen, Naphthalin-1,4-diyl, Naphthalin-2,6-diyl, Phenanthren-2,7-diyl, Anthracen-2,7-diyl, Fluoren-2,7-diyl, Cumarin, Flavon, wobei in diesen Gruppen auch eine oder mehrere CH-Gruppen durch N ersetzt sein können, Cyclohexan-1,4-diyl, worin auch eine oder mehrere nicht-benachbarte CH₂-Gruppen durch O und/oder S ersetzt sein können, 1,4-Cyclohexenylen, Bicyclo[1.1.1 ]pentan-1,3-diyl, Bicyclo[2.2.2]octan-1,4-diyl, Spiro[3.3]heptan-2,6-diyl, Piperidin-1,4-diyl, Decahydronaphthalin-2,6-diyl, 1,2,3,4-Tetrahydronaphthalin-2,6-diyl, Indan-2,5-diyl oder Octahydro-4,7-methano-indan-2,5-diyl, wobei alle diese Gruppen unsubstituiert oder durch L ein- oder mehrfach substituiert sein können,
- L: P, P-Sp-, OH, CH₂OH, F, Cl, Br, I, -CN, -NO₂, -NCO, -NCS,-OCN, -SCN, -C(=O)N(R^{x})₂, -C(=O)Y¹, -C(=O)R^{x}, -N(R^{x})₂, optional substituiertes Silyl, optional substituiertes Aryl mit 6 bis 20 C Atomen, oder geradkettiges oder verzweigtes Alkyl, Alkoxy, Alkylcarbonyl, Alkoxycarbonyl, Alkylcarbonlyoxy oder Alkoxycarbonyloxy mit 1 bis 25 C-Atomen, worin auch ein oder mehrere H-Atome durch F, Cl, P oder P-Sp- ersetzt sein können,
- P: eine polymerisierbare Gruppe,
- Y¹: Halogen,
- R^{x}: P, P-Sp-, H, Halogen, geradkettiges, verzweigtes oder cyclisches Alkyl mit 1 bis 25 C-Atomen, worin auch eine oder mehrere nicht benachbarte CH₂-Gruppen durch -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, P oder P-Sp- ersetzt sein können, eine optional substituierte Aryl- oder Aryloxygruppe mit 6 bis 40 C-Atomen, oder eine optional substituierte Heteroaryl- oder Heteroaryloxygruppe mit 2 bis 40 C-Atomen,
bedeuten.

Ganz besonders bevorzugt sind Verbindungen der Formel M, worin einer von R^{Ma} und R^{Mb} oder beide P oder P-Sp- bedeuten.

Geeignete und bevorzugte RMs oder Monomere bzw. Comonomere für die Verwendung in erfindungsgemäßen flüssigkristallinen Medien und PS-VA-Anzeigen oder PSA-Anzeigen, sind beispielsweise ausgewählt aus den folgenden Formeln: 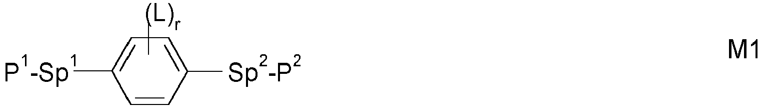 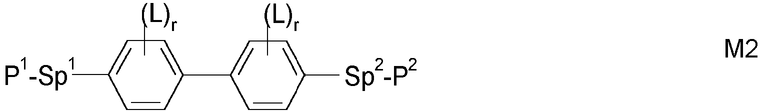 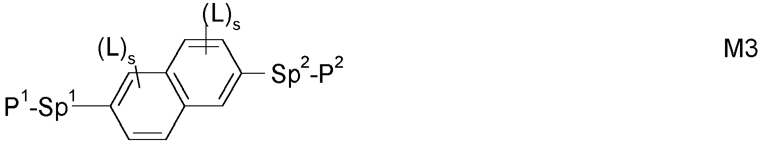 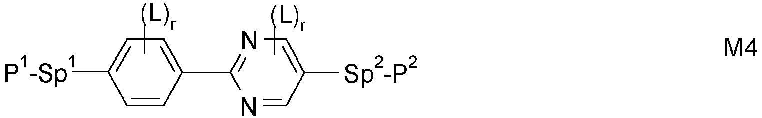 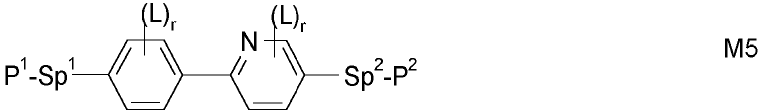 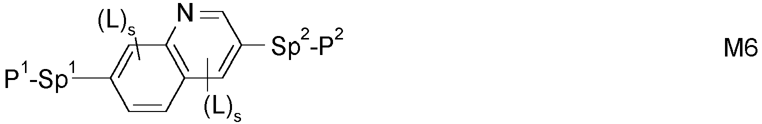 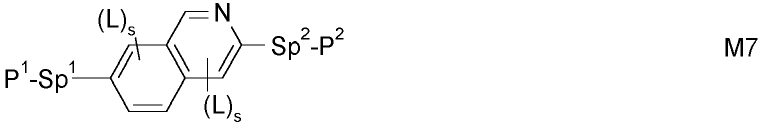 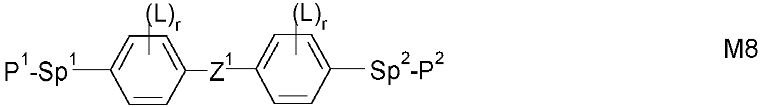 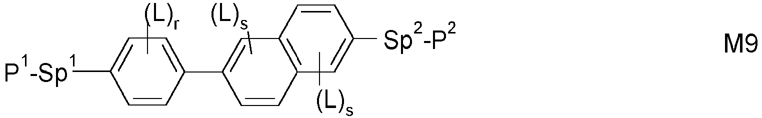 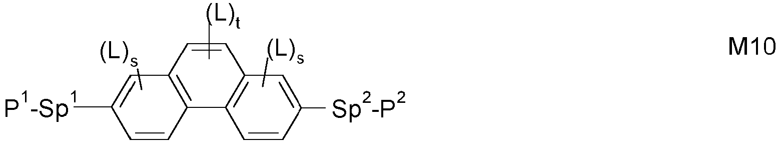 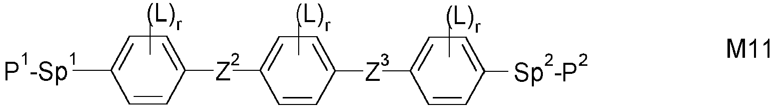 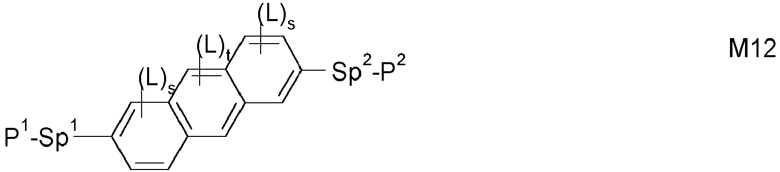 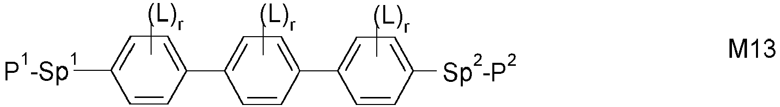 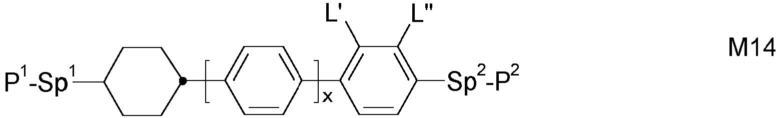 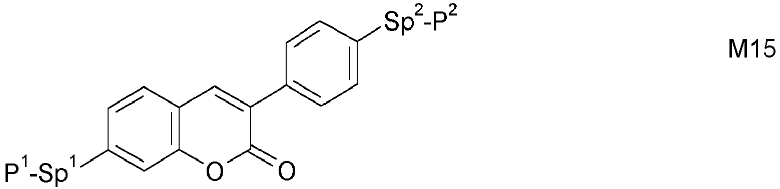 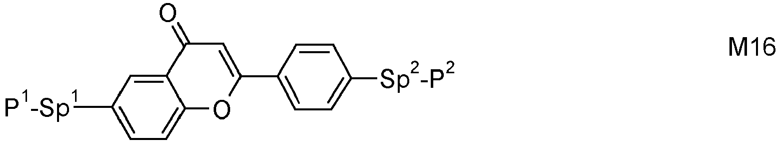 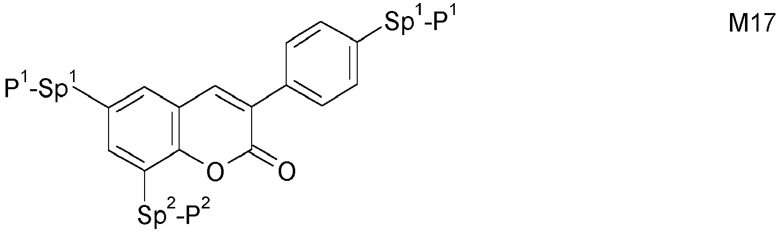 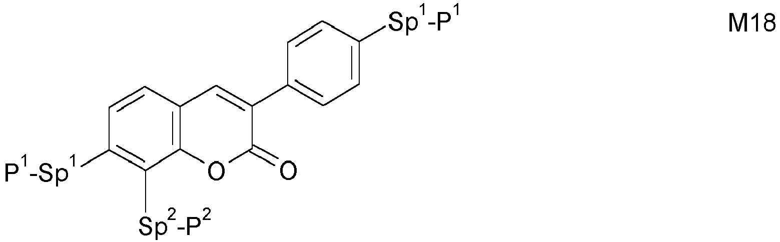 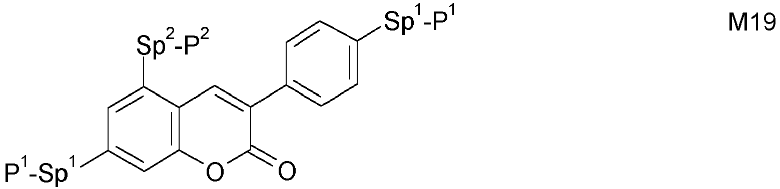 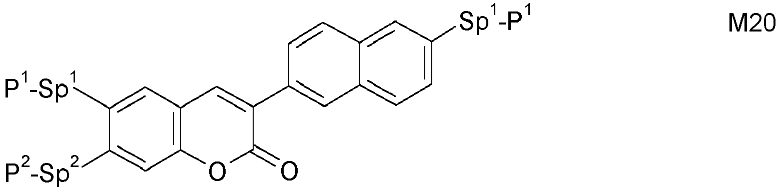 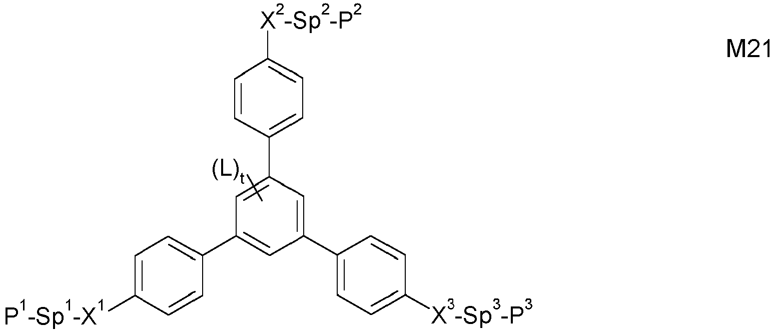 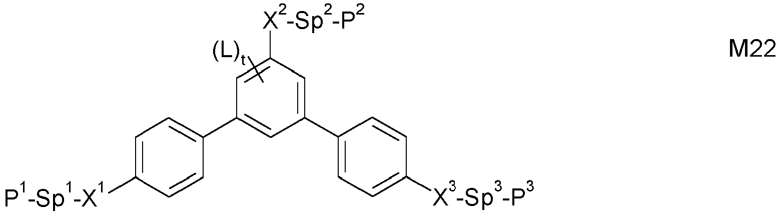 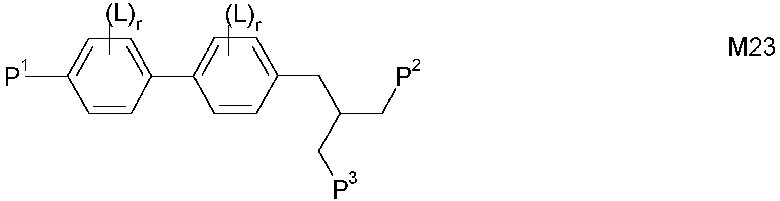 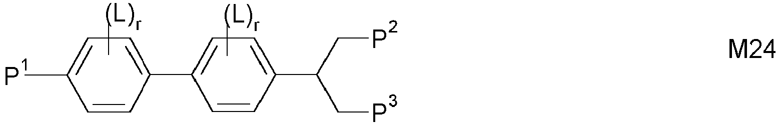 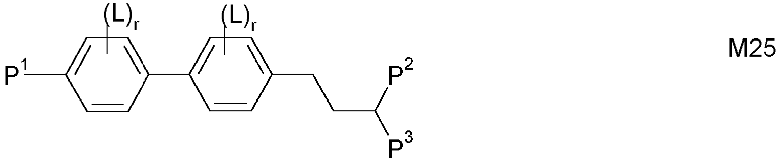 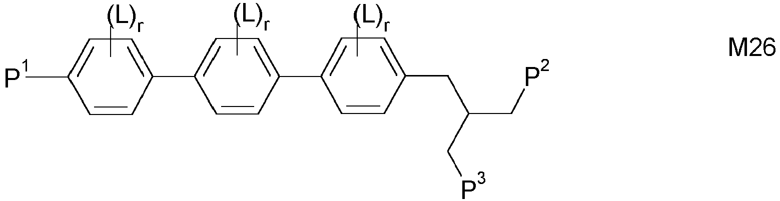 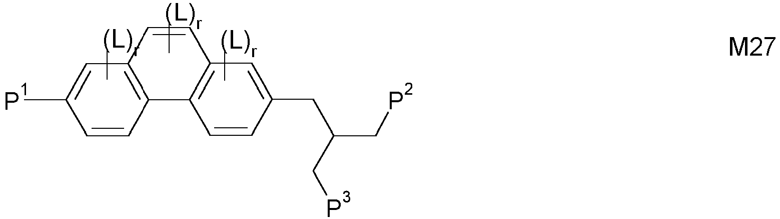 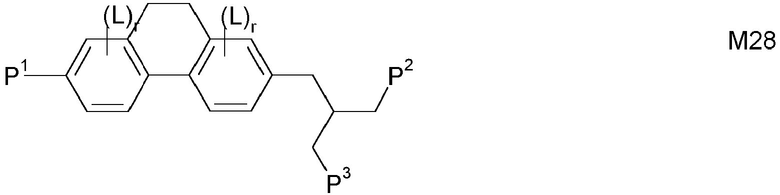 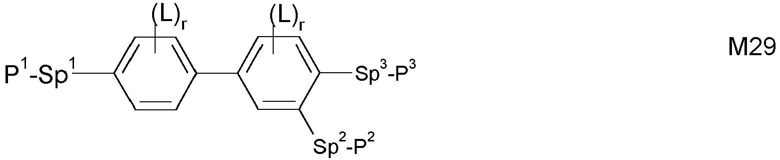 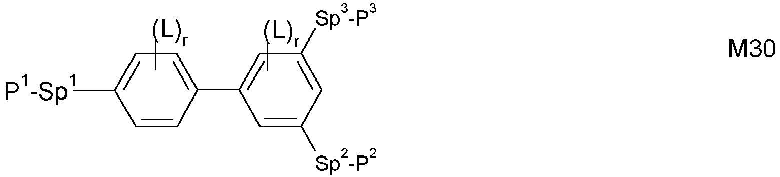 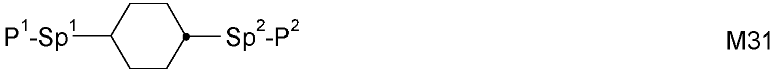 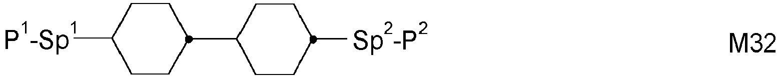 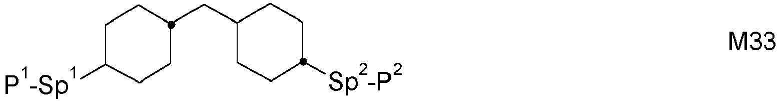 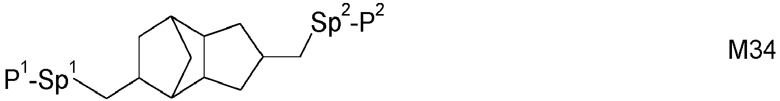 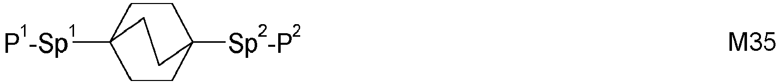 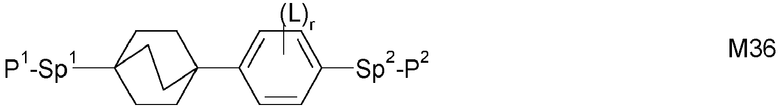 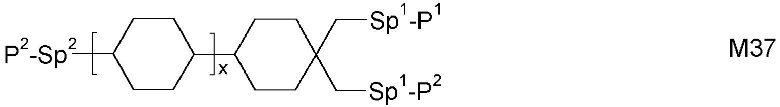 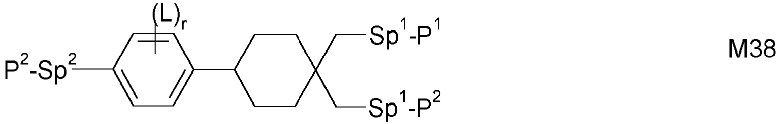 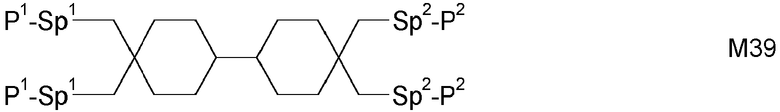 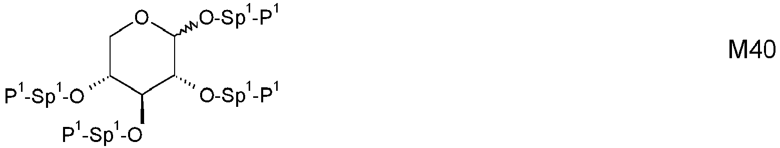 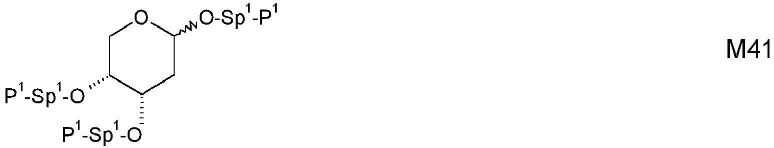 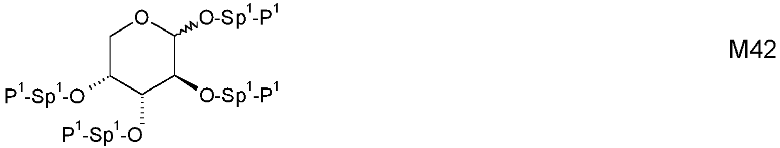 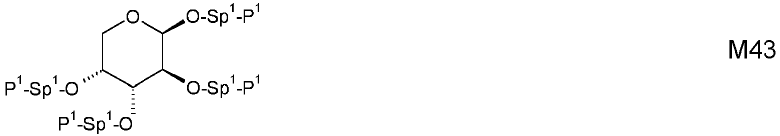 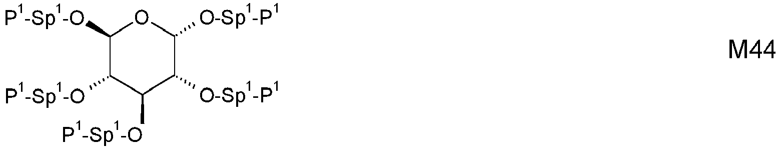 worin die einzelnen Reste folgende Bedeutung besitzen:
- P¹,P² und P³: jeweils unabhängig voneinander eine polymerisierbare Gruppe, vorzugsweise mit einer der vor- und nachstehend für P angegebenen Bedeutungen, besonders bevorzugt eine Acrylat-, Methacrylat-, Fluoracrylat-, Oxetan-, Vinyloxy- oder Epoxygruppe,
- Sp¹, Sp² und Sp³: jeweils unabhängig voneinander eine Einfachbindung oder eine Abstandsgruppe, vorzugsweise mit einer der vor- und nachstehend für Sp^{a} angegebenen Bedeutungen, und besonders bevorzugt -(CH₂)ₚ₁-, -(CH₂)ₚ₁-O-, -(CH₂)ₚ₁-CO-O- oder -(CH₂)ₚ₁-O-CO-O-, worin p1 eine ganze Zahl von 1 bis 12 ist, und wobei in den letztgenannten Gruppen die Verknüpfung zur benachbarten Ring über das O-Atom erfolgt, wobei auch einer oder mehrere der Reste P¹-Sp¹-, P²-Sp²- und P³-Sp³- einen Rest R^{aa} bedeuten können, mit der Maßgabe dass mindestens einer der vorhandenen Reste P¹-Sp¹-, P²-Sp²- und P³-Sp³- nicht R^{aa} bedeutet,
- R^{aa}: H, F, Cl, CN oder geradkettiges oder verzweigtes Alkyl mit 1 bis 25 C-Atomen, worin auch eine oder mehrere nicht benachbarte CH₂-Gruppen jeweils unabhängig voneinander durch C(R⁰)=C(R⁰⁰)-, -C≡C-, -N(R⁰)-, -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, CN oder P¹-Sp¹-ersetzt sein können, besonders bevorzugt geradkettiges oder verzweigtes, optional ein- oder mehrfach fluoriertes, Alkyl, Alkoxy, Alkenyl, Alkinyl, Alkylcarbonyl, Alkoxycarbonyl, oder Alkylcarbonyloxy mit 1 bis 12 C-Atomen (wobei die Alkenyl- und Alkinylreste mindestens zwei und die verzweigten Reste mindestens drei C-Atome aufweisen),
- R⁰, R⁰⁰: jeweils unabhängig voneinander und bei jedem Auftreten gleich oder verschieden H oder Alkyl mit 1 bis 12 C-Atomen,
- R^{y} und R^{z}: jeweils unabhängig voneinander H, F, CH₃ oder CF₃,
- X¹, X² und X³: jeweils unabhängig voneinander -CO-O-, O-CO- oder eine Einfachindung,
- Z¹: -O-, -CO-, -C(R^{y}R^{z})-,oder -CF₂CF₂-,
- Z² und Z³: jeweils unabhängig voneinander -CO-O-, -O-CO-, -CH₂O-, -OCH₂-, -CF₂O-, -OCF₂-, oder -(CH₂)ₙ-, wobei n 2, 3 oder 4 ist,
- L: bei jedem Auftreten gleich oder verschieden F, Cl, CN, SCN, SF₅ oder geradkettiges oder verzweigtes, optional ein- oder mehrfach fluoriertes, Alkyl, Alkoxy, Alkenyl, Alkinyl, Alkylcarbonyl, Alkoxycarbonyl, Alkylcarbonyloxy oder Alkoxycarbonyloxy mit 1 bis 12 C-Atomen vorzugsweise F,
- L' und L": jeweils unabhängig voneinander H, F oder Cl,
- r: 0, 1, 2, 3 oder 4,
- s: 0, 1, 2 oder 3,
- t: 0, 1 oder 2,
- x: 0 oder 1.

In den Verbindungen der Formeln M1 bis M44 bedeutet 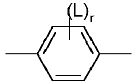 vorzugsweise 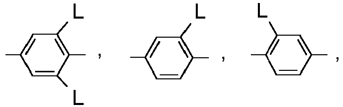 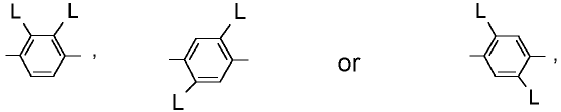 worin L, bei jedem Auftreten gleich oder verschieden, eine der vorstehenden Bedeutungen hat und vorzusgweise F, Cl, CN, NO₂, CH₃, C₂H₅, C(CH₃)₃, CH(CH₃)₂, CH₂CH(CH₃)C₂H₅, OCH₃, OC₂H₅, COCH₃, COC₂H₅, COOCH₃, COOC₂H₅, CF₃, OCF₃, OCHF₂, OC₂F₅ oder P-Sp-, besonders bevorzugt F, Cl, CN, CH₃, C₂H₅, OCH₃, COCH₃, OCF₃ oder P-Sp-, ganz besonders bevorzugt F, Cl, CH₃, OCH₃, COCH₃ oder OCF₃, insbesondere F oder CH₃ bedeutet.

Geeignete polymerisierbare Verbindungen sind beispielsweise in Tabelle D gelistet.

Bevorzugt enthalten die flüssigkristallinen Medien gemäß der vorliegenden Anmeldung insgesamt 0,1 bis 10 %, bevorzugt 0,2 bis 4,0 %, besonders bevorzugt 0,2 bis 2,0 %, an polymerisierbaren Verbindungen.

Insbesondere bevorzugt sind die polymerisierbaren Verbindungen der Formel M und der Formeln RM-1 bis RM-94.

Die erfindungsgemäßen Mischungen können weiterhin übliche Zusätze oder Additive enthalten, wie z.B. Stabilisatoren, Antioxidantien, UV-Absorber, Nanopartikel, Mikropartikel, etc.

Der Aufbau der erfindungsgemäßen Flüssigkristallanzeigen entspricht der üblichen Geometrie, wie sie z.B. in EP-OS 0 240 379, beschrieben wird.

Die folgenden Beispiele sollen die Erfindung erläutern, ohne sie zu begrenzen. Vor- und nachstehend bedeuten Prozentangaben Gewichtsprozent; alle Temperaturen sind in Grad Celsius angegeben.

In der gesamten Patentanmeldung werden 1,4-Cyclohexylenringe und 1,4-Phenylenringe wie folgt dargestellt:

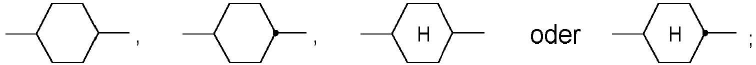

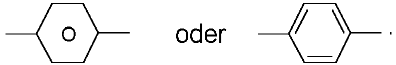

Bei den Cyclohexylenringen handelt es sich um trans-1,4-Cyclohexylenringe.

In der gesamten Patentanmeldung sowie in den Ausführungsbeispielen sind die Strukturen der Flüssigkristallverbindungen durch Acronyme angegeben. Sofern nicht anders angegeben, erfolgt die Transformation in chemische Formeln gemäß der Tabellen 1-3. Alle Reste CₙH₂ₙ₊₁, CₘH₂ₘ₊₁, und C_{m'}H_{2m'+1} bzw. CₙH₂ₙ und CₘH₂ₘ sind geradkettige Alkylreste bzw. Alkylenreste jeweils mit n, m, m' bzw. z C-Atomen.n, m, m', z bedeuten jeweils unabhängig voneinander 1, 2, 3, 4, 5, 6, 7, 8, 9, 10, 11, 12, vorzugsweise 1, 2, 3, 4, 5 oder 6. In Tabelle 1 sind die Ringelemente der jeweiligen Verbindung codiert, in Tabelle 2 sind die Brückenglieder aufgelistet und in Tabelle 3 sind die Bedeutungen der Symbole für die linken bzw. rechten Seitenketten der Verbindungen angegeben.

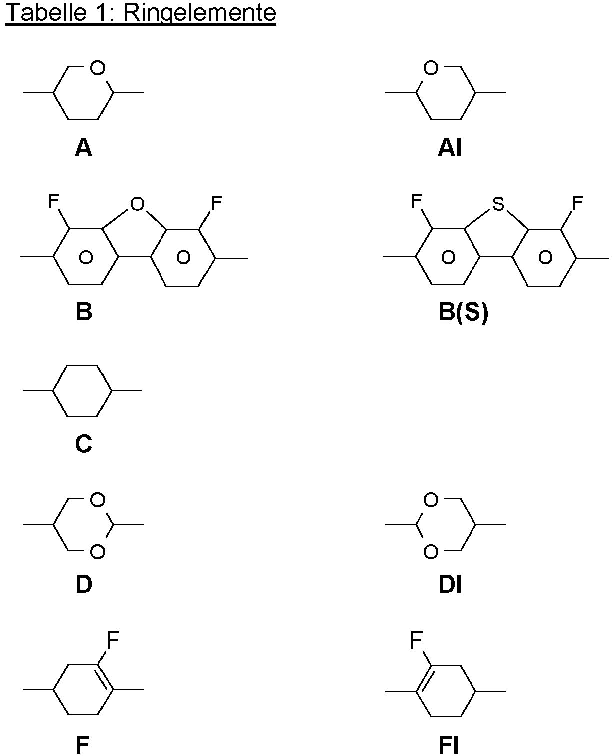

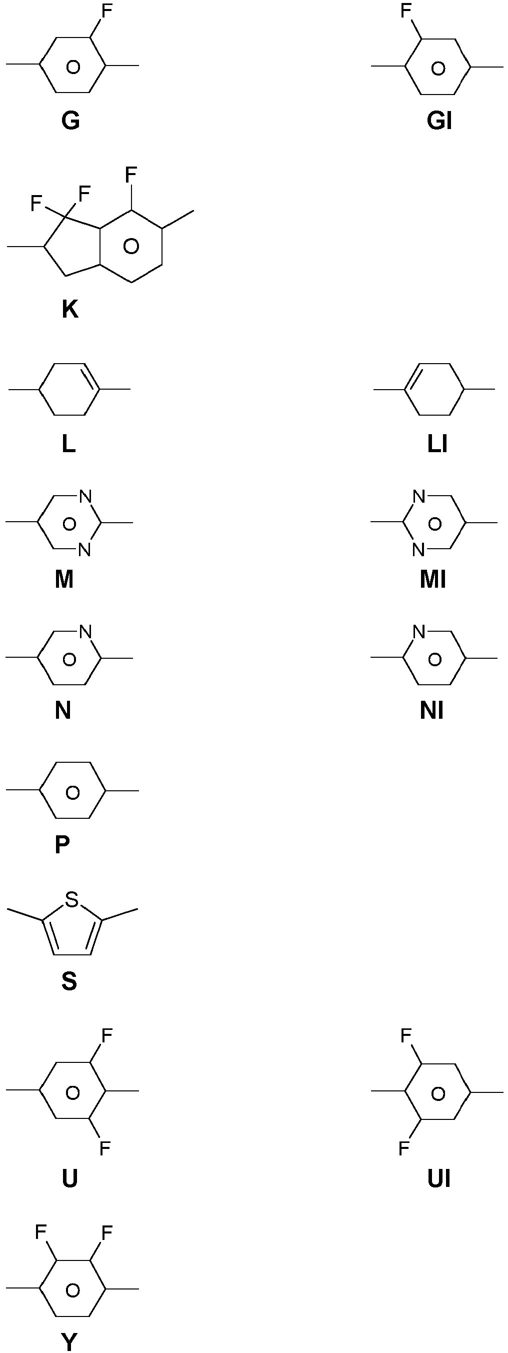

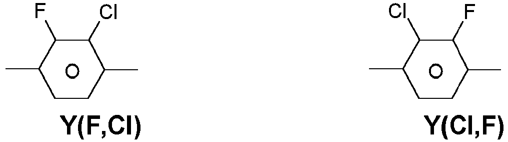

**Tabelle 2: Brückenglieder**

| | | | |
|---|---|---|---|
| E | -CH₂CH₂- | | |
| V | -CH=CH- | | |
| T | -C=C- | | |
| W | -CF₂CF₂- | | |
| Z | -COO- | **ZI** | -OCO- |
| O | -CH₂O- | OI | -OCH₂- |
| Q | -CF₂O- | QI | -OCF₂- |

**Tabelle 3: Seitenketten**

| Seitenkette links | | Seitenkette rechts | |
|---|---|---|---|
| n- | CnH₂ₙ₊₁- | -n | -CₙH₂ₙ₊₁ |
| **nO-** | CₙH₂ₙ₊₁-O- | **-On** | -O-CₙH₂ₙ₊₁ |
| **V-** | CH₂=CH- | **-V** | -CH=CH₂ |
| **nV-** | CₙH₂ₙ₊₁-CH=CH- | **-nV** | -CₙH₂ₙ-CH=CH₂ |
| **Vn-** | CH₂=CH- CₙH₂ₙ- | **-Vn** | -CH=CH-CₙH₂ₙ₊₁ |
| **nVm-** | CₙH₂ₙ₊₁-CH=CH-CₘH₂ₘ- | **-nVm** | - CₙH₂ₙ-CH=CH-CₘH₂ₘ₊₁ |
| **N-** | N=C- | **-N** | -C=N |
| **F-** | F- | **-F** | -F |
| Cl- | Cl- | -Cl | -Cl |
| **M-** | CFH₂- | **-M** | -CFH₂ |
| **D-** | CF₂H- | **-D** | -CF₂H |
| **T-** | CF₃- | **-T** | -CF₃ |
| **MO-** | CFH₂O- | **-OM** | -OCFH₂ |
| **DO-** | CF₂HO- | **-OD** | -OCF₂H |
| **TO-** | CF₃O- | **-OT** | -OCF₃ |
| **T-** | CF₃- | **-T** | -CF₃ |
| **A-** | H-C=C- | **-A** | -C=C-H |

Vorzugsweise enthalten die erfindungsgemäßen Mischungen neben den Verbindungen der Formel I eine oder mehrere Verbindungen der nachfolgend genannten Verbindungen aus der Tabelle A.

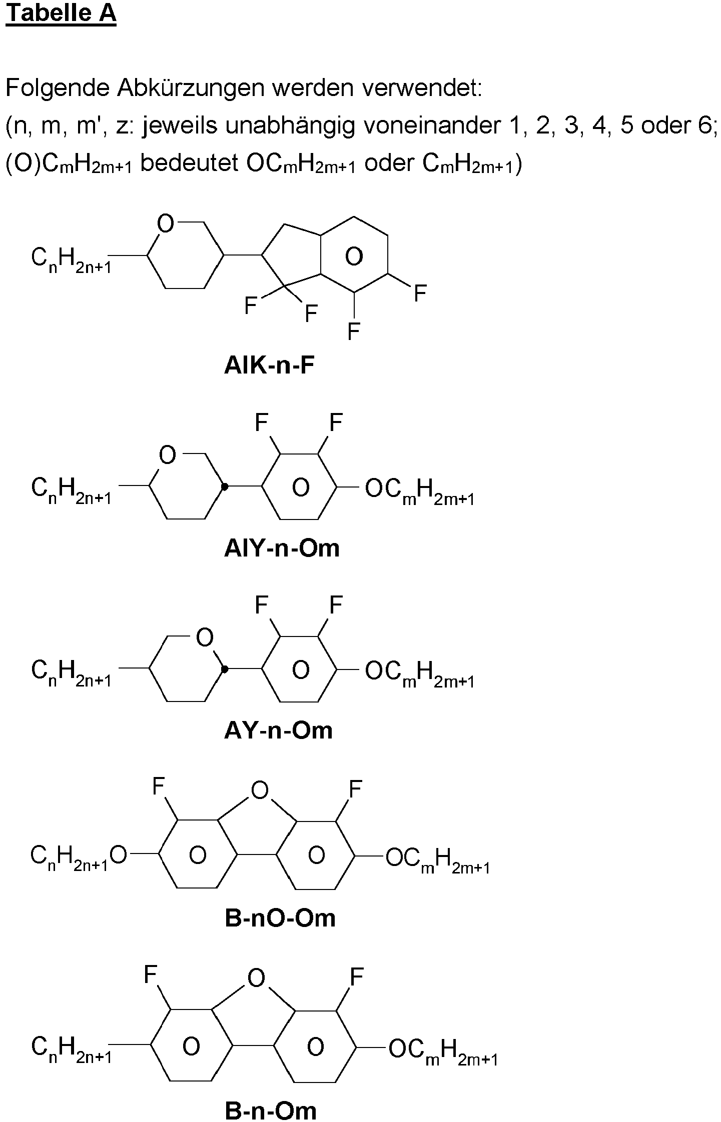

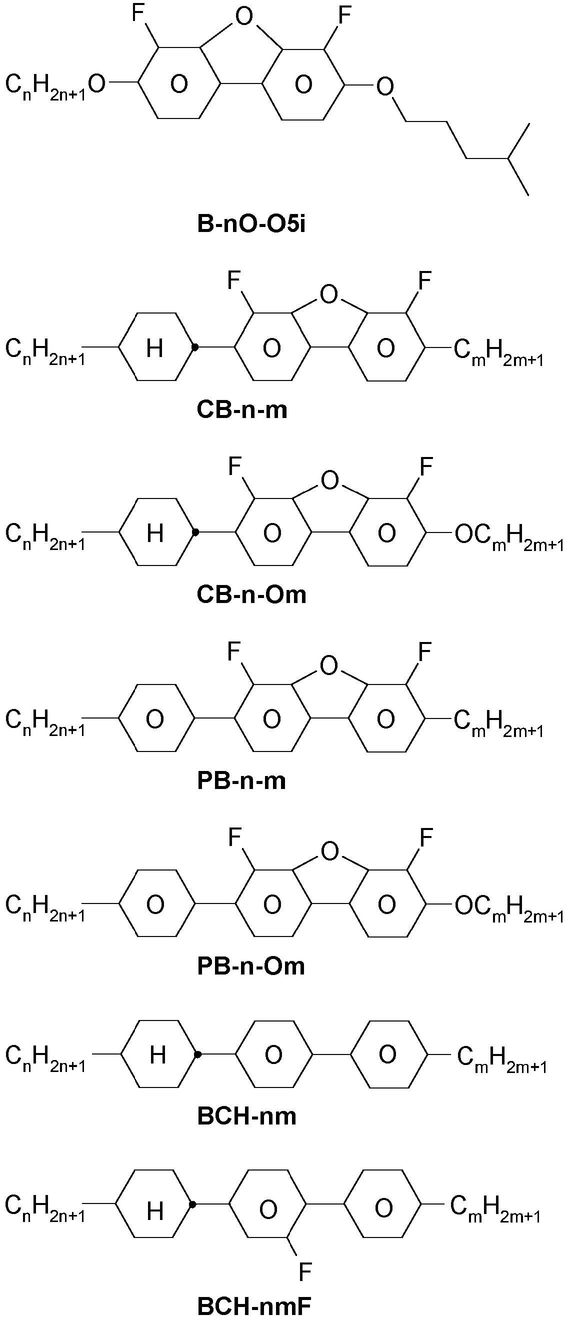

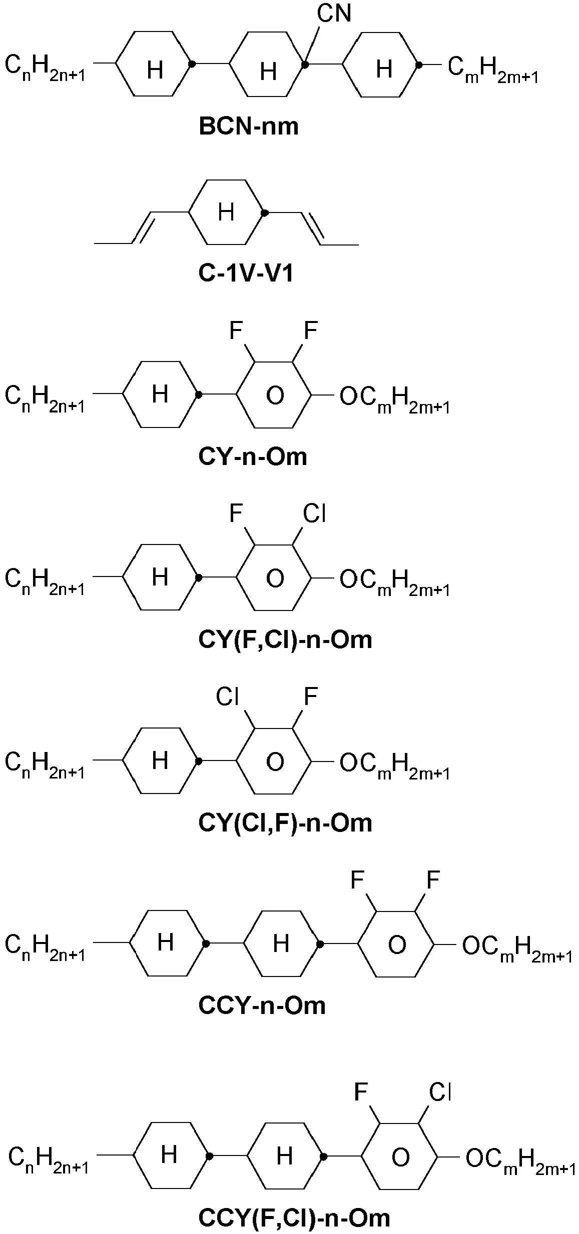

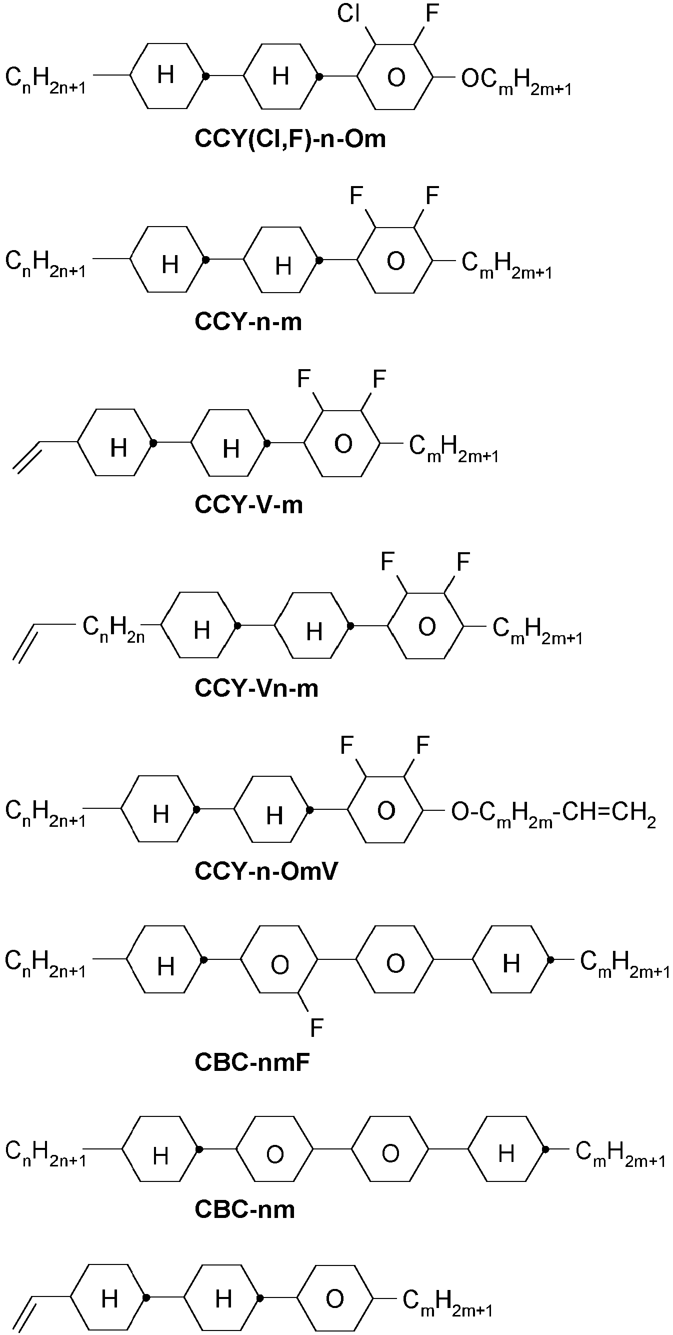

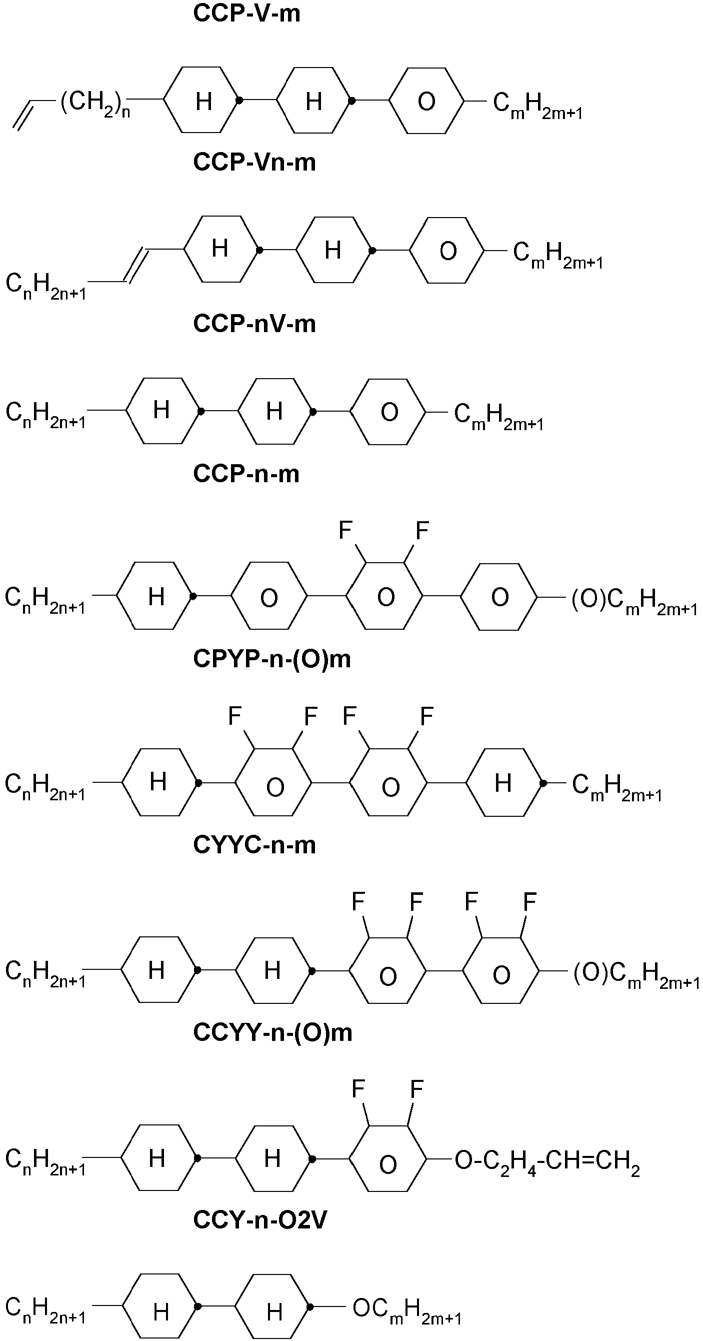

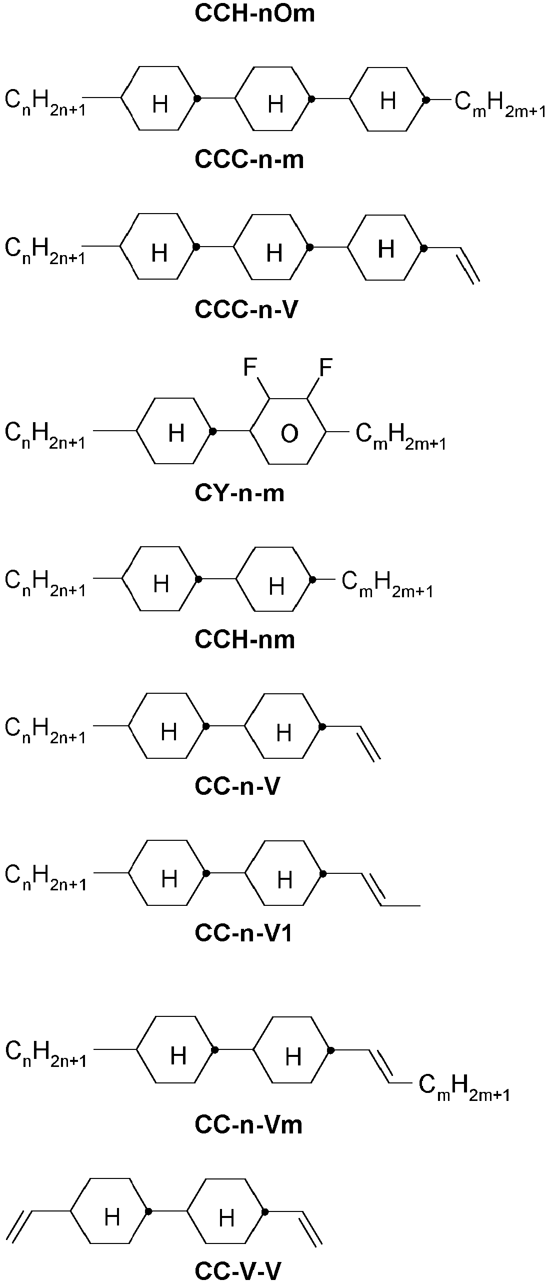

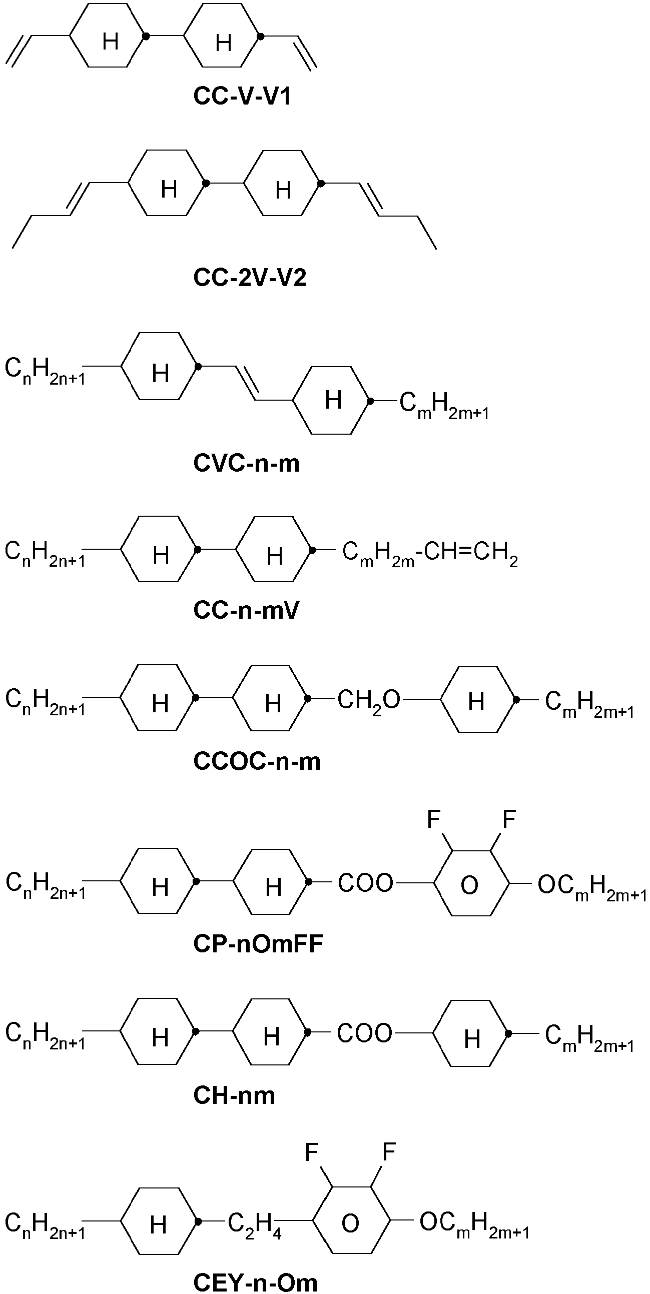

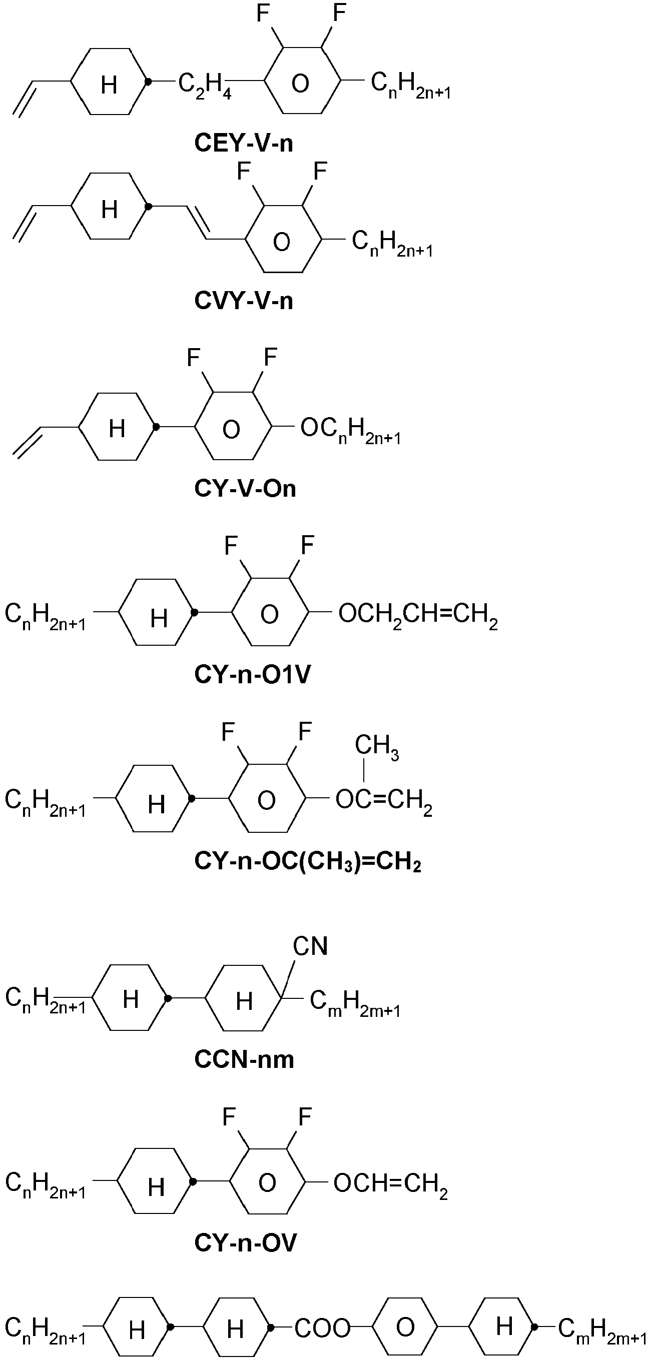

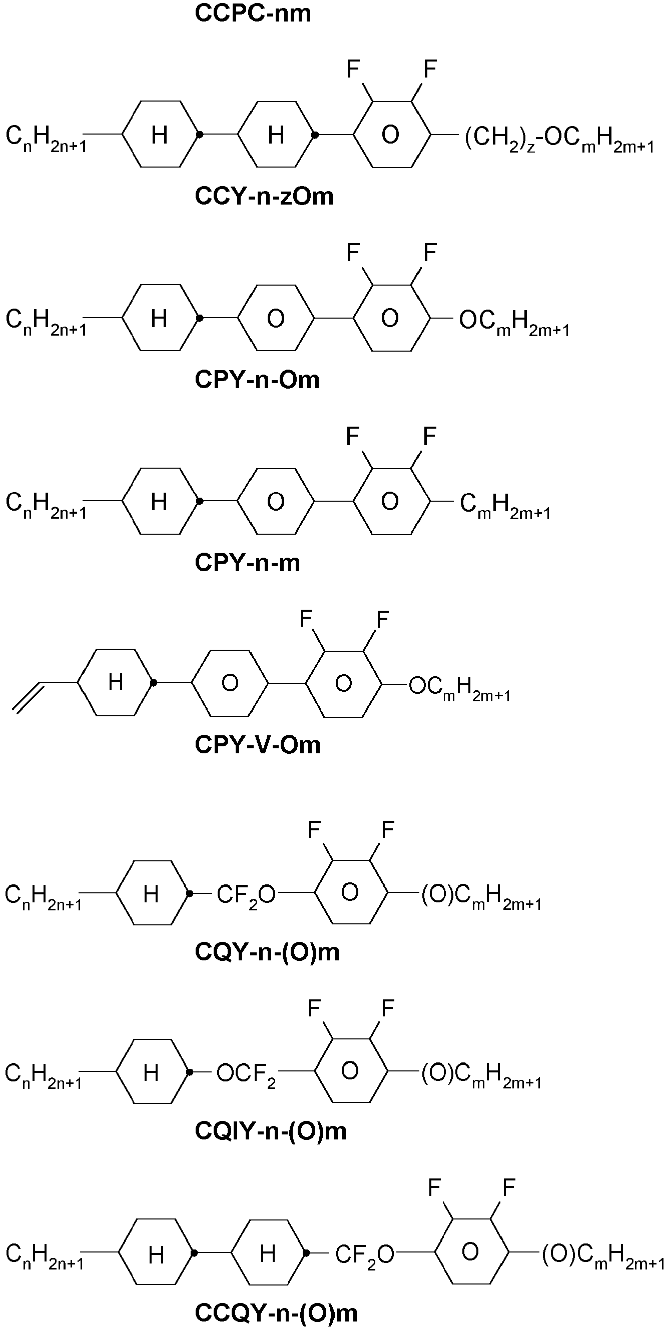

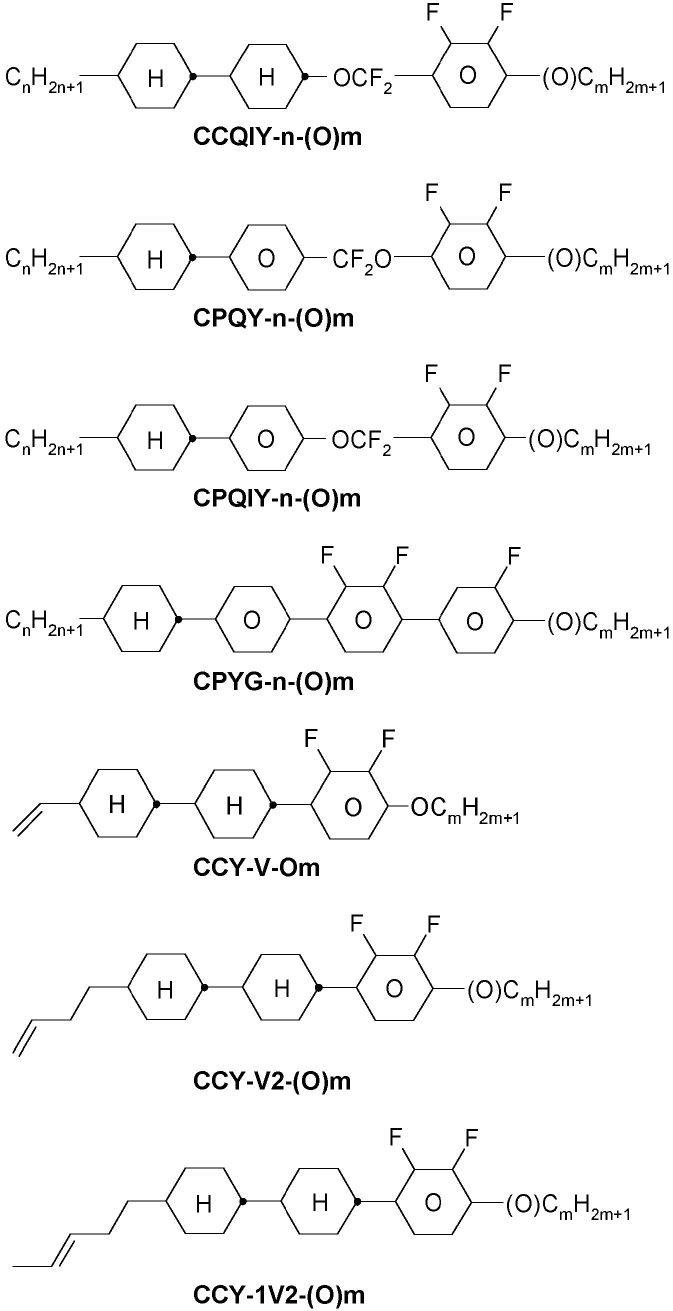

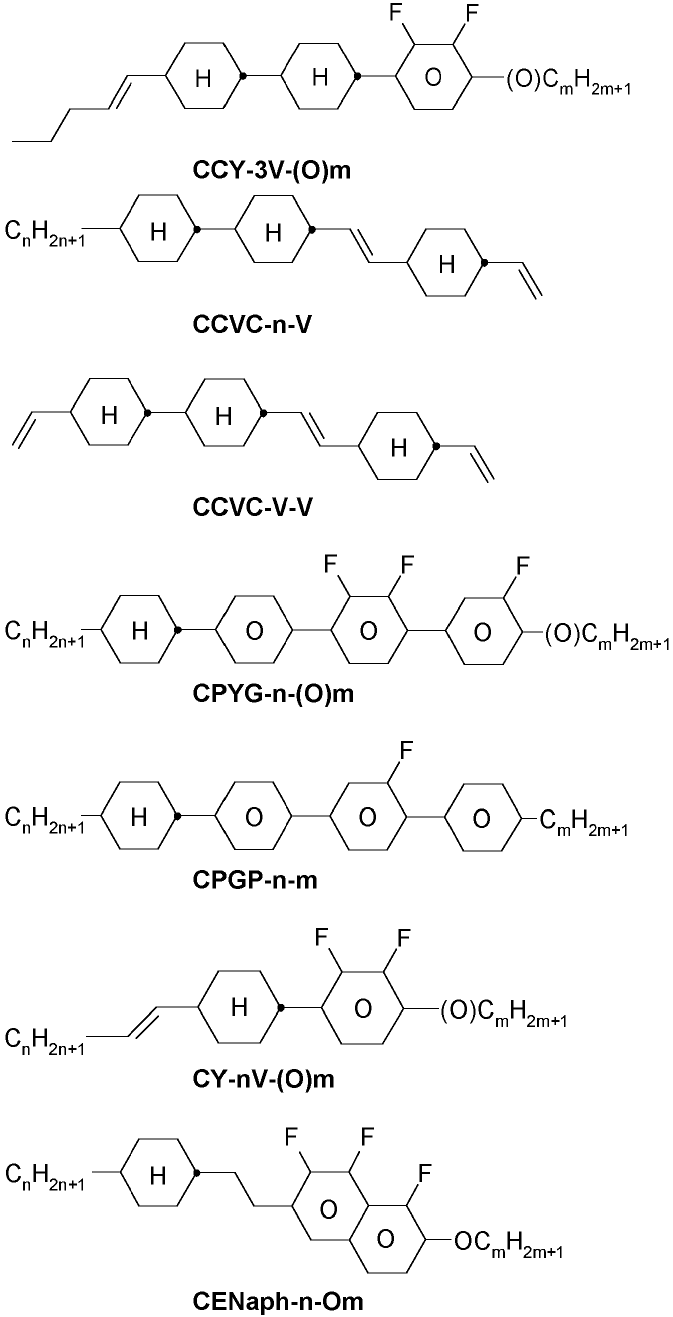

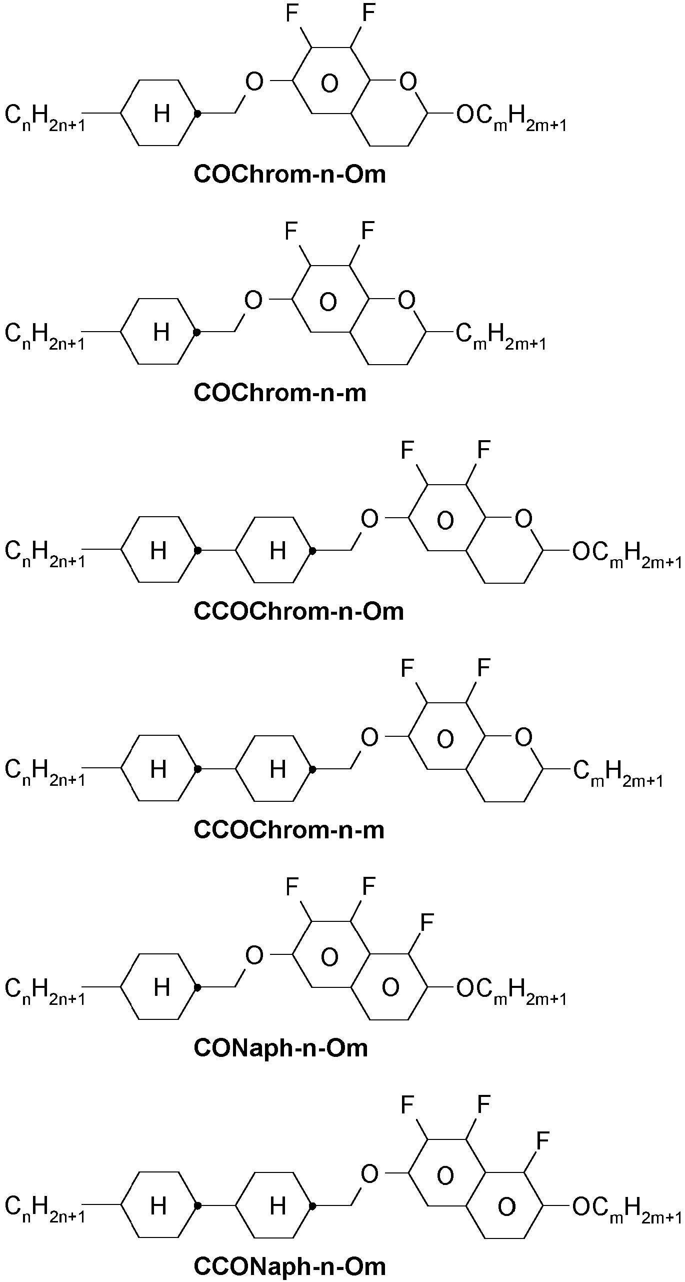

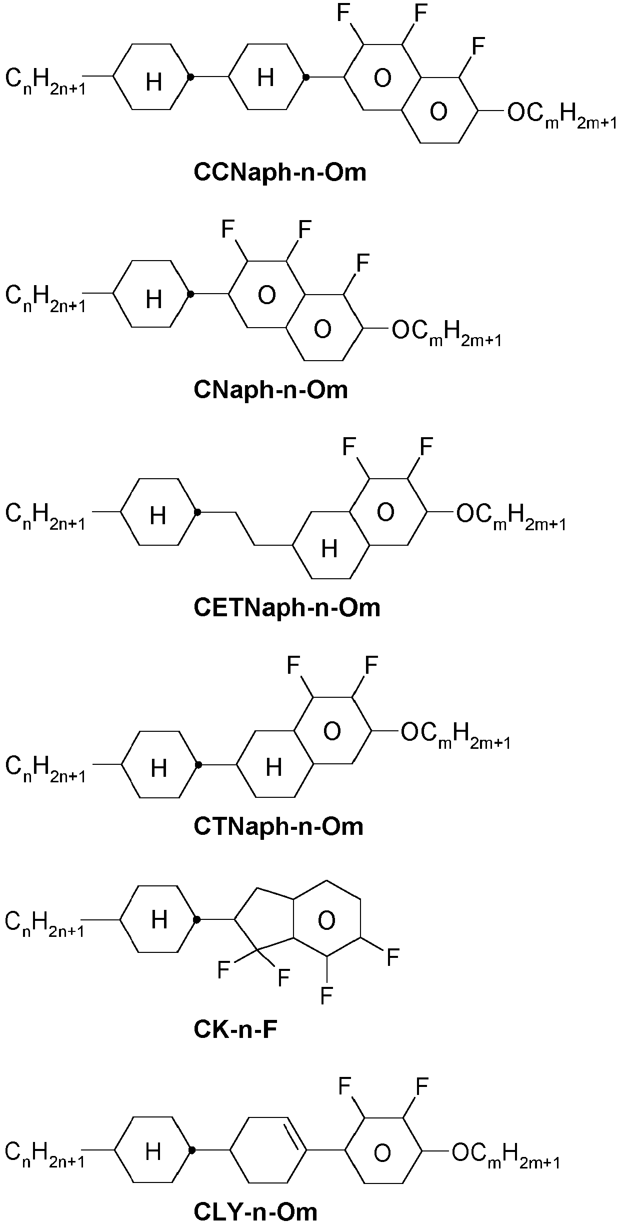

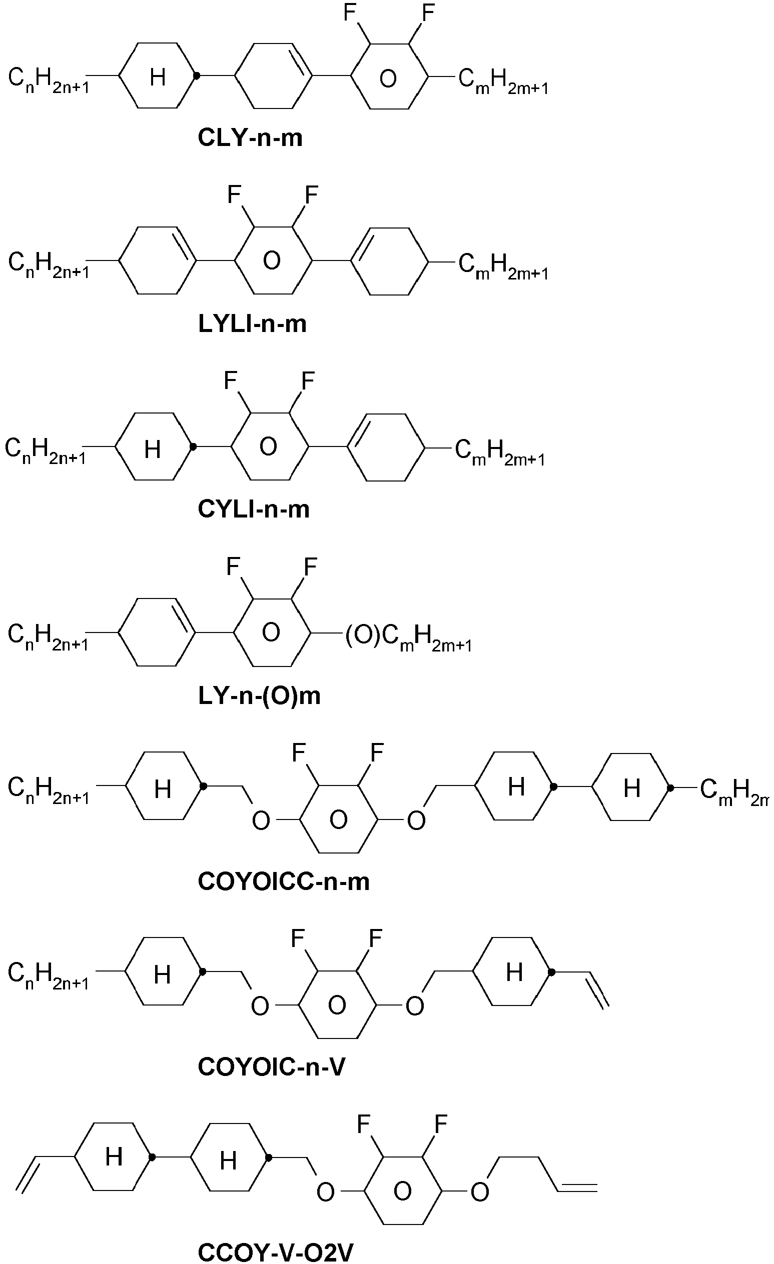

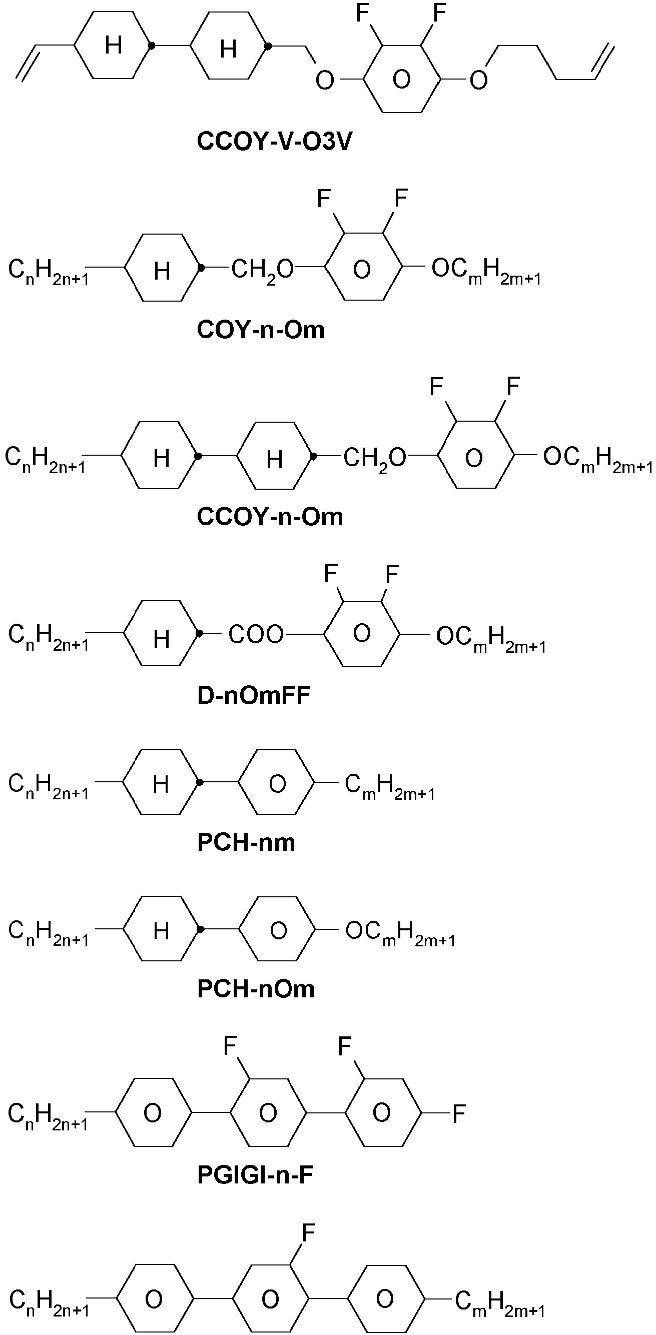

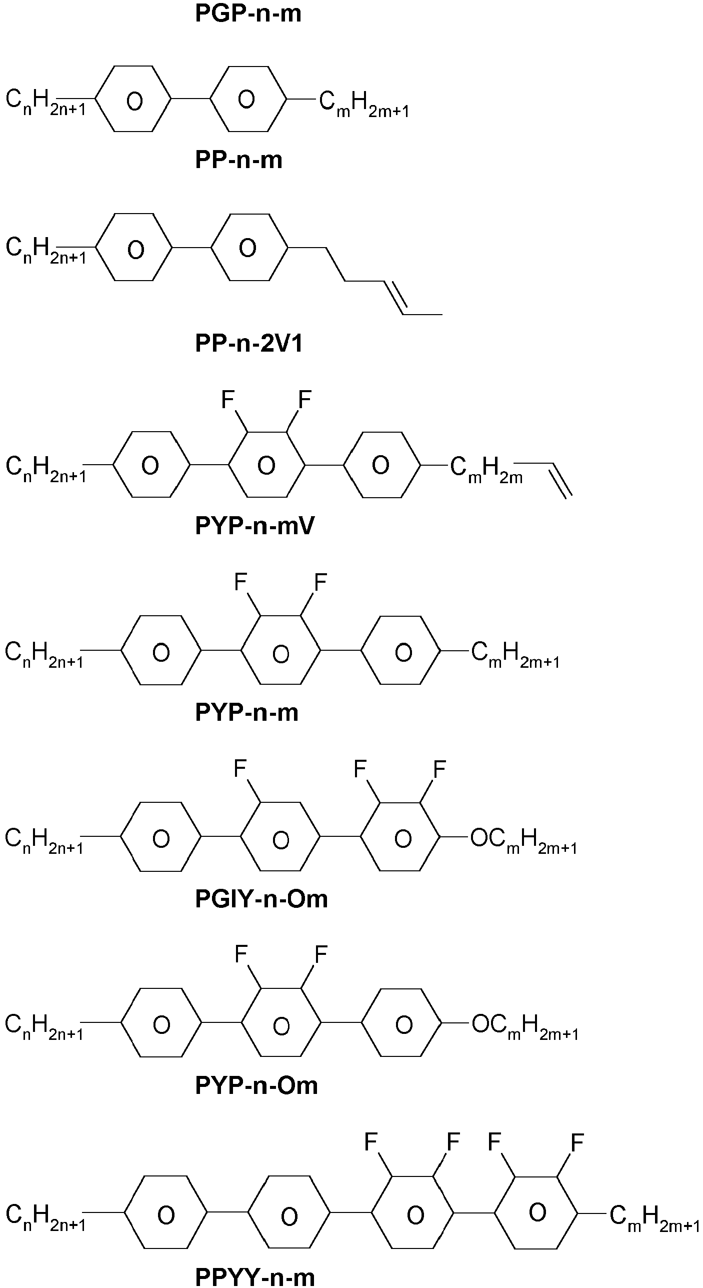

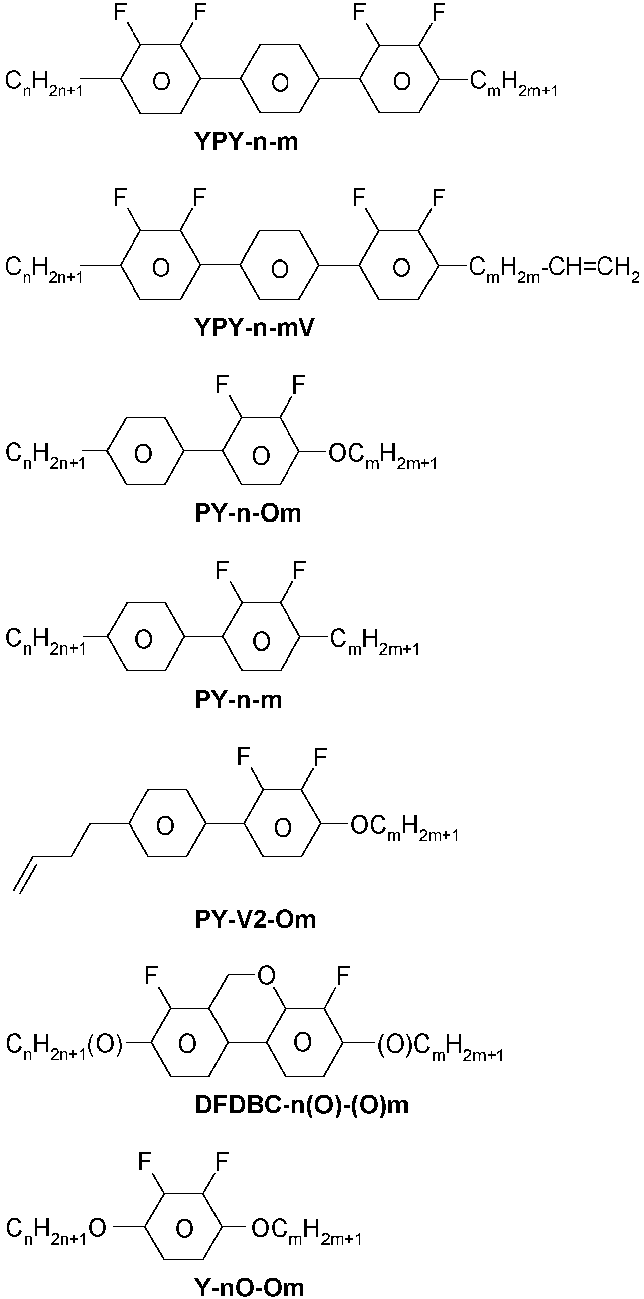

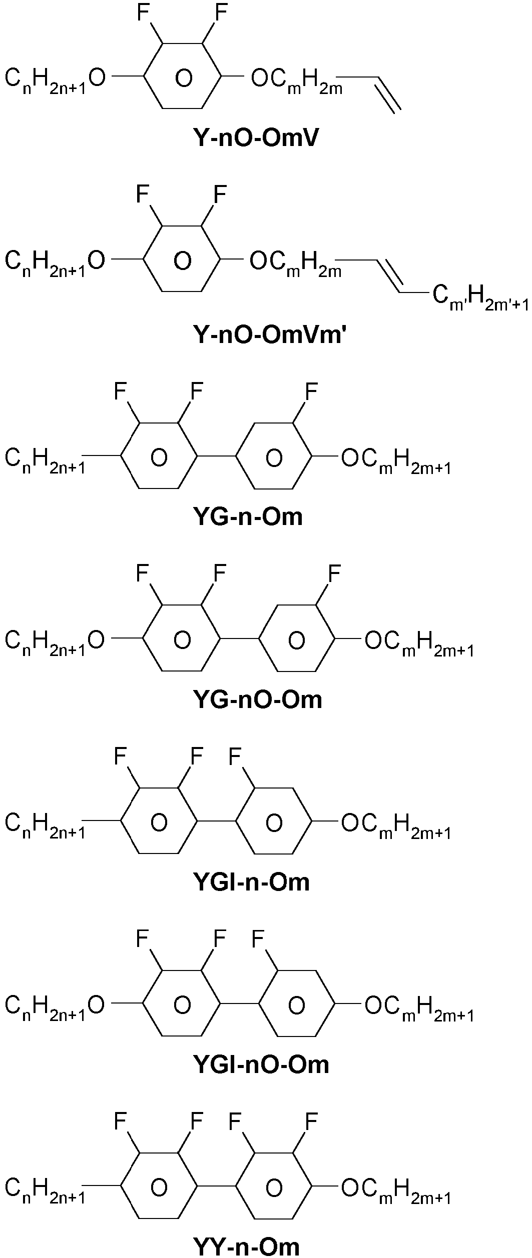

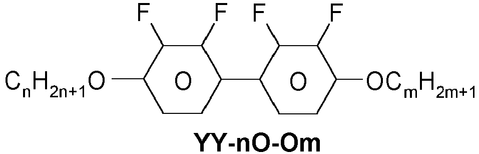

Die Herstellung der erfindungsgemäß verwendbaren Flüssigkristallmischungen erfolgt in an sich üblicher Weise. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich, Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation.

Mittels geeigneter Zusatzstoffe können die erfindungsgemäßen Flüssigkristallphasen derart modifiziert werden, dass sie in jeder bisher bekannt gewordenen Art von z. B. ECB-, VAN-, IPS-, GH- oder ASM-VA LCD-Anzeige einsetzbar sind.

Die Dielektrika können auch weitere, dem Fachmann bekannte und in der Literatur beschriebene Additive, wie z. B. UV-Absorber, Antioxidantien, Nanoteilchen, Radikalfänger, enthalten. Beispielsweise können 0-15 % pleochroitische Farbstoffe, Stabilisatoren oder chirale Dotierstoffe zugesetzt werden. Geeignete Stabilisatoren für die erfindungsgemäßen Mischungen sind insbesondere solche die in Tabelle B gelistet sind.

Beispielsweise können 0-15 % pleochroitische Farbstoffe zugesetzt werden, ferner Leitsalze, vorzugsweise Ethyldimethyldodecylammonium-4-hexoxybenzoat, Tetrabutylammoniumtetraphenylboranat oder Komplexsalze von Kronenethern (vgl. z.B. Haller et al., Mol. Cryst. Liq. Cryst. Band 24, Seiten 249- 258 (1973)) zur Verbesserung der Leitfähigkeit oder Substanzen zur Veränderung der dielektrischen Anisotropie, der Viskosität und/oder der Orientierung der nematischen Phasen. Derartige Substanzen sind z. B. in den DE-OS 22 09 127, 22 40 864, 23 21 632, 23 38 281, 24 50 088, 26 37 430 und 28 53 728 beschrieben.

In der Tabelle B werden mögliche Dotierstoffe angegeben, die den erfindungsgemäßen Mischungen zugesetzt werden können. Sofern die Mischungen einen Dotierstoff enthalten, wird er in Mengen von 0,01-4 Gew.%, vorzugsweise 0,1-1,0 Gew.%, eingesetzt.

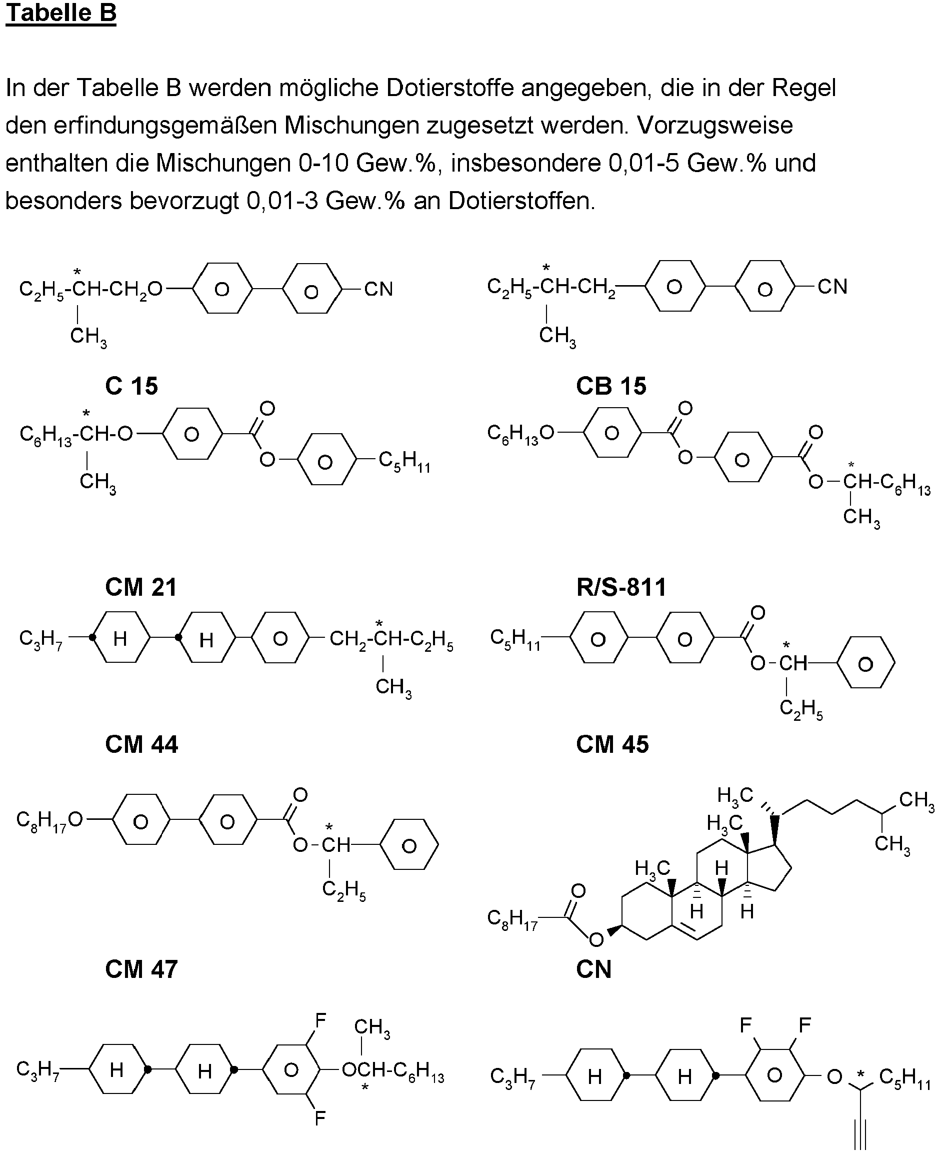

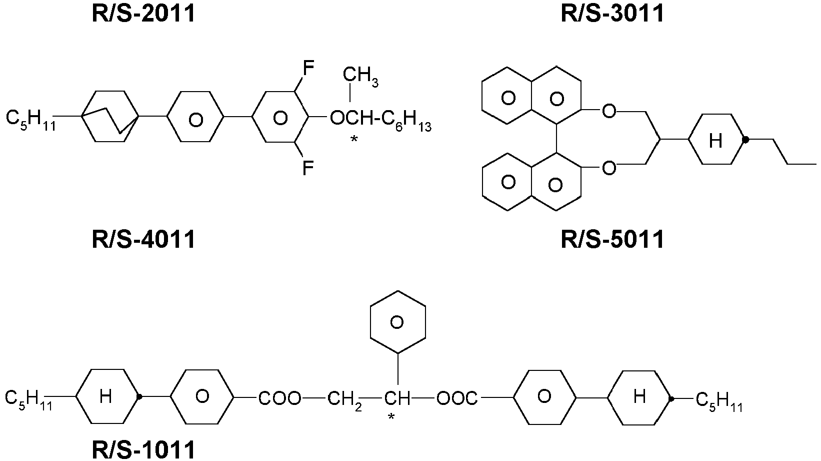

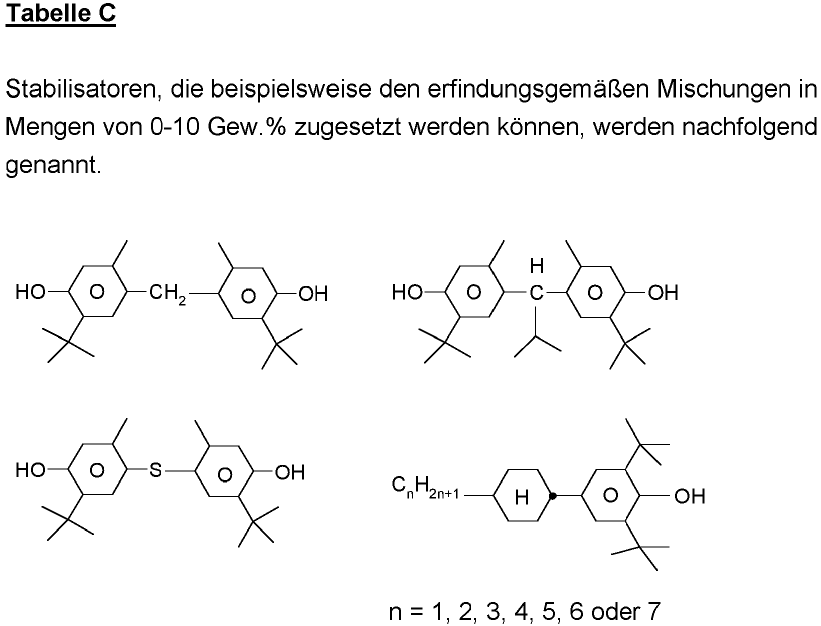

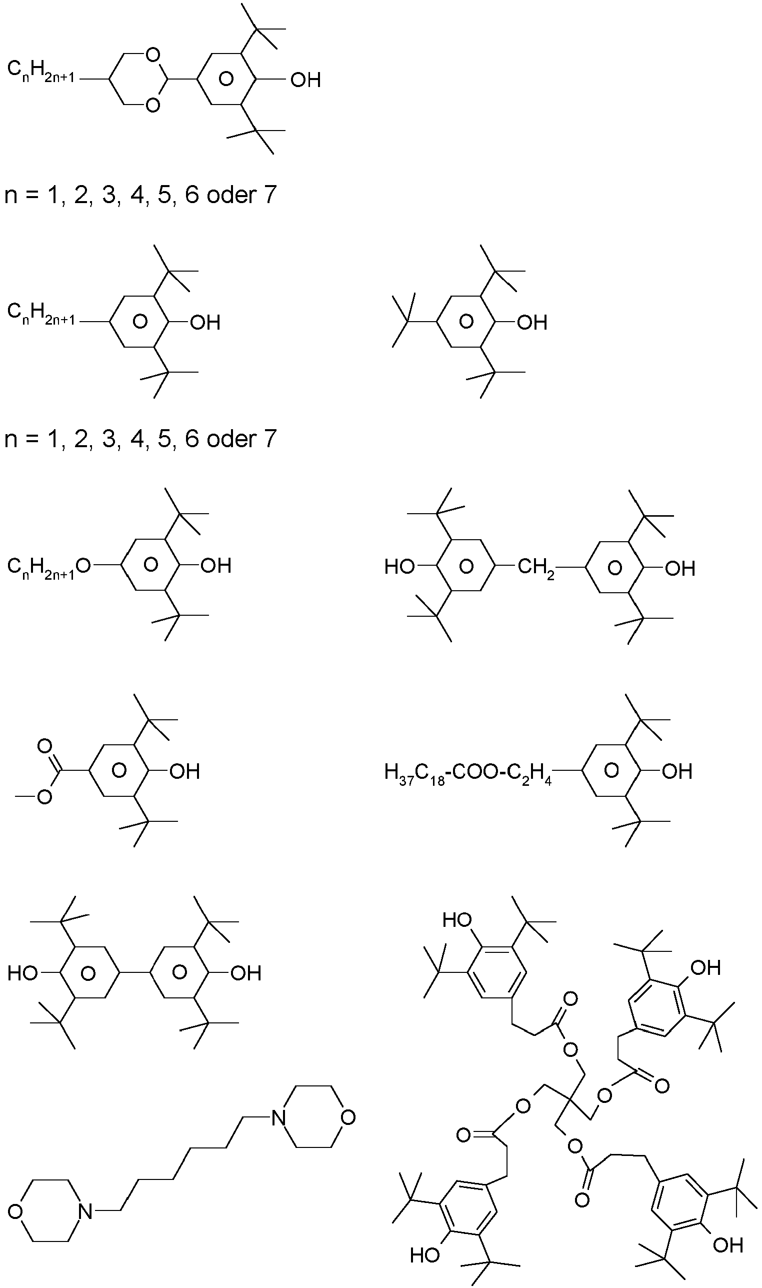

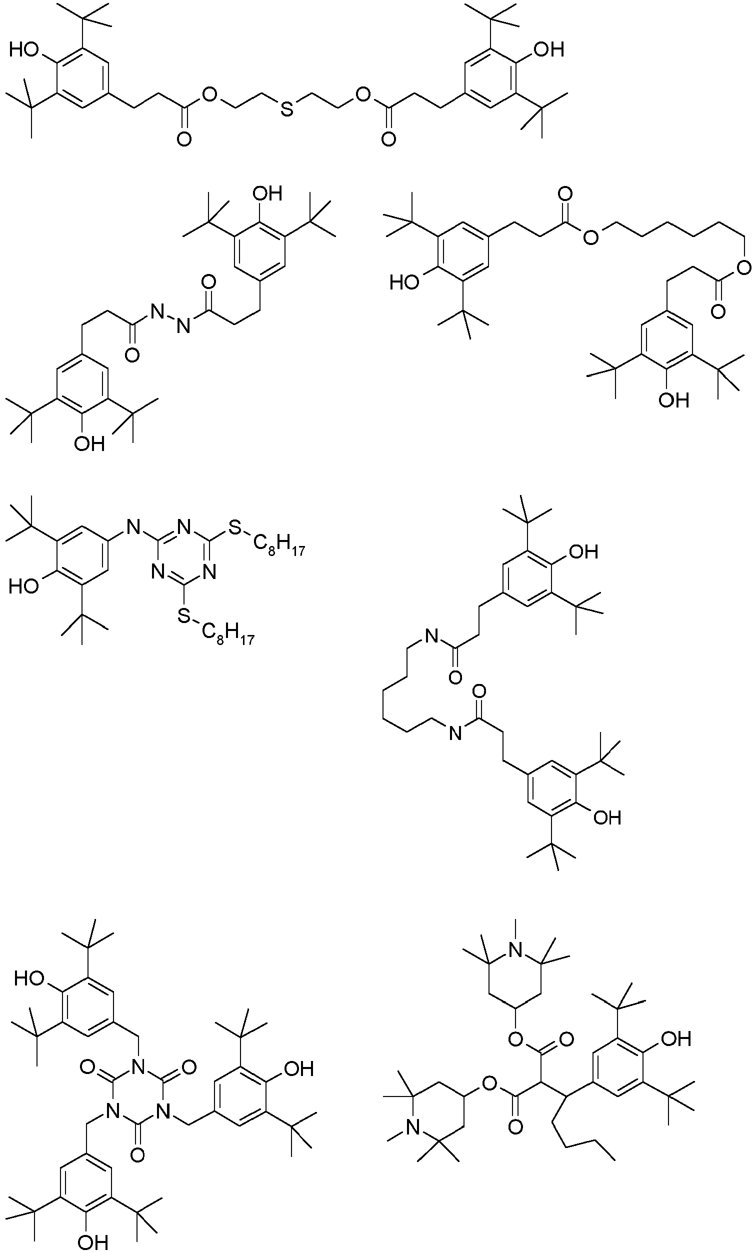

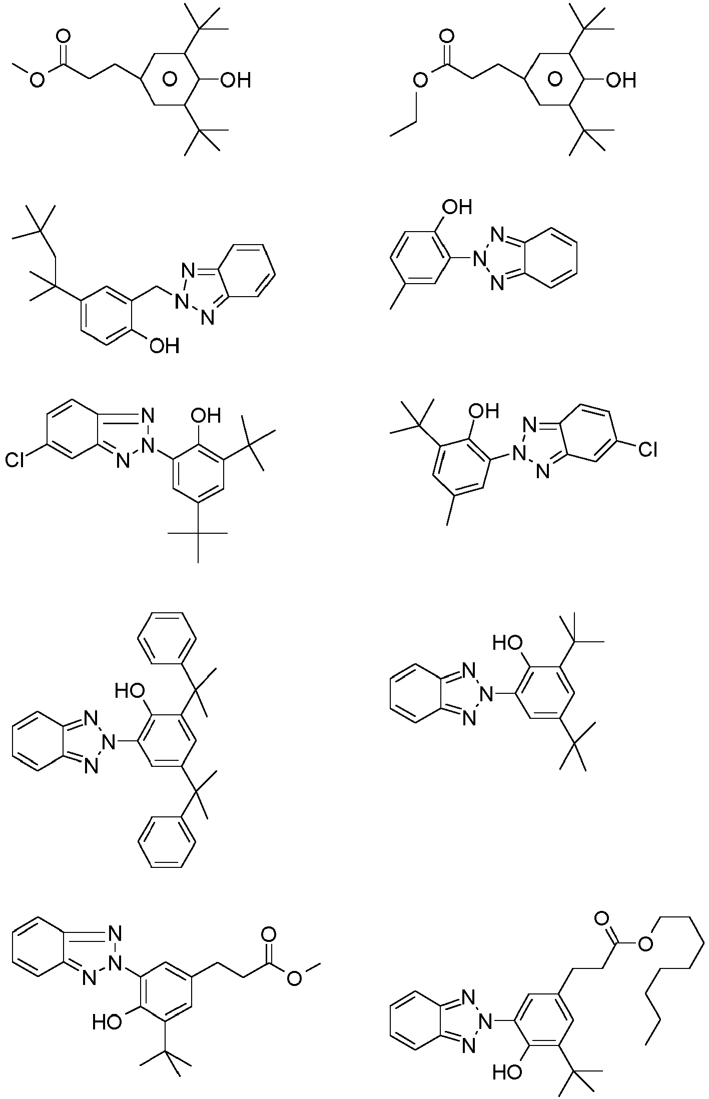

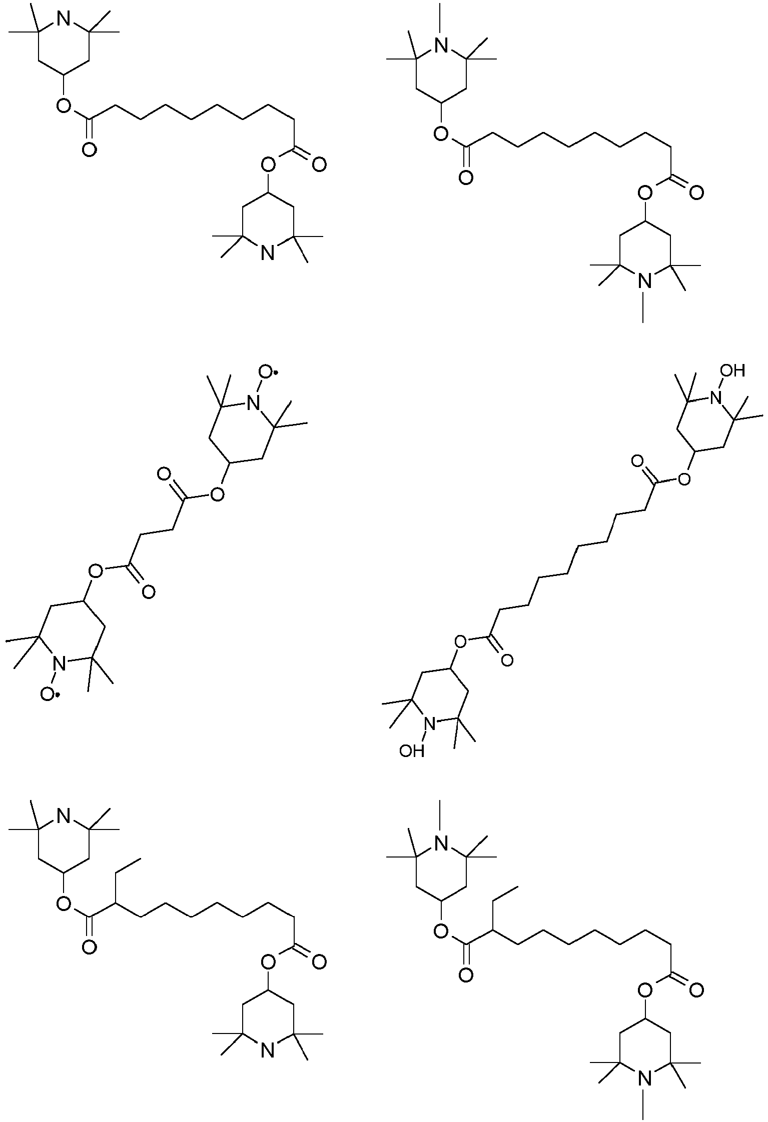

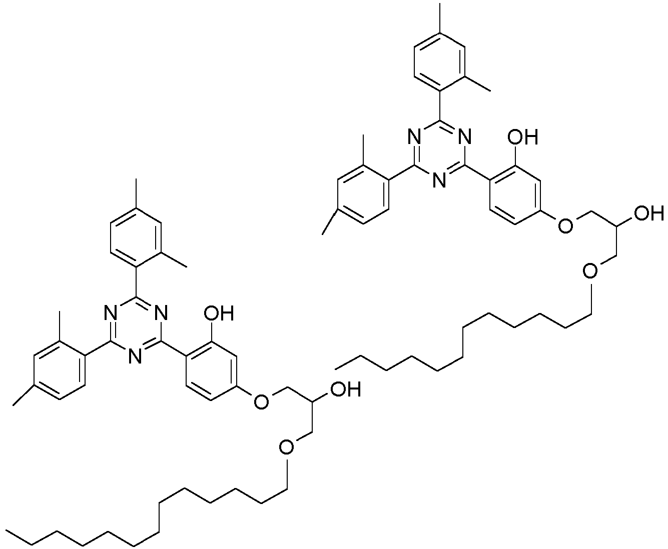

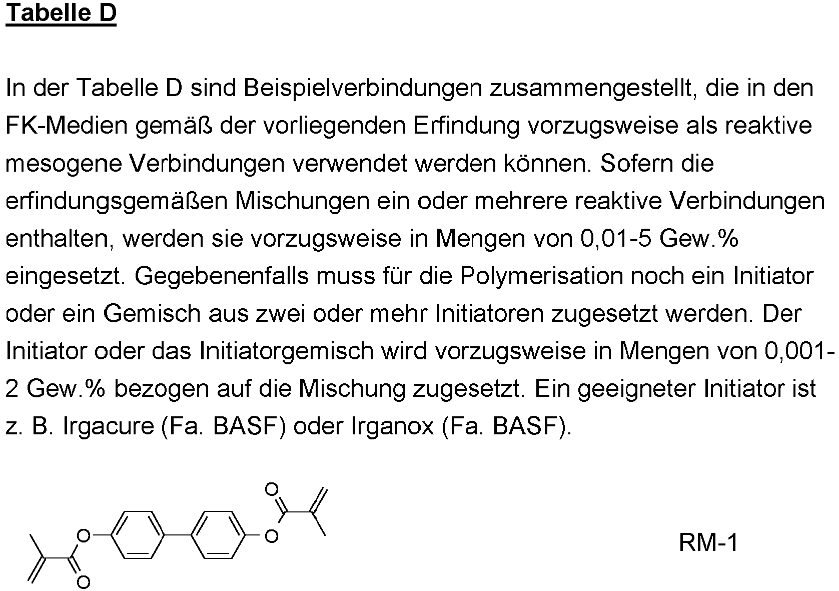

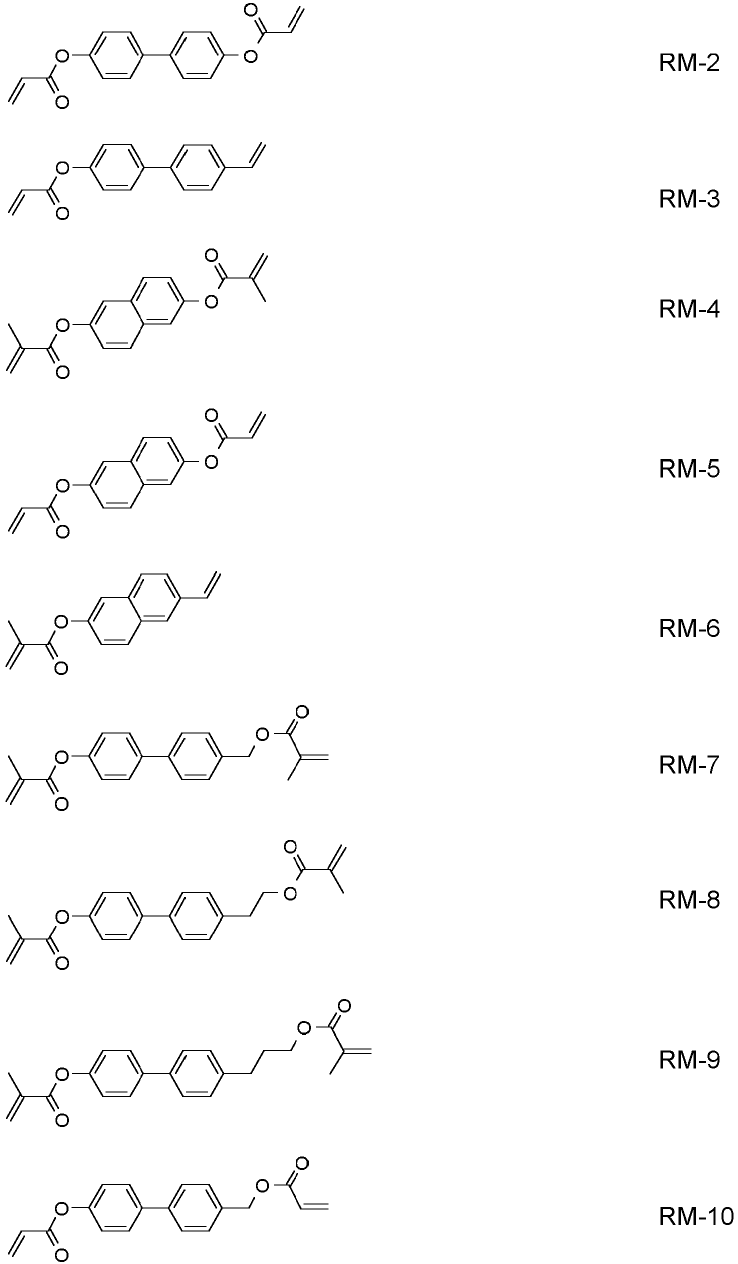

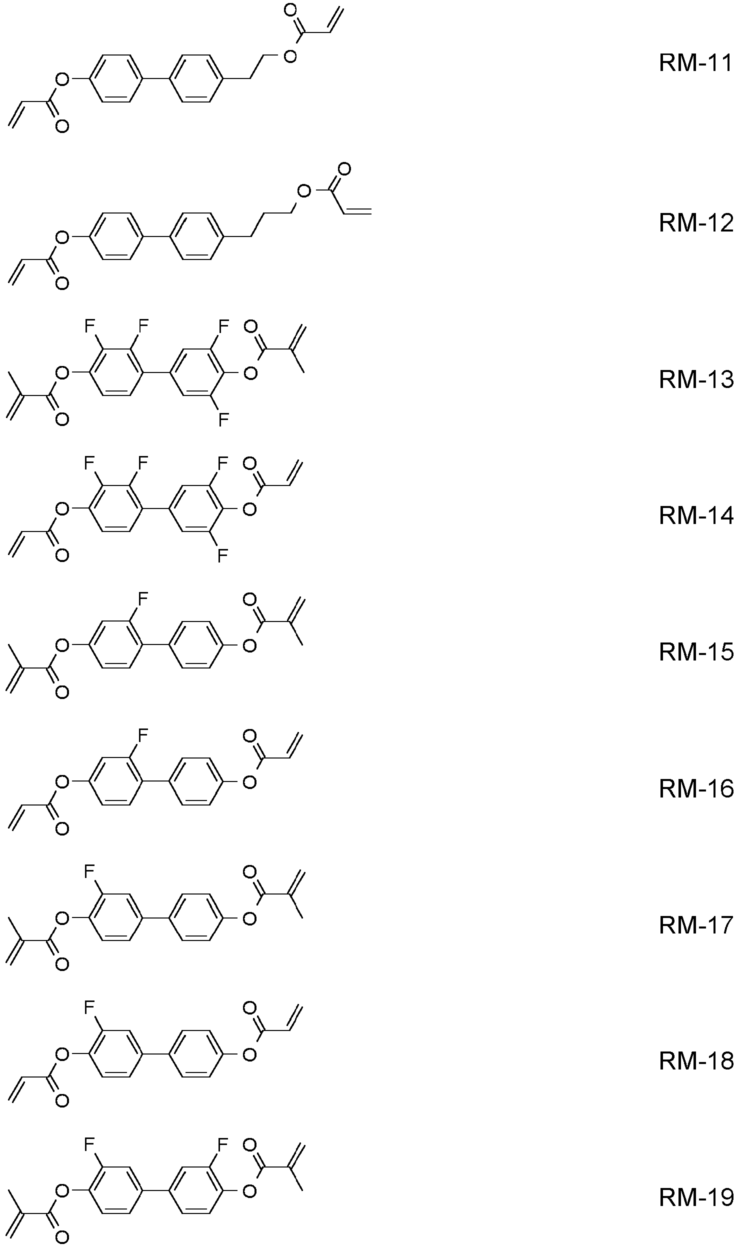

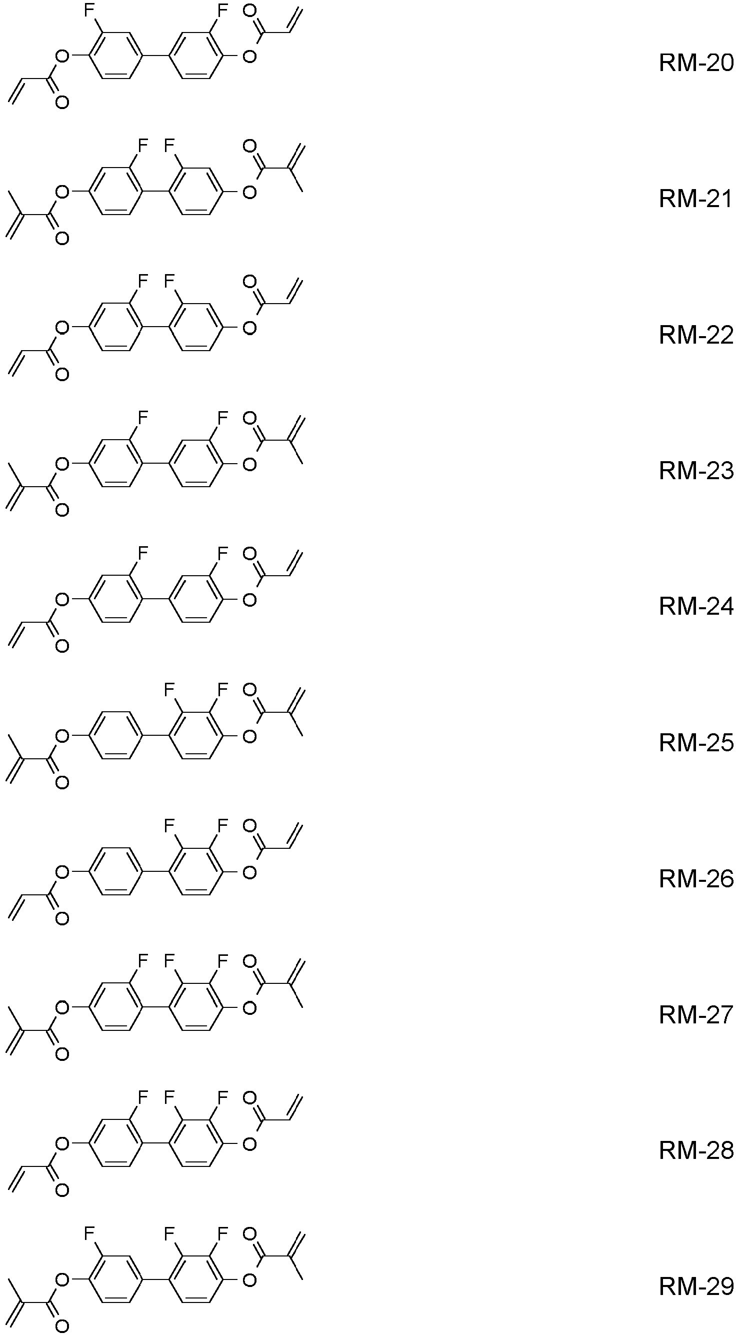

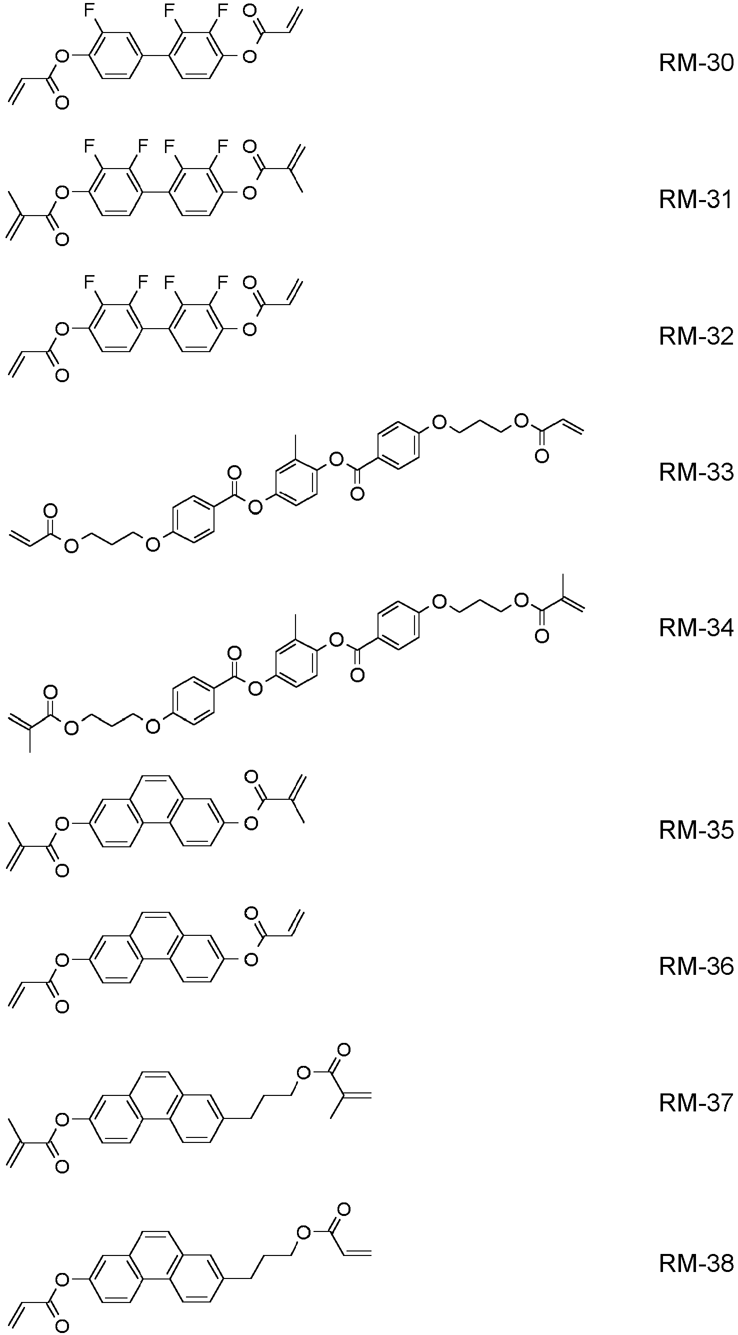

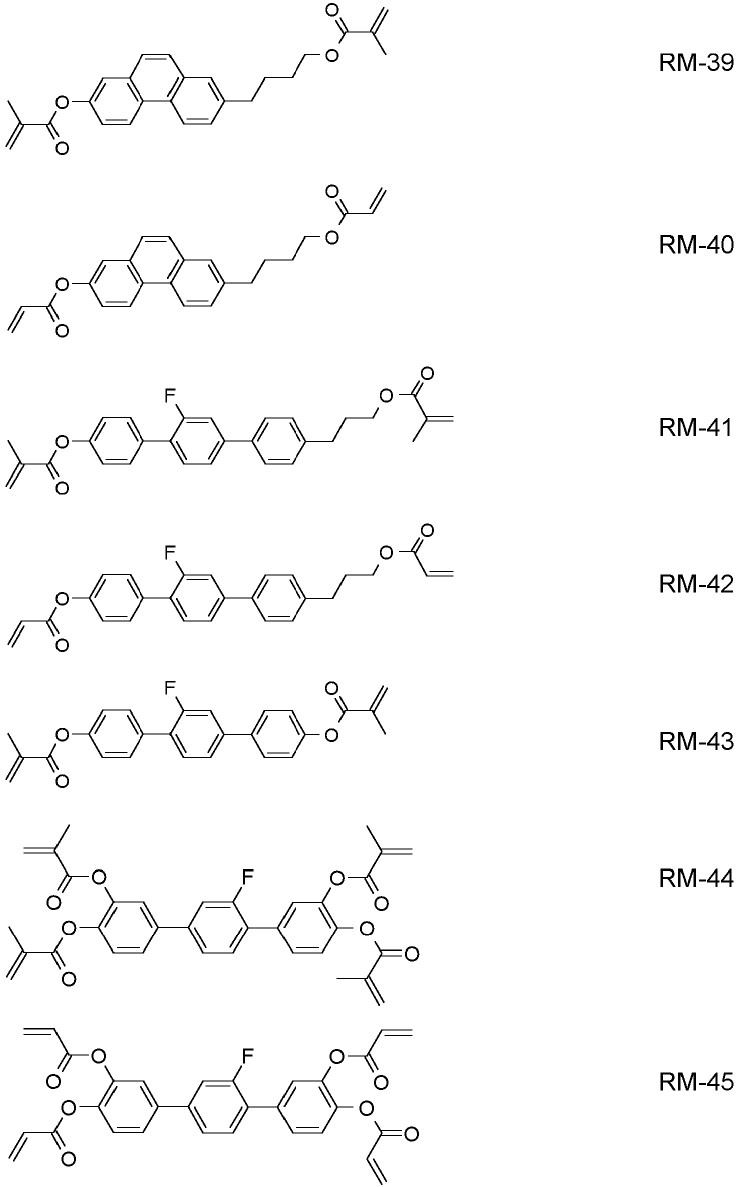

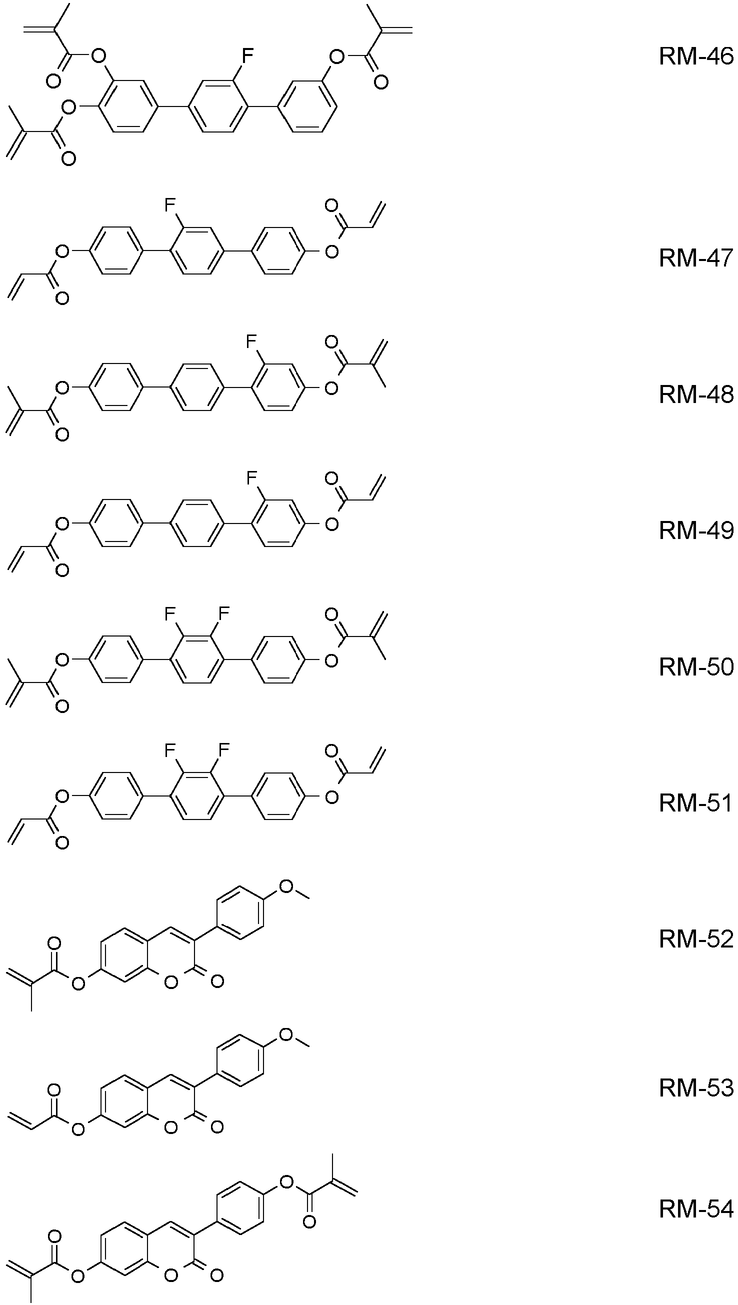

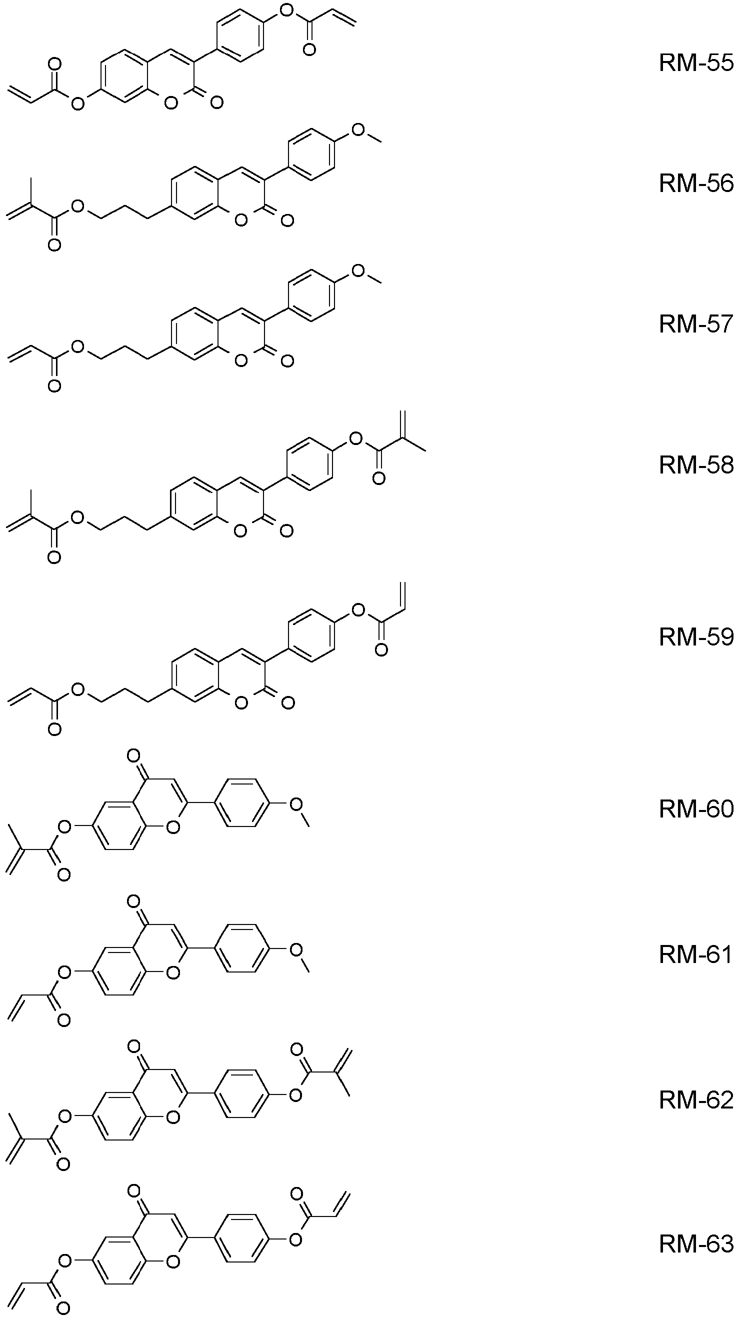

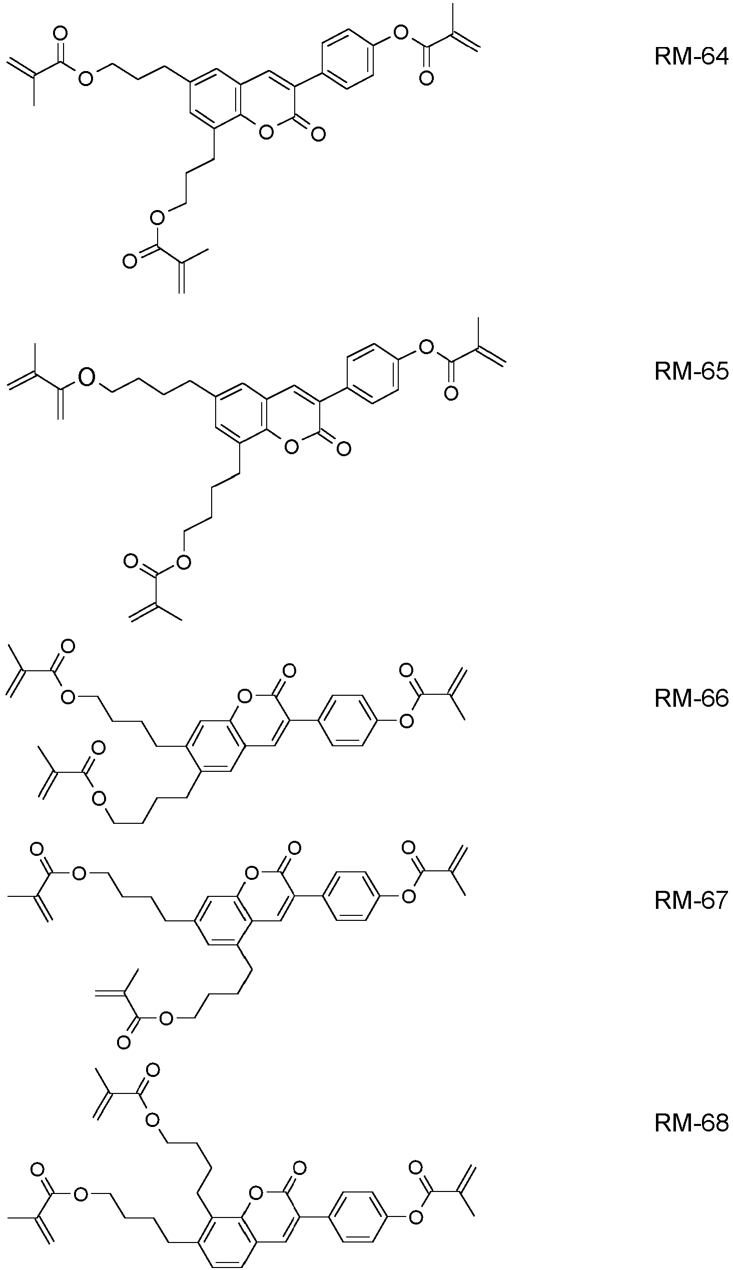

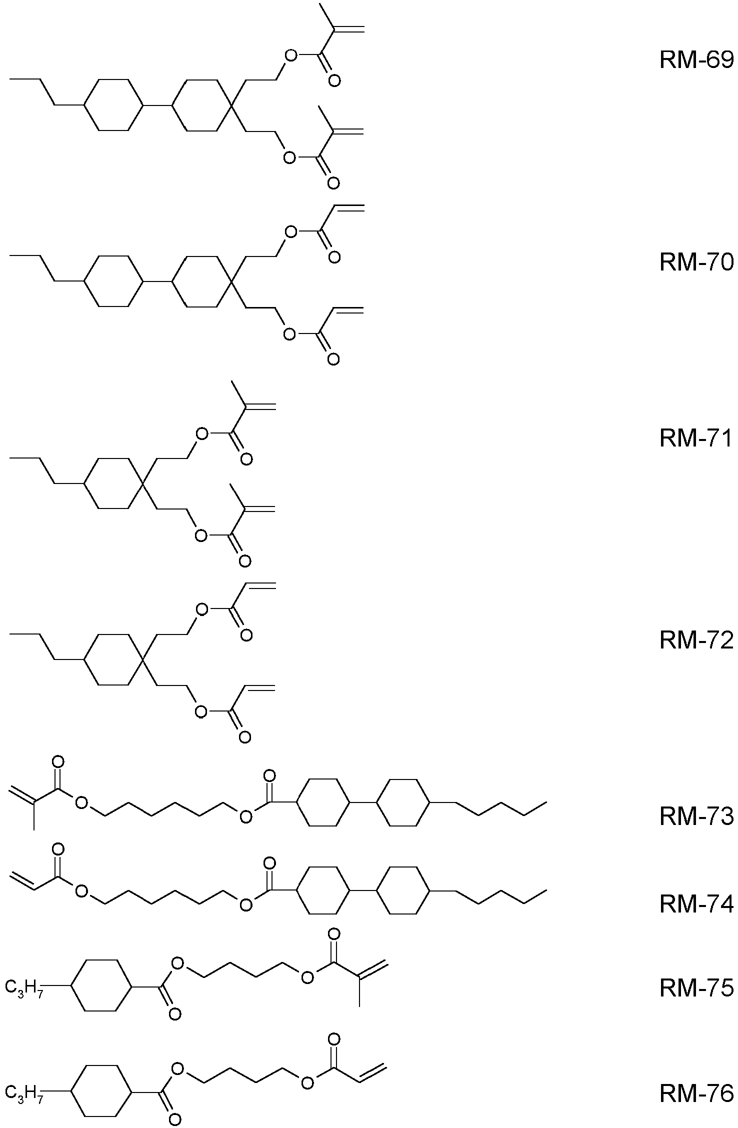

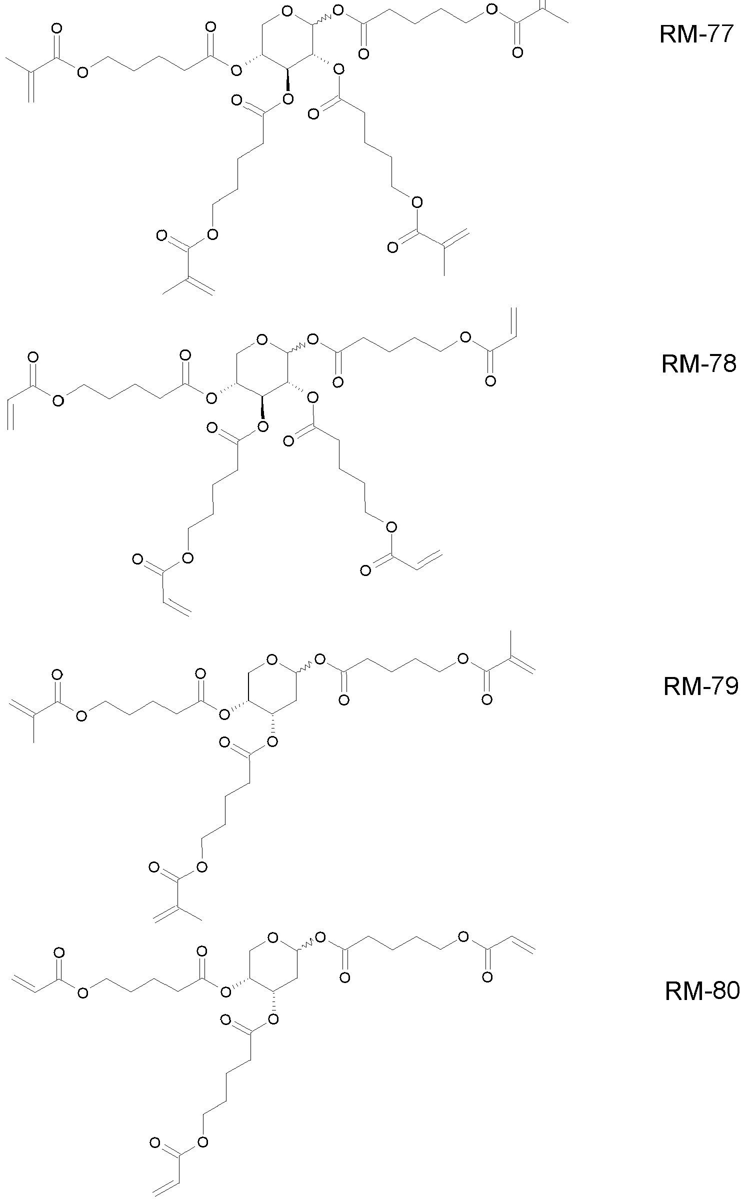

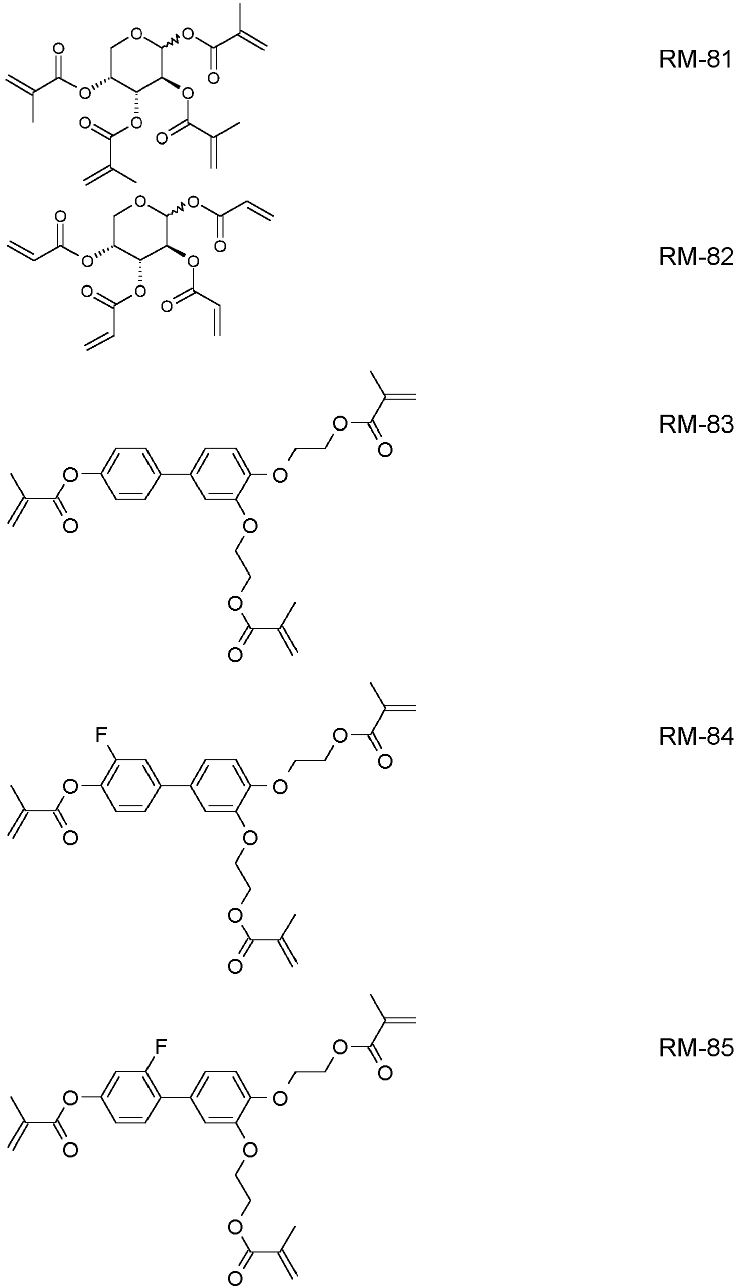

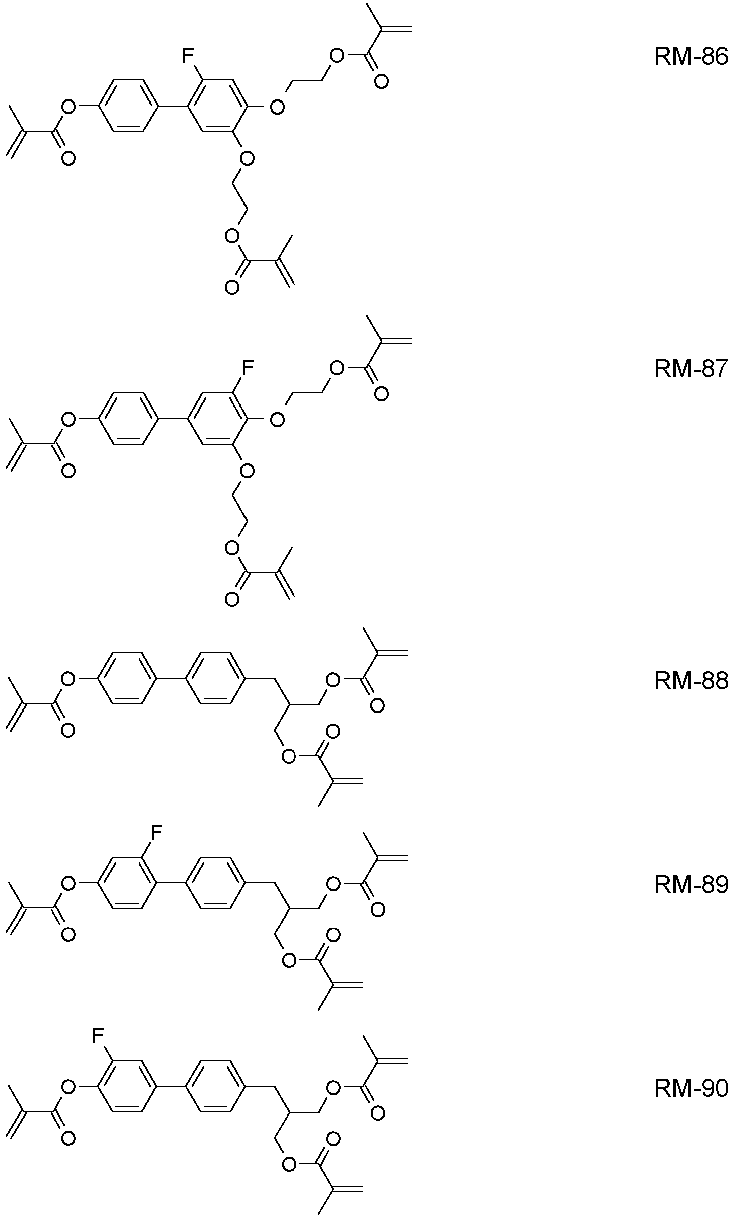

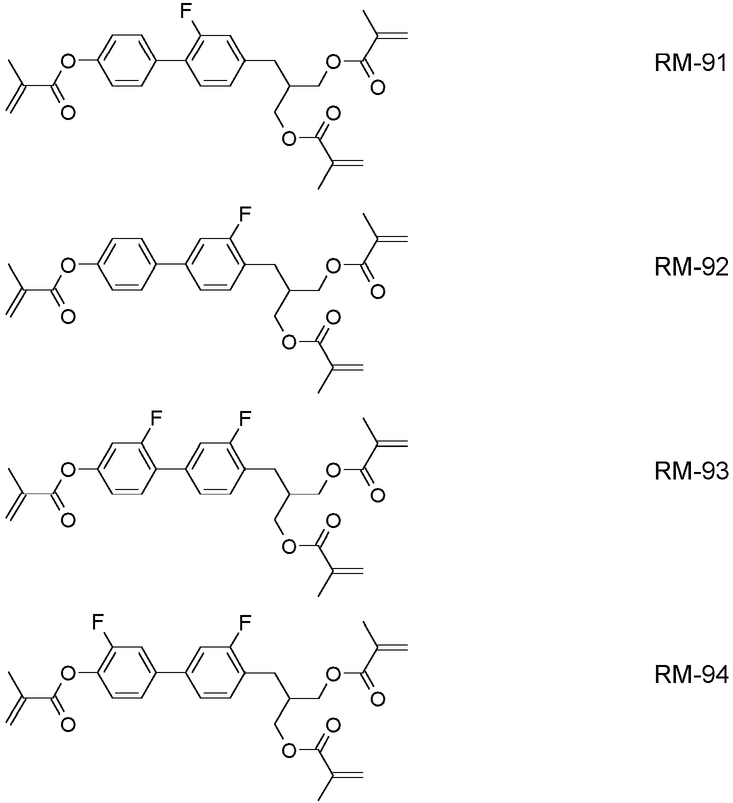

In einer bevorzugten Ausführungsform enthalten die erfindungsgemäßen Mischungen eine oder mehrere polymerisierbare Verbindungen, vorzugsweise ausgewählt aus den polymerisierbaren Verbindungen der Formeln RM-1 bis RM-94. Derartige Medien sind insbesondere für PS-FFS- und PS-IPS-Anwendungen geeignet. Von den in der Tabelle D genannten reaktiven Mesogenen sind die Verbindungen RM-1, RM-2, RM-3, RM-4, RM-5, RM-11, RM-17, RM-35, RM-41, RM-44, RM-62 und RM-81 insbesondere bevorzugt.

### Ausführungsbeispiele:

Die folgenden Beispiele sollen die Erfindung erläutern, ohne sie zu begrenzen. In den Beispielen bedeuten F. den Schmelzpunkt und K den Klärpunkt einer flüssigkristallinen Substanz in Grad Celsius; Siedetemperaturen sind mit Kp. bezeichnet. Es bedeuten ferner:
K: kristallin-fester Zustand, S: smektische Phase (der Index bezeichnet den Phasentyp), N: nematischer Zustand, Ch: cholesterische Phase, I: isotrope Phase, T_{g}: Glastemperatur. Die zwischen zwei Symbolen stehende Zahl gibt die Umwandlungstemperatur in Grad Celsius an.

Als Hostmischung zur Bestimmung der optischen Anisotropie Δn der Verbindungen der Formel I wird die Verkaufsmischung ZLI-4792 (Fa. Merck KGaA) verwendet. Für die Bestimmung der dielektrischen Anisotropie Δε wird die Verkaufsmischung ZLI-2857 verwendet. Aus der Änderung der Dielektrizitätskonstanten der Hostmischung nach Zugabe der zu untersuchenden Verbindung und Extrapolation auf 100 % der eingesetzten Verbindung werden die physikalischen Daten der zu untersuchenden Verbindung erhalten. Die zu untersuchende Verbindung wird in Abhängigkeit der Löslichkeit in der Regel zu 10 % in der Hostmischung gelöst.

Sofern nichts anderes angegeben ist, bedeuten Angaben von Teilen oder Prozent Gewichtsteile bzw. Gewichtsprozent.

Vor- und nachstehend bedeuten:
- Vₒ: Schwellenspannung, kapazitiv [V] bei 20°C,
- nₑ: außerordentlicher Brechungsindex bei 20°C und 589 nm,
- nₒ: ordentlicher Brechungsindex bei 20°C und 589 nm,
- Δn: optische Anisotropie bei 20°C und 589 nm,
- ε⊥: dielektrische Permittivität senkrecht zum Direktor bei 20°C und 1 kHz,
- εll: dielektrische Permittivität parallel zum Direktor bei 20°C und 1 kHz,
- Δε: dielektrische Anisotropie bei 20°C und 1 kHz,
- Kp., T(N,I): Klärpunkt [°C],
- γ₁: Rotationsviskosität gemessen bei 20 °C [mPa·s], bestimmt nach dem Rotationsverfahren in einem magnetischen Feld
- K₁: elastische Konstante, "splay"-Deformation bei 20°C [pN],
- K₂: elastische Konstante, "twist"-Deformation bei 20°C [pN],
- K₃: elastische Konstante, "bend"-Deformation bei 20°C [pN].
- LTS: Tieftemperaturstabilität [Low temperature stability (nematische Phase)], bestimmt in Testzellen.

Soweit nicht explizit anders vermerkt, sind in der vorliegenden Anmeldung alle angegebenen Werte für Temperaturen, wie z. B. der Schmelzpunkt T(C,N), der Übergang von der smektischen (S) zur nematischen (N) Phase T(S,N) und der Klärpunkt T(N,I), in Grad Celsius (°C) angegeben. Fp. bedeutet Schmelzpunkt, Kp. = Klärpunkt. Ferner bedeuten Tg = Glaszustand, K = kristalliner Zustand, N = nematische Phase, S = smektische Phase und I = isotrope Phase. Die Angaben zwischen diesen Symbolen stellen die Übergangstemperaturen dar.

Alle physikalischen Eigenschaften werden und wurden nach "Merck Liquid Crystals, Physical Properties of Liquid Crystals", Status Nov. 1997, Merck KGaA, Deutschland bestimmt und gelten für eine Temperatur von 20°C und Δn wird bei 589 nm und Δε bei 1 kHz bestimmt, sofern nicht jeweils explizit anders angegeben.

Der Begriff "Schwellenspannung" bezieht sich für die vorliegende Erfindung auf die kapazitive Schwelle (V₀), auch Freedericksz-Schwelle genannt, sofern nicht explizit anders angegeben. In den Beispielen kann auch, wie allgemein üblich, die optische Schwelle für 10 % relativen Kontrast (V₁₀) angegeben werden.

Die zur Messung der kapazitiven Schwellenspannung verwendete Anzeige besteht aus zwei planparallelen Glasträgerplatten im Abstand von 20 µm, welche auf den Innenseiten jeweils ein Elektrodenschicht sowie eine darüberliegende, ungeriebene Orientierungsschicht aus Polyimid aufweisen, die eine homöotrope Randorientierung der Flüssigkristallmoleküle bewirken.

Die zur Messung der Tiltwinkel verwendete Anzeige bzw. Testzelle besteht aus zwei planparallelen Glasträgerplatten im Abstand von 4 µm, welche auf den Innenseiten jeweils eine Elektrodenschicht sowie eine darüberliegende Orientierungsschicht aus Polyimid aufweisen, wobei die beiden Polyimidschichten antiparallel zueinander gerieben werden und eine homöotrope Randorientierung der Flüssigkristallmoleküle bewirken.

Die polymerisierbaren Verbindungen werden in der Anzeige bzw. Testzelle durch Bestrahlung mit UVA-Licht (üblicherweise 365nm) einer definierten Intensität für eine vorgegebene Zeit polymerisiert, wobei gleichzeitig eine Spannung an die Anzeige angelegt wird (üblicherweise 10V bis 30V Wechselstrom, 1 kHz). In den Beispielen wird, falls nicht anders angegeben, eine Quecksilberdampflampe mit 50 mW/cm² verwendet, die Intensität wird mit einem Standard-UV-Meter (Fabrikat Ushio UNI meter) gemessen, der mit einem Bandpassfilter bei 365nm ausgerüstet ist.

Der Tiltwinkel wird per Drehkristall-Experiment (Autronic-Melchers TBA-105) bestimmt. Ein niedriger Wert (d.h. eine große Abweichung vom 90°-Winkel) entspricht dabei einem großen Tilt.

Der VHR -Wert wird wie folgt gemessen: Zur FK-Host-Mischung werden 0.3% einer polymerisierbaren monomeren Verbindung zugesetzt, und die dadurch entstandene Mischung in TN-VHR-Testzellen gefüllt (90° gerieben, Orientierungsschicht TN-Polyimid, Schichtdicke d ≈ 6 µm). Der HR-Wert wird nach 5 min bei 100 °C vor und nach 2h UV-Belastung (suntest) bei 1 V, 60 Hz, 64 µs pulse bestimmt (Messgerät: Autronic-Melchers VHRM-105).

Zur Untersuchung der Tieftemperaturstabilität, auch als "LTS" (low temperature stability) bezeichnet, d.h. der Stabilität der FK-Mischung gegen spontane Auskristallisation einzelner Komponenten bei tiefen Temperaturen, werden Fläschchen mit 1 g FK/RM-Mischung bei -10 °C eingelagert und es wird regelmäßig überprüft, ob die Mischungen auskristallisiert waren.

Die sogenannte "HTP" ("helical twisting power") bezeichnet das helikale Verdrillungsvermögen einer optisch aktiven bzw. chiralen Substanz in einerm FK-Medium (in µm). Falls nicht anders angegeben, wird die HTP in der kommerziell erhältlichen nematischen FK-Hostmischung MLD-6260 (Merck KGaA) bei einer Temperatur von 20°C gemessen.

Soweit nicht explizit anders vermerkt, sind in der vorliegenden Anmeldung alle Konzentrationen in Gewichtsprozent angegeben und beziehen sich auf die entsprechende Gesamtmischung, enthaltend alle festen oder flüssigkristallinen Komponenten, ohne Lösungsmittel. Alle physikalischen Eigenschaften werden nach "Merck Liquid Crystals, Physical Properties of Liquid Crystals", Status November 1997, Merck KGaA, Deutschland bestimmt und gelten für eine Temperatur von 20 °C, sofern nicht explizit anders angegeben.

### Mischungsbeispiele

**Beispiel M1**

| | | | |
|---|---|---|---|
| CC-3-V | 41,00 % | Klärpunkt [°C]: | 74,0 |
| CCY-3-O2 | 11,00 % | Δn [589 nm, 20 °C]: | 0,1005 |
| CCY-3-O1 | 2,50 % | Δε [1 kHz, 20 °C]: | -3,7 |
| CLY-3-O2 | 4,00 % | ^{ε}∥ [1 kHz, 20 °C]: | 3,7 |
| CLY-3-O3 | 4,00 % | K₁ [pN, 20 °C]: | 13,3 |
| CPY-2-O2 | 4,00 % | K₃ [pN, 20 °C]: | 15,0 |
| CPY-3-O2 | 11,00 % | γ₁ [mPa·s, 20 °C]: | 87 |
| CY-3-O2 | 7,50 % | V₀ [20 °C, V]: | 2,14 |
| PY-3-O2 | 12,00 % | | |
| B-30-05 | 3,00 % | | |

**Beispiel M2**

| | | | |
|---|---|---|---|
| CC-3-V | 42,00 % | Klärpunkt [°C]: | 73,5 |
| CCY-3-O1 | 5,00 % | Δn [589 nm, 20 °C]: | 0,1009 |
| CCY-3-O2 | 10,00 % | Δε [1 kHz, 20 °C]: | -3,5 |
| CCY-4-O2 | 2,50 % | ^{ε}∥ [1 kHz, 20 °C]: | 3,6 |
| CPY-2-O2 | 10,00 % | K₁ [pN, 20 °C]: | 12,9 |
| CPY-3-O2 | 10,00 % | K₃ [pN, 20 °C]: | 14,5 |
| CY-3-O2 | 6,50 % | γ₁ [mPa·s, 20 °C]: | 88 |
| PY-3-O2 | 11,00 % | V₀ [20 °C, V]: | 2,14 |
| B-30-05 | 3,00 % | | |

**Beispiel M3**

| | | | |
|---|---|---|---|
| CC-3-V | 43,00 % | Klärpunkt [°C]: | 73,5 |
| CCY-3-O1 | 5,00 % | Δn [589 nm, 20 °C]: | 0,1011 |
| CCY-3-O2 | 10,00 % | Δε [1 kHz, 20 °C]: | -3,6 |
| CCY-4-O2 | 2,00 % | ^{ε}\|\| [1 kHz, 20 °C]: | 3,7 |
| CPY-2-O2 | 10,00 % | K₁ [pN, 20 °C]: | 13,3 |
| CPY-3-O2 | 10,00 % | K₃[pN,20°C]: | 14,7 |
| CY-3-O2 | 5,00 % | γ₁ [mPa·s, 20 °C]: | 83 |
| PY-3-O2 | 11,00 % | Vo [20 °C, V]: | 2,14 |
| B-30-05 | 4,00 % | | |

**Beispiel M4**

| | | | |
|---|---|---|---|
| CC-3-V | 40,50 % | Klärpunkt [°C]: | 74,0 |
| CCY-3-O1 | 5,00 % | Δn [589 nm, 20 °C]: | 0,1005 |
| CCY-3-O2 | 10,00 % | Δε [1 kHz, 20 °C]: | -3,6 |
| CCY-4-O2 | 3,50 % | ^{ε}∥ [1 kHz, 20 °C]: | 3,6 |
| CPY-2-O2 | 10,00 % | K₁ [pN, 20 °C]: | 12,9 |
| CPY-3-O2 | 10,00 % | K₃ [pN, 20 °C]: | 14,6 |
| CY-3-O2 | 8,00 % | γ₁ [mPa·s, 20 °C]: | 88 |
| PY-3-O2 | 11,00 % | V₀ [20 °C, V]: | 2,14 |
| B-3O-O4 | 2,00 % | | |

**Beispiel M5**

| | | | |
|---|---|---|---|
| CC-3-V | 42,00 % | Klärpunkt [°C]: | 73,5 |
| CCY-3-O1 | 5,00 % | Δn [589 nm, 20 °C]: | 0,1011 |
| CCY-3-O2 | 10,00 % | Δε [1 kHz, 20 °C]: | -3,6 |
| CCY-4-02 | 2,50 % | ^{ε}∥ [1 kHz, 20 °C]: | 3,7 |
| CPY-2-02 | 10,00 % | K₁ [pN, 20 °C]: | 12,9 |
| CPY-3-02 | 10,00 % | K₃ [pN, 20 °C]: | 14,6 |
| CY-3-02 | 6,50 % | γ₁ [mPa·s, 20 °C]: | 85 |
| PY-3-02 | 11,00 % | V₀ [20 °C, V]: | 2,14 |
| B-20-04 | 3,00 % | | |

**Beispiel M6**

| | | | |
|---|---|---|---|
| CC-3-V | 42,00 % | Klärpunkt [°C]: | 73,5 |
| CCY-3-01 | 5,00 % | Δn [589 nm, 20 °C]: | 0,1001 |
| CCY-3-02 | 10,00 % | Δε [1 kHz, 20 °C]: | -3,5 |
| CCY-4-02 | 2,50 % | ^{ε}∥ [1 kHz, 20 °C]: | 3,6 |
| CPY-2-02 | 10,00 % | K₁ [pN, 20 °C]: | 13,0 |
| CPY-3-02 | 10,00 % | K₃ [pN, 20 °C]: | 14,5 |
| CY-3-02 | 6,50 % | γ₁ [mPa·s, 20 °C]: | 84 |
| PY-3-02 | 11,00 % | V₀ [20 °C, V]: | 2,15 |
| B-30-06 | 3,00 % | | |

**Beispiel M7**

| | | | |
|---|---|---|---|
| CC-3-V | 42,00 % | Klärpunkt [°C]: | 73,5 |
| CCY-3-O1 | 5,00 % | Δn [589 nm, 20 °C]: | 0,1007 |
| CCY-3-02 | 10,00 % | Δε [1 kHz, 20 °C]: | -3,5 |
| CCY-4-02 | 2,50 % | ^{ε}∥ [1 kHz, 20 °C]: | 3,7 |
| CPY-2-02 | 10,00 % | K₁ [pN, 20 °C]: | 12,9 |
| CPY-3-02 | 10,00 % | K₃ [pN, 20 °C]: | 14,6 |
| CY-3-02 | 6,50 % | γ₁ [mPa·s, 20 °C]: | 84 |
| PY-3-02 | 11,00 % | V₀ [20 °C, V]: | 2,15 |
| B-20-05 | 3,00 % | | |

**Beispiel M8**

| | | | |
|---|---|---|---|
| CC-3-V | 45,50 % | Klärpunkt [°C]: | 73,0 |
| CCY-3-01 | 3,00 % | Δn [589 nm, 20 °C]: | 0,1011 |
| CCY-3-02 | 11,00 % | Δε [1 kHz, 20 °C]: | -3,5 |
| CCY-4-02 | 3,50 % | ^{ε}∥ [1 kHz, 20 °C]: | 3,6 |
| CPY-2-02 | 7,50 % | K₁ [pN, 20 °C]: | 13,1 |
| CPY-3-02 | 10,00 % | K₃ [pN, 20 °C]: | 14,3 |
| CY-3-02 | 2,00 % | γ₁ [mPa·s, 20 °C]: | 79 |
| PY-3-02 | 11,50 % | V₀ [20 °C, V]: | 2,15 |
| B-20-05 | 6,00 % | | |

**Beispiel M9**

| | | | |
|---|---|---|---|
| CC-3-V | 42,00 % | Klärpunkt [°C]: | 73,5 |
| CCY-3-01 | 5,00 % | Δn [589 nm, 20 °C]: | 0,0993 |
| CCY-3-02 | 10,00 % | Δε [1 kHz, 20 °C]: | -3,5 |
| CCY-4-02 | 2,50 % | ^{ε}∥ [1 kHz, 20 °C]: | 3,6 |
| CPY-2-02 | 10,00 % | K₁ [pN, 20 °C]: | 12,9 |
| CPY-3-02 | 10,00 % | K₃ [pN, 20 °C]: | 14,5 |
| CY-3-02 | 6,50 % | γ₁ [mPa·s, 20 °C]: | 84 |
| PY-3-02 | 11,00 % | V₀ [20 °C, V]: | 2,14 |
| B-30-03 | 3,00 % | | |

**Beispiel M10**

| | | | |
|---|---|---|---|
| CC-3-V | 45,50 % | Klärpunkt [°C]: | 73,5 |
| CCY-3-01 | 3,00 % | Δn [589 nm, 20 °C]: | 0,1009 |
| CCY-3-02 | 11,00 % | Δε [1 kHz, 20 °C]: | -3,5 |
| CCY-4-02 | 3,00 % | ^{ε}\|\| [1 kHz, 20 °C]: | 3,6 |
| CPY-2-02 | 8,50 % | K₁ [pN, 20 °C]: | 13,2 |
| CPY-3-02 | 10,00 % | K₃ [pN, 20 °C]: | 14,3 |
| CY-3-02 | 2,00 % | γ₁ [mPa·s, 20 °C]: | 80 |
| PY-3-02 | 11,00 % | V₀ [20 °C, V]: | 2,15 |
| B-20-05 | | | 3,00 % |
| B-30-05 | | | 3,00 % |

**Beispiel M11**

| | | | |
|---|---|---|---|
| CC-3-V | 35,50 % | Klärpunkt [°C]: | 73,0 |
| CCY-3-01 | 6,00 % | Δn [589 nm, 20 °C]: | 0,1006 |
| CCY-3-02 | 11,00 % | Δε [1 kHz, 20 °C]: | -4,4 |
| CCY-4-02 | 6,50 % | ^{ε}∥ [1 kHz, 20 °C]: | 3,9 |
| CPY-2-02 | 4,00 % | K₁ [pN, 20 °C]: | 12,9 |
| CPY-3-02 | 10,00 % | K₃ [pN, 20 °C]: | 14,6 |
| CY-3-02 | 12,00 % | γ₁ [mPa·s, 20 °C]: | 99 |
| PY-3-02 | 10,00 % | V₀ [20 °C, V]: | 1,93 |
| B-30-05 | 2,50 % | | |
| B-30-04 | 2,50 % | | |

**Beispiel M12**

| | | | |
|---|---|---|---|
| CC-3-V | 34,50 % | Klärpunkt [°C]: | 75,0 |
| CC-3-V1 | 8,00 % | Δn [589 nm, 20 °C]: | 0,1084 |
| CCY-3-01 | 6,00 % | Δε [1 kHz, 20 °C]: | -3,1 |
| CCY-3-02 | 11,50 % | ^{ε}\|\| [1 kHz, 20 °C]: | 3,5 |
| CCY-4-O2 | 3,50 % | K₁ [pN, 20 °C]: | 14,3 |
| CPY-3-02 | 11,50 % | K₃ [pN, 20 °C]: | 15,9 |
| PY-3-02 | 16,00 % | γ₁ [mPa·s, 20 °C]: | 87 |
| PYP-2-3 | 3,00 % | V₀ [20 °C, V]: | 2,40 |
| PP-1-2V1 | 3,00 % | | |
| B-30-05 | 3,00 % | | |

**Beispiel M13**

| | | | |
|---|---|---|---|
| CC-3-V | 34,50 % | Klärpunkt [°C]: | 75,0 |
| CC-3-V1 | 8,00 % | Δn [589 nm, 20 °C]: | 0,1075 |
| CCY-3-01 | 7,00 % | Δε [1 kHz, 20 °C]: | -3,1 |
| CCY-3-02 | 11,50 % | ^{ε}\|\| [1 kHz, 20 °C]: | 3,5 |
| CCY-4-02 | 3,50 % | K₁ [pN, 20 °C]: | 14,7 |
| CPY-3-02 | 11,50 % | K₃ [pN, 20 °C]: | 16,4 |
| PY-3-02 | 13,00 % | γ₁ [mPa·s, 20 °C]: | 84 |
| PP-1-2V1 | 6,00 % | V₀ [20 °C, V]: | 2,41 |
| B-20-05 | 5,00 % | | |

**Beispiel M14**

| | | | |
|---|---|---|---|
| CC-V-V | 37,00 % | Klärpunkt [°C]: | 73,5 |
| CC-V-V1 | 10,00 % | Δn [589 nm, 20 °C]: | 0,1026 |
| CCVC-V-V | 7,00 % | Δε [1 kHz, 20 °C]: | -3,0 |
| CCVC-3-V | 7,00 % | ^{ε}\|\| [1 kHz, 20 °C]: | 3,7 |
| CCC-2-V | 3,00 % | K₁ [pN, 20 °C]: | 11,8 |
| CCC-3-V | 4,00 % | K₃ [pN, 20 °C]: | 11,9 |
| B-40-04 | 3,00 % | γ₁ [mPa·s, 20 °C]: | 57 |
| B-30-04 | 8,50 % | V₀ [20 °C, V]: | 2,10 |
| B-30-05 | 8,50 % | | |
| PB-3-O4 | 7,00 % | | |
| CB-3-04 | 5,00 % | | |

**Beispiel M15**

| | | | |
|---|---|---|---|
| CC-V-V | 18,00 % | Klärpunkt [°C]: | 77,8 |
| CC-3-V | 21,00 % | Δn [589 nm, 20 °C]: | 0,1040 |
| CC-V-V1 | 10,00 % | Δε [1 kHz, 20 °C]: | -3,2 |
| CCVC-V-V | 7,00 % | ^{ε}\|\| [1 kHz, 20 °C]: | 3,7 |
| CCVC-3-V | 7,00 % | K₁ [pN, 20 °C]: | 13,3 |
| CCC-2-V | 2,00 % | K₃ [pN, 20 °C]: | 12,2 |
| CCC-3-V | 3,00 % | γ₁ [mPa·s, 20 °C]: | 64 |
| B-40-04 | 3,00 % | V₀ [20 °C, V]: | 2,07 |
| B-30-04 | 8,50 % | | |
| B-30-05 | 8,50 % | | |
| PB-3-04 | 7,00 % | | |
| CB-3-04 | 5,00 % | | |

**Beispiel M16**

| | | | |
|---|---|---|---|
| CC-V-V | 38,00 % | Klärpunkt [°C]: | 74,0 |
| CCVC-V-V | 5,00 % | Δn [589 nm, 20 °C]: | 0,1119 |
| CCVC-3-V | 10,00 % | Δε [1 kHz, 20 °C]: | -4,8 |
| CCC-2-V | 3,00 % | ^{ε}\|\| [1 kHz, 20 °C]: | 4,3 |
| CCC-3-V | 4,00 % | K₁ [pN, 20 °C]: | 12,3 |
| B-40-04 | 2,00 % | K₃ [pN, 20 °C]: | 11,0 |
| B-30-04 | 5,00 % | γ₁ [mPa·s, 20 °C]: | 76 |
| B-30-05 | 7,00 % | V₀ [20 °C, V]: | 1,60 |
| PB-3-04 | 6,00 % | | |
| CB-3-04 | 7,00 % | | |
| B-20-05 | 8,00 % | | |
| B-20-06 | 5,00 % | | |

**Beispiel M17**

| | | | |
|---|---|---|---|
| CC-V-V | 25,00 % | Klärpunkt [°C]: | 81,0 |
| CCVC-V-V | 4,00 % | Δn [589 nm, 20 °C]: | 0,1068 |
| CCVC-3-V | 9,00 % | Δε [1 kHz, 20 °C]: | -4,2 |
| CCC-2-V | 3,00 % | ^{ε}\|\| [1 kHz, 20 °C]: | 4,1 |
| CCC-3-V | 3,00 % | K₁ [pN, 20 °C]: | 14,6 |
| B-30-04 | 4,00 % | K₃ [pN, 20 °C]: | 11,8 |
| B-30-05 | 5,00 % | γ₁ [mPa·s, 20 °C]: | 90 |
| PB-3-04 | 4,00 % | V₀ [20 °C, V]: | 1,70 |
| CB-3-04 | 7,00 % | | |
| B-20-05 | 6,00 % | | |
| B-20-06 | | | 5,00 % |
| B-20-04 | | | 3,00 % |
| B-1O-O4 | | | 3,00 % |
| CCOC-3-3 | | | 3,00 % |
| CCH-23 | | | 3,00 % |
| CCH-34 | | | 10,00 % |
| CCOC-4-3 | | | 3,00 % |

**Beispiel M18**

| | | | |
|---|---|---|---|
| CC-V-V | 28,00 % | Klärpunkt [°C]: | 55,5 |
| CCVC-V-V | 5,00 % | Δε [1 kHz, 20 °C]: | -7,6 |
| CCVC-3-V | 10,00 % | ^{ε}\|\| [1 kHz, 20 °C]: | 5,8 |
| CCC-2-V | 3,00 % | K₁ [pN, 20 °C]: | 11,1 |
| CCC-3-V | 3,00 % | K₃ [pN, 20 °C]: | 8,6 |
| B-40-04 | 3,00 % | γ₁ [mPa·s, 20 °C]: | 90 |
| B-30-04 | 4,00 % | V₀ [20 °C, V]: | 1,12 |
| B-30-05 | 3,00 % | | |
| B-40-05 | 3,00 % | | |
| B-20-04 | 3,00 % | | |
| B-20-05 | 5,00 % | | |
| B-20-06 | 3,00 % | | |
| B-1O-O5 | 3,00 % | | |
| B-3O-O3 | 4,00 % | | |
| B-30-06 | 3,00 % | | |
| B-1O-O4 | 5,00 % | | |
| B-20-05i | 5,00 % | | |
| CCP-V-1 | 7,00 % | | |

**Beispiel M19**

| | | | |
|---|---|---|---|
| CC-V-V | 35,00 % | Klärpunkt [°C]: | 67,5 |
| CCVC-V-V | 5,00 % | Δε [1 kHz, 20 °C]: | -6,2 |
| CCVC-3-V | 8,00 % | ^{ε}\|\| [1 kHz, 20 °C]: | 4,8 |
| CCC-2-V | 3,00 % | K₁ [pN, 20 °C]: | 11,9 |
| CCC-3-V | 4,00 % | K₃ [pN, 20 °C]: | 9,6 |
| B-40-04 | 2,00 % | γ₁ [mPa·s, 20 °C]: | 86 |
| B-30-04 | 5,00 % | V₀ [20 °C, V]: | 1,32 |
| B-30-05 | 7,00 % | | |
| PB-3-04 | 6,00 % | | |
| CB-3-04 | 7,00 % | | |
| B-20-05 | 8,00 % | | |
| B-20-06 | 5,00 % | | |
| B-30-03 | 5,00 % | | |

**Beispiel M20**

| | | | |
|---|---|---|---|
| CC-3-V | 10,00 % | Klärpunkt [°C]: | 74,5 |
| CC-3-V1 | 8,00 % | Δn [589 nm, 20 °C]: | 0,1078 |
| CCH-23 | 10,00 % | Δε [1 kHz, 20 °C]: | -3,3 |
| CCH-34 | 5,00 % | ^{ε}\|\| [1 kHz, 20 °C]: | 3,5 |
| CCP-3-1 | 7,00 % | K₁ [pN, 20 °C]: | 14,6 |
| CCY-3-01 | 5,00 % | K₃ [pN, 20 °C]: | 15,7 |
| CCY-3-02 | 11,00 % | γ₁ [mPa·s, 20 °C]: | 101 |
| CCY-4-02 | 4,50 % | V₀ [20 °C, V]: | 2,32 |
| CPY-3-02 | 3,00 % | | |
| CY-3-02 | 12,00 % | | |
| PY-3-O2 | 8,50 % | | |
| PYP-2-3 | 8,00 % | | |
| B-20-05 | 4,00 % | | |
| PP-1-2V1 | 4,00 % | | |

**Beispiel M21**

| | | | |
|---|---|---|---|
| CC-3-V | 15,00 % | Klärpunkt [°C]: | 74,0 |
| CC-3-V1 | 8,00 % | Δn [589 nm, 20 °C]: | 0,1078 |
| CCH-23 | 10,00 % | Δε [1 kHz, 20 °C]: | -3,1 |
| CCH-34 | 7,00 % | ^{ε}\|\| [1 kHz, 20 °C]: | 3,5 |
| CCY-3-01 | 8,00 % | K₁ [pN, 20 °C]: | 14,6 |
| CCY-3-02 | 11,00 % | K₃ [pN, 20 °C]: | 15,1 |
| CCY-4-02 | 3,50 % | γ₁ [mPa·s, 20 °C]: | 94 |
| CPY-3-02 | 6,00 % | V₀ [20 °C, V]: | 2,32 |
| CY-3-02 | 5,50 % | | |
| PY-3-02 | 10,00 % | | |
| PYP-2-3 | 8,00 % | | |
| B-20-05 | 4,00 % | | |
| PP-1-2V1 | 4,00 % | | |

**Beispiel M22**

| | | | |
|---|---|---|---|
| CC-3-V | 42,50 % | Klärpunkt [°C]: | 75,0 |
| CCP-3-1 | 4,50 % | Δn [589 nm, 20 °C]: | 0,0984 |
| CCY-3-01 | 9,00 % | Δε [1 kHz, 20 °C]: | -3,2 |
| CCY-3-02 | 11,00 % | ^{ε}\|\| [1 kHz, 20 °C]: | 3,6 |
| CPY-3-02 | 8,00 % | K₁ [pN, 20 °C]: | 13,5 |
| CPY-2-02 | 4,50 % | K₃ [pN, 20 °C]: | 15,1 |
| CY-3-02 | 3,00 % | γ₁ [mPa·s, 20 °C]: | 81 |
| PY-3-02 | 13,50 % | V₀ [20 °C, V]: | 2,29 |
| B-20-05 | 4,00 % | | |

**Beispiel M23**

| | | | |
|---|---|---|---|
| CC-3-V | 45,00 % | Klärpunkt [°C]: | 75,0 |
| CCP-3-1 | 3,00 % | Δn [589 nm, 20 °C]: | 0,0991 |
| CCY-3-01 | 8,50 % | Δε [1 kHz, 20 °C]: | -3,3 |
| CCY-3-02 | 11,00 % | ^{ε}\|\| [1 kHz, 20 °C]: | 3,6 |
| CCY-4-02 | 1,50 % | K₁ [pN, 20 °C]: | 13,8 |
| CPY-3-02 | 11,50 % | K₃ [pN, 20 °C]: | 15,3 |
| PY-3-02 | 12,50 % | γ₁ [mPa·s, 20 °C]: | 80 |
| B-20-05 | 4,00 % | V₀ [20 °C, V]: | 2,28 |
| B-3-O2 | 3,00 % | | |

**Beispiel M24**

| | | | |
|---|---|---|---|
| CC-3-V1 | 8,00 % | Klärpunkt [°C]: | 74,0 |
| CCH-23 | 18,00 % | Δn [589 nm, 20 °C]: | 0,0978 |
| CCH-34 | 3,00 % | Δε [1 kHz, 20 °C]: | -3,4 |
| CCH-35 | 4,00 % | ^{ε}\|\| [1 kHz, 20 °C]: | 3,6 |
| CCP-3-1 | 14,00 % | K₁ [pN, 20 °C]: | 14,9 |
| CCY-3-02 | 11,00 % | K₃ [pN, 20 °C]: | 16,1 |
| CCY-3-01 | 2,00 % | γ₁ [mPa·s, 20 °C]: | 102 |
| CPY-3-02 | 11,00 % | V₀ [20 °C, V]: | 2,28 |
| CY-3-02 | 10,50 % | | |
| PY-3-02 | 12,50 % | | |
| B-20-05 | 3,00 % | | |
| Y-40-04 | 3,00 % | | |

**Beispiel M25**

| | | | |
|---|---|---|---|
| CC-3-V1 | 7,00 % | Klärpunkt [°C]: | 73,0 |
| CCH-23 | 18,00 % | Δn [589 nm, 20 °C]: | 0,0969 |
| CCH-34 | 3,00 % | Δε [1 kHz, 20 °C]: | -3,4 |
| CCH-35 | 4,00 % | ^{ε}\|\| [1 kHz, 20 °C]: | 3,6 |
| CCP-3-1 | 14,00 % | K₁ [pN, 20 °C]: | 14,6 |
| CCY-3-02 | 11,00 % | K₃ [pN, 20 °C]: | 15,8 |
| CCY-3-O1 | 3,00 % | γ₁ [mPa·s, 20 °C]: | 102 |
| CPY-3-02 | 11,00 % | V₀ [20 °C, V]: | 2,29 |
| CY-3-02 | 12,00 % | | |
| PY-3-02 | 11,00 % | | |
| Y-40-04 | 3,00 % | | |
| B-2-O2 | 3,00 % | | |

**Beispiel M26**

| | | | |
|---|---|---|---|
| CC-V-V | 31,50 % | Klärpunkt [°C]: | 75,0 |
| CCP-3-1 | 5,00 % | Δn [589 nm, 20 °C]: | 0,0949 |
| CCY-2-1 | 12,00 % | Δε [1 kHz, 20 °C]: | -3,8 |
| CCY-3-01 | 7,50 % | ^{ε}\|\| [1 kHz, 20 °C]: | 3,7 |
| CCY-3-02 | 8,00 % | K₁ [pN, 20 °C]: | 11,4 |
| CLY-3-02 | 5,50 % | K₃ [pN, 20 °C]: | 14,2 |
| CLY-3-03 | 4,00 % | γ₁ [mPa·s, 20 °C]: | 88 |
| CPY-2-02 | 4,50 % | V₀ [20 °C, V]: | 2,04 |
| CPY-3-02 | 3,00 % | | |
| CY-3-02 | 11,00 % | | |
| PY-1-O4 | 4,00 % | | |
| B-20-05 | 4,00 % | | |

**Beispiel M27**

| | | | |
|---|---|---|---|
| CC-3-V | 36,50 % | Klärpunkt [°C]: | 80,0 |
| CCY-3-01 | 6,00 % | Δn [589 nm, 20 °C]: | 0,1028 |
| CCY-3-02 | 6,00 % | Δε [1 kHz, 20 °C]: | -4,4 |
| CCY-4-02 | 2,50 % | ^{ε}∥ [1 kHz, 20 °C]: | 3,8 |
| CLY-3-02 | 7,00 % | K₁ [pN, 20 °C]: | 14,2 |
| CLY-3-03 | 3,00 % | K₃ [pN, 20 °C]: | 15,9 |
| CPY-2-O2 | 7,00 % | γ₁ [mPa·s, 20 °C]: | 108 |
| CPY-3-02 | 10,00 % | V₀ [20 °C, V]: | 2,01 |
| CY-3-02 | 11,50 % | | |
| PY-3-02 | 5,50 % | | |
| B-20-05 | 5,00 % | | |

**Beispiel M28**

| | | | |
|---|---|---|---|
| CY-3-02 | 9,00 % | Klärpunkt [°C]: | 87,0 |
| CY-3-04 | 7,00 % | Δn [589 nm, 20 °C]: | 0,1026 |
| PY-3-02 | 3,00 % | Δε [1 kHz, 20 °C]: | -4,9 |
| CCY-3-01 | 8,00 % | ^{ε}\|\| [1 kHz, 20 °C]: | 3,9 |
| CCY-3-02 | 11,00 % | K₁ [pN, 20 °C]: | 14,5 |
| CCY-4-02 | 10,00 % | K₃ [pN, 20 °C]: | 16,7 |
| CPY-2-02 | 6,50 % | γ₁ [mPa·s, 20 °C]: | 142 |
| CPY-3-02 | 12,00 % | V₀ [20 °C, V]: | 1,95 |
| CC-3-V | 29,50 % | | |
| B-20-05 | 4,00 % | | |

**Beispiel M29**

| | | | |
|---|---|---|---|
| CY-3-02 | 12,50 % | Klärpunkt [°C]: | 87,0 |
| CCY-3-01 | 9,00 % | Δn [589 nm, 20 °C]: | 0,1025 |
| CCY-3-02 | 11,00 % | Δε [1 kHz, 20 °C]: | -4,8 |
| CCY-4-02 | 7,00 % | ^{ε}\|\| [1 kHz, 20 °C]: | 3,8 |
| CPY-3-02 | 3,00 % | K₁ [pN, 20 °C]: | 14,1 |
| CC-3-V | 31,00 % | K₃ [pN, 20 °C]: | 16,8 |
| B-20-05 | 4,00 % | γ₁ [mPa·s, 20 °C]: | 127 |
| PY-V2-02 | 5,50 % | V₀ [20 °C, V]: | 1,97 |
| CPY-V-O2 | 6,00 % | | |
| CPY-V-04 | 5,00 % | | |
| CCY-V-02 | 6,00 % | | |

**Beispiel M30**

| | | | |
|---|---|---|---|
| CC-3-V | 34,50 % | Klärpunkt [°C]: | 74,0 |
| CC-3-V1 | 8,00 % | Δn [589 nm, 20 °C]: | 0,1072 |
| CCY-3-01 | 5,50 % | Δε [1 kHz, 20 °C]: | -3,0 |
| CCY-3-02 | 11,50 % | ^{ε}\|\| [1 kHz, 20 °C]: | 3,5 |
| PY-3-02 | 7,50 % | K₁ [pN, 20 °C]: | 14,0 |
| PP-1-2V1 | 7,00 % | K₃ [pN, 20 °C]: | 15,8 |
| B-20-05 | 4,00 % | γ₁ [mPa·s, 20 °C]: | 78 |
| PY-V2-02 | 5,00 % | V₀ [20 °C, V]: | 2,43 |
| CPY-V-02 | 6,00 % | | |
| CPY-V-04 | 5,00 % | | |
| CCY-V-02 | 6,00 % | | |

**Beispiel M31**

| | | | |
|---|---|---|---|
| CC-3-V | 42,00 % | Klärpunkt [°C]: | 74,5 |
| CCP-3-1 | 5,00 % | Δn [589 nm, 20 °C]: | 0,0997 |
| CCY-3-01 | 3,00 % | Δε [1 kHz, 20 °C]: | -3,3 |
| CCY-3-02 | 11,00 % | ^{ε}\|\| [1 kHz, 20 °C]: | 3,6 |
| CCY-V-02 | 6,00 % | K₁ [pN, 20 °C]: | 13,0 |
| CPY-V-02 | 6,50 % | K₃ [pN, 20 °C]: | 14,9 |
| CPY-V-04 | 5,00 % | γ₁ [mPa·s, 20 °C]: | 75 |
| CY-3-02 | 3,50 % | V₀ [20 °C, V]: | 2,26 |
| PY-3-02 | 5,00 % | | |
| B-20-05 | 4,00 % | | |
| PY-V2-02 | 9,00 % | | |

### Beispiel M32

Zur Herstellung einer PS-VA-Mischung werden 99,7 % der Mischung gemäß Beispiel M1 mit 0,3 % der polymerisierbaren Verbindung der Formel 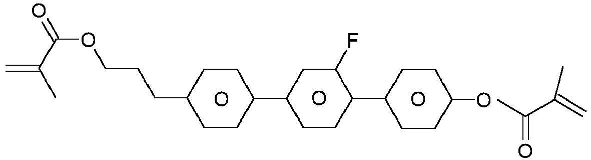 versetzt.

### Beispiel M33

Zur Herstellung einer PS-VA-Mischung werden 99,75 % der Mischung gemäß Beispiel M1 mit 0,25 % der polymerisierbaren Verbindung der Formel 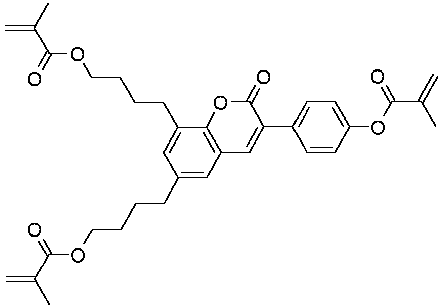 versetzt.

### Beispiel M34

Zur Herstellung einer PS-VA-Mischung werden 99,8 % der Mischung gemäß Beispiel M1 mit 0,2 % der polymerisierbaren Verbindung der Formel 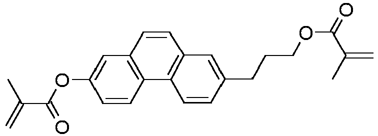 versetzt.

### Beispiel M35

Zur Herstellung einer PS-VA-Mischung werden 99,75 % der Mischung gemäß Beispiel M1 mit 0,25 % der polymerisierbaren Verbindung der Formel 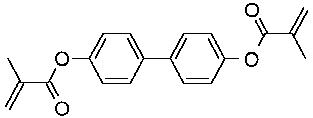 versetzt.

### Beispiel M36

Zur Herstellung einer PS-VA-Mischung werden 99,75 % der Mischung gemäß Beispiel M1 mit 0,25 % der polymerisierbaren Verbindung der Formel 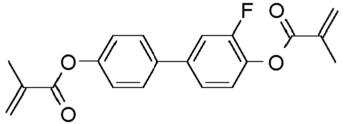 versetzt.

### Beispiel M37

Zur Herstellung einer PS-VA-Mischung werden 99,75 % der Mischung gemäß Beispiel M1 mit 0,3 % der polymerisierbaren Verbindung der Formel 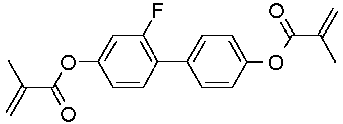 versetzt.

### Beispiel M38

Zur Herstellung einer PS-VA-Mischung werden 99,8 % der Mischung gemäß Beispiel M1 mit 0,2 % der polymerisierbaren Verbindung der Formel 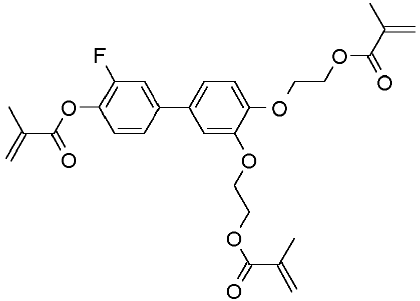 versetzt.

### Beispiel M39

Zur Herstellung einer PS-VA-Mischung werden 99,8 % der Mischung gemäß Beispiel M1 mit 0,2 % der polymerisierbaren Verbindung der Formel 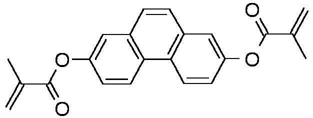 versetzt.

### Beispiel M40

Zur Herstellung einer PS-VA-Mischung werden 99,75 % der Mischung gemäß Beispiel M2 mit 0,25 % der polymerisierbaren Verbindung der Formel 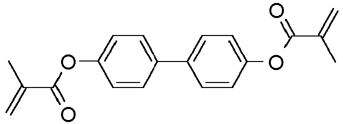 versetzt.

### Beispiel M41

Zur Herstellung einer PS-VA-Mischung werden 99,7 % der Mischung gemäß Beispiel M2 mit 0,3 % der polymerisierbaren Verbindung der Formel 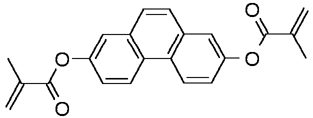 versetzt.

### Beispiel M42

Zur Herstellung einer PS-VA-Mischung werden 99,7 % der Mischung gemäß Beispiel M2 mit 0,3 % der polymerisierbaren Verbindung der Formel 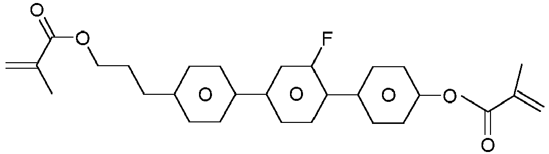 versetzt.

### Beispiel M43

Zur Herstellung einer PS-VA-Mischung werden 99,75 % der Mischung gemäß Beispiel M2 mit 0,25 % der polymerisierbaren Verbindung der Formel 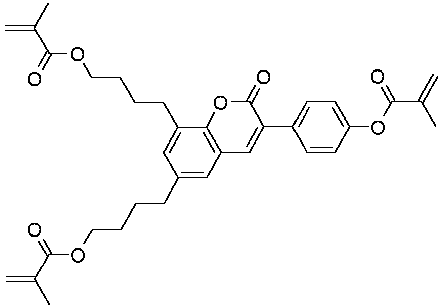 versetzt.

### Beispiel M44

Zur Herstellung einer PS-VA-Mischung werden 99,7 % der Mischung gemäß Beispiel M2 mit 0,3 % der polymerisierbaren Verbindung der Formel 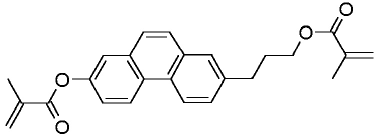 versetzt.

### Beispiel M45

Zur Herstellung einer PS-VA-Mischung werden 99,75 % der Mischung gemäß Beispiel M2 mit 0,25 % der polymerisierbaren Verbindung der Formel 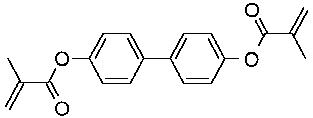 versetzt.

### Beispiel M46

Zur Herstellung einer PS-VA-Mischung werden 99,75 % der Mischung gemäß Beispiel M2 mit 0,25 % der polymerisierbaren Verbindung der Formel 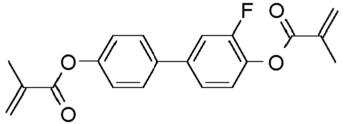 versetzt.

### Beispiel M47

Zur Herstellung einer PS-VA-Mischung werden 99,75 % der Mischung gemäß Beispiel M2 mit 0,25 % der polymerisierbaren Verbindung der Formel 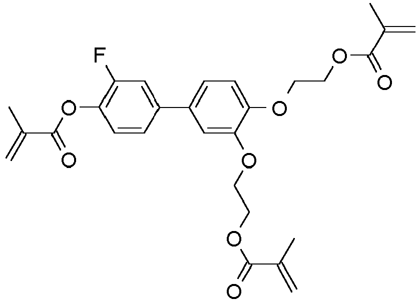 versetzt.

### Beispiel M48

Zur Herstellung einer PS-VA-Mischung werden 99,75 % der Mischung gemäß Beispiel M3 mit 0,25 % der polymerisierbaren Verbindung der Formel 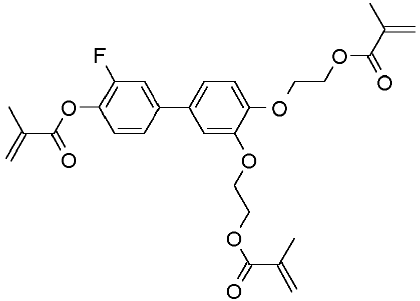 versetzt.

### Beispiel M49

Zur Herstellung einer PS-VA-Mischung werden 99,8 % der Mischung gemäß Beispiel M3 mit 0,3 % der polymerisierbaren Verbindung der Formel 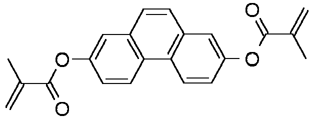 versetzt.

### Beispiel M50

Zur Herstellung einer PS-VA-Mischung werden 99,7 % der Mischung gemäß Beispiel M3 mit 0,3 % der polymerisierbaren Verbindung der Formel 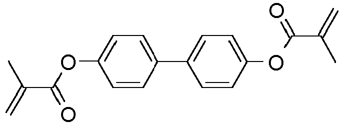 versetzt.

### Beispiel M51

Zur Herstellung einer PS-VA-Mischung werden 99,7 % der Mischung gemäß Beispiel M3 mit 0,3 % der polymerisierbaren Verbindung der Formel 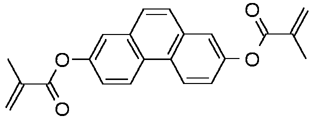 versetzt.

### Beispiel M52

Zur Herstellung einer PS-VA-Mischung werden 99,7 % der Mischung gemäß Beispiel M3 mit 0,3 % der polymerisierbaren Verbindung der Formel 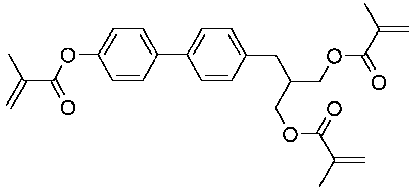 versetzt.

### Beispiel M53

Zur Herstellung einer PS-VA-Mischung werden 99,7 % der Mischung gemäß Beispiel M3 mit 0,3 % der polymerisierbaren Verbindung der Formel 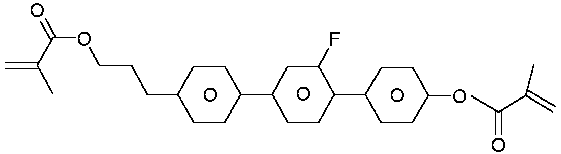 versetzt.

### Beispiel M54

Zur Herstellung einer PS-VA-Mischung werden 99,7 % der Mischung gemäß Beispiel M4 mit 0,3 % der polymerisierbaren Verbindung der Formel 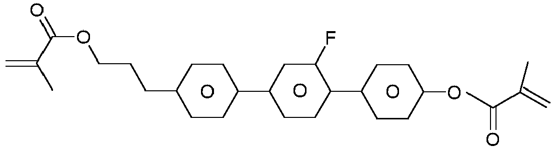 versetzt.

### Beispiel M55

Zur Herstellung einer PS-VA-Mischung werden 99,75 % der Mischung gemäß Beispiel M4 mit 0,25 % der polymerisierbaren Verbindung der Formel 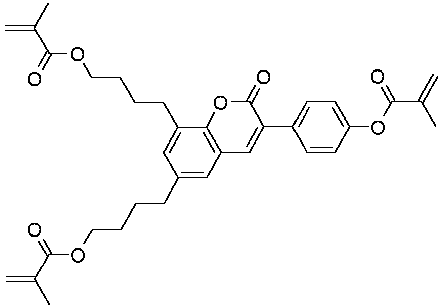 versetzt.

### Beispiel M56

Zur Herstellung einer PS-VA-Mischung werden 99,75 % der Mischung gemäß Beispiel M17 mit 0,25 % der polymerisierbaren Verbindung der Formel 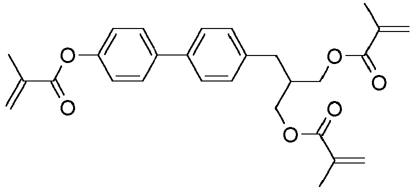 versetzt.

### Beispiel M57

Zur Herstellung einer PS-VA-Mischung werden 99,7 % der Mischung gemäß Beispiel M21 mit 0,3 % der polymerisierbaren Verbindung der Formel 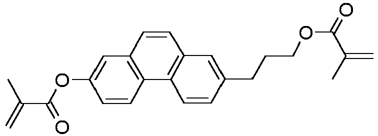 versetzt.

### Beispiel M58

Zur Herstellung einer PS-VA-Mischung werden 99,75 % der Mischung gemäß Beispiel M25 mit 0,25 % der polymerisierbaren Verbindung der Formel 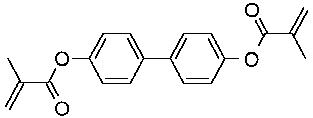 versetzt.

### Beispiel M59

Zur Herstellung einer PS-VA-Mischung werden 99,75 % der Mischung gemäß Beispiel M28 mit 0,25 % der polymerisierbaren Verbindung der Formel 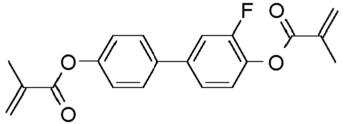 versetzt.

### Beispiel M60

Zur Herstellung einer PS-VA-Mischung werden 99,7 % der Mischung gemäß Beispiel M29 mit 0,3 % der polymerisierbaren Verbindung der Formel 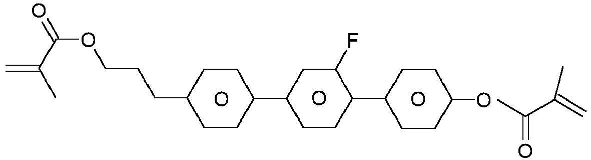 versetzt.

### Beispiel M61

Zur Herstellung einer PS-VA-Mischung werden 99,75 % der Mischung gemäß Beispiel M30 mit 0,25 % der polymerisierbaren Verbindung der Formel 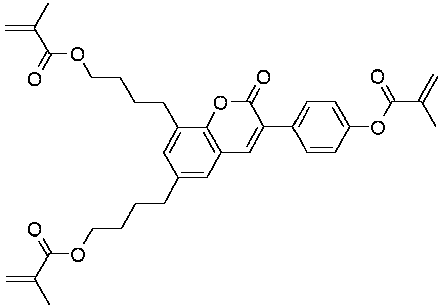 versetzt.

### Beispiel M62

Zur Herstellung einer PS-VA-Mischung werden 99,8 % der Mischung gemäß Beispiel M31 mit 0,2 % der polymerisierbaren Verbindung der Formel 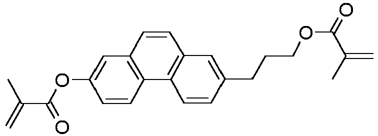 versetzt.

### Beispiel M63

Zur Herstellung einer PS-VA-Mischung werden 99,75 % der Mischung gemäß Beispiel M19 mit 0,25 % der polymerisierbaren Verbindung der Formel 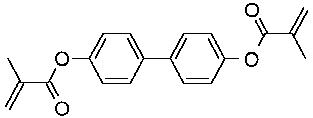 versetzt.

### Beispiel M64

Zur Herstellung einer PS-VA-Mischung werden 99,75 % der Mischung gemäß Beispiel M20 mit 0,25 % der polymerisierbaren Verbindung der Formel 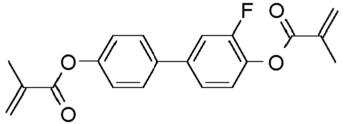 versetzt.

### Beispiel M65

Zur Herstellung einer PS-VA-Mischung werden 99,75 % der Mischung gemäß Beispiel M27 mit 0,25 % der polymerisierbaren Verbindung der Formel 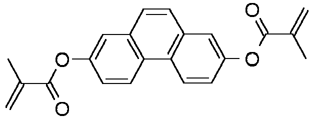 versetzt.

### Beispiel M66

Zur Herstellung einer PS-VA-Mischung werden 99,75 % der Mischung gemäß Beispiel M27 mit 0,25 % der polymerisierbaren Verbindung der Formel 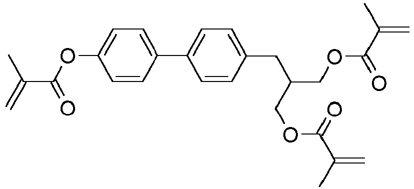 versetzt.

**Beispiel M67**

| | | | |
|---|---|---|---|
| CC-3-V | 17,00% | Klärpunkt [°C]: | 75,5 |
| CC-V-V | 20,00% | Δn [589 nm, 20°C]: | 0,1079 |
| CC-3-V1 | 8,00% | ^{ε}∥ [1 kHz, 20°C]: | 3,4 |
| CCY-3-01 | 6,00% | ε⊥ [1 kHz, 20°C]: | 6,1 |
| CCY-3-02 | 10,00% | Δε [1 kHz, 20°C]: | -2,7 |
| CCY-4-02 | 2,50% | K₁ [pN, 20°C]: | 12,5 |
| CPY-2-02 | 5,50% | K₃[pN, 20°C]: | 14,8 |
| CPY-3-02 | 11,50% | V₀ [pN, 20°C]: | 2,45 |
| PY-3-02 | 8,50% | γ₁ [mPa·s, 20 °C]: | 75 |
| PYP-2-3 | 5,00% | | |
| PP-1-2V1 | 3,00% | | |
| B-30-05 | 3,00% | | |

**Beispiel M68**

| | | | |
|---|---|---|---|
| CC-3-V | 20,50% | Klärpunkt [°C]: | 74,5 |
| CC-V-V | 15,00% | Δn [589 nm, 20°C]: | 0,1095 |
| CC-3-V1 | 8,00% | ^{ε}∥ [1 kHz, 20°C]: | 3,5 |
| CLY-3-02 | 6,00% | ε⊥ [1 kHz, 20°C]: | 6,3 |
| CCY-3-02 | 11,50% | Δε [1 kHz, 20°C]: | -2,9 |
| CCY-4-02 | 4,00% | K₁ [pN, 20°C]: | 13,5 |
| CPY-3-O2 | 7,50% | K₃ [pN, 20°C]: | 15,2 |
| BCH-32 | 3,50% | V₀ [pN, 20°C]: | 2,43 |
| PY-3-02 | 11,50% | γ₁ [mPa·s, 20 °C]: | 77 |
| PGIY-2-04 | 4,50% | | |
| PP-1-2V1 | 4,00% | | |
| B-20-05 | 4,00% | | |

**Beispiel M69**

| | | | |
|---|---|---|---|
| CC-V-V | 36,00% | Klärpunkt [°C]: | 75 |
| CC-3-V1 | 8,00% | Δn [589 nm, 20°C]: | 0,1076 |
| CLY-3-02 | 6,00% | ^{ε}∥ [1 kHz, 20°C]: | 3,4 |
| CCY-3-02 | 11,50% | ε⊥ [1 kHz, 20°C]: | 6,0 |
| CCY-4-02 | 5,00% | Δε [1 kHz, 20°C]: | -2,7 |
| CPY-3-02 | 7,50% | K₁ [pN, 20°C]: | 12,0 |
| BCH-32 | 7,00% | K₃ [pN, 20°C]: | 14,3 |
| PY-3-02 | 8,00% | V₀ [pN, 20°C]: | 2,46 |
| PGIY-2-04 | 4,50% | γ₁ [mPa·s, 20 °C]: | 70 |
| PP-1-2V1 | 2,50% | | |
| B-20-05 | 4,00% | | |

**Beispiel M70**

| | | | |
|---|---|---|---|
| CC-V-V | 34,00% | Klärpunkt [°C]: | 75,5 |
| CC-3-V1 | 8,00% | Δn [589 nm, 20°C]: | 0,1085 |
| CCY-3-01 | 7,00% | ^{ε}∥ [1 kHz, 20°C]: | 3,4 |
| CCY-3-02 | 11,50% | ε⊥ [1 kHz, 20°C]: | 6,1 |
| CCY-4-02 | 5,00% | Δε [1 kHz, 20°C]: | -2,7 |
| CPY-3-02 | 7,50% | K₁ [pN, 20°C]: | 12,0 |
| BCH-32 | 7,00% | K₃ [pN, 20°C]: | 14,4 |
| PY-3-O2 | 8,00% | V₀ [pN, 20°C]: | 2,44 |
| PGIY-2-04 | 4,50% | γ₁ [mPa·s, 20 °C]: | 74 |
| PP-1-2V1 | 3,50% | | |
| B-20-05 | 4,00% | | |

**Beispiel M71**

| | | | |
|---|---|---|---|
| CC-3-V | 9,50% | Klärpunkt [°C]: | 74,0 |
| CC-V-V | 29,00% | Δn [589 nm, 20°C]: | 0,0989 |
| CCP-3-1 | 10,00% | ^{ε}\|\| [1 kHz, 20°C]: | 3,6 |
| CCY-3-01 | 8,50% | ε⊥ [1 kHz, 20°C]: | 6,7 |
| CCY-3-02 | 11,00% | Δε [1 kHz, 20°C]: | -3,2 |
| CPY-2-02 | 2,00% | K₁ [pN, 20°C]: | 11,8 |
| CPY-3-02 | 11,00% | K₃ [pN, 20°C]: | 14,8 |
| CY-3-02 | 5,00% | V₀ [pN, 20°C]: | 2,28 |
| PY-3-02 | 10,00% | γ₁ [mPa·s, 20 °C]: | 76 |
| B-20-05 | 4,00% | | |

**Beispiel M72**

| | | | |
|---|---|---|---|
| CC-3-V | 15,50% | Klärpunkt [°C]: | 74,5 |
| CC-V-V | 20,00% | Δn [589 nm, 20°C]: | 0,1075 |
| CC-3-V1 | 8,00% | ^{ε}\|\| [1 kHz, 20°C]: | 3,5 |
| CCY-3-01 | 8,00% | ε⊥ [1 kHz, 20°C]: | 6,5 |
| CCY-3-02 | 11,50% | Δε [1 kHz, 20°C]: | -3,0 |
| CCY-4-02 | 4,50% | K₁ [pN, 20°C]: | 12,9 |
| CPY-3-02 | 8,50% | K₃ [pN, 20°C]: | 15,0 |
| PY-2-02 | 6,50% | V₀ [pN, 20°C]: | 2,35 |
| PGIY-2-04 | 5,00% | γ₁ [mPa·s, 20 °C]: | 76 |
| PP-1-2V1 | 6,50% | LTS bulk [-20 °C]: | > 1000 h |
| B(S)-20-05 | 3,00% | | |
| B-20-05 | 3,00% | | |

**Beispiel M73**

| | | | |
|---|---|---|---|
| CC-3-V | 17,50% | Klärpunkt [°C]: | 74 |
| CC-V-V | 20,00% | Δn [589 nm, 20°C]: | 0,1074 |
| CC-3-V1 | 8,00% | ^{ε}\|\| [1 kHz, 20°C]: | 3,5 |
| CCY-3-01 | 8,00% | ε⊥ [1 kHz, 20°C]: | 6,4 |
| CCY-3-02 | 12,00% | Δε [1 kHz, 20°C]: | -2,9 |
| CPY-3-02 | 12,00% | K₁ [pN, 20°C]: | 12,7 |
| PY-2-02 | 6,00% | K₃ [pN, 20°C]: | 15,1 |
| PGIY-2-04 | 4,50% | V₀ [pN, 20°C]: | 2,41 |
| PP-1-2V1 | 6,00% | γ₁ [mPa·s, 20 °C]: | 72 |
| B(S)-20-05 | 3,00% | | |
| B-20-05 | | | 3,00% |

**Beispiel M74**

| | | | |
|---|---|---|---|
| CC-3-V | 22,00% | Klärpunkt [°C]: | 76 |
| CC-V-V | 20,00% | Δn [589 nm, 20°C]: | 0,0946 |
| CCY-3-01 | 8,00% | ^{ε}∥ [1 kHz, 20°C]: | 3,7 |
| CCY-3-02 | 12,00% | ε⊥ [1 kHz, 20°C]: | 7,5 |
| CCY-4-02 | 2,50% | Δε [1 kHz, 20°C]: | -3,9 |
| CLY-3-02 | 6,00% | K₁ [pN, 20°C]: | 12,4 |
| CLY-3-03 | 9,50% | K₃ [pN, 20°C]: | 14,3 |
| CPY-3-02 | 1,50% | V₀ [pN, 20°C]: | 2,04 |
| CY-3-02 | 2,50% | γ₁ [mPa·s, 20 °C]: | 78 |
| B-20-05 | 3,00% | | |
| B(S)-20-05 | 3,00% | | |
| PY-2-02 | 10,00% | | |

**Beispiel M75**

| | | | |
|---|---|---|---|
| CC-3-V | 20,50% | Klärpunkt [°C]: | 76 |
| CC-V-V | 20,00% | Δn [589 nm, 20°C]: | 0,0945 |
| CCY-3-01 | 8,00% | ^{ε}∥ [1 kHz, 20°C]: | 3,7 |
| CCY-3-O2 | 11,50% | ε⊥ [1 kHz, 20°C]: | 7,6 |
| CCY-4-02 | 4,00% | Δε [1 kHz, 20°C]: | -3,9 |
| CPY-2-02 | 6,00% | K₁ [pN, 20°C]: | 11,9 |
| CPY-3-02 | 11,00% | K₃ [pN, 20°C]: | 14,7 |
| CY-3-02 | 13,50% | V₀ [pN, 20°C]: | 2,05 |
| B-20-05 | 3,00% | γ₁ [mPa·s, 20 °C]: | 84 |
| B(S)-20-05 | 2,50% | | |

**Beispiel M76**

| | | | |
|---|---|---|---|
| CC-3-V | 19,50% | Klärpunkt [°C]: | 75,5 |
| CC-V-V | 23,00% | Δn [589 nm, 20°C]: | 0,0989 |
| CCP-3-1 | 5,50% | ^{ε}\|\| [1 kHz, 20°C]: | 3,5 |
| CCY-3-01 | 8,00% | ε⊥ [1 kHz, 20°C]: | 6,6 |
| CCY-3-02 | 11,00% | Δε [1 kHz, 20°C]: | -3,1 |
| CCY-4-02 | 4,00% | K₁ [pN, 20°C]: | 12,2 |
| CPY-3-02 | 12,00% | K₃ [pN, 20°C]: | 14,8 |
| PY-3-02 | 13,00% | V₀ [pN, 20°C]: | 2,30 |
| B-20-05 | 4,00% | γ₁ [mPa·s, 20 °C]: | 75 |

**Beispiel M77**

| | | | |
|---|---|---|---|
| CC-V-V | 15,00% | Klärpunkt [°C]: | 74,5 |
| CC-3-V1 | 7,00% | Δn [589 nm, 20°C]: | 0,1071 |
| CCH-23 | 6,50% | ^{ε}∥ [1 kHz, 20°C]: | 3,6 |
| CCH-34 | 4,00% | ε⊥ [1 kHz, 20°C]: | 6,7 |
| CCP-3-1 | 16,00% | Δε [1 kHz, 20°C]: | -3,2 |
| CCY-3-01 | 4,50% | K₁ [pN, 20°C]: | 13,4 |
| CCY-3-02 | 12,00% | K₃ [pN, 20°C]: | 15,0 |
| CY-3-02 | 8,50% | V₀ [pN, 20°C]: | 2,29 |
| PY-3-02 | 11,50% | γ₁ [mPa·s, 20 °C]: | 88 |
| PYP-2-3 | 8,00% | | |
| B-20-05 | 4,00% | | |
| B(S)-20-05 | 3,00% | | |

**Beispiel M78**

| | | | |
|---|---|---|---|
| CC-3-V | 23,00% | Klärpunkt [°C]: | 74,5 |
| CC-V-V | 20,00% | Δn [589 nm, 20°C]: | 0,0974 |
| CCP-3-1 | 5,00% | ^{ε}∥ [1 kHz, 20°C]: | 3,5 |
| CCY-3-01 | 7,50% | ε⊥ [1 kHz, 20°C]: | 6,6 |
| CCY-3-02 | 11,00% | Δε [1 kHz, 20°C]: | -3,1 |
| CCY-4-02 | 5,00% | K₁ [pN, 20°C]: | 12,3 |
| CPY-3-02 | 11,00% | K₃ [pN, 20°C]: | 14,7 |
| PY-3-02 | 13,50% | V₀ [pN, 20°C]: | 2,30 |
| B-20-05 | 4,00% | γ₁ [mPa·s, 20 °C]: | 74 |

**Beispiel M79**

| | | | |
|---|---|---|---|
| BCH-32 | 8,50% | Klärpunkt [°C]: | 73,0 |
| CC-3-V | 15,00% | Δn [589 nm, 20°C]: | 0,1052 |
| CC-V-V | 14,00% | ε_{∥} [1 kHz, 20°C]: | 3,4 |
| CCP-3-1 | 11,00% | ε_{⊥} [1 kHz, 20°C]: | 6,0 |
| CCY-3-01 | 7,00% | Δε [1 kHz, 20°C]: | -2,6 |
| CCY-3-02 | 8,50% | K₁ [pN, 20°C]: | 12,5 |
| CPY-3-02 | 7,00% | K₃ [pN, 20°C]: | 14,7 |
| CY-3-02 | 17,00% | V₀ [pN, 20°C]: | 2,53 |
| PP-1-3 | 7,00% | γ₁ [mPa·s, 20 °C]: | 79 |
| B-20-05 | 4,00% | | |
| PYP-2-3 | 1,00% | | |

**Beispiel M80**

| | | | |
|---|---|---|---|
| CC-V-V | 31,50% | Klärpunkt [°C]: | 75,0 |
| CCP-3-1 | 5,00% | Δn [589 nm, 20°C]: | 0,0949 |
| CCY-2-1 | 12,00% | ε_{∥} [1 kHz, 20°C]: | 3,7 |
| CCY-3-01 | 7,50% | ε_{⊥} [1 kHz, 20°C]: | 7,5 |
| CCY-3-O2 | 8,00% | Δε [1 kHz, 20°C]: | -3,8 |
| CLY-3-02 | 5,50% | K₁ [pN, 20°C]: | 11,4 |
| CLY-3-03 | 4,00% | K₃ [pN, 20°C]: | 14,2 |
| CPY-2-02 | 4,50% | V₀ [pN, 20°C]: | 2,04 |
| CPY-3-02 | 3,00% | γ₁ [mPa·s, 20 °C]: | 88 |
| CY-3-02 | 11,00% | | |
| PY-1-O4 | 4,00% | | |
| B-20-05 | 4,00% | | |

**Beispiel M81**

| | | | |
|---|---|---|---|
| CC-V-V | 31,50% | Klärpunkt [°C]: | 74,5 |
| CCP-3-1 | 4,00% | Δn [589 nm, 20°C]: | 0,0945 |
| CCY-2-1 | 12,00% | ε_{∥} [1 kHz, 20°C]: | 3,7 |
| CCY-3-01 | 7,50% | ε_{⊥} [1 kHz, 20°C]: | 7,6 |
| CCY-3-02 | 11,50% | Δε [1 kHz, 20°C]: | -3,8 |
| CLY-3-02 | 5,00% | K₁ [pN, 20°C]: | 11,2 |
| CPY-3-02 | 4,50% | K₃ [pN, 20°C]: | 14,4 |
| CY-3-02 | 14,00% | V₀ [pN, 20°C]: | 2,05 |
| PY-4-02 | 2,00% | γ₁ [mPa·s, 20 °C]: | 90 |
| PGIY-2-04 | 3,00% | | |
| B-20-05 | 4,00% | | |
| CCPC-33 | 1,00% | | |

**Beispiel M82**

| | | | |
|---|---|---|---|
| CC-3-V | 14,50% | Klärpunkt [°C]: | 74 |
| CC-V-V | 20,00% | Δn [589 nm, 20°C]: | 0,1074 |
| CC-3-V1 | 8,00% | Δε [1 kHz, 20°C]: | -3,0 |
| CCY-3-01 | 5,50% | K₁ [pN, 20°C]: | 12,7 |
| CCY-3-02 | 11,50% | K₃ [pN, 20°C]: | 15,4 |
| CPY-3-02 | 4,00% | V₀ [pN, 20°C]: | 2,42 |
| PY-3-O2 | 3,50% | γ₁ [mPa·s, 20 °C]: | 73 |
| PP-1-2V1 | 7,00% | | |
| B-20-05 | 4,00% | | |
| PY-V2-02 | 5,00% | | |
| CPY-V-02 | 6,00% | | |
| CPY-V-04 | 5,00% | | |
| CCY-V-02 | 6,00% | | |

**Beispiel M83**

| | | | |
|---|---|---|---|
| CCP-V-1 | 4,00% | Klärpunkt [°C]: | 92,5 |
| CCY-3-02 | 7,50% | Δn [589 nm, 20°C]: | 0,1074 |
| CLY-3-02 | 8,00% | Δε [1 kHz, 20°C]: | -2,5 |
| CLY-3-03 | 5,00% | ε_{∥} [1 kHz, 20°C]: | 3,2 |
| CPY-3-02 | 10,00% | ε_{⊥} [1 kHz, 20°C]: | 5,7 |
| PGIY-2-04 | 5,00% | K₁ [pN, 20°C]: | 16,4 |
| PYP-2-3 | 6,00% | K₃ [pN, 20°C]: | 18,0 |
| B-20-05 | 5,00% | V₀ [pN, 20°C]: | 2,82 |
| CC-3-V | 41,50% | γ₁ [mPa·s, 20 °C]: | 96 |
| CC-3-V1 | 8,00% | | |

**Beispiel M84**

| | | | |
|---|---|---|---|
| CLY-3-02 | 10,00% | Klärpunkt [°C]: | 80 |
| CLY-3-03 | 1,50% | Δn [589 nm, 20°C]: | 0,1080 |
| CPY-2-02 | 4,00% | Δε [1 kHz, 20°C]: | -2,4 |
| CPY-3-02 | 10,00% | ε_{∥} [1 kHz, 20°C]: | 3,3 |
| PGIY-2-04 | 5,00% | ε_{⊥} [1 kHz, 20°C]: | 5,7 |
| PYP-2-3 | 8,00% | K₁ [pN, 20°C]: | 14,0 |
| B-20-05 | 4,50% | K₃ [pN, 20°C]: | 15,8 |
| CC-3-V | 44,50% | V₀ [pN, 20°C]: | 2,68 |
| CC-3-V1 | 8,00% | γ₁ [mPa·s, 20 °C]: | 80 |
| CY-3-02 | 2,50% | | |
| CY-5-02 | 2,00% | | |

**Beispiel M85**

| | | | |
|---|---|---|---|
| CCP-V2-1 | 5,00% | Klärpunkt [°C]: | 79,5 |
| CLY-3-02 | 10,00% | Δn [589 nm, 20°C]: | 0,1079 |
| CPY-3-02 | 9,00% | Δε [1 kHz, 20°C]: | -2,0 |
| PGIY-2-04 | 2,50% | ε_{∥} [1 kHz, 20°C]: | 3,2 |
| PYP-2-3 | 8,00% | ε_{⊥} [1 kHz, 20°C]: | 5,3 |
| PYP-2-4 | 5,00% | K₁ [pN, 20°C]: | 14,4 |
| B-20-05 | 4,00% | K₃ [pN, 20°C]: | 15,5 |
| CC-3-V | 43,00% | V₀ [pN, 20°C]: | 2,92 |
| CC-3-V1 | 7,50% | γ₁ [mPa·s, 20 °C]: | 75 |
| CY-3-02 | 6,00% | | |

**Beispiel M86**

| | | | |
|---|---|---|---|
| CCY-3-01 | 4,00% | Klärpunkt [°C]: | 79,8 |
| CCY-3-02 | 8,50% | Δn [589 nm, 20°C]: | 0,1013 |
| CCY-4-02 | 5,00% | Δε [1 kHz, 20°C]: | -3,7 |
| CLY-3-02 | 10,00% | ε_{∥} [1 kHz, 20°C]: | 3,6 |
| CLY-3-03 | 4,00% | ε_{⊥} [1 kHz, 20°C]: | 7,3 |
| PGIY-2-04 | 5,00% | K₁ [pN, 20°C]: | 14,7 |
| PYP-2-3 | 1,00% | K₃ [pN, 20°C]: | 16,3 |
| B-20-05 | 5,00% | V₀ [pN, 20°C]: | 2,20 |
| CC-3-V | 34,50% | γ₁ [mPa·s, 20 °C]: | 97 |
| CC-3-V1 | 8,50% | | |
| CY-3-02 | 5,00% | | |
| PY-3-02 | 9,50% | | |

**Beispiel M87**

| | | | |
|---|---|---|---|
| CCY-3-02 | 7,00% | Klärpunkt [°C]: | 80 |
| CCY-4-02 | 2,00% | Δn [589 nm, 20°C]: | 0,1009 |
| CLY-3-02 | 8,00% | Δε [1 kHz, 20°C]: | -3,7 |
| CLY-3-03 | 4,00% | ε_{∥} [1 kHz, 20°C]: | 3,6 |
| CPY-2-02 | 3,00% | ε_{⊥} [1 kHz, 20°C]: | 7,3 |
| CPY-3-02 | 8,00% | K₁ [pN, 20°C]: | 14,4 |
| PGIY-2-04 | 5,00% | K₃ [pN, 20°C]: | 16,4 |
| PYP-2-3 | 1,00% | V₀ [pN, 20°C]: | 2,21 |
| B-20-05 | 5,00% | γ₁ [mPa·s, 20 °C]: | 99 |
| CC-3-V | 35,00% | | |
| CC-3-V1 | 8,00% | | |
| CY-3-02 | | | 12,00% |
| CY-5-02 | | | 2,00% |

**Beispiel M88**

| | | | |
|---|---|---|---|
| CY-3-02 | 4,00% | Klärpunkt [°C]: | 100 |
| CY-3-04 | 18,00% | Δn [589 nm, 20°C]: | 0,0955 |
| CCY-3-01 | 5,00% | Δε [1 kHz, 20°C]: | -5,0 |
| CCY-3-02 | 6,00% | ε_{∥} [1 kHz, 20°C]: | 3,8 |
| CCY-3-03 | 6,00% | ε_{⊥} [1 kHz, 20°C]: | 8,8 |
| CCY-4-02 | 6,00% | K₁ [pN, 20°C]: | 15,2 |
| CLY-3-02 | 2,50% | K₃ [pN, 20°C]: | 16,0 |
| CPY-2-02 | 8,00% | V₀ [pN, 20°C]: | 1,90 |
| CC-4-V | 18,00% | γ₁ [mPa·s, 20 °C]: | 226 |
| CC-5-V | 4,00% | | |
| CH-33 | 3,00% | | |
| CH-35 | 3,00% | | |
| CCPC-33 | 4,50% | | |
| CCPC-34 | 4,50% | | |
| B-20-05 | 7,50% | | |

**Beispiel M89**

| | | | |
|---|---|---|---|
| CCY-3-01 | 8,00% | Klärpunkt [°C]: | 81,5 |
| CCY-4-02 | 6,00% | Δn [589 nm, 20°C]: | 0,1075 |
| CLY-3-02 | 10,00% | Δε [1 kHz, 20°C]: | -3,1 |
| CPY-3-02 | 10,50% | ε_{∥} [1 kHz, 20°C]: | 3,5 |
| PYP-2-3 | 9,00% | ε_{⊥} [1 kHz, 20°C]: | 6,6 |
| B-20-05 | 5,00% | K₁ [pN, 20°C]: | 14,3 |
| CC-3-V | 45,00% | K₃ [pN, 20°C]: | 15,7 |
| PY-3-02 | 5,00% | V₀ [pN, 20°C]: | 2,38 |
| Y-40-04 | 1,50% | γ₁ [mPa·s, 20 °C]: | 90 |

**Beispiel M90**

| | | | |
|---|---|---|---|
| CC-3-V | 35,00% | Klärpunkt [°C]: | 86 |
| CCY-3-01 | 5,00% | Δn [589 nm, 20°C]: | 0,1208 |
| CCY-3-02 | 7,50% | Δε [1 kHz, 20°C]: | -4,2 |
| CLY-3-02 | 8,00% | ε_{∥} [1 kHz, 20°C]: | 3,8 |
| CPY-2-02 | 10,00% | ε_{⊥} [1 kHz, 20°C]: | 8,0 |
| CPY-3-02 | 10,00% | K₁ [pN, 20°C]: | 14,3 |
| PY-3-02 | 12,50% | K₃ [pN, 20°C]: | 15,6 |
| PGIY-2-04 | 8,00% | V₀ [pN, 20°C]: | 2,04 |
| B-20-05 | 4,00% | γ₁ [mPa·s, 20 °C]: | 129 |

**Beispiel M91**

| | | | |
|---|---|---|---|
| CCY-3-01 | 7,00% | Klärpunkt [°C]: | 80 |
| CLY-3-02 | 10,00% | Δn [589 nm, 20°C]: | 0,1141 |
| CPY-2-02 | 10,00% | Δε [1 kHz, 20°C]: | -4,0 |
| CPY-3-02 | 10,00% | ε_{∥} [1 kHz, 20°C]: | 3,7 |
| PYP-2-3 | 3,00% | ε_{⊥} [1 kHz, 20°C]: | 7,7 |
| B-20-05 | 4,00% | K₁ [pN, 20°C]: | 14,9 |
| CC-3-V | 38,00% | K₃ [pN, 20°C]: | 15,6 |
| PY-1-O4 | 10,00% | V₀ [pN, 20°C]: | 2,09 |
| PY-3-02 | 4,50% | γ₁ [mPa·s, 20 °C]: | 108 |
| CCY-3-O2 | 3,50% | | |

**Beispiel M92**

| | | | |
|---|---|---|---|
| CCY-3-01 | 7,00% | Klärpunkt [°C]: | 90 |
| CCY-4-02 | 4,00% | Δn [589 nm, 20°C]: | 0,1139 |
| CLY-3-02 | 10,00% | Δε [1 kHz, 20°C]: | -4,2 |
| CPY-2-02 | 10,00% | ε_{∥} [1 kHz, 20°C]: | 3,7 |
| CPY-3-02 | 10,00% | ε_{⊥} [1 kHz, 20°C]: | 7,9 |
| PYP-2-3 | 2,50% | K₁ [pN, 20°C]: | 16,2 |
| B-20-05 | 4,00% | K₃ [pN, 20°C]: | 17,0 |
| CC-3-V | 35,50% | V₀ [pN, 20°C]: | 2,12 |
| PY-1-O4 | 10,00% | γ₁ [mPa·s, 20 °C]: | 131 |
| PY-3-02 | 1,00% | | |
| CCY-3-02 | 6,00% | | |

**Beispiel M93**

| | | | |
|---|---|---|---|
| B-20-05 | 5,00% | Klärpunkt [°C]: | 80,1 |
| BCH-32 | 7,00% | Δn [589 nm, 20°C]: | 0,1121 |
| CC-3-V | 34,50% | Δε [1 kHz, 20°C]: | -3,9 |
| CCP-V-1 | 2,00% | K₁ [pN, 20°C]: | 14,0 |
| CCY-3-01 | 5,00% | K₃ [pN, 20°C]: | 14,5 |
| CCY-3-02 | 4,00% | V₀ [pN, 20°C]: | 2,03 |
| CCY-4-02 | 2,00% | γ₁ [mPa·s, 20 °C]: | 104 |
| CLY-3-02 | 8,00% | | |
| CPY-2-02 | 10,00% | | |
| CPY-3-O2 | 7,00% | | |
| PGIY-2-04 | 6,00% | | |
| PY-3-02 | 2,00% | | |
| Y-40-04 | 7,50% | | |

**Beispiel M94**

| | | | |
|---|---|---|---|
| CCY-3-01 | 7,00% | Klärpunkt [°C]: | 80,5 |
| CCY-4-02 | 1,50% | Δn [589 nm, 20°C]: | 0,1070 |
| CLY-3-02 | 10,00% | Δε [1 kHz, 20°C]: | -3,9 |
| CPY-2-02 | 9,50% | K₁ [pN, 20°C]: | 15,0 |
| CPY-3-02 | 8,00% | K₃ [pN, 20°C]: | 15,7 |
| B-20-05 | 4,00% | V₀ [pN, 20°C]: | 2,12 |
| CC-3-V | 40,00% | γ₁ [mPa·s, 20 °C]: | 104 |
| PY-1-O4 | 9,50% | | |
| PY-3-02 | 4,50% | | |
| CCY-3-02 | 6,00% | | |

**Beispiel M95**

| | | | |
|---|---|---|---|
| CCY-3-01 | 7,00% | Klärpunkt [°C]: | 80,5 |
| CLY-3-02 | 10,00% | Δn [589 nm, 20°C]: | 0,1140 |
| CPY-2-02 | 10,00% | Δε [1 kHz, 20°C]: | -4,0 |
| CPY-3-02 | 10,00% | K₁ [pN, 20°C]: | 14,8 |
| PYP-2-3 | 3,00% | K₃ [pN, 20°C]: | 15,6 |
| B-20-05 | 5,00% | V₀ [pN, 20°C]: | 2,09 |
| CC-3-V | 38,50% | γ₁ [mPa·s, 20 °C]: | 107 |
| PY-1-O4 | 10,00% | | |
| PY-3-02 | 3,00% | | |
| CCY-3-02 | 3,50% | | |

**Beispiel M96**

| | | | |
|---|---|---|---|
| CCY-3-01 | 7,00% | Klärpunkt [°C]: | 78,5 |
| CLY-3-02 | 10,00% | Δn [589 nm, 20°C]: | 0,1142 |
| CPY-2-02 | 10,00% | Δε [1 kHz, 20°C]: | -4,0 |
| CPY-3-02 | 10,00% | K₁ [pN, 20°C]: | 14,3 |
| PGIY-2-04 | 5,00% | K₃ [pN, 20°C]: | 14,8 |
| PYP-2-3 | 1,00% | V₀ [pN, 20°C]: | 45,66 |
| B-20-05 | 5,00% | γ₁ [mPa·s, 20 °C]: | 103 |
| CC-3-V | 39,50% | | |
| PY-1-O4 | 10,00% | | |
| Y-40-04 | 1,50% | | |
| CCY-3-02 | 1,00% | | |

**Beispiel M97**

| | | | |
|---|---|---|---|
| CCY-3-01 | 7,00% | Klärpunkt [°C]: | 74,5 |
| CLY-3-02 | 10,00% | Δn [589 nm, 20°C]: | 0,1014 |
| CPY-2-02 | 10,50% | Δε [1 kHz, 20°C]: | -3,7 |
| CPY-3-02 | 9,50% | γ₁ [mPa·s, 20 °C]: | 89 |
| B-20-05 | 3,50% | | |
| CC-3-V | | | 42,00% |
| PY-1-O4 | | | 10,00% |
| Y-40-04 | | | 3,50% |
| CCY-3-02 | | | 4,00% |

**Beispiel M98**

| | | | |
|---|---|---|---|
| CCY-3-01 | 7,00% | Klärpunkt [°C]: | 76,5 |
| CLY-3-02 | 10,00% | Δn [589 nm, 20°C]: | 0,1003 |
| CPY-2-02 | 10,50% | Δε [1 kHz, 20°C]: | -3,7 |
| CPY-3-02 | 10,00% | K₁ [pN, 20°C]: | 14,0 |
| B-20-05 | 5,00% | K₃ [pN, 20°C]: | 14,7 |
| CC-3-V | 43,50% | V₀ [pN, 20°C]: | 2,09 |
| PY-1-O4 | 6,00% | γ₁ [mPa·s, 20 °C]: | 89 |
| Y-4O-O4 | 4,00% | | |
| CCY-3-02 | 4,00% | | |

**Beispiel M99**

| | | | |
|---|---|---|---|
| B-20-05 | 5,00% | Klärpunkt [°C]: | 80 |
| CC-3-V | 37,00% | Δn [589 nm, 20°C]: | 0,1094 |
| CCP-V-1 | 4,50% | Δε [1 kHz, 20°C]: | -3,7 |
| CCY-3-O1 | 5,00% | ε_{∥} [1 kHz, 20°C]: | 3,7 |
| CCY-3-02 | 6,00% | ε_{⊥} [1 kHz, 20°C]: | 7,4 |
| CCY-4-02 | 5,00% | K₁ [pN, 20°C]: | 13,9 |
| CLY-3-02 | 8,00% | K₃ [pN, 20°C]: | 14,4 |
| CPY-2-02 | 9,50% | V₀ [pN, 20°C]: | 2,09 |
| PGIY-2-04 | 6,00% | γ₁ [mPa·s, 20 °C]: | 106 |
| PY-3-02 | 14,00% | | |

**Beispiel M100**

| | | | |
|---|---|---|---|
| CCY-3-01 | 7,00% | Klärpunkt [°C]: | 90 |
| CCY-4-02 | 4,00% | Δn [589 nm, 20°C]: | 0,1139 |
| CLY-3-02 | 10,00% | Δε [1 kHz, 20°C]: | -4,2 |
| CPY-2-02 | 10,00% | ε_{∥} [1 kHz, 20°C]: | 3,7 |
| CPY-3-02 | 10,00% | ε_{⊥} [1 kHz, 20°C]: | 7,9 |
| PYP-2-3 | 2,50% | K₁ [pN, 20°C]: | 16,2 |
| B-20-05 | 4,00% | K₃ [pN, 20°C]: | 17,0 |
| CC-3-V | 35,50% | V₀ [pN, 20°C]: | 2,12 |
| PY-1-O4 | 10,00% | γ₁ [mPa·s, 20 °C]: | 131 |
| PY-3-02 | 1,00% | | |
| CCY-3-02 | 6,00% | | |

**Beispiel M101**

| | | | |
|---|---|---|---|
| CC-3-V | 35,50% | Klärpunkt [°C]: | 75,1 |
| CC-3-V1 | 10,00% | Δn [589 nm, 20°C]: | 0,1096 |
| CCP-3-1 | 1,50% | Δε [1 kHz, 20°C]: | -3,2 |
| CLY-3-02 | 10,00% | ε_{∥} [1 kHz, 20°C]: | 3,5 |
| CLY-3-03 | 3,00% | ε_{⊥} [1 kHz, 20°C]: | 6,7 |
| CPY-2-02 | 9,00% | K₁ [pN, 20°C]: | 14,3 |
| CPY-3-02 | 10,50% | K₃ [pN, 20°C]: | 15,9 |
| PY-3-O2 | 16,50% | V₀ [pN, 20°C]: | 2,37 |
| PYP-2-3 | 1,00% | γ₁ [mPa·s, 20 °C]: | 84 |
| B-20-05 | 3,00% | | |

### Beispiel M102

Zur Herstellung einer PS-VA-Mischung werden 99,9 % der Mischung gemäß Beispiel M101 mit 0,1 % der polymerisierbaren Verbindung der Formel 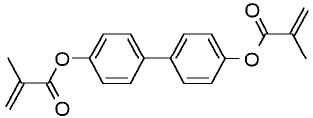 versetzt.

### Beispiel M103

Zur Herstellung einer PS-VA-Mischung werden 99,6 % der Mischung gemäß Beispiel M101 mit 0,4 % der polymerisierbaren Verbindung der Formel 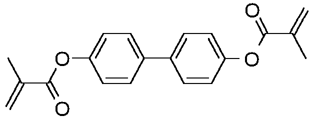 versetzt.

### Beispiel M104

Zur Herstellung einer PS-VA-Mischung werden 99,7 % der Mischung gemäß Beispiel M101 mit 0,3 % der polymerisierbaren Verbindung der Formel 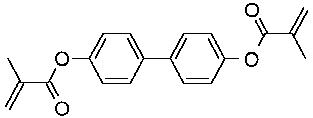 versetzt.

### Beispiel M105

Zur Herstellung einer PS-VA-Mischung werden 99,8 % der Mischung gemäß Beispiel M101 mit 0,2 % der polymerisierbaren Verbindung der Formel 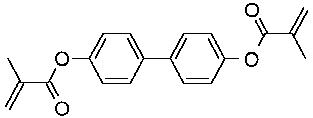 versetzt.

**Beispiel M106**

| | | | |
|---|---|---|---|
| CC-3-V | 36,50% | Klärpunkt [°C]: | 74,1 |
| CC-3-V1 | 6,00% | Δn [589 nm, 20°C]: | 0,1087 |
| CCY-3-01 | 7,00% | Δε [1 kHz, 20°C]: | -3,2 |
| CCY-3-02 | 9,00% | ε_{∥} [1 kHz, 20°C]: | 3,6 |
| CCY-5-02 | 2,00% | ε_{⊥} [1 kHz, 20°C]: | 6,8 |
| CLY-3-02 | 10,00% | K₁ [pN, 20°C]: | 14,1 |
| PY-1-O4 | 3,00% | K₃ [pN, 20°C]: | 15,7 |
| PY-3-02 | 14,00% | V₀ [pN, 20°C]: | 2,33 |
| PYP-2-3 | 9,50% | γ₁ [mPa·s, 20 °C]: | 87 |
| B-20-05 | 3,00% | | |

### Beispiel M107

Zur Herstellung einer PS-VA-Mischung werden 99,6 % der Mischung gemäß Beispiel M106 mit 0,4 % der polymerisierbaren Verbindung der Formel 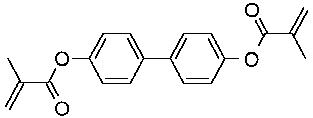 versetzt.

**Beispiel M108**

| | | | |
|---|---|---|---|
| CY-3-02 | 3,50% | Klärpunkt [°C]: | 74,3 |
| CCY-3-01 | 5,00% | Δn [589 nm, 20°C]: | 0,1096 |
| CCY-3-02 | 2,50% | Δε [1 kHz, 20°C]: | -4,0 |
| CPY-2-02 | 8,00% | ε_{∥} [1 kHz, 20°C]: | 4,0 |
| CPY-3-02 | 10,00% | ε_{⊥} [1 kHz, 20°C]: | 8,0 |
| PYP-2-3 | 4,00% | K₁ [pN, 20°C]: | 13,0 |
| CLY-3-02 | 7,00% | K₃ [pN, 20°C]: | 13,7 |
| CLY-3-03 | 4,00% | V₀ [pN, 20°C]: | 1,94 |
| Y-40-04 | 7,00% | γ₁ [mPa·s, 20 °C]: | 96 |
| PGIY-2-04 | 7,00% | | |
| B-20-05 | 4,00% | | |
| CC-3-V | 38,00% | | |

**Beispiel M109**

| | | | |
|---|---|---|---|
| CCY-3-01 | 6,00% | Klärpunkt [°C]: | 80 |
| CCY-3-02 | 8,00% | Δn [589 nm, 20°C]: | 0,1010 |
| CCY-4-02 | 4,00% | Δε [1 kHz, 20°C]: | -3,7 |
| CCY-5-O2 | 2,50% | ε_{∥} [1 kHz, 20°C]: | 3,6 |
| CLY-3-02 | 8,00% | ε_{⊥} [1 kHz, 20°C]: | 7,3 |
| CLY-3-03 | 4,00% | K₁ [pN, 20°C]: | 15,0 |
| PGIY-2-04 | 5,00% | K₃ [pN, 20°C]: | 16,3 |
| B-20-05 | 4,00% | V₀ [pN, 20°C]: | 2,20 |
| CC-3-V | 36,00% | γ₁ [mPa·s, 20 °C]: | 98 |
| CC-3-V1 | 7,50% | | |
| CY-3-02 | 2,00% | | |
| PY-3-02 | 12,00% | | |

**Beispiel M110**

| | | | |
|---|---|---|---|
| CCY-3-02 | 6,00% | Klärpunkt [°C]: | 80,4 |
| CCY-4-02 | 3,00% | Δn [589 nm, 20°C]: | 0,1014 |
| CLY-3-02 | 8,00% | Δε [1 kHz, 20°C]: | -3,7 |
| CLY-3-03 | 2,00% | ε_{∥} [1 kHz, 20°C]: | 3,6 |
| CPY-2-02 | 6,50% | ε_{⊥} [1 kHz, 20°C]: | 7,3 |
| CPY-3-02 | 8,00% | K₁ [pN, 20°C]: | 13,9 |
| PGIY-2-04 | 5,00% | K₃ [pN, 20°C]: | 15,8 |
| B-20-05 | 5,00% | V₀ [pN, 20°C]: | 2,20 |
| CC-3-V | 36,00% | γ₁ [mPa·s, 20 °C]: | 99 |
| CC-3-V1 | 7,50% | | |
| CY-3-02 | 11,00% | | |
| CY-5-02 | 2,00% | | |

**Beispiel M111**

| | | | |
|---|---|---|---|
| BCH-32 | 3,00% | Klärpunkt [°C]: | 74,7 |
| CC-3-V | 15,00% | Δn [589 nm, 20°C]: | 0,1086 |
| CC-3-V1 | 9,00% | Δε [1 kHz, 20°C]: | -3,2 |
| CCH-34 | 4,00% | ε_{∥} [1 kHz, 20°C]: | 3,6 |
| CCH-35 | 5,00% | ε_{⊥} [1 kHz, 20°C]: | 6,7 |
| CCP-3-1 | 8,00% | K₁ [pN, 20°C]: | 13,4 |
| CCP-3-3 | 5,00% | K₃ [pN, 20°C]: | 15,6 |
| CPY-2-02 | 10,50% | V₀ [pN, 20°C]: | 2,31 |
| CPY-3-02 | 10,50% | γ₁ [mPa·s, 20 °C]: | 109 |
| CY-3-02 | 15,00% | | |
| PY-3-02 | 12,00% | | |
| B-20-05 | 3,00% | | |

**Beispiel M112**

| | | | |
|---|---|---|---|
| BCH-32 | 2,50% | Klärpunkt [°C]: | 74,4 |
| CCP-3-1 | 8,00% | Δn [589 nm, 20°C]: | 0,1093 |
| CCY-3-01 | 8,00% | Δε [1 kHz, 20°C]: | -3,1 |
| CCY-3-02 | 11,00% | ε_{∥} [1 kHz, 20°C]: | 3,5 |
| CCY-5-02 | 1,50% | ε_{⊥} [1 kHz, 20°C]: | 6,6 |
| PGIY-2-04 | 5,00% | K₁ [pN, 20°C]: | 15,3 |
| B-20-05 | 4,00% | K₃ [pN, 20°C]: | 15,8 |
| CC-3-V | 5,00% | V₀ [pN, 20°C]: | 2,37 |
| CC-3-V1 | 7,00% | γ₁ [mPa·s, 20 °C]: | 105 |
| CCH-23 | 11,00% | | |
| CCH-34 | 9,00% | | |
| CCH-35 | 2,00% | | |
| CY-3-02 | 2,50% | | |
| PCH-301 | 1,00% | | |
| PP-1-2V1 | 4,50% | | |
| PY-3-02 | 18,00% | | |

**Beispiel M113**

| | | | |
|---|---|---|---|
| BCH-32 | 6,50 % | Klärpunkt [°C]: | 74,2 |
| CCP-3-1 | 8,00 % | Δn [589 nm, 20°C]: | 0,1086 |
| CCY-3-O2 | 11,00 % | Δε [1 kHz, 20°C]: | -3,2 |
| CCY-5-02 | 7,50 % | ε_{∥} [1 kHz, 20°C]: | 3,6 |
| PGIY-2-04 | 5,00 % | ε_{⊥} [1 kHz, 20°C]: | 6,8 |
| B-20-05 | 3,00 % | K₁ [pN, 20°C]: | 14,2 |
| CC-3-V | 10,00 % | K₃ [pN, 20°C]: | 15,9 |
| CC-3-V1 | 8,00 % | V₀ [pN, 20°C]: | 2,35 |
| CCH-23 | 10,00 % | γ₁ [mPa·s, 20 °C]: | 105 |
| CCH-34 | 3,00 % | | |
| CY-3-02 | 9,00 % | | |
| PCH-301 | 1,50 % | | |
| PP-1-2V1 | 1,50 % | | |
| PY-3-02 | 16,00 % | | |

**Beispiel M114**

| | | | |
|---|---|---|---|
| B-20-05 | 3,00 % | Klärpunkt [°C]: | 74,5 |
| CC-3-V | 15,00 % | Δn [589 nm, 20°C]: | 0,1092 |
| CC-3-V1 | 10,00 % | Δε [1 kHz, 20°C]: | -3,3 |
| CCH-34 | 9,00 % | ε_{∥} [1 kHz, 20°C]: | 3,6 |
| CCP-3-1 | 8,00 % | ε_{⊥} [1 kHz, 20°C]: | 6,9 |
| CCP-3-3 | 6,00 % | K₁ [pN, 20°C]: | 14,0 |
| CPY-2-02 | 8,50 % | K₃ [pN, 20°C]: | 15,7 |
| CPY-3-02 | 11,00 % | V₀ [pN, 20°C]: | 2,31 |
| CY-3-02 | 15,00 % | γ₁ [mPa·s, 20 °C]: | 102 |
| PGIY-2-04 | 4,00 % | | |
| PY-3-02 | 10,50 % | | |

**Beispiel M115**

| | | | |
|---|---|---|---|
| B-20-05 | 3,00 % | Klärpunkt [°C]: | 74,5 |
| BCH-32 | 2,00 % | Δn [589 nm, 20°C]: | 0,1090 |
| CC-3-V | 37,00 % | Δε [1 kHz, 20°C]: | -3,2 |
| CC-3-V1 | 6,50 % | ε_{∥} [1 kHz, 20°C]: | 3,6 |
| CCY-3-01 | 6,50 % | ε_{⊥} [1 kHz, 20°C]: | 6,8 |
| CCY-3-02 | 3,50 % | K₁ [pN, 20°C]: | 14,1 |
| CLY-3-02 | 10,00 % | K₃ [pN, 20°C]: | 15,9 |
| CPY-3-O2 | 10,50 % | V₀ [pN, 20°C]: | 2,35 |
| PY-3-02 | 18,00 % | γ₁ [mPa·s, 20 °C]: | 86 |
| PYP-2-3 | 3,00 % | | |

**Beispiel M116**

| | | | |
|---|---|---|---|
| B-20-05 | 3,00 % | Klärpunkt [°C]: | 74,6 |
| CC-3-V | 36,50 % | Δn [589 nm, 20°C]: | 0,1092 |
| CC-3-V1 | 9,00 % | Δε [1 kHz, 20°C]: | -3,2 |
| CCY-3-01 | 5,50 % | ε_{∥} [1 kHz, 20°C]: | 3,5 |
| CLY-3-02 | 10,00 % | ε_{⊥} [1 kHz, 20°C]: | 6,7 |
| CPY-2-02 | 6,50 % | K₁ [pN, 20°C]: | 14,2 |
| CPY-3-02 | 10,50 % | K₃ [pN, 20°C]: | 15,7 |
| PY-3-02 | 16,00 % | V₀ [pN, 20°C]: | 2,34 |
| PYP-2-3 | 3,00 % | γ₁ [mPa·s, 20 °C]: | 86 |

### Beispiel M117

Zur Herstellung einer PS-VA-Mischung werden 99,7 % der Mischung gemäß Beispiel M111 mit 0,3 % der polymerisierbaren Verbindung der Formel 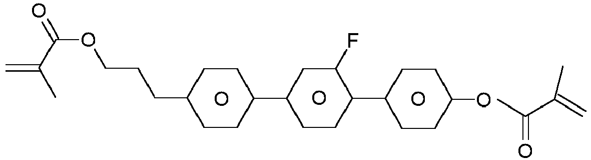 versetzt.

**Beispiel M118**

| | | | |
|---|---|---|---|
| B-20-05 | 4,00 % | Klärpunkt [°C]: | 74,6 |
| BCH-32 | 0,50 % | Δn [589 nm, 20°C]: | 0,1036 |
| CC-3-V | 33,00 % | Δε [1 kHz, 20°C]: | -3,4 |
| CC-3-V1 | 8,00 % | ε_{∥} [1 kHz, 20°C]: | 3,6 |
| CCH-301 | 1,00 % | ε_{⊥} [1 kHz, 20°C]: | 7,0 |
| CCY-3-1 | 2,50 % | K₁ [pN, 20°C]: | 13,4 |
| CCY-3-01 | 9,00 % | K₃ [pN, 20°C]: | 14,9 |
| CCY-4-02 | 5,00 % | V₀ [pN, 20°C]: | 2,21 |
| CPY-2-02 | 5,50 % | γ₁ [mPa·s, 20 °C]: | 92 |
| CPY-3-02 | 12,50 % | | |
| CY-3-02 | 7,00 % | | |
| PY-1-O4 | 1,50 % | | |
| PY-3-02 | 8,00 % | | |
| PYP-2-3 | 2,50 % | | |

### Beispiel M119

Zur Herstellung einer PS-VA-Mischung werden 99,7 % der Mischung gemäß Beispiel M118 mit 0,001 % Irganox 1076 und 0,3 % der polymerisierbaren Verbindung der Formel 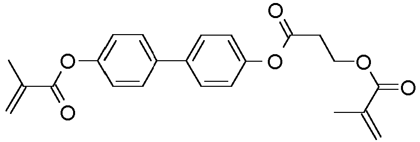 versetzt.

**Beispiel M120**

| | | | |
|---|---|---|---|
| CC-3-V | 34,50 % | Klärpunkt [°C]: | 74,7 |
| CC-3-V1 | 10,00 % | Δn [589 nm, 20°C]: | 0,1094 |
| CCP-3-1 | 1,00 % | Δε [1 kHz, 20°C]: | -3,2 |
| CLY-3-02 | 10,00 % | ε_{∥} [1 kHz, 20°C]: | 3,5 |
| CLY-3-03 | 3,00 % | ε_{⊥} [1 kHz, 20°C]: | 6,8 |
| CPY-2-02 | 8,00 % | K₁ [pN, 20°C]: | 13,9 |
| CPY-3-02 | 10,50 % | K₃ [pN, 20°C]: | 15,8 |
| CY-3-02 | 4,50 % | V₀ [pN, 20°C]: | 2,34 |
| PY-3-02 | 12,50 % | γ₁ [mPa·s, 20 °C]: | 87 |
| PYP-2-3 | 3,00 % | | |
| B-20-05 | 3,00 % | | |

### Beispiel M121

Zur Herstellung einer PS-VA-Mischung werden 99,6 % der Mischung gemäß Beispiel M120 mit 0,4 % der polymerisierbaren Verbindung der Formel 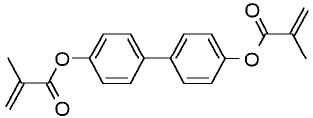 versetzt.

**Beispiel M122**

| | | | |
|---|---|---|---|
| B-20-05 | 3,00 % | Klärpunkt [°C]: | 74,8 |
| BCH-32 | 2,50 % | Δn [589 nm, 20°C]: | 0,1096 |
| CC-3-V | 42,00 % | Δε [1 kHz, 20°C]: | -3,2 |
| CC-3-V1 | 1,00 % | ε_{∥} [1 kHz, 20°C]: | 3,5 |
| CCY-3-02 | 9,00 % | ε_{⊥} [1 kHz, 20°C]: | 6,7 |
| CLY-3-02 | 10,00 % | K₁ [pN, 20°C]: | 14,1 |
| CPY-2-02 | 2,00 % | K₃ [pN, 20°C]: | 15,7 |
| CPY-3-02 | 10,50 % | V₀ [pN, 20°C]: | 2,34 |
| PY-3-02 | 17,00 % | γ₁ [mPa·s, 20 °C]: | 85 |
| PYP-2-3 | 3,00 % | | |

**Beispiel M123**

| | | | |
|---|---|---|---|
| CY-3-02 | 3,00 % | Klärpunkt [°C]: | 74,9 |
| CCY-3-01 | 5,00 % | Δn [589 nm, 20°C]: | 0,1032 |
| CCY-3-02 | 4,50 % | Δε [1 kHz, 20°C]: | -4,0 |
| CPY-2-02 | 8,00 % | ε_{∥} [1 kHz, 20°C]: | 3,9 |
| CPY-3-02 | 10,00 % | ε_{⊥} [1 kHz, 20°C]: | 7,9 |
| CLY-3-02 | 7,00 % | K₁ [pN, 20°C]: | 13,2 |
| CLY-3-03 | 4,00 % | K₃ [pN, 20°C]: | 13,9 |
| Y-40-04 | 7,00 % | V₀ [pN, 20°C]: | 1,96 |
| PGIY-2-04 | 7,00 % | γ₁ [mPa·s, 20 °C]: | 92 |
| B-20-05 | 4,00 % | | |
| CC-3-V | 40,50 % | | |

**Beispiel M124**

| | | | |
|---|---|---|---|
| CC-3-V | 37,50 % | Klärpunkt [°C]: | 75 |
| CCY-3-01 | 5,00 % | Δn [589 nm, 20°C]: | 0,1033 |
| CCY-3-02 | 13,75 % | Δε [1 kHz, 20°C]: | -3,6 |
| CCY-4-02 | 4,25 % | ε_{∥} [1 kHz, 20°C]: | 3,6 |
| CPY-3-02 | 13,50 % | ε_{⊥} [1 kHz, 20°C]: | 7,2 |
| CY-3-02 | 7,50 % | K₁ [pN, 20°C]: | 13,5 |
| PY-3-02 | 15,50 % | K₃ [pN, 20°C]: | 15,8 |
| PYP-2-3 | 3,00 % | V₀ [pN, 20°C]: | 1,17 |
| | | γ₁ [mPa·s, 20 °C]: | 98 |

### Beispiel M125

Zur Herstellung einer PS-VA-Mischung werden 99,7 % der Mischung gemäß Beispiel M124 mit 0,3 % der polymerisierbaren Verbindung der Formel 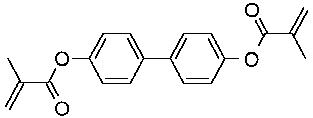 versetzt.

**Beispiel M126**

| | | | |
|---|---|---|---|
| CCP-V-1 | 2,00 % | Klärpunkt [°C]: | 75 |
| CCY-3-01 | 7,00 % | Δn [589 nm, 20°C]: | 0,1050 |
| CCY-3-02 | 7,00 % | Δε [1 kHz, 20°C]: | -3,7 |
| CCY-4-02 | 3,00 % | ε_{∥} [1 kHz, 20°C]: | 3,7 |
| CCY-5-02 | 1,50 % | ε_{⊥} [1 kHz, 20°C]: | 7,4 |
| CLY-3-02 | 8,00 % | K₁ [pN, 20°C]: | 14,8 |
| CLY-3-03 | 2,00 % | K₃ [pN, 20°C]: | 15,4 |
| PGIY-2-04 | 5,00 % | V₀ [pN, 20°C]: | 2,15 |
| B-20-05 | 5,00 % | γ₁ [mPa·s, 20 °C]: | 93 |
| CC-3-V | 34,00 % | | |
| CC-3-V1 | 8,00 % | | |
| PY-1-O4 | 3,50 % | | |
| PY-3-02 | 14,00 % | | |

### Beispiel M127

Zur Herstellung einer PS-VA-Mischung werden 99,7 % der Mischung gemäß Beispiel M126 mit 0,001 % Irganox 1076 und 0,3 % der polymerisierbaren Verbindung der Formel 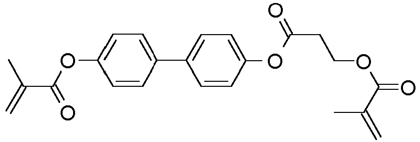 versetzt.

**Beispiel M128**

| | | | |
|---|---|---|---|
| CCP-V-1 | 2,00 % | Klärpunkt [°C]: | 75 |
| CCY-3-01 | 5,00 % | Δn [589 nm, 20°C]: | 0,1013 |
| CCY-3-O2 | 7,50 % | Δε [1 kHz, 20°C]: | -3,7 |
| CCY-4-02 | 3,50 % | ε_{∥} [1 kHz, 20°C]: | 3,7 |
| CLY-3-02 | 10,00 % | ε_{⊥} [1 kHz, 20°C]: | 7,4 |
| CLY-3-03 | 2,00 % | K₁ [pN, 20°C]: | 14,4 |
| PGIY-2-04 | 5,00 % | K₃ [pN, 20°C]: | 15,5 |
| B-20-05 | 5,00 % | V₀ [pN, 20°C]: | 2,15 |
| CC-3-V | 34,00 % | γ₁ [mPa·s, 20 °C]: | 91 |
| CC-3-V1 | 8,00 % | | |
| CY-3-02 | 6,00 % | | |
| PY-3-02 | 12,00 % | | |

**Beispiel M129**

| | | | |
|---|---|---|---|
| CCP-V-1 | 1,00 % | Klärpunkt [°C]: | 75 |
| CCY-3-01 | 7,00 % | Δn [589 nm, 20°C]: | 0,1081 |
| CCY-3-02 | 7,00 % | Δε [1 kHz, 20°C]: | -3,7 |
| CCY-4-02 | 3,00 % | ε_{∥} [1 kHz, 20°C]: | 3,7 |
| CCY-5-02 | 1,00 % | ε_{⊥} [1 kHz, 20°C]: | 7,4 |
| CLY-3-02 | 8,00 % | K₁ [pN, 20°C]: | 14,5 |
| CLY-3-03 | 2,00 % | K₃ [pN, 20°C]: | 15,2 |
| PGIY-2-04 | 5,00 % | V₀ [pN, 20°C]: | 2,14 |
| PYP-2-3 | 2,50 % | γ₁ [mPa·s, 20 °C]: | 93 |
| B-20-05 | 5,00 % | | |
| CC-3-V | 34,00 % | | |
| CC-3-V1 | 7,50 % | | |
| PY-1-O4 | 2,00 % | | |
| PY-3-02 | 15,00 % | | |

**Beispiel M130**

| | | | |
|---|---|---|---|
| CY-3-02 | 3,00 % | Klärpunkt [°C]: | 75,1 |
| CCY-3-01 | 5,00 % | Δn [589 nm, 20°C]: | 0,1021 |
| CCY-3-02 | 3,00 % | Δε [1 kHz, 20°C]: | -3,7 |
| CPY-2-02 | 8,00 % | ε_{∥} [1 kHz, 20°C]: | 3,8 |
| CPY-3-O2 | 10,00 % | ε_{⊥} [1 kHz, 20°C]: | 7,5 |
| CLY-3-02 | 7,00 % | K₁ [pN, 20°C]: | 13,3 |
| CLY-3-03 | 4,00 % | K₃ [pN, 20°C]: | 14,0 |
| Y-40-04 | 6,00 % | V₀ [pN, 20°C]: | 2,04 |
| PGIY-2-04 | 7,00 % | γ₁ [mPa·s, 20 °C]: | 87 |
| B-20-05 | 4,00 % | | |
| CC-3-V | 43,00 % | | |

**Beispiel M131**

| | | | |
|---|---|---|---|
| CCY-3-01 | 8,00 % | Klärpunkt [°C]: | 75,5 |
| CCY-4-02 | 3,00 % | Δn [589 nm, 20°C]: | 0,1024 |
| CLY-3-02 | 8,00 % | Δε [1 kHz, 20°C]: | -3,8 |
| CLY-3-03 | 4,00 % | ε_{∥} [1 kHz, 20°C]: | 3,7 |
| CPY-2-02 | 7,50 % | ε_{⊥} [1 kHz, 20°C]: | 7,5 |
| CPY-3-02 | 3,00 % | | |
| B-20-05 | 4,00 % | | |
| CC-3-V | 41,50 % | | |
| PY-1-O4 | 5,00 % | | |
| PY-3-02 | 11,50 % | | |
| CCY-3-02 | 4,50 % | | |

**Beispiel M132**

| | | | |
|---|---|---|---|
| CCY-3-01 | 6,50 % | Klärpunkt [°C]: | 79,5 |
| CLY-3-02 | 10,00 % | Δn [589 nm, 20°C]: | 0,1070 |
| CPY-2-02 | 10,00 % | Δε [1 kHz, 20°C]: | -3,9 |
| CPY-3-02 | 10,00 % | ε_{∥} [1 kHz, 20°C]: | 3,7 |
| PYP-2-3 | 5,50 % | ε_{⊥} [1 kHz, 20°C]: | 7,6 |
| B-20-05 | 4,00 % | K₁ [pN, 20°C]: | 13,9 |
| CC-3-V | 37,00 % | K₃ [pN, 20°C]: | 15,5 |
| CY-3-O2 | 14,00 % | V₀ [pN, 20°C]: | 2,09 |
| CCY-3-02 | 1,50 % | γ₁ [mPa·s, 20 °C]: | 104 |
| CY-5-02 | 1,50 % | | |

**Beispiel M133**

| | | | |
|---|---|---|---|
| CCP-V2-1 | 5,00 % | Klärpunkt [°C]: | 79,5 |
| CLY-3-02 | 10,00 % | Δn [589 nm, 20°C]: | 0,1079 |
| CPY-3-02 | 9,00 % | Δε [1 kHz, 20°C]: | -2,0 |
| PGIY-2-04 | 2,50 % | ε_{∥} [1 kHz, 20°C]: | 3,2 |
| PYP-2-3 | 8,00 % | ε_{⊥} [1 kHz, 20°C]: | 5,3 |
| PYP-2-4 | 5,00 % | K₁ [pN, 20°C]: | 14,4 |
| B-20-05 | 4,00 % | K₃ [pN, 20°C]: | 15,5 |
| CC-3-V | 43,00 % | V₀ [pN, 20°C]: | 2,92 |
| CC-3-V1 | 7,50 % | γ₁ [mPa·s, 20 °C]: | 75 |
| CY-3-02 | 6,00 % | | |

**Beispiel M134**

| | | | |
|---|---|---|---|
| CCY-3-01 | 1,00 % | Klärpunkt [°C]: | 79,5 |
| CLY-3-02 | 10,00 % | Δn [589 nm, 20°C]: | 0,1151 |
| CLY-3-03 | 1,50 % | Δε [1 kHz, 20°C]: | -4,0 |
| CPY-2-02 | 10,00 % | ε_{∥} [1 kHz, 20°C]: | 3,7 |
| CPY-3-02 | 10,00 % | ε_{⊥} [1 kHz, 20°C]: | 7,7 |
| PGIY-2-04 | 5,00 % | K₁ [pN, 20°C]: | 14,4 |
| PYP-2-3 | 6,00 % | K₃ [pN, 20°C]: | 15,7 |
| B-20-05 | 2,50 % | V₀ [pN, 20°C]: | 2,09 |
| CC-3-V | 27,00 % | γ₁ [mPa·s, 20 °C]: | 115 |
| CC-3-V1 | 8,00 % | | |
| CY-3-02 | 11,00 % | | |
| CY-5-02 | 6,50 % | | |
| CY-3-04 | 1,50 % | | |

**Beispiel M135**

| | | | |
|---|---|---|---|
| CLY-3-02 | 10,00 % | Klärpunkt [°C]: | 79,5 |
| CLY-3-03 | 2,00 % | Δn [589 nm, 20°C]: | 0,1157 |
| CPY-2-02 | 10,50 % | Δε [1 kHz, 20°C]: | -4,0 |
| CPY-3-02 | 9,50 % | ε_{∥} [1 kHz, 20°C]: | 3,7 |
| PGIY-2-04 | 5,00 % | ε_{⊥} [1 kHz, 20°C]: | 7,7 |
| PYP-2-3 | 5,50 % | K₁ [pN, 20°C]: | 14,6 |
| B-20-05 | 5,00 % | K₃ [pN, 20°C]: | 15,5 |
| CC-3-V | 30,00 % | V₀ [pN, 20°C]: | 2,07 |
| CC-3-V1 | 7,50 % | γ₁ [mPa·s, 20 °C]: | 111 |
| CY-3-02 | 11,00 % | | |
| CY-5-02 | | | 4,00 % |

**Beispiel M136**

| | | | |
|---|---|---|---|
| CCY-3-01 | 4,00 % | Klärpunkt [°C]: | 79,8 |
| CCY-3-02 | 8,50 % | Δn [589 nm, 20°C]: | 0,1013 |
| CCY-4-02 | 5,00 % | Δε [1 kHz, 20°C]: | -3,7 |
| CLY-3-02 | 10,00 % | ε_{∥} [1 kHz, 20°C]: | 3,6 |
| CLY-3-03 | 4,00 % | ε_{⊥} [1 kHz, 20°C]: | 7,3 |
| PGIY-2-04 | 5,00 % | K₁ [pN, 20°C]: | 14,7 |
| PYP-2-3 | 1,00 % | K₃ [pN, 20°C]: | 16,3 |
| B-20-05 | 5,00 % | V₀ [pN, 20°C]: | 2,20 |
| CC-3-V | 34,50 % | γ₁ [mPa·s, 20 °C]: | 97 |
| CC-3-V1 | 8,50 % | | |
| CY-3-02 | 5,00 % | | |
| PY-3-02 | 9,50 % | | |

**Beispiel M137**

| | | | |
|---|---|---|---|
| B-20-05 | 4,00 % | Klärpunkt [°C]: | 79,9 |
| CC-3-V | 32,00 % | Δn [589 nm, 20°C]: | 0,1036 |
| CCP-3-1 | 2,00 % | Δε [1 kHz, 20°C]: | -4,4 |
| CCY-3-O2 | 8,00 % | ε_{∥} [1 kHz, 20°C]: | 4,0 |
| CCY-4-02 | 8,00 % | ε_{⊥} [1 kHz, 20°C]: | 8,3 |
| CLY-3-02 | 6,50 % | K₁ [pN, 20°C]: | 13,4 |
| CLY-3-03 | 6,50 % | K₃ [pN, 20°C]: | 14,4 |
| CPY-2-02 | 8,00 % | V₀ [pN, 20°C]: | 1,92 |
| CPY-3-02 | 8,00 % | γ₁ [mPa·s, 20 °C]: | 89 |
| CY-3-02 | 2,00 % | | |
| PY-3-02 | 10,00 % | | |
| Y-40-04 | 5,00 % | | |

**Beispiel M138**

| | | | |
|---|---|---|---|
| B-20-05 | 4,00 % | Klärpunkt [°C]: | 79,9 |
| CC-3-V | 32,00 % | Δn [589 nm, 20°C]: | 0,1058 |
| CCP-3-1 | 2,00 % | Δε [1 kHz, 20°C]: | -4,6 |
| CCY-3-02 | 8,00 % | K₁ [pN, 20°C]: | 14,3 |
| CCY-4-02 | 8,00 % | K₃ [pN, 20°C]: | 15,1 |
| CLY-3-02 | 6,50 % | V₀ [pN, 20°C]: | 1,91 |
| CLY-3-03 | 6,50 % | γ₁ [mPa·s, 20 °C]: | 113 |
| CPY-2-02 | 8,00 % | | |
| CPY-3-02 | 8,00 % | | |
| CY-3-02 | 2,00 % | | |
| PY-3-02 | 10,00 % | | |
| Y-40-04 | 5,00 % | | |

**Beispiel M139**

| | | | |
|---|---|---|---|
| BCH-32 | 5,00 % | Klärpunkt [°C]: | 80,4 |
| CC-3-V | 32,50 % | Δn [589 nm, 20°C]: | 0,1099 |
| CCY-3-01 | 5,00 % | Δε [1 kHz, 20°C]: | -3,7 |
| CCY-3-02 | 8,00 % | ε_{∥} [1 kHz, 20°C]: | 3,9 |
| CCY-4-02 | 2,50 % | ε_{⊥} [1 kHz, 20°C]: | 7,6 |
| CLY-3-02 | 8,00 % | K₁ [pN, 20°C]: | 13,3 |
| CPY-2-O2 | 7,00 % | K₃ [pN, 20°C]: | 14,3 |
| CPY-3-02 | 10,00 % | V₀ [pN, 20°C]: | 2,05 |
| PGIY-2-04 | 7,00 % | γ₁ [mPa·s, 20 °C]: | 108 |
| PY-3-02 | 7,00 % | | |
| Y-40-04 | 8,00 % | | |

**Beispiel M140**

| | | | |
|---|---|---|---|
| B-20-05 | 5,00 % | Klärpunkt [°C]: | 80 |
| CC-3-V | 37,00 % | Δn [589 nm, 20°C]: | 0,1094 |
| CCP-V-1 | 4,50 % | Δε [1 kHz, 20°C]: | -3,7 |
| CCY-3-01 | 5,00 % | ε_{∥} [1 kHz, 20°C]: | 3,7 |
| CCY-3-02 | 6,00 % | ε_{⊥} [1 kHz, 20°C]: | 7,4 |
| CCY-4-02 | 5,00 % | K₁ [pN, 20°C]: | 13,9 |
| CLY-3-02 | 8,00 % | K₃ [pN, 20°C]: | 14,4 |
| CPY-2-02 | 9,50 % | V₀ [pN, 20°C]: | 2,09 |
| PGIY-2-04 | 6,00 % | γ₁ [mPa·s, 20 °C]: | 106 |
| PY-3-02 | 14,00 % | | |

**Beispiel M141**

| | | | |
|---|---|---|---|
| B-20-05 | 5,00 % | Klärpunkt [°C]: | 80,1 |
| BCH-32 | 7,00 % | Δn [589 nm, 20°C]: | 0,1096 |
| CC-3-V | 34,50 % | Δε [1 kHz, 20°C]: | -3,7 |
| CCP-V-1 | 2,00 % | ε_{∥} [1 kHz, 20°C]: | 3,9 |
| CCY-3-01 | 5,00 % | ε_{⊥} [1 kHz, 20°C]: | 7,6 |
| CCY-3-02 | 4,00 % | K₁ [pN, 20°C]: | 13,3 |
| CCY-4-02 | 2,00 % | K₃ [pN, 20°C]: | 13,7 |
| CLY-3-02 | 8,00 % | V₀ [pN, 20°C]: | 2,04 |
| CPY-2-02 | 10,00 % | γ₁ [mPa·s, 20 °C]: | 104 |
| CPY-3-02 | 7,00 % | | |
| PGIY-2-04 | 6,00 % | | |
| PY-3-02 | 2,00 % | | |
| Y-40-04 | 7,50 % | | |

### Beispiel M142

Zur Herstellung einer PS-VA-Mischung werden 99,7 % der Mischung gemäß Beispiel M140 mit 0,3 % der polymerisierbaren Verbindung der Formel 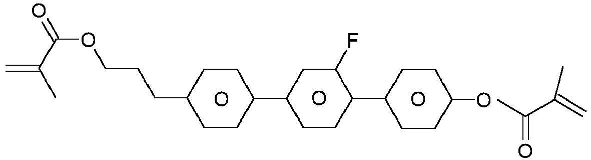 versetzt.

### Beispiel M143

Zur Herstellung einer PS-VA-Mischung werden 99,75 % der Mischung gemäß Beispiel M140 mit 0,25 % der polymerisierbaren Verbindung der Formel 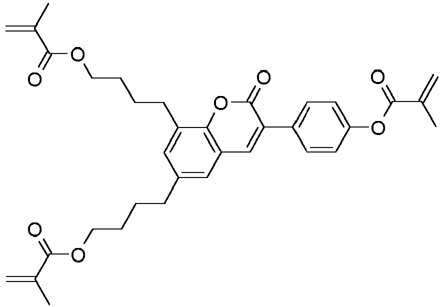 versetzt.

### Beispiel M144

Zur Herstellung einer PS-VA-Mischung werden 99,8 % der Mischung gemäß Beispiel M140 mit 0,2 % der polymerisierbaren Verbindung der Formel 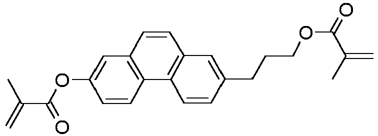 versetzt.

### Beispiel M145

Zur Herstellung einer PS-VA-Mischung werden 99,75 % der Mischung gemäß Beispiel M141 mit 0,3 % der polymerisierbaren Verbindung der Formel 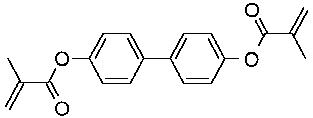 versetzt.

### Beispiel M146

Zur Herstellung einer PS-VA-Mischung werden 99,75 % der Mischung gemäß Beispiel M141 mit 0,25 % der polymerisierbaren Verbindung der Formel 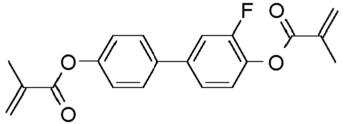 versetzt.

### Beispiel M147

Zur Herstellung einer PS-VA-Mischung werden 99,75 % der Mischung gemäß Beispiel M141 mit 0,3 % der polymerisierbaren Verbindung der Formel 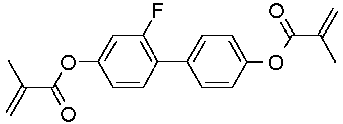 versetzt.

### Beispiel M148

Zur Herstellung einer PS-VA-Mischung werden 99,8 % der Mischung gemäß Beispiel M141 mit 0,2 % der polymerisierbaren Verbindung der Formel 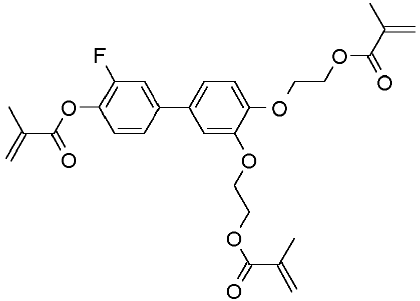 versetzt.

### Beispiel M149

Zur Herstellung einer PS-VA-Mischung werden 99,7 % der Mischung gemäß Beispiel M141 mit 0,3 % der polymerisierbaren Verbindung der Formel 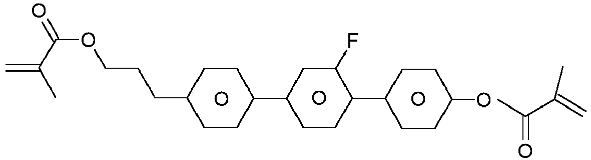 versetzt.

### Beispiel M150

Zur Herstellung einer PS-VA-Mischung werden 99,75 % der Mischung gemäß Beispiel M141 mit 0,25 % der polymerisierbaren Verbindung der Formel 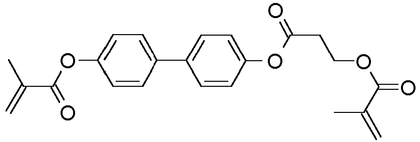 versetzt.

**Beispiel M151**

| | | | |
|---|---|---|---|
| CCY-3-01 | 7,00 % | Klärpunkt [°C]: | 80 |
| CCY-3-02 | 6,00 % | Δn [589 nm, 20°C]: | 0,1073 |
| CCY-4-02 | 6,50 % | Δε [1 kHz, 20°C]: | -3,9 |
| CCY-5-02 | 3,00 % | ε_{∥} [1 kHz, 20°C]: | 3,7 |
| CLY-3-02 | 10,00 % | ε_{⊥} [1 kHz, 20°C]: | 7,6 |
| PGIY-2-04 | 5,00 % | γ₁ [mPa·s, 20 °C]: | 90 |
| PYP-2-3 | 2,00 % | | |
| B-20-05 | 5,00 % | | |
| CC-3-V | 33,50 % | | |
| CC-3-V1 | 7,00 % | | |
| PY-1-O4 | 5,00 % | | |
| PY-3-02 | 10,00 % | | |

**Beispiel M152**

| | | | |
|---|---|---|---|
| CLY-3-02 | 10,00 % | Klärpunkt [°C]: | 80 |
| CLY-3-03 | 1,50 % | Δn [589 nm, 20°C]: | 0,1080 |
| CPY-2-02 | 4,00 % | Δε [1 kHz, 20°C]: | -2,4 |
| CPY-3-02 | 10,00 % | ε_{∥} [1 kHz, 20°C]: | 3,3 |
| PGIY-2-04 | 5,00 % | ε_{⊥} [1 kHz, 20°C]: | 5,7 |
| PYP-2-3 | 8,00 % | K₁ [pN, 20°C]: | 14,0 |
| B-20-05 | 4,50 % | K₃ [pN, 20°C]: | 15,8 |
| CC-3-V | 44,50 % | V₀ [pN, 20°C]: | 2,68 |
| CC-3-V1 | 8,00 % | γ₁ [mPa·s, 20 °C]: | 80 |
| CY-3-02 | 2,50 % | | |
| CY-5-02 | 2,00 % | | |

**Beispiel M153**

| | | | |
|---|---|---|---|
| CCY-3-02 | 7,00 % | Klärpunkt [°C]: | 80 |
| CCY-4-02 | 2,00 % | Δn [589 nm, 20°C]: | 0,1009 |
| CLY-3-02 | 8,00 % | Δε [1 kHz, 20°C]: | -3,7 |
| CLY-3-03 | 4,00 % | ε_{∥} [1 kHz, 20°C]: | 3,6 |
| CPY-2-02 | 3,00 % | ε_{⊥} [1 kHz, 20°C]: | 7,3 |
| CPY-3-02 | 8,00 % | K₁ [pN, 20°C]: | 14,4 |
| PGIY-2-04 | 5,00 % | K₃ [pN, 20°C]: | 16,4 |
| PYP-2-3 | 1,00 % | V₀ [pN, 20°C]: | 2,21 |
| B-20-05 | 5,00 % | γ₁ [mPa·s, 20 °C]: | 99 |
| CC-3-V | 35,00 % | | |
| CC-3-V1 | 8,00 % | | |
| CY-3-02 | 12,00 % | | |
| CY-5-02 | 2,00 % | | |

**Beispiel M154**

| | | | |
|---|---|---|---|
| CC-3-V | 37,50 % | Klärpunkt [°C]: | 80,2 |
| CCY-3-01 | 5,00 % | Δn [589 nm, 20°C]: | 0,1097 |
| CCY-3-02 | 3,00 % | Δε [1 kHz, 20°C]: | -3,9 |
| CCY-4-02 | 7,00 % | ε_{∥} [1 kHz, 20°C]: | 3,7 |
| CLY-3-02 | 8,00 % | ε_{⊥} [1 kHz, 20°C]: | 7,6 |
| CPY-2-O2 | 10,00 % | K₁ [pN, 20°C]: | 13,5 |
| CPY-3-02 | 8,00 % | K₃ [pN, 20°C]: | 14,5 |
| PY-1-O4 | 3,50 % | V₀ [pN, 20°C]: | 1,08 |
| PY-3-02 | 12,00 % | γ₁ [mPa·s, 20 °C]: | 110 |
| PGIY-2-04 | 2,00 % | | |
| B-20-05 | 4,00 % | | |

**Beispiel M155**

| | | | |
|---|---|---|---|
| B-20-05 | 4,00 % | Klärpunkt [°C]: | 80,4 |
| CC-3-V | 24,50 % | Δn [589 nm, 20°C]: | 0,1030 |
| CC-3-V1 | 5,00 % | Δε [1 kHz, 20°C]: | -4,4 |
| CCP-3-1 | 3,00 % | ε_{\|\|} [1 kHz, 20°C]: | 4,0 |
| CCY-3-02 | 8,00 % | ε_{⊥} [1 kHz, 20°C]: | 8,4 |
| CCY-4-02 | 8,00 % | K₁ [pN, 20°C]: | 13,3 |
| CLY-3-02 | 6,00 % | K₃ [pN, 20°C]: | 14,3 |
| CLY-3-03 | 6,00 % | V₀ [pN, 20°C]: | 1,91 |
| CPY-2-02 | 6,50 % | γ₁ [mPa·s, 20 °C]: | 96 |
| CPY-3-02 | 8,00 % | | |
| CY-3-02 | 8,00 % | | |
| PYP-2-3 | 5,00 % | | |
| Y-40-04 | 8,00 % | | |

**Beispiel M156**

| | | | |
|---|---|---|---|
| BCH-32 | 0,50 % | Klärpunkt [°C]: | 80,4 |
| CC-3-V | 37,00 % | Δn [589 nm, 20°C]: | 0,1195 |
| CCY-3-01 | 5,00 % | Δε [1 kHz, 20°C]: | -3,9 |
| CCY-3-02 | 3,50 % | ε_{∥} [1 kHz, 20°C]: | 3,8 |
| CLY-3-02 | 8,00 % | ε_{⊥} [1 kHz, 20°C]: | 7,7 |
| CPY-2-02 | 10,00 % | K₁ [pN, 20°C]: | 13,5 |
| CPY-3-02 | 10,00 % | K₃ [pN, 20°C]: | 14,5 |
| PY-3-02 | 14,00 % | V0 [pN, 20°C]: | 2,04 |
| PGIY-2-04 | 8,00 % | γ₁ [mPa·s, 20 °C]: | 114 |
| B-2O-O5 | 4,00 % | | |

**Beispiel M157**

| | | | |
|---|---|---|---|
| CCY-3-02 | 6,00 % | Klärpunkt [°C]: | 80,4 |
| CCY-4-02 | 3,00 % | Δn [589 nm, 20°C]: | 0,1014 |
| CLY-3-02 | 8,00 % | Δε [1 kHz, 20°C]: | -3,7 |
| CLY-3-03 | 2,00 % | ε_{∥} [1 kHz, 20°C]: | 3,6 |
| CPY-2-02 | 6,50 % | ε_{⊥} [1 kHz, 20°C]: | 7,3 |
| CPY-3-02 | 8,00 % | K₁ [pN, 20°C]: | 13,9 |
| PGIY-2-04 | 5,00 % | K₃ [pN, 20°C]: | 15,8 |
| B-20-05 | 5,00 % | V₀ [pN, 20°C]: | 2,20 |
| CC-3-V | 36,00 % | γ₁ [mPa·s, 20 °C]: | 99 |
| CC-3-V1 | 7,50 % | | |
| CY-3-02 | 11,00 % | | |
| CY-5-02 | 2,00 % | | |

**Beispiel M 158**

| | | | |
|---|---|---|---|
| CC-3-V | 25,00 % | Klärpunkt [°C]: | 80,5 |
| CC-3-V1 | 7,00 % | Δn [589 nm, 20°C]: | 0,0995 |
| CCP-3-1 | 7,00 % | Δε [1 kHz, 20°C]: | -3,8 |
| CCY-3-01 | 4,50 % | ε_{\|\|} [1 kHz, 20°C]: | 3,7 |
| CCY-3-02 | 6,50 % | ε_{⊥} [1 kHz, 20°C]: | 7,5 |
| CCY-4-02 | 5,00 % | K₁ [pN, 20°C]: | 13,7 |
| CCY-5-02 | 4,00 % | K₃ [pN, 20°C]: | 15,8 |
| CLY-3-02 | 8,00 % | V₀ [pN, 20°C]: | 2,12 |
| CY-3-02 | 12,00 % | γ₁ [mPa·s, 20 °C]: | 111 |
| CY-3-04 | 4,00 % | | |
| PY-3-02 | 7,00 % | | |
| PGIY-2-04 | 6,00 % | | |
| B-20-05 | 4,00 % | | |

**Beispiel M159**

| | | | |
|---|---|---|---|
| CC-3-V | 26,00 % | Klärpunkt [°C]: | 80,8 |
| CC-3-V1 | 7,00 % | Δn [589 nm, 20°C]: | 0,1099 |
| CCP-3-1 | 6,00 % | Δε [1 kHz, 20°C]: | -3,9 |
| CCY-3-01 | 2,00 % | ε_{\|\|} [1 kHz, 20°C]: | 3,7 |
| CCY-3-02 | 7,00 % | ε_{⊥} [1 kHz, 20°C]: | 7,6 |
| CCY-4-02 | 7,00 % | K₁ [pN, 20°C]: | 14,2 |
| CCY-5-02 | 4,00 % | K₃ [pN, 20°C]: | 15,6 |
| CLY-3-02 | 8,00 % | V₀ [pN, 20°C]: | 2,12 |
| CY-3-02 | 4,00 % | γ₁ [mPa·s, 20 °C]: | 121 |
| PY-1-O4 | 5,00 % | | |
| PY-3-02 | | | 12,00 % |
| PGIY-2-04 | | | 8,00 % |
| B-20-05 | | | 4,00 % |

**Beispiel M160**

| | | | |
|---|---|---|---|
| CCY-3-01 | 8,00 % | Klärpunkt [°C]: | 82 |
| CCY-3-02 | 1,00 % | Δn [589 nm, 20°C]: | 0,1081 |
| CLY-3-02 | 10,00 % | Δε [1 kHz, 20°C]: | -3,5 |
| CPY-2-02 | 9,50 % | ε_{∥} [1 kHz, 20°C]: | 3,6 |
| CPY-3-02 | 10,00 % | ε_{⊥} [1 kHz, 20°C]: | 7,2 |
| PGIY-2-04 | 5,00 % | K₁ [pN, 20°C]: | 14,4 |
| PYP-2-3 | 1,00 % | K₃ [pN, 20°C]: | 15,3 |
| B-20-05 | 5,00 % | V₀ [pN, 20°C]: | 2,20 |
| CC-3-V | 44,50 % | γ₁ [mPa·s, 20 °C]: | 95 |
| PY-1-O4 | 4,00 % | | |
| Y-40-04 | 2,00 % | | |

**Beispiel M161**

| | | | |
|---|---|---|---|
| B-20-05 | 4,00 % | Klärpunkt [°C]: | 84,6 |
| CC-3-V | 31,00 % | Δn [589 nm, 20°C]: | 0,1069 |
| CC-3-V1 | 4,00 % | Δε [1 kHz, 20°C]: | -3,8 |
| CCP-3-1 | 3,00 % | ε_{∥} [1 kHz, 20°C]: | 3,7 |
| CCY-3-02 | 8,50 % | ε_{⊥} [1 kHz, 20°C]: | 7,5 |
| CCY-4-02 | 6,00 % | K₁ [pN, 20°C]: | 14,1 |
| CLY-3-02 | 6,00 % | K₃ [pN, 20°C]: | 15,0 |
| CLY-3-03 | 6,00 % | V₀ [pN, 20°C]: | 2,09 |
| CPY-2-02 | 8,00 % | γ₁ [mPa·s, 20 °C]: | 88 |
| CPY-3-02 | 8,00 % | | |
| PY-3-02 | 8,00 % | | |
| PY-4-02 | 3,00 % | | |
| PYP-2-4 | 1,50 % | | |
| Y-40-04 | 3,00 % | | |

**Beispiel M162**

| | | | |
|---|---|---|---|
| CCY-3-02 | 10,00 % | Klärpunkt [°C]: | 85 |
| CCY-5-02 | 7,00 % | Δn [589 nm, 20°C]: | 0,1047 |
| CPY-2-02 | 10,00 % | Δε [1 kHz, 20°C]: | -3,4 |
| CPY-3-02 | 10,00 % | ε_{∥} [1 kHz, 20°C]: | 3,5 |
| PYP-2-3 | 5,50 % | ε_{⊥} [1 kHz, 20°C]: | 6,9 |
| B-20-05 | 4,00 % | K₁ [pN, 20°C]: | 14,6 |
| CC-3-V | 32,00 % | K₃ [pN, 20°C]: | 17,4 |
| CC-3-V1 | 10,00 % | V0 [pN, 20°C]: | 2,37 |
| CY-3-02 | 10,00 % | γ₁ [mPa·s, 20 °C]: | 108 |
| CY-5-02 | 1,50 % | | |

**Beispiel M163**

| | | | |
|---|---|---|---|
| CC-3-V | 35,00 % | Klärpunkt [°C]: | 86 |
| CCY-3-01 | 5,00 % | Δn [589 nm, 20°C]: | 0,1208 |
| CCY-3-02 | 7,50 % | Δε [1 kHz, 20°C]: | -4,2 |
| CLY-3-02 | 8,00 % | ε_{∥} [1 kHz, 20°C]: | 3,8 |
| CPY-2-02 | 10,00 % | ε_{⊥} [1 kHz, 20°C]: | 8,0 |
| CPY-3-02 | 10,00 % | K₁ [pN, 20°C]: | 14,3 |
| PY-3-02 | 12,50 % | K₃ [pN, 20°C]: | 15,6 |
| PGIY-2-04 | 8,00 % | V₀ [pN, 20°C]: | 2,04 |
| B-2O-O5 | 4,00 % | γ₁ [mPa·s, 20 °C]: | 129 |

**Beispiel M164**

| | | | |
|---|---|---|---|
| CC-3-V | 35,00 % | Klärpunkt [°C]: | 86,1 |
| CCY-3-01 | 5,00 % | Δn [589 nm, 20°C]: | 0,1103 |
| CCY-3-02 | 6,00 % | Δε [1 kHz, 20°C]: | -4,1 |
| CCY-3-03 | 2,00 % | ε_{∥} [1 kHz, 20°C]: | 3,7 |
| CCY-4-02 | 6,00 % | ε_{⊥} [1 kHz, 20°C]: | 7,8 |
| CLY-3-02 | 8,00 % | K₁ [pN, 20°C]: | 14,2 |
| CPY-2-02 | 10,00 % | K₃ [pN, 20°C]: | 15,7 |
| CPY-3-02 | 9,50 % | V₀ [pN, 20°C]: | 2,06 |
| PY-1-O4 | 3,50 % | γ₁ [mPa·s, 20 °C]: | 125 |
| PY-3-02 | 11,00 % | | |
| B-20-05 | 4,00 % | | |

**Beispiel M165**

| | | | |
|---|---|---|---|
| CCY-3-01 | 3,50 % | Klärpunkt [°C]: | 86,5 |
| CCY-3-02 | 4,50 % | Δn [589 nm, 20°C]: | 0,1053 |
| CLY-3-02 | 9,00 % | Δε [1 kHz, 20°C]: | -3,4 |
| CPY-2-02 | 10,50 % | ε_{∥} [1 kHz, 20°C]: | 3,5 |
| CPY-3-02 | 11,00 % | ε_{⊥} [1 kHz, 20°C]: | 6,9 |
| PYP-2-3 | 3,50 % | K₁ [pN, 20°C]: | 14,8 |
| CC-3-V | 32,00 % | K₃ [pN, 20°C]: | 17,8 |
| CC-3-V1 | 12,00 % | V0 [pN, 20°C]: | 2,41 |
| CY-3-02 | 10,00 % | γ₁ [mPa·s, 20 °C]: | 105 |
| B-20-05 | 4,00 % | | |

**Beispiel M166**

| | | | |
|---|---|---|---|
| CC-3-V | 22,50 % | Klärpunkt [°C]: | 97,2 |
| CC-3-V1 | 7,00 % | Δn [589 nm, 20°C]: | 0,1005 |
| CCP-3-1 | 2,00 % | Δε [1 kHz, 20°C]: | -4,6 |
| CCY-3-01 | 5,00 % | ε_{∥} [1 kHz, 20°C]: | 3,6 |
| CCY-3-02 | 7,00 % | ε_{⊥} [1 kHz, 20°C]: | 8,2 |
| CCY-3-03 | 5,00 % | K₁ [pN, 20°C]: | 15,8 |
| CCY-4-02 | 7,00 % | K₃ [pN, 20°C]: | 18,6 |
| CCY-5-02 | 5,00 % | V0 [pN, 20°C]: | 2,13 |
| CLY-3-02 | 8,00 % | γ₁ [mPa·s, 20 °C]: | 172 |
| CPY-3-02 | 8,00 % | | |
| CY-3-02 | 12,00 % | | |
| CY-5-02 | 4,50 % | | |
| PGIY-2-04 | 3,00 % | | |
| B-20-05 | 4,00 % | | |

**Beispiel M167**

| CC-3-V | 21,50 % | Klärpunkt [°C]: | 98,6 |
|---|---|---|---|
| CC-3-V1 | 7,00 % | Δn [589 nm, 20°C]: | 0,1103 |
| CCP-3-1 | 3,00 % | Δε [1 kHz, 20°C]: | -4,6 |
| CCY-3-01 | 5,00 % | ε_{∥} [1 kHz, 20°C]: | 3,7 |
| CCY-3-02 | 7,00 % | ε_{⊥} [1 kHz, 20°C]: | 8,3 |
| CCY-4-02 | 7,00 % | K₁ [pN, 20°C]: | 16,3 |
| CCY-5-02 | 4,00 % | K₃ [pN, 20°C]: | 18,7 |
| CLY-3-02 | 8,00 % | V₀ [pN, 20°C]: | 2,12 |
| CPY-3-02 | 10,50 % | γ₁ [mPa·s, 20 °C]: | 175 |
| CY-3-02 | 12,00 % | | |
| CY-5-02 | 3,00 % | | |
| PGIY-2-04 | 8,00 % | | |
| B-20-05 | 4,00 % | | |

**Beispiel M168**

| CC-3-V1 | 7,00 % | Klärpunkt [°C]: | 109 |
|---|---|---|---|
| CCP-3-1 | 10,00 % | Δn [589 nm, 20°C]: | 0,1012 |
| CCP-3-3 | 6,50 % | Δε [1 kHz, 20°C]: | -5,2 |
| CCY-3-01 | 5,00 % | ε_{∥} [1 kHz, 20°C]: | 3,7 |
| CCY-3-02 | 6,00 % | ε_{⊥} [1 kHz, 20°C]: | 8,9 |
| CCY-3-O3 | 7,50 % | K₁ [pN, 20°C]: | 18,2 |
| CCY-4-02 | 8,00 % | K₃ [pN, 20°C]: | 21,4 |
| CCY-5-02 | 4,00 % | V0 [pN, 20°C]: | 2,13 |
| CCY-3-1 | 8,00 % | γ₁ [mPa·s, 20 °C]: | 287 |
| CLY-3-02 | 8,00 % | | |
| CY-3-02 | 12,00 % | | |
| CY-3-04 | 14,00 % | | |
| B-20-05 | 4,00 % | | |

**Beispiel M169**

| CC-3-V | 10,75 % | Klärpunkt [°C]: | 111,8 |
|---|---|---|---|
| CC-3-V1 | 3,50 % | Δn [589 nm, 20°C]: | 0,1104 |
| CCP-3-1 | 7,50 % | Δε [1 kHz, 20°C]: | -5,2 |
| CCY-3-01 | 5,00 % | ε_{\|\|} [1 kHz, 20°C]: | 3,7 |
| CCY-3-02 | 7,50 % | ε_{⊥} [1 kHz, 20°C]: | 8,9 |
| CCY-4-02 | 7,50 % | K₁ [pN, 20°C]: | 17,9 |
| CCY-5-02 | 4,50 % | K₃ [pN, 20°C]: | 21,0 |
| CLY-3-02 | 8,00 % | V₀ [pN, 20°C]: | 2,13 |
| CPY-3-02 | 8,00 % | γ₁ [mPa·s, 20 °C]: | 267 |
| CY-3-02 | 12,00 % | | |
| CY-5-02 | 5,00 % | | |
| PGIY-2-04 | 4,00 % | | |
| B-20-05 | 4,00 % | | |
| CCP-3-3 | 1,75 % | | |
| CCY-3-03 | 3,00 % | | |
| CCY-2-1 | 4,00 % | | |
| CCY-3-1 | 4,00 % | | |

**Beispiel M170**

| CCP-3-1 | 12,00 % | Klärpunkt [°C]: | 126 |
|---|---|---|---|
| CCP-3-3 | 3,50 % | Δn [589 nm, 20°C]: | 0,1103 |
| CCY-3-01 | 5,00 % | Δε [1 kHz, 20°C]: | -5,8 |
| CCY-3-02 | 8,00 % | ε_{\|\|} [1 kHz, 20°C]: | 3,7 |
| CCY-3-03 | 6,00 % | ε_{⊥} [1 kHz, 20°C]: | 9,5 |
| CCY-4-02 | 8,00 % | K₁ [pN, 20°C]: | 20,3 |
| CCY-5-02 | 5,00 % | K₃ [pN, 20°C]: | 23,8 |
| CCY-2-1 | 8,00 % | V0 [pN, 20°C]: | 2,14 |
| CCY-3-1 | 8,00 % | γ₁ [mPa·s, 20 °C]: | 422 |
| CLY-3-02 | 8,00 % | | |
| CPY-3-02 | 5,50 % | | |
| CY-3-02 | 12,00 % | | |
| CY-5-02 | 7,00 % | | |
| B-2O-O5 | 4,00 % | | |

**Beispiel M171**

| CC-3-V | 12,50 % | Klärpunkt [°C]: | 110,3 |
|---|---|---|---|
| CC-3-V1 | 6,50 % | Δn [589 nm, 20°C]: | 0,1100 |
| CCP-3-1 | 12,50 % | Δε [1 kHz, 20°C]: | -4,8 |
| CCY-3-O1 | 5,00 % | ε_{\|\|} [1 kHz, 20°C]: | 3,6 |
| CCY-3-O2 | 7,50 % | ε_{⊥} [1 kHz, 20°C]: | 8,4 |
| CCY-4-O2 | 3,00 % | K₁ [pN, 20°C]: | 17,9 |
| CCY-5-O2 | 3,50 % | K₃ [pN, 20°C]: | 20,8 |
| CLY-2-O4 | 5,00 % | V₀ [pN, 20°C]: | 2,22 |
| CLY-3-O2 | 7,00 % | γ₁ [mPa·s, 20 °C]: | 233 |
| CLY-3-O3 | 6,00 % | | |
| CPY-3-O2 | 8,00 % | | |
| CY-3-O2 | 12,00 % | | |
| CY-5-O2 | 4,00 % | | |
| PGIY-2-O4 | 3,50 % | | |
| B-2O-O5 | 4,00 % | | |

### Beispiel M172

Zur Herstellung einer PS-VA-Mischung werden 99,7 % der Mischung gemäß Beispiel M171 mit 0,3 % der polymerisierbaren Verbindung der Formel 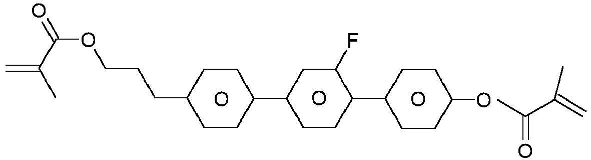 versetzt.

### Beispiel M173

Zur Herstellung einer PS-VA-Mischung werden 99,75 % der Mischung gemäß Beispiel M171 mit 0,25 % der polymerisierbaren Verbindung der Formel 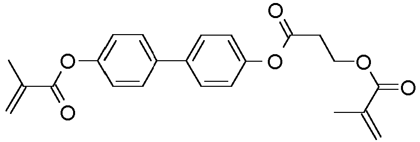 versetzt.

**Beispiel M174**

| CC-3-V | 14,00 % | Klärpunkt [°C]: | 111 |
|---|---|---|---|
| CC-3-V1 | 6,00 % | Δn [589 nm, 20°C]: | 0,1102 |
| CCP-3-1 | 7,00 % | Δε [1 kHz, 20°C]: | -5,2 |
| CCY-3-O1 | 5,00 % | ε_{∥} [1 kHz, 20°C]: | 3,7 |
| CCY-3-O2 | 8,00 % | ε_{⊥} [1 kHz, 20°C]: | 8,9 |
| CCY-4-O2 | 5,50 % | K₁ [pN, 20°C]: | 18,5 |
| CCY-5-O2 | 2,00 % | K₃ [pN, 20°C]: | 20,5 |
| CLY-2-O4 | 7,00 % | V₀ [pN, 20°C]: | 2,10 |
| CLY-3-O2 | 8,00 % | γ₁ [mPa·s, 20 °C]: | 241 |
| CLY-3-O3 | 7,00 % | | |
| CPY-3-O2 | 10,00 % | | |
| CY-3-O2 | 12,00 % | | |
| CY-5-O2 | 2,50 % | | |
| PGIY-2-O4 | 2,00 % | | |
| B-2O-O5 | 4,00 % | | |

**Beispiel M175**

| CC-3-V | 12,25 % | Klärpunkt [°C]: | 110,9 |
|---|---|---|---|
| CC-3-V1 | 6,00 % | Δn [589 nm, 20°C]: | 0,1101 |
| CCP-3-1 | 11,00 % | Δε [1 kHz, 20°C]: | -5,0 |
| CCY-3-O1 | 5,00 % | ε_{\|\|} [1 kHz, 20°C]: | 3,6 |
| CCY-3-O2 | 7,75 % | ε_{⊥} [1 kHz, 20°C]: | 8,6 |
| CCY-4-O2 | 5,00 % | K₁ [pN, 20°C]: | 18,5 |
| CCY-5-O2 | 3,50 % | K₃ [pN, 20°C]: | 20,8 |
| CLY-2-O4 | 5,00 % | V₀ [pN, 20°C]: | 2,16 |
| CLY-3-O2 | 7,00 % | γ₁ [mPa·s, 20 °C]: | 240 |
| CLY-3-O3 | 6,00 % | | |
| CPY-3-O2 | 8,00 % | | |
| CY-3-O2 | 12,00 % | | |
| CY-5-O2 | 4,25 % | | |
| PGIY-2-O4 | 3,25 % | | |
| B-2O-O5 | 4,00 % | | |

### Beispiel M176

Zur Herstellung einer PS-VA-Mischung werden 99,8 % der Mischung gemäß Beispiel M174 mit 0,2 % der polymerisierbaren Verbindung der Formel 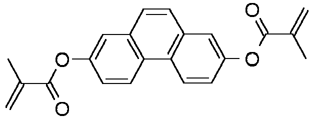 versetzt.

### Beispiel M177

Zur Herstellung einer PS-VA-Mischung werden 99,75 % der Mischung gemäß Beispiel M174 mit 0,25 % der polymerisierbaren Verbindung der Formel 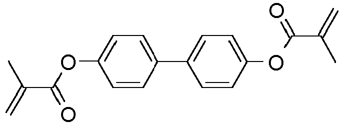 versetzt.

### Beispiel M178

Zur Herstellung einer PS-VA-Mischung werden 99,7 % der Mischung gemäß Beispiel M174 mit 0,3 % der polymerisierbaren Verbindung der Formel 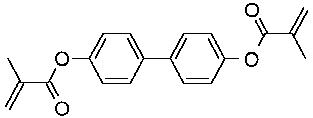 versetzt.

### Beispiel M179

Zur Herstellung einer PS-VA-Mischung werden 99,7 % der Mischung gemäß Beispiel M174 mit 0,3 % der polymerisierbaren Verbindung der Formel 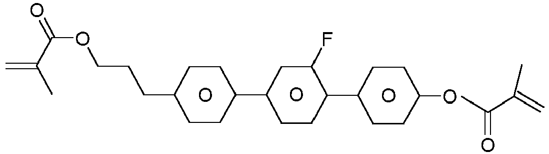 versetzt.

### Beispiel M180

Zur Herstellung einer PS-VA-Mischung werden 99,75 % der Mischung gemäß Beispiel M174 mit 0,25 % der polymerisierbaren Verbindung der Formel 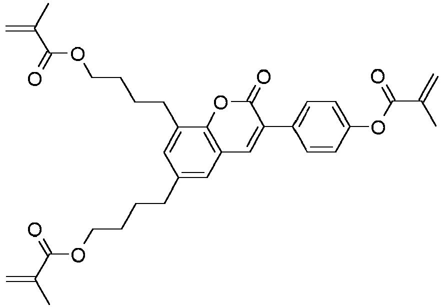 versetzt.

### Beispiel M181

Zur Herstellung einer PS-VA-Mischung werden 99,7 % der Mischung gemäß Beispiel M174 mit 0,3 % der polymerisierbaren Verbindung der Formel 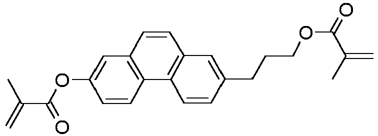 versetzt.

### Beispiel M182

Zur Herstellung einer PS-VA-Mischung werden 99,75 % der Mischung gemäß Beispiel M175 mit 0,25 % der polymerisierbaren Verbindung der Formel 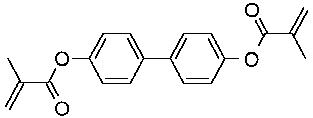 versetzt.

### Beispiel M183

Zur Herstellung einer PS-VA-Mischung werden 99,75 % der Mischung gemäß Beispiel M175 mit 0,25 % der polymerisierbaren Verbindung der Formel 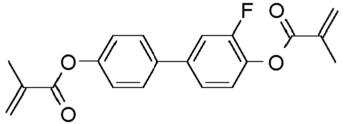 versetzt.

### Beispiel M184

Zur Herstellung einer PS-VA-Mischung werden 99,75 % der Mischung gemäß Beispiel M175 mit 0,25 % der polymerisierbaren Verbindung der Formel 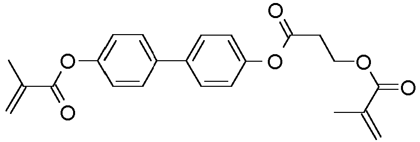 versetzt.

### Beispiel M185

Zur Herstellung einer PS-VA-Mischung werden 99,75 % der Mischung gemäß Beispiel M175 mit 0,25 % der polymerisierbaren Verbindung der Formel 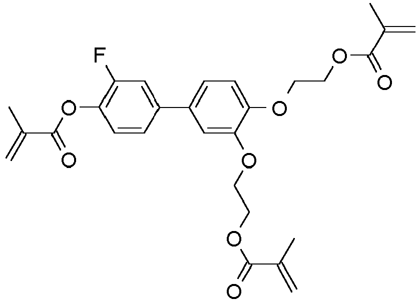 versetzt.

### Beispiel M186

Zur Herstellung einer PS-VA-Mischung werden 99,8 % der Mischung gemäß Beispiel M175 mit 0,3 % der polymerisierbaren Verbindung der Formel 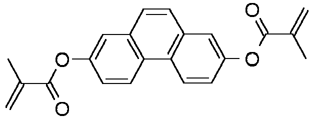 versetzt.

### Beispiel M187

Zur Herstellung einer PS-VA-Mischung werden 99,7 % der Mischung gemäß Beispiel M166 mit 0,3 % der polymerisierbaren Verbindung der Formel 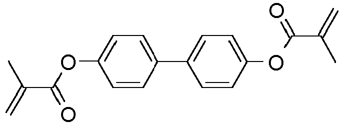 versetzt.

### Beispiel M188

Zur Herstellung einer PS-VA-Mischung werden 99,7 % der Mischung gemäß Beispiel M167 mit 0,3 % der polymerisierbaren Verbindung der Formel 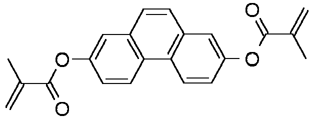 versetzt.

### Beispiel M189

Zur Herstellung einer PS-VA-Mischung werden 99,7 % der Mischung gemäß Beispiel M167 mit 0,3 % der polymerisierbaren Verbindung der Formel 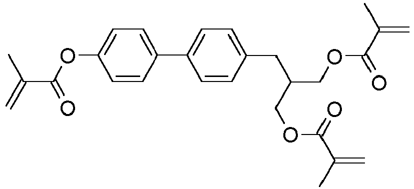 versetzt.

### Beispiel M190

Zur Herstellung einer PS-VA-Mischung werden 99,7 % der Mischung gemäß Beispiel M167 mit 0,3 % der polymerisierbaren Verbindung der Formel 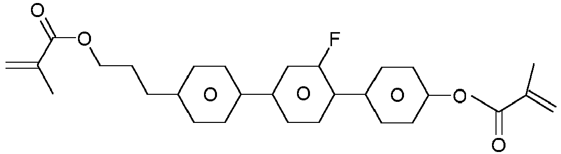 versetzt.

**Beispiel M191**

| CC-3-V | 12,00 % | Klärpunkt [°C]: | 111,6 |
|---|---|---|---|
| CC-3-V1 | 5,50 % | Δn [589 nm, 20°C]: | 0,1101 |
| CCP-3-1 | 9,50 % | Δε [1 kHz, 20°C]: | -5,2 |
| CCY-3-O1 | 5,00 % | ε_{∥} [1 kHz, 20°C]: | 3,7 |
| CCY-3-O2 | 8,00 % | ε_{⊥} [1 kHz, 20°C]: | 8,9 |
| CCY-4-O2 | 7,00 % | K₁ [pN, 20°C]: | 18,6 |
| CCY-5-O2 | 3,50 % | K₃ [pN, 20°C]: | 20,6 |
| CLY-2-O4 | 5,00 % | V₀ [pN, 20°C]: | 2,11 |
| CLY-3-O2 | 7,00 % | γ₁ [mPa·s, 20 °C]: | 252 |
| CLY-3-O3 | 6,00 % | | |
| CPY-3-O2 | 8,00 % | | |
| CY-3-O2 | 12,00 % | | |
| CY-5-O2 | 4,50 % | | |
| PGIY-2-O4 | 3,00 % | | |
| B-2O-O5 | 4,00 % | | |

### Beispiel M192

Zur Herstellung einer PS-VA-Mischung werden 99,7 % der Mischung gemäß Beispiel M191 mit 0,3 % der polymerisierbaren Verbindung der Formel 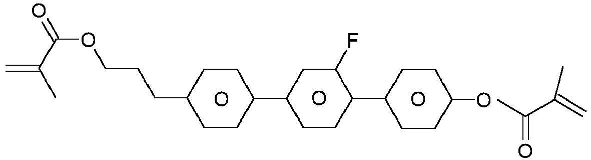 versetzt.

### Beispiel M193

Zur Herstellung einer PS-VA-Mischung werden 99,75 % der Mischung gemäß Beispiel M191 mit 0,25 % der polymerisierbaren Verbindung der Formel 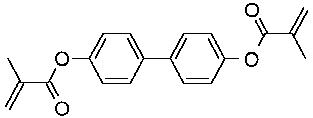 versetzt.

### Beispiel M194

Zur Herstellung einer PS-VA-Mischung werden 99,75 % der Mischung gemäß Beispiel M191 mit 0,25 % der polymerisierbaren Verbindung der Formel 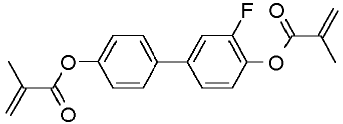 versetzt.

**Beispiel M195**

| CC-3-V | 45,50 % | Klärpunkt [°C]: | 73,5 |
|---|---|---|---|
| CCY-3-O1 | 5,50 % | Δn [589 nm, 20 °C]: | 0,1013 |
| CCY-3-O2 | 11,00 % | Δε [1 kHz, 20 °C]: | -3,4 |
| CPY-2-O2 | 9,00 % | K₁ [pN, 20 °C]: | 13,1 |
| CPY-3-O2 | 10,50 % | K₃ [pN, 20 °C]: | 14,4 |
| CY-3-O2 | 3,00 % | γ₁ [mPa·s, 20 °C]: | 80 |
| PY-3-O2 | 11,00 % | | |
| B-3O-O5 | 2,50 % | | |
| B-3O-O4 | 2,50 % | | |

### Beispiel M196

Zur Herstellung einer PS-VA-Mischung werden 99,75 % der Mischung gemäß Beispiel M195 mit 0,25 % der polymerisierbaren Verbindung der Formel 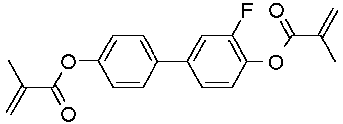 versetzt.

### Beispiel M197

Zur Herstellung einer PS-VA-Mischung werden 99,7 % der Mischung gemäß Beispiel M195 mit 0,3 % der polymerisierbaren Verbindung der Formel 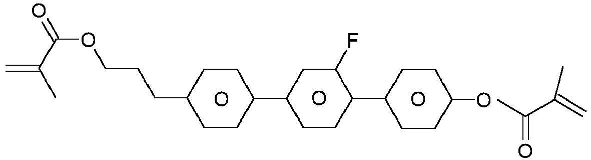 versetzt.

**Beispiel M198**

| CC-3-V | 41,00 % | Klärpunkt [°C]: | 73,9 |
|---|---|---|---|
| CCY-3-O1 | 6,00 % | Δn [589 nm, 20 °C]: | 0,1011 |
| CCY-3-O2 | 11,00 % | Δε [1 kHz, 20 °C]: | -3,6 |
| CCY-4-O2 | 6,00 % | K₁ [pN, 20 °C]: | 13,2 |
| CPY-3-O2 | 4,00 % | K₃ [pN, 20 °C]: | 14,6 |
| CPY-3-O2 | 11,00 % | γ₁ [mPa·s, 20 °C]: | 90 |
| CY-3-O2 | 3,00 % | | |
| PY-3-O2 | 12,00 % | | |
| B-3-O3 | 6,00 % | | |

### Beispiel M199

Zur Herstellung einer PS-VA-Mischung werden 99,75 % der Mischung gemäß Beispiel M198 mit 0,25 % der polymerisierbaren Verbindung der Formel 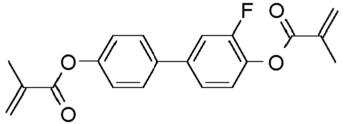 versetzt.

### Beispiel M200

Zur Herstellung einer PS-VA-Mischung werden 99,75 % der Mischung gemäß Beispiel M198 mit 0,25 % der polymerisierbaren Verbindung der Formel 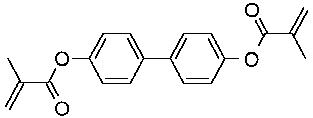 versetzt.

## Patentansprüche

1. Flüssigkristallines Medium auf der Basis eines Gemisches von polaren Verbindungen, **dadurch gekennzeichnet, dass** es mindestens eine Verbindung der Formel I, 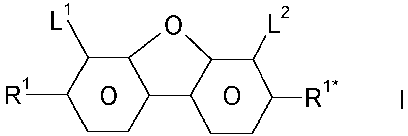 worin
R¹ und R^{1*} jeweils unabhängig voneinander einen Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -OCF₂- , -CH=CH-, 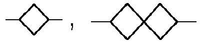 , -O-, -CO-O- , -O-CO- so ersetzt sein können, dass O-Atome nicht direkt miteinander verknüpft sind, und worin auch ein oder mehrere H-Atome durch Halogen ersetzt sein können,
L¹ und L² jeweils unabhängig voneinander F, Cl, CF₃ oder CHF₂,
bedeuten,
enthält, und
zusätzlich eine oder mehrere Verbindungen der Formeln O-1 bis O-18, 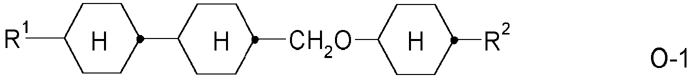 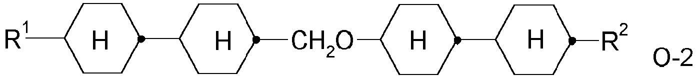 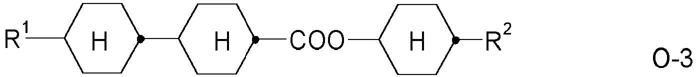 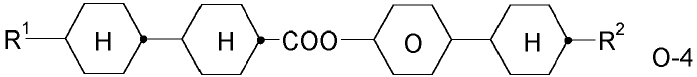 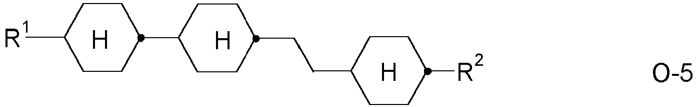 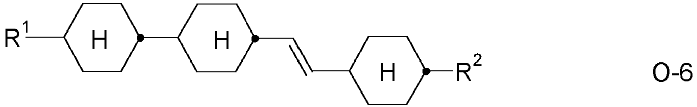 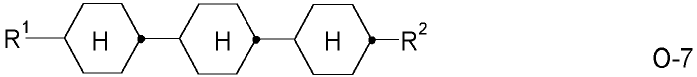 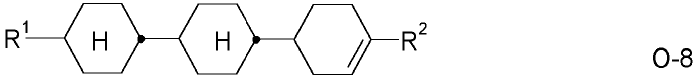 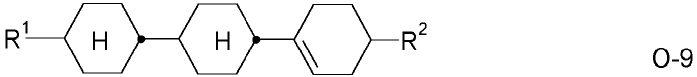 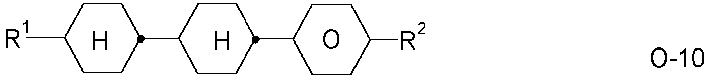 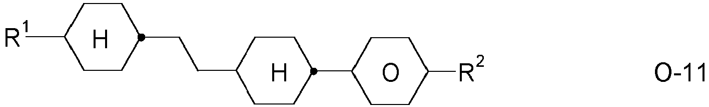 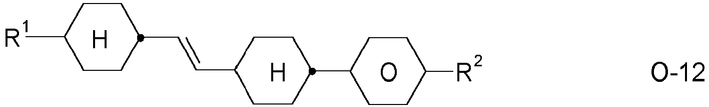 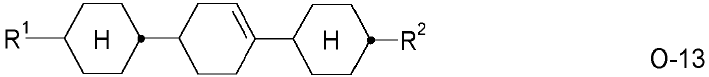 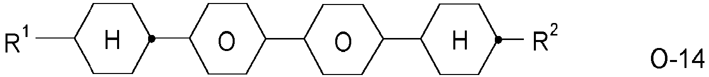 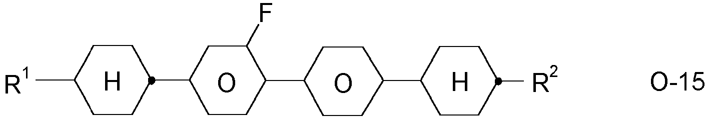 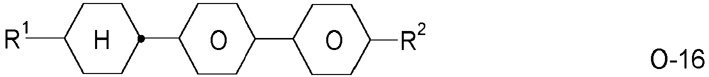 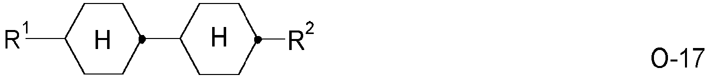 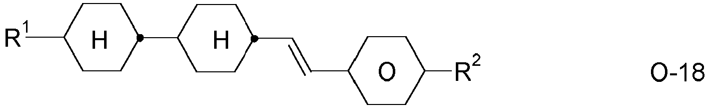
worin
R¹ und R² jeweils unabhängig voneinander H, einen unsubstituierten, einen einfach durch CN oder CF₃ oder mindestens einfach durch Halogen substituierten Alkyl- oder Alkenylrest mit bis zu 15 C-Atomen, wobei in diesen Resten auch eine oder mehrere CH₂-Gruppen durch -O-, -S-, 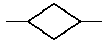 , -C≡C-, -CF₂O-, -OCF₂-, -OC-O- oder-O-CO- so ersetzt sein können, dass O-Atome nicht direkt miteinander verknüpft sind,
bedeuten,
enthält.

2. Flüssigkristallines Medium nach Anspruch 1, **dadurch gekennzeichnet, dass** das Medium mindestens eine Verbindung der Formeln I-1 bis I-10, 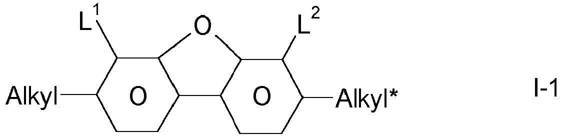 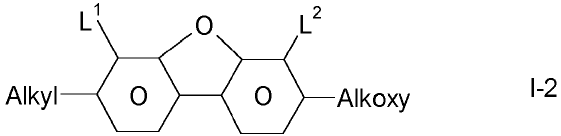 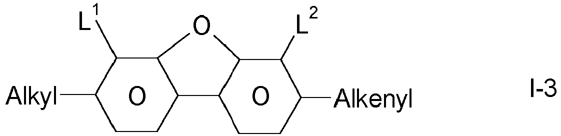 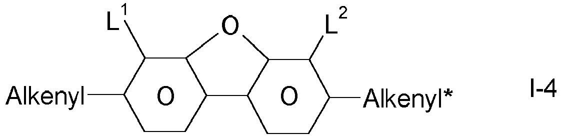 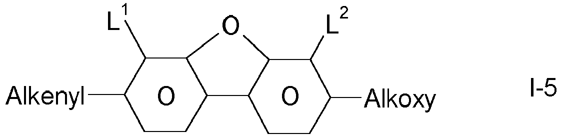 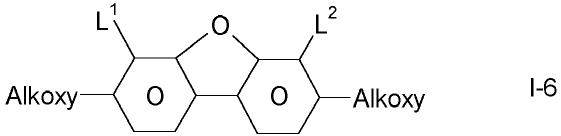 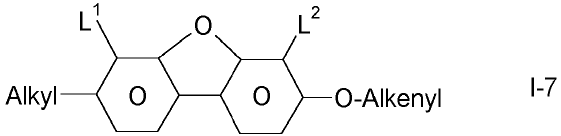 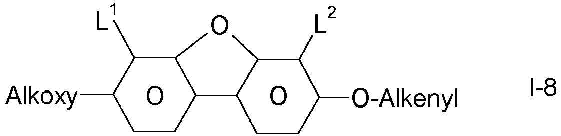 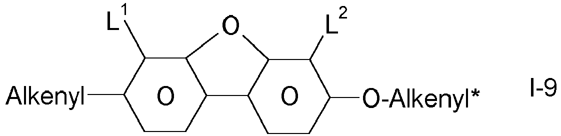 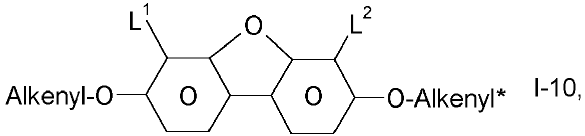 worin
Alkyl und Alkyl* jeweils unabhängig voneinander einen geradkettigen Alkylrest mit 1-6 C-Atomen,
Alkenyl und Alkenyl* jeweils unabhängig voneinander einen geradkettigen Alkenylrest mit 2-6 C-Atomen,
Alkoxy und Alkoxy* jeweils unabhängig voneinander einen geradkettigen Alkoxyrest mit 1-6 C-Atomen, und
L¹ und L² jeweils unabhängig voneinander F, Cl, CF₃ oder CHF₂,
bedeuten, enthält.

3. Flüssigkristallines Medium nach Anspruch 1 oder 2, **dadurch gekennzeichnet, dass** das Medium mindestens eine Verbindung aus der Gruppe der Verbindungen der Formeln I-2.1 bis I-2.49, I-6.1 bis I-6.28 und I-6B.1 bis I-6B.3, 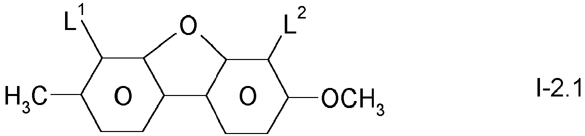 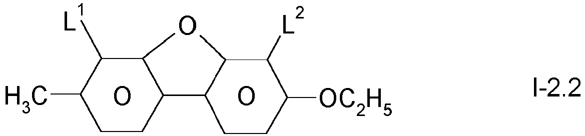 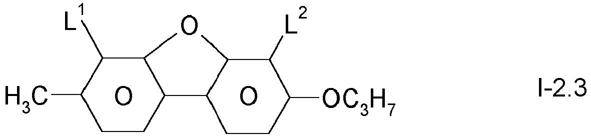 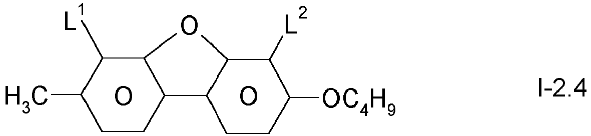 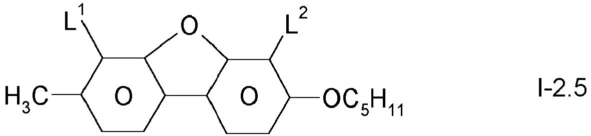 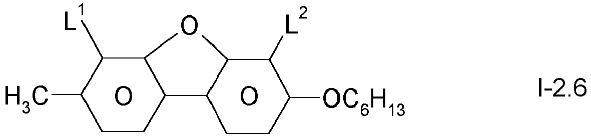 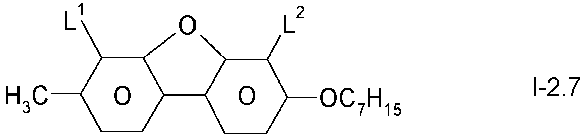 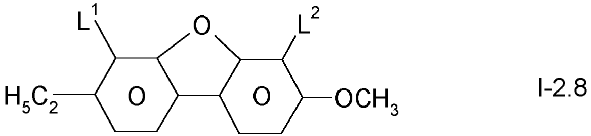 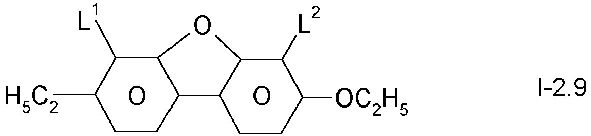 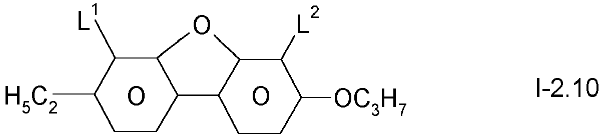 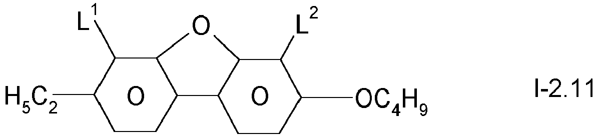 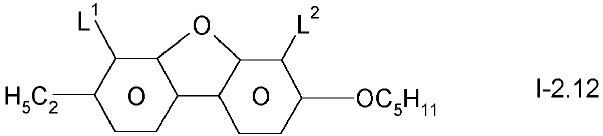 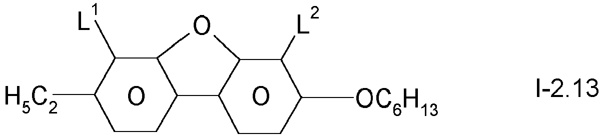 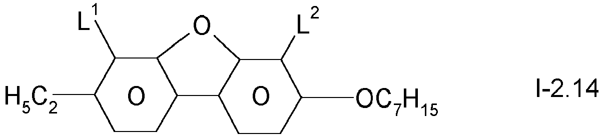 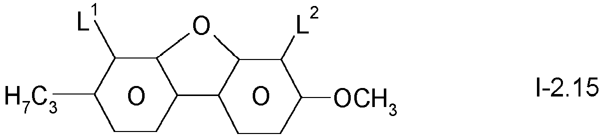 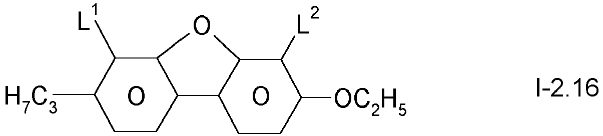 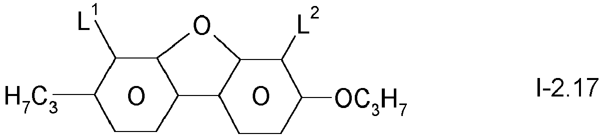 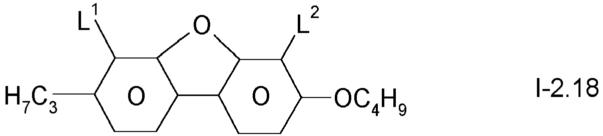 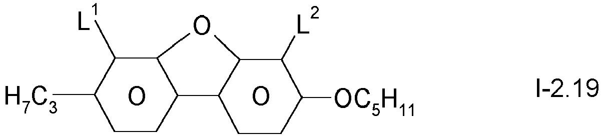 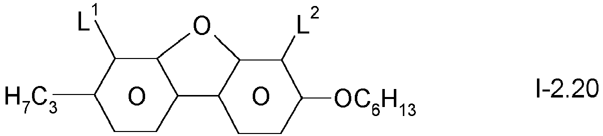 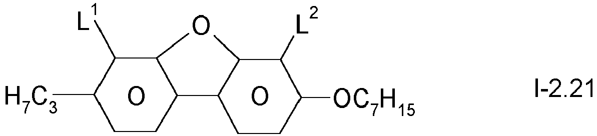 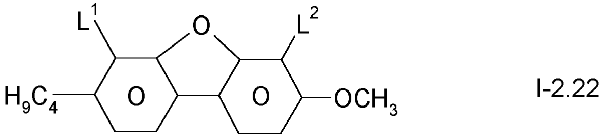 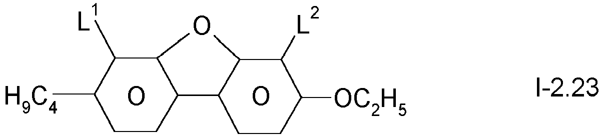 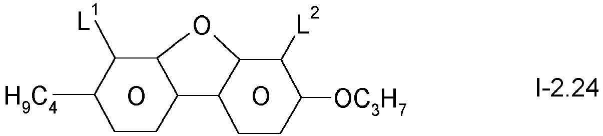 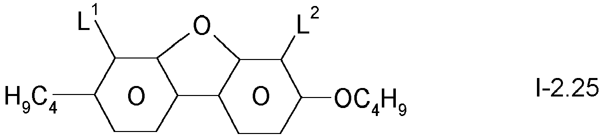 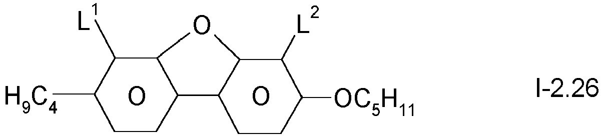 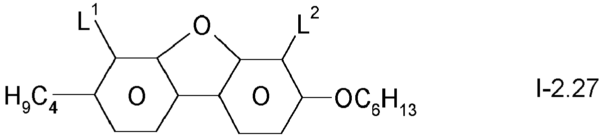 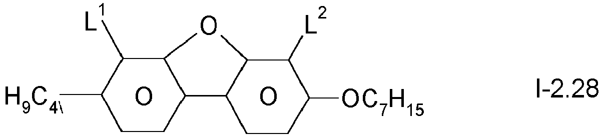 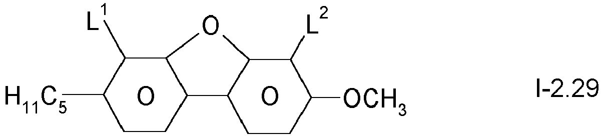 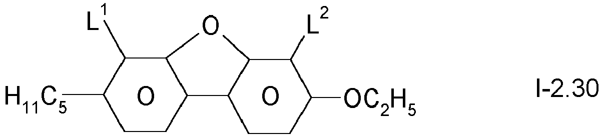 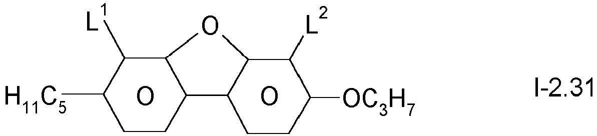 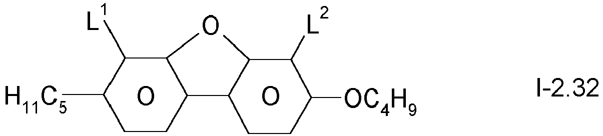 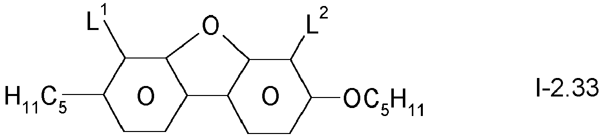 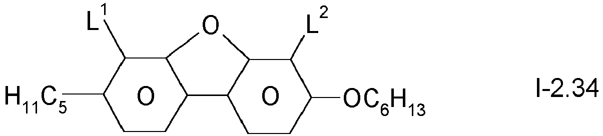 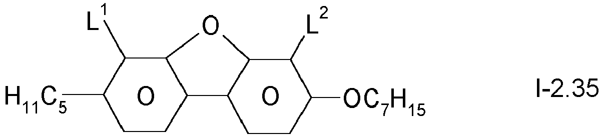 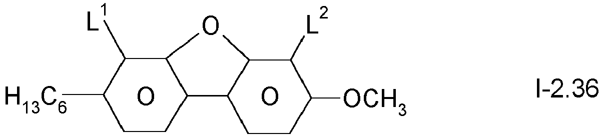 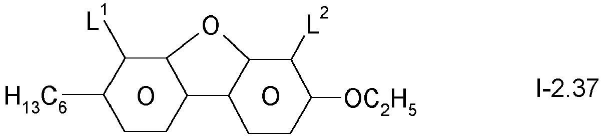 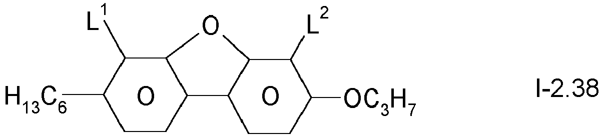 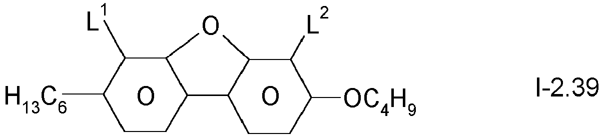 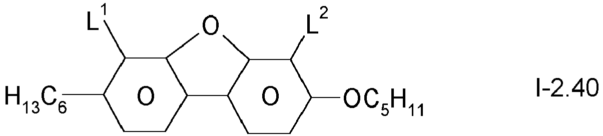 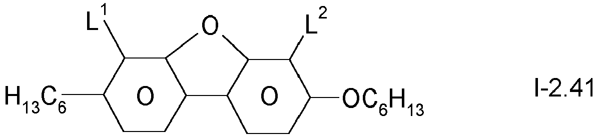 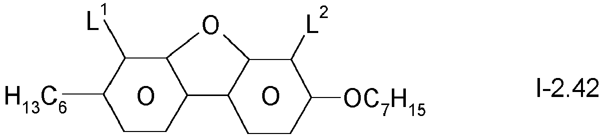 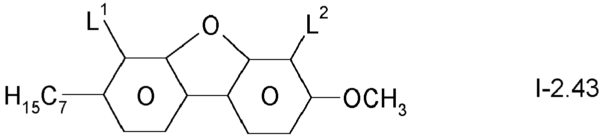 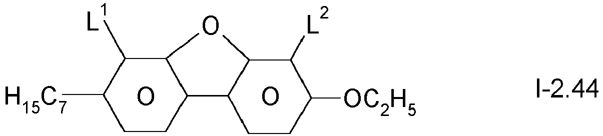 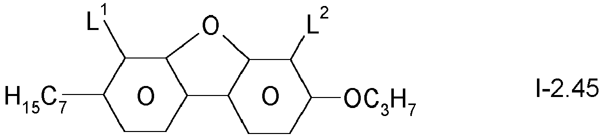 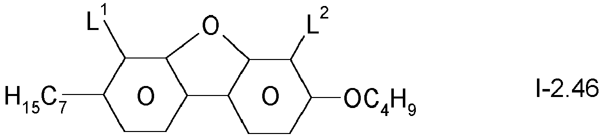 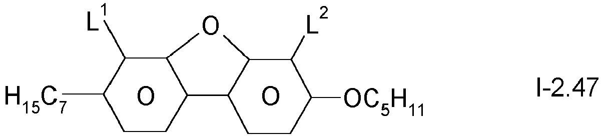 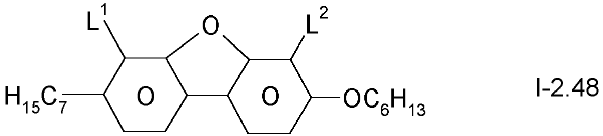 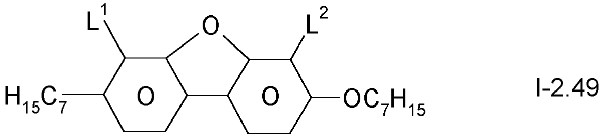 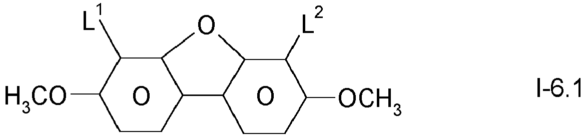 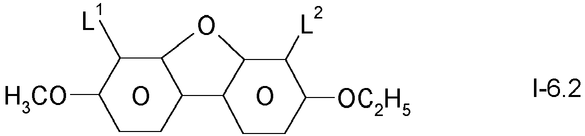 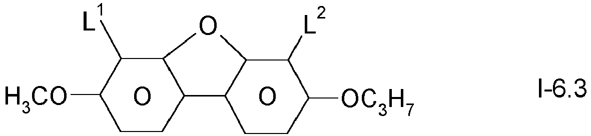 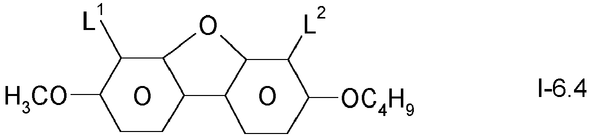 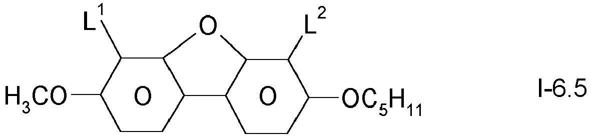 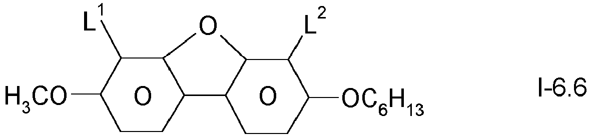 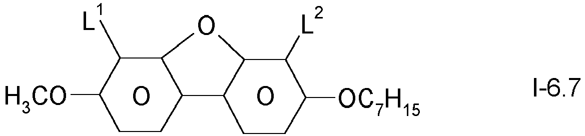 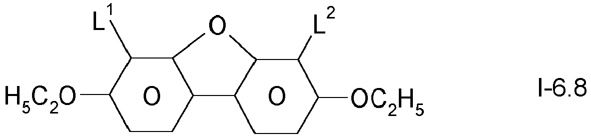 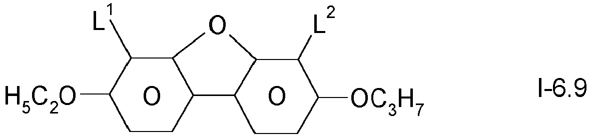 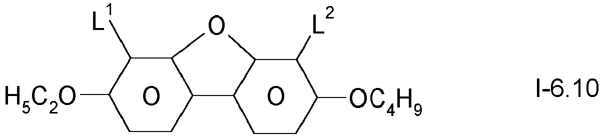 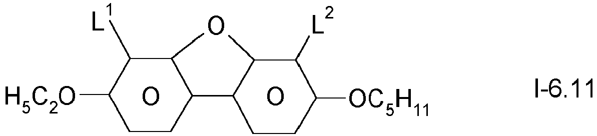 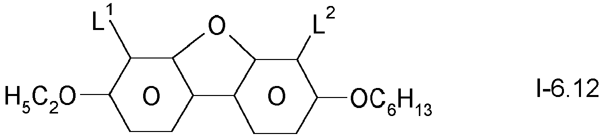 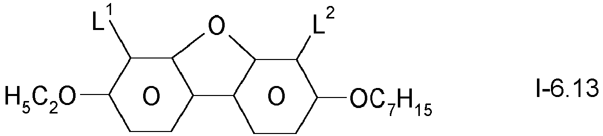 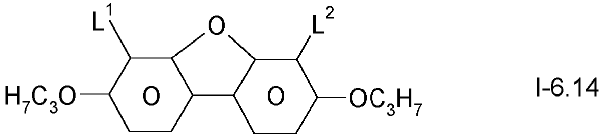 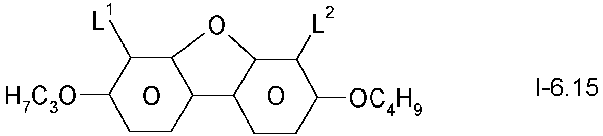 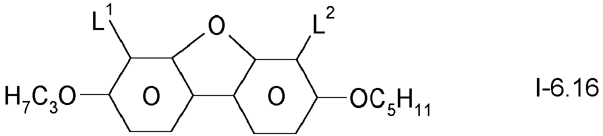 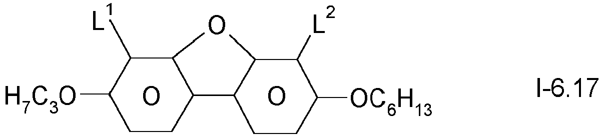 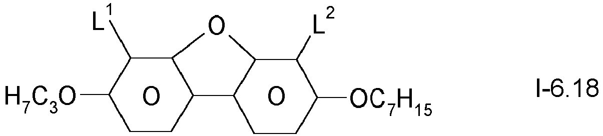 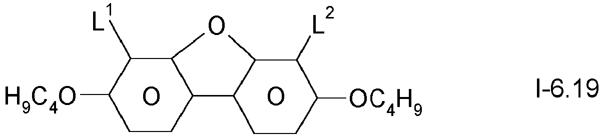 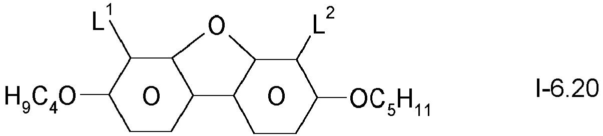 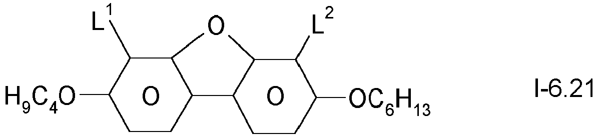 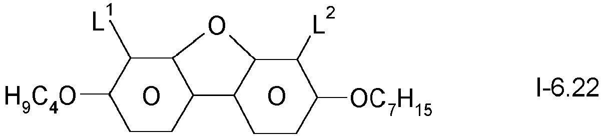 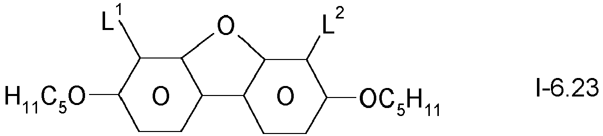 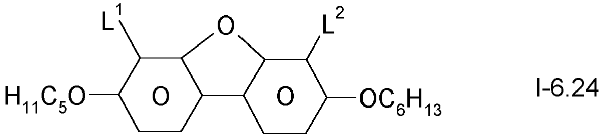 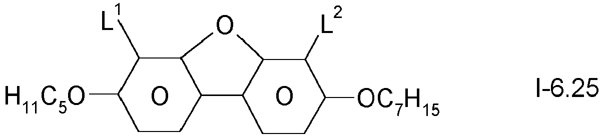 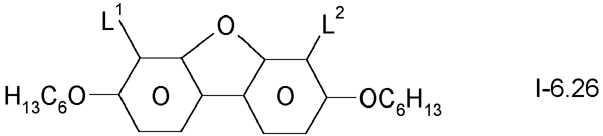 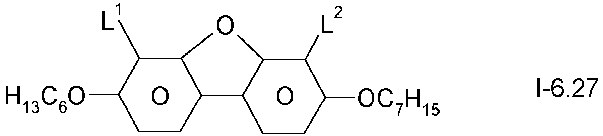 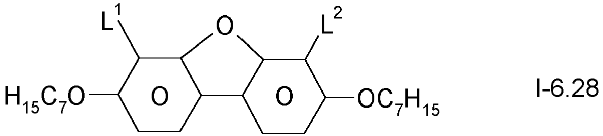 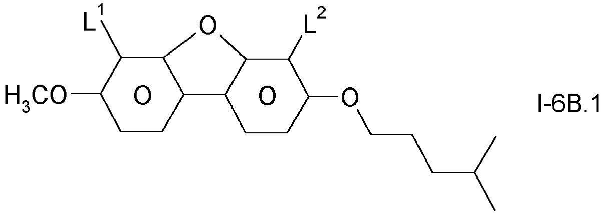 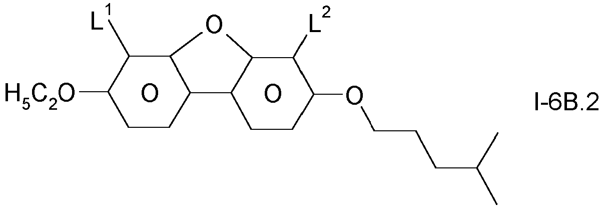 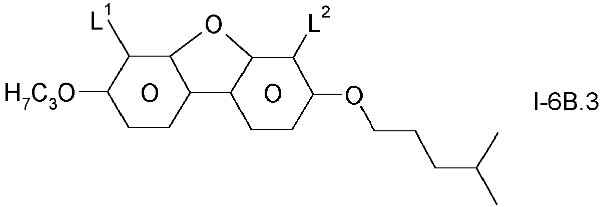 worin L¹ und L² die in Anspruch 1 angegebenen Bedeutungen haben, enthält.

4. Flüssigkristallines Medium nach einem oder mehreren der Ansprüche 1 bis 3, **dadurch gekennzeichnet, dass** in der Formel I L¹ und L² jeweils F bedeuten.

5. Flüssigkristallines Medium nach einem oder mehreren der Ansprüche 1 bis 4, **dadurch gekennzeichnet, dass** es zusätzlich eine oder mehrere Verbindungen ausgewählt aus der Gruppe der Verbindungen der Formeln IIA, IIB und IIC, 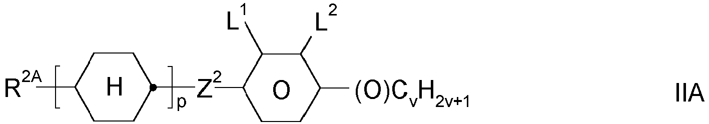 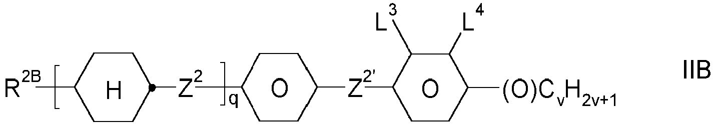 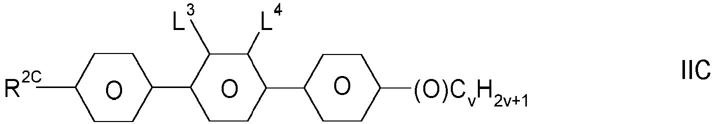 worin
R^{2A}, R^{2B} und R^{2C} jeweils unabhängig voneinander H, einen unsubstituierten, einen einfach durch CN oder CF₃ oder mindestens einfach durch Halogen substituierten Alkyl- oder Alkenylrest mit bis zu 15 C-Atomen, wobei in diesen Resten auch eine oder mehrere CH₂-Gruppen durch -O-, -S-, 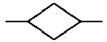 , -C≡C-, -CF₂O-, -OCF₂-, -OC-O- oder-O-CO- so ersetzt sein können, dass O-Atome nicht direkt miteinander verknüpft sind,
L¹⁻⁴ jeweils unabhängig voneinander F oder Cl,
Z² und Z²' jeweils unabhängig voneinander Einfachbindung, -CH₂CH₂-, -CH=CH-, -CF₂O-, -OCF₂-, -CH₂O-, -OCH₂-, -COO-, -OCO-, -C₂F₄-, -CF=CF-, -CH=CHCH₂O-,
p 0, 1 oder 2,
q 0 oder 1, und
v 1 bis 6
bedeuten, enthält.

6. Flüssigkristallines Medium nach einem oder mehreren der Ansprüche 1 bis 5, **dadurch gekennzeichnet, dass** das Medium zusätzlich eine oder mehrere Verbindungen der Formel III, 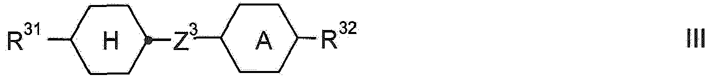 worin
R³¹ und R³² jeweils unabhängig voneinander einen geradkettigen Alkyl-, Alkoxyalkyl- oder Alkoxyrest mit bis zu 12 C-Atomen, und 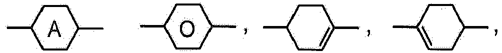 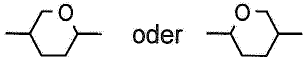
Z³ Einfachbindung, -CH₂CH₂-, -CH=CH-, -CF₂O-, -OCF₂-,-CH₂O-, -OCH₂-, -COO-, -OCO-, -C₂F₄-, -C₄H9-, -CF=CF-
bedeuten,
enthält.

7. Flüssigkristallines Medium nach einem oder mehreren der Ansprüche 1 bis 6, **dadurch gekennzeichnet, dass** das Medium zusätzlich eine oder mehrere Verbindungen der Formeln L-1 bis L-11, 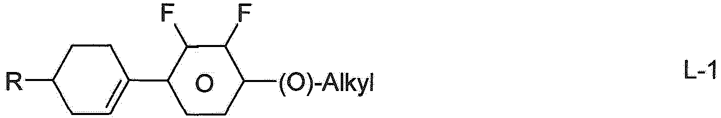 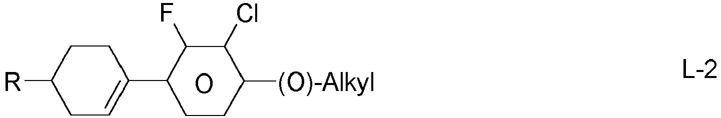 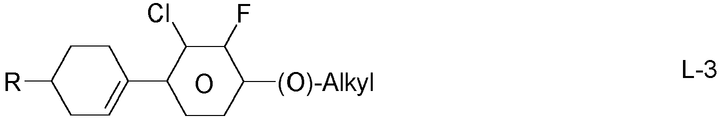 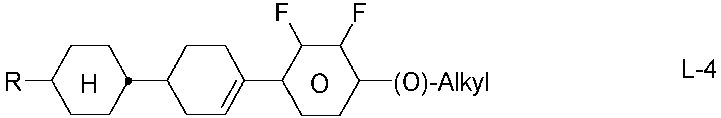 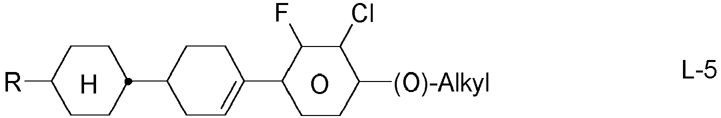 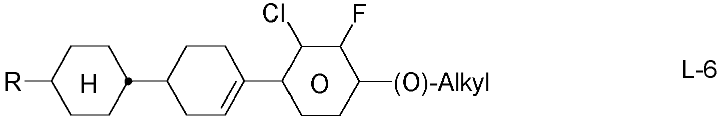 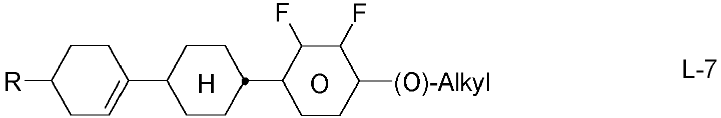 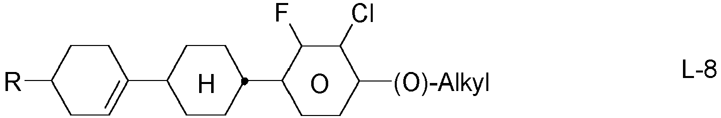 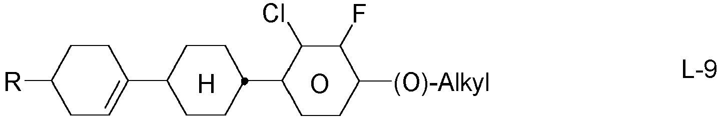 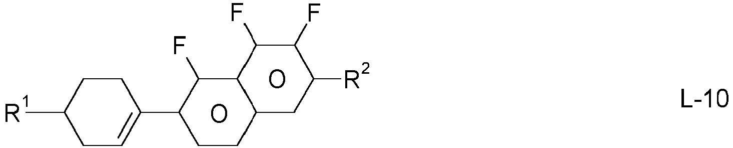 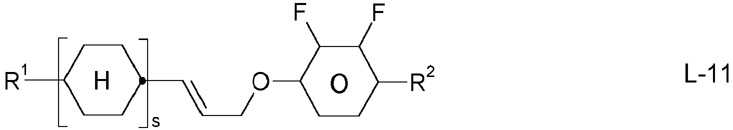 worin
R, R¹ und R² jeweils unabhängig voneinander die für R^{2A} in Anspruch 5 angegebenen Bedeutungen haben und Alkyl ein Alkylrest mit 1-6 C-Atomen, und
s 1 oder 2
bedeuten,
enthält.

8. Flüssigkristallines Medium nach einem oder mehreren der Ansprüche 1 bis 6, **dadurch kennzeichnet, dass** das Medium zusätzlich eine oder mehrere Terphenyle der Formeln T-1 bis T-21, 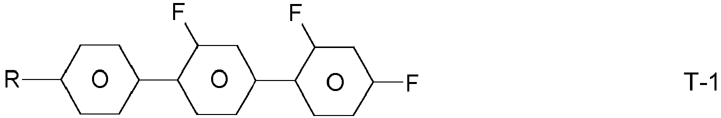 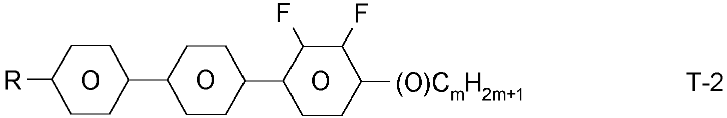 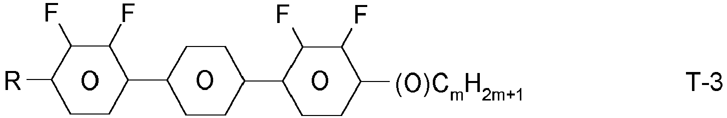 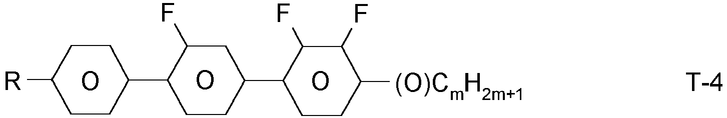 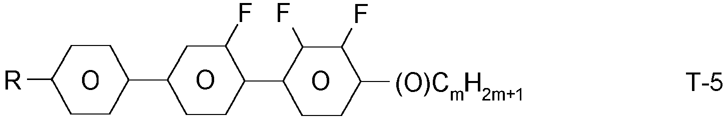 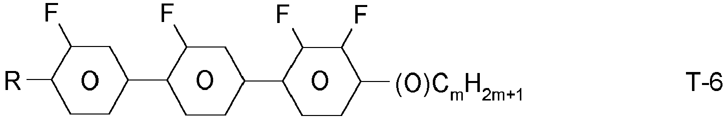 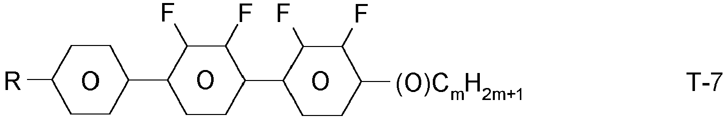 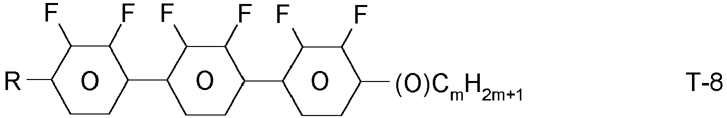 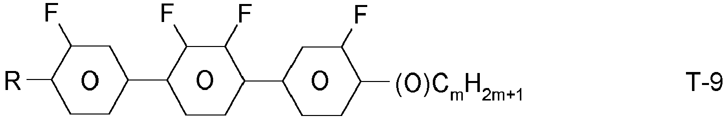 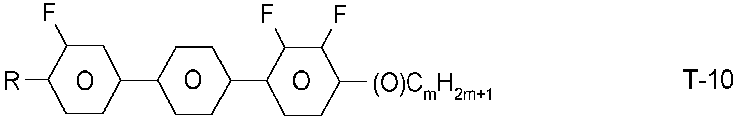 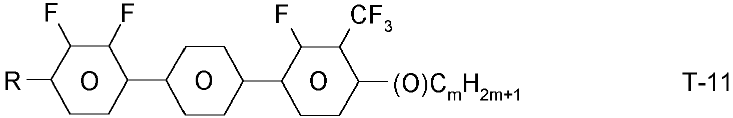 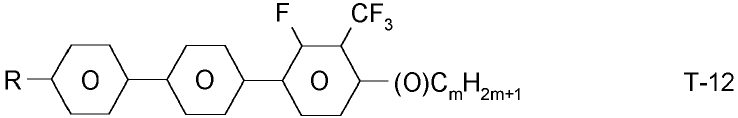 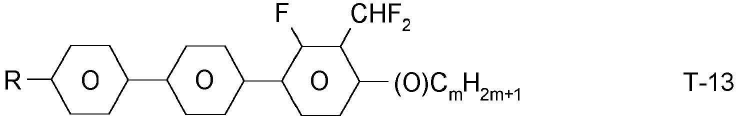 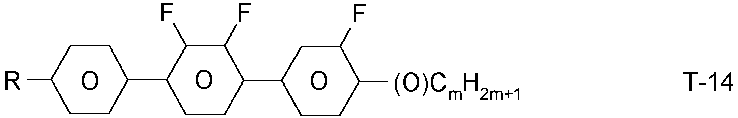 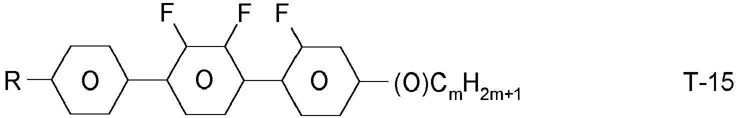 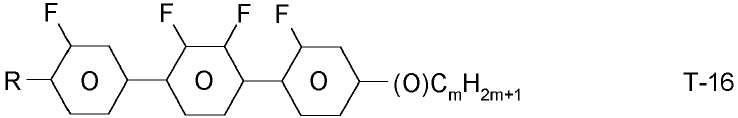 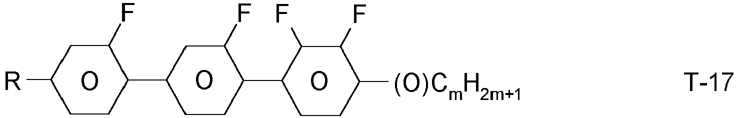 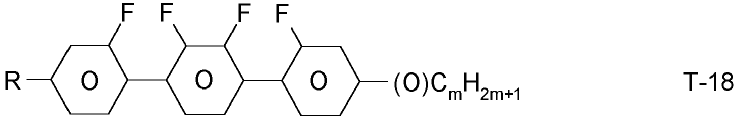 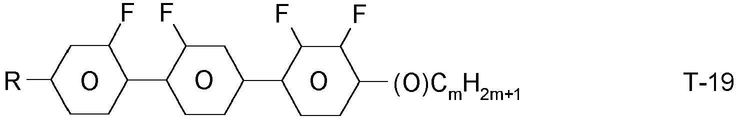 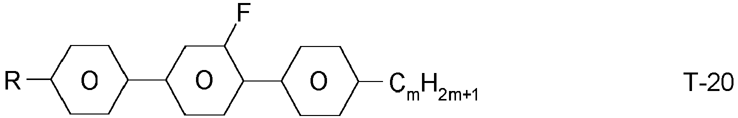 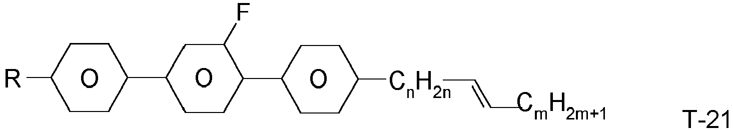 worin
R geradkettiger Alkyl- oder Alkoxyrest mit 1-7 C-Atomen bedeutet, und
m 1-6
bedeuten,
enthält.

9. Flüssigkristallines Medium nach einem oder mehreren der Ansprüche 1 bis 8, **dadurch gekennzeichnet, dass** das Medium zusätzlich eine oder mehrere Verbindungen ausgewählt aus der Gruppe der Verbindungen der Formeln O-6, O-7 und O-17, 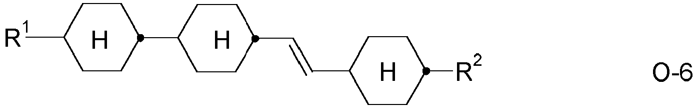 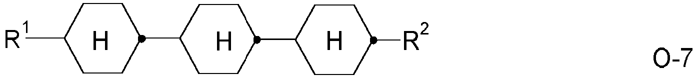 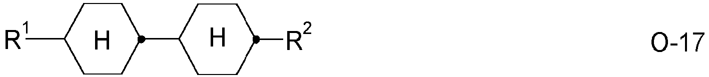 worin
R¹ Alkyl oder Alkenyl mit 1-6 bzw. 2-6 C-Atomen und R² Alkenyl mit 2-6 C-Atomen bedeuten,
enthält.

10. Flüssigkristallines Medium nach einem oder mehreren der Ansprüche 1 bis 9, **dadurch gekennzeichnet, dass** das Medium zusätzlich eine oder mehrere Indan-Verbindungen der Formel In, 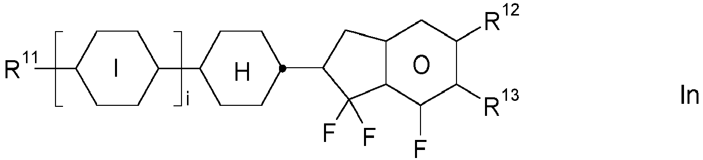 worin
R¹¹, R¹², R¹³ ein geradkettiger Alkyl-, oder Alkoxy-, Alkoxyalkyl-, Alkenylrest mit 1-5 C-Atomen,
R¹² und R¹³ zusätzlich auch Halogen, 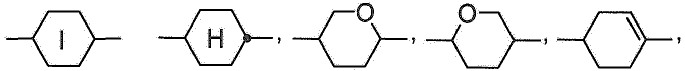 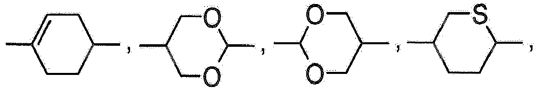 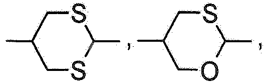
i 0, 1 oder 2
bedeuten,
enthält.

11. Flüssigkristallines Medium nach einem oder mehreren der Ansprüche 1 bis 10, **dadurch gekennzeichnet, dass** das Medium zusätzlich eine oder mehrere Verbindungen der Formeln BF-1 und BF-2, 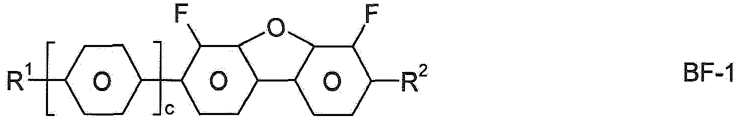 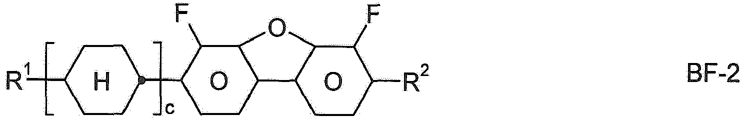 worin
R¹ und R² jeweils unabhängig voneinander die Bedeutung von R^{2A} aufweisen, und
c 1 oder 2
bedeuten,
enthält.

12. Flüssigkristallines Medium nach einem oder mehreren der Ansprüche 1 bis 11, **dadurch gekennzeichnet, dass** der Anteil an Verbindungen der Formel I im Gesamtgemisch 1 - 40 Gew.% beträgt.

13. Flüssigkristallines Medium nach einem oder mehreren der Ansprüche 1 bis 12, **dadurch gekennzeichnet, dass** das Medium mindestens eine polymerisierbare Verbindung enthält.

14. Flüssigkristallines Medium nach einem oder mehreren der Ansprüche 1 bis 13, **dadurch gekennzeichnet, dass** das Medium ein oder mehrere Additive enthält.

15. Flüssigkristallines Medium nach einem oder mehreren der Ansprüche 1 bis 14, **dadurch gekennzeichnet, dass** das Additiv ausgewählt ist aus der Gruppe Radikalfänger, Antioxidans und/oder UV-Stabilisator.

16. Verfahren zur Herstellung eines flüssigkristallinen Mediums nach einem oder mehreren der Ansprüche 1 bis 15, **dadurch gekennzeichnet, dass** man mindestens eine Verbindung der Formel I mit mindestens einer weiteren flüssigkristallinen Verbindung mischt und gegebenenfalls ein oder mehrere Additive und gegebenenfalls mindestens eine polymerisierbare Verbindung zusetzt.

17. Verwendung des flüssigkristallinen Mediums nach einem oder mehreren der Ansprüche 1 bis 15 in elektrooptischen Anzeigen.

18. Elektrooptische Anzeige mit einer Aktivmatrix-Addressierung, **dadurch gekennzeichnet, dass** sie als Dielektrikum ein flüssigkristallines Medium nach einem oder mehreren der Ansprüche 1 bis 15 enthält.

19. Elektrooptische Anzeige nach Anspruch 18, **dadurch gekennzeichnet, dass** es sich um eine VA-, PSA-, PA-VA-, PS-VA-, PALC-, IPS-, PS-IPS-, FFS-, PS-FFS-Anzeige handelt.

## Claims

1. Liquid-crystalline medium based on a mixture of polar compounds, **characterised in that** it comprises at least one compound of the formula I, 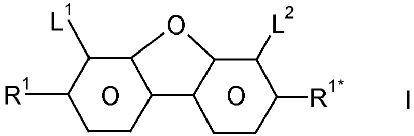 in which
R¹ and R^{1*} in each case, independently of one another, denote an alkyl or alkoxy radical having 1 to 15 C atoms, where, in addition, one or more CH₂ groups in these radicals may in each case be replaced, independently of one another, by -C≡C-, -CF₂O-, -OCF₂-, -CH=CH-, 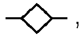 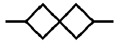 , -O-, -CO-O-, -O-CO- in such a way that O atoms are not linked directly to one another, and in which, in addition, one or more H atoms may be replaced by halogen,
L¹ and L² in each case, independently of one another, denote F, Cl, CF₃ or CHF₂,
and
additionally one or more compounds of the formulae 0-1 to 0-18, 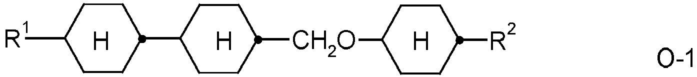 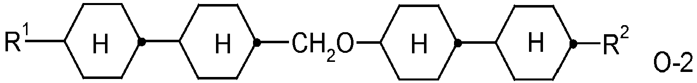 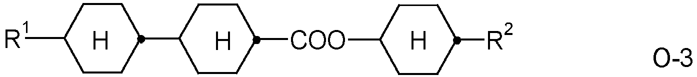 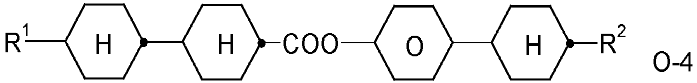 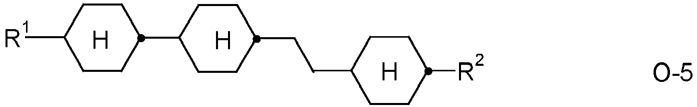 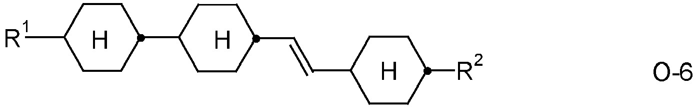 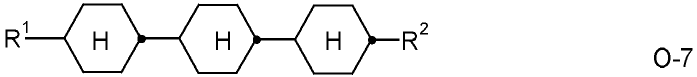 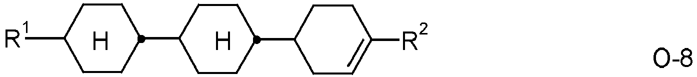 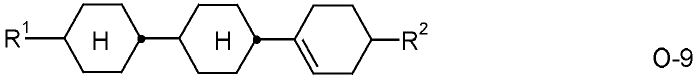 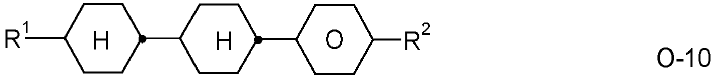 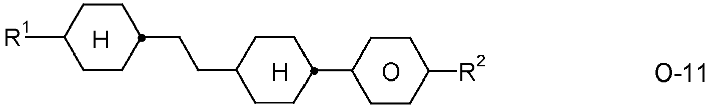 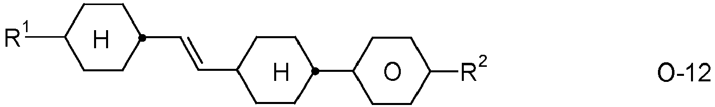 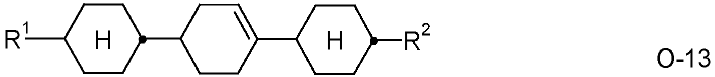 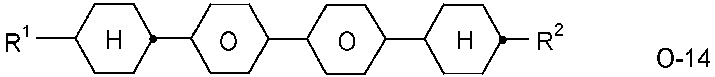 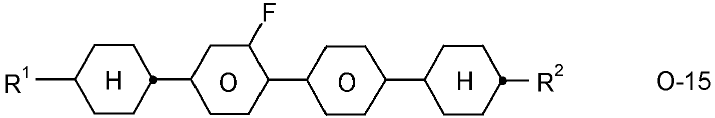 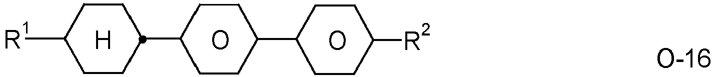 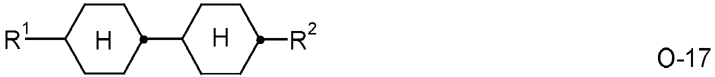 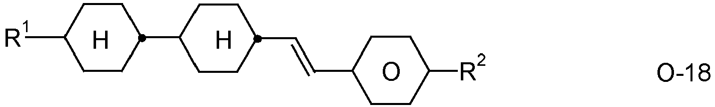 in which
R¹ and R² in each case, independently of one another, denote H, an alkyl or alkenyl radical having up to 15 C atoms which is unsubstituted, monosubstituted by CN or CF₃ or at least monosubstituted by halogen, where, in addition, one or more CH₂ groups in these radicals may be replaced by -O-, -S-, 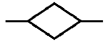 , -C≡C-, -CF₂O-, -OCF₂-, -OC-O- or -O-CO- in such a way that O atoms are not linked directly to one another.

2. Liquid-crystalline medium according to Claim 1, **characterised in that** the medium comprises at least one compound of the formulae I-1 to I-10, 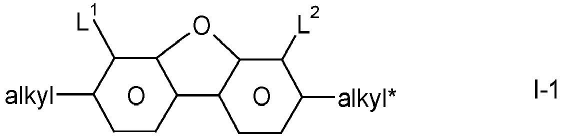 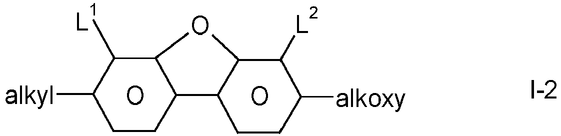 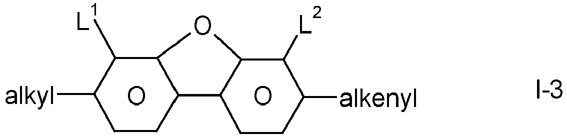 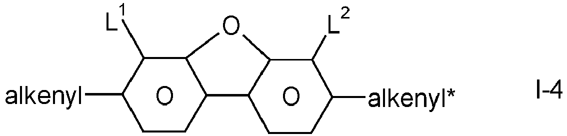 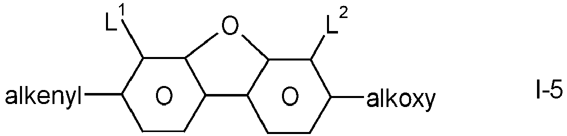 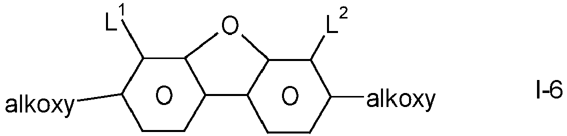 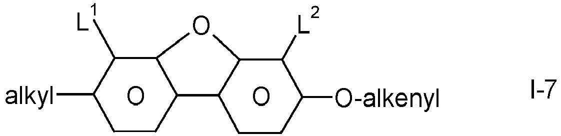 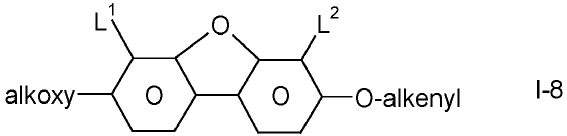 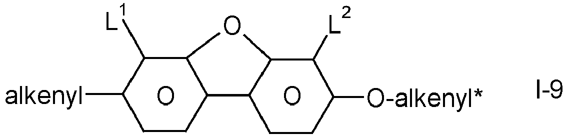 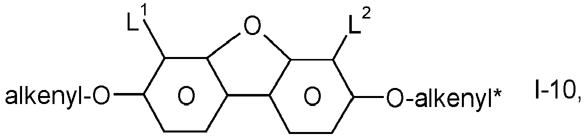 in which
alkyl and alkyl* in each case, independently of one another, denote a straight-chain alkyl radical having 1-6 C atoms,
alkenyl and alkenyl* in each case, independently of one another, denote a straight-chain alkenyl radical having 2-6 C atoms,
alkoxy and alkoxy* in each case, independently of one another, denote a straight-chain alkoxy radical having 1-6 C atoms, and
L¹ and L² in each case, independently of one another, denote F, Cl, CF₃ or CHF₂.

3. Liquid-crystalline medium according to Claim 1 or 2, **characterised in that** the medium comprises at least one compound from the group of the compounds of the formulae I-2.1 to I-2.49, 1-6.1 to I-6.28 and I-6B.1 to I-6B.3, 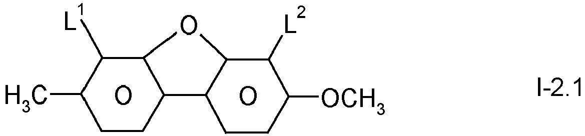 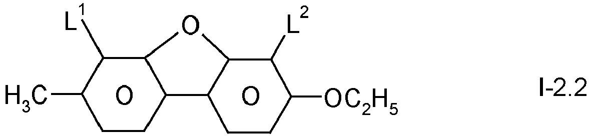 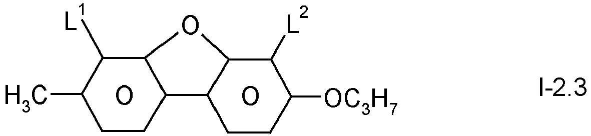 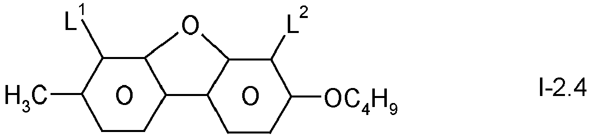 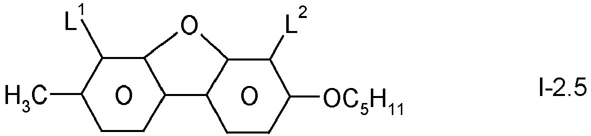 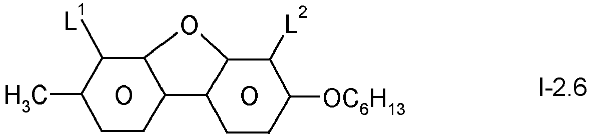 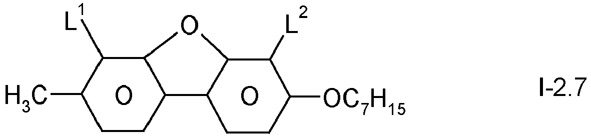 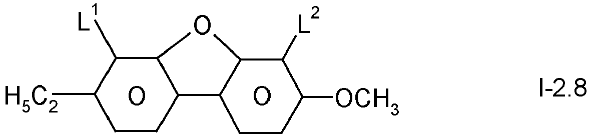 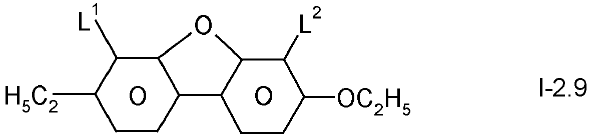 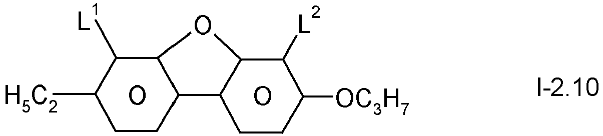 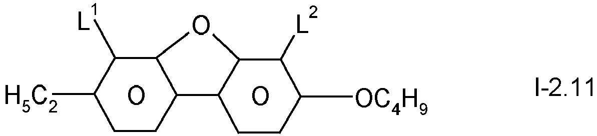 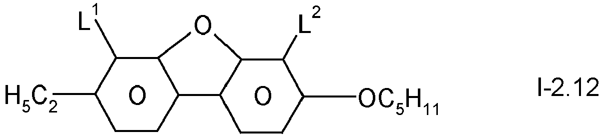 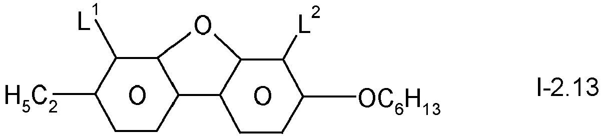 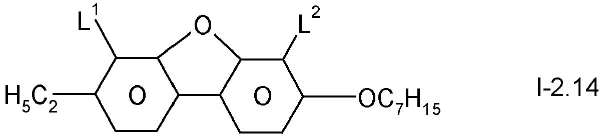 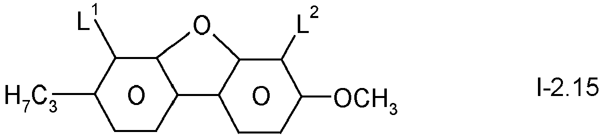 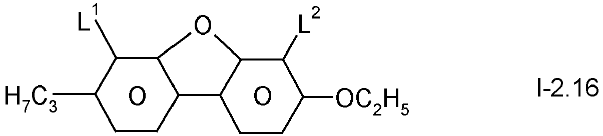 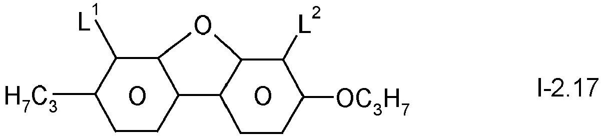 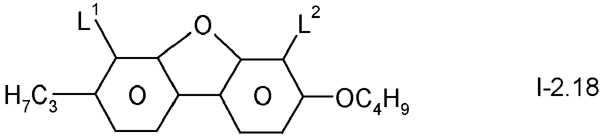 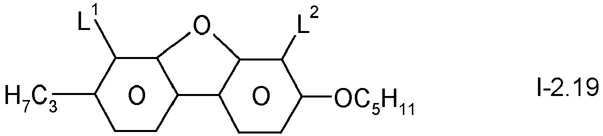 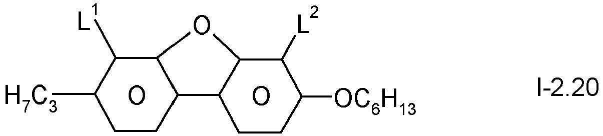 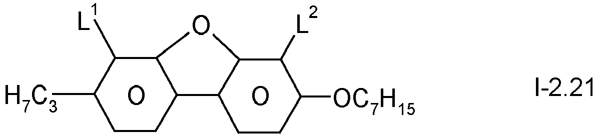 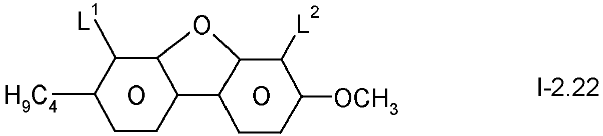 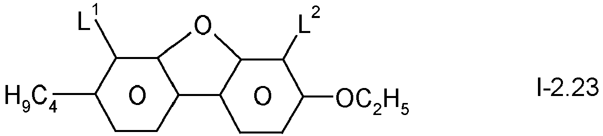 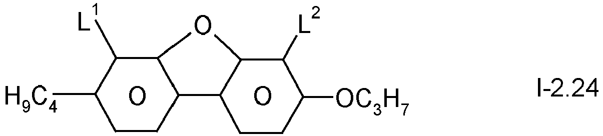 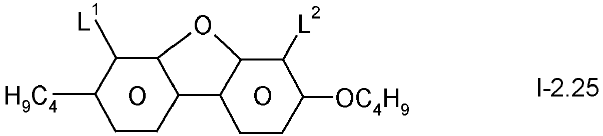 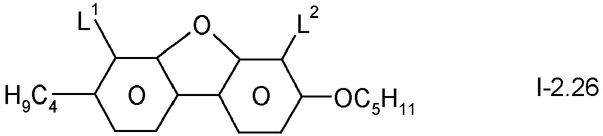 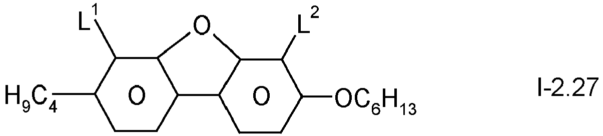 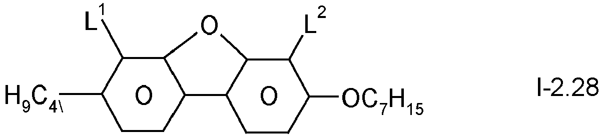 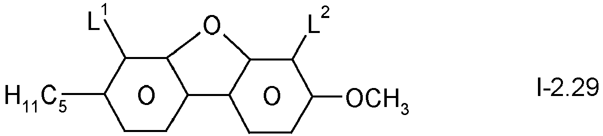 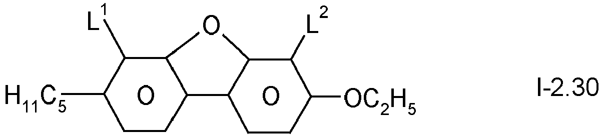 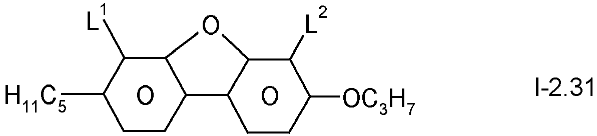 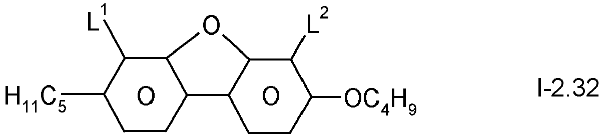 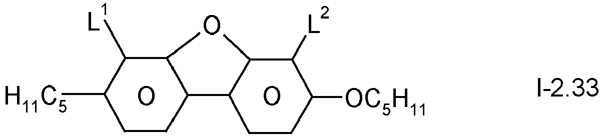 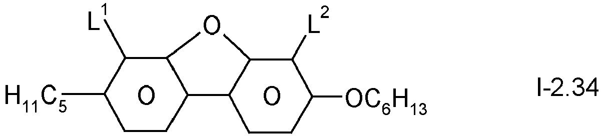 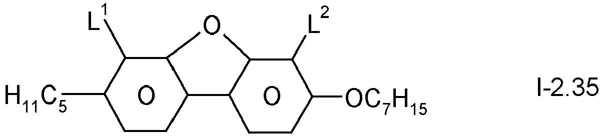 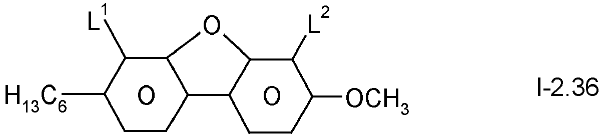 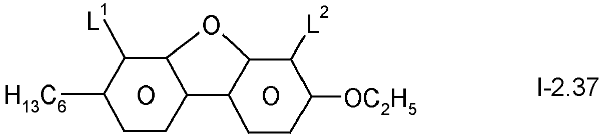 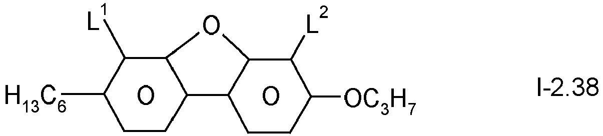 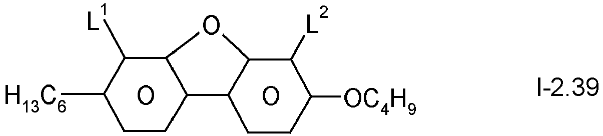 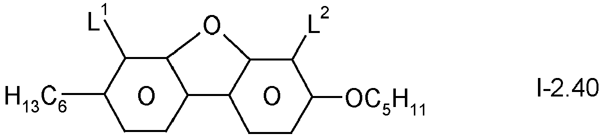 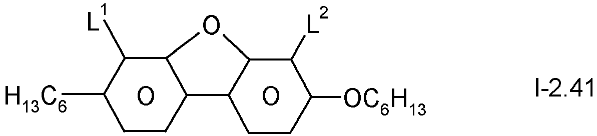 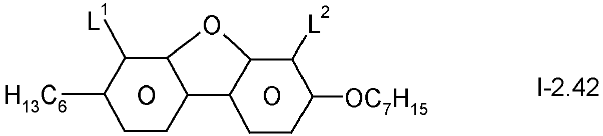 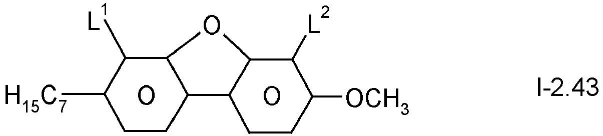 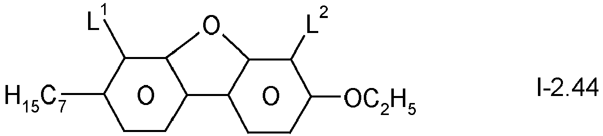 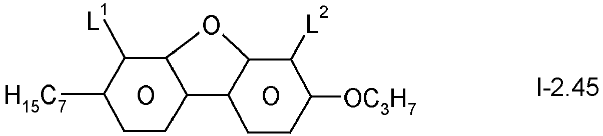 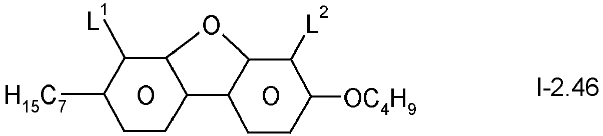 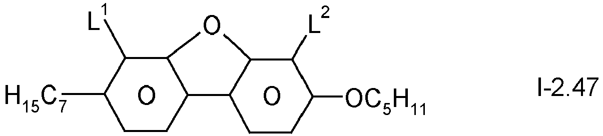 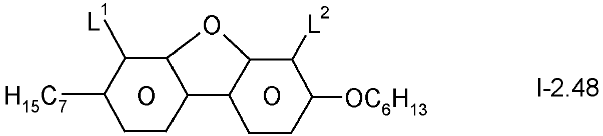 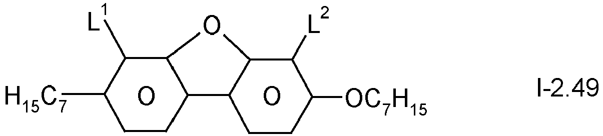 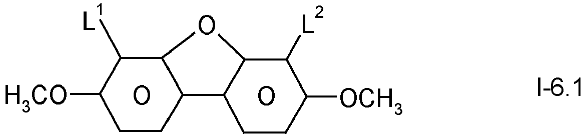 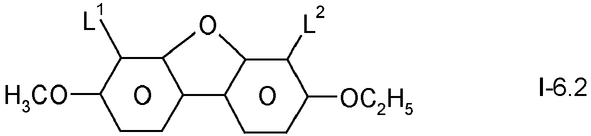 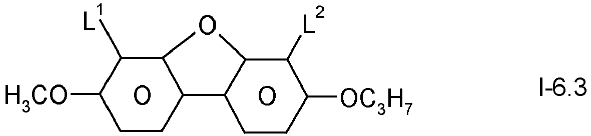 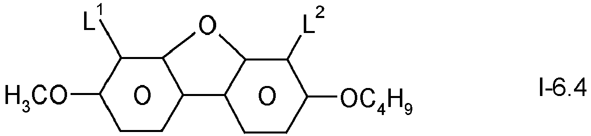 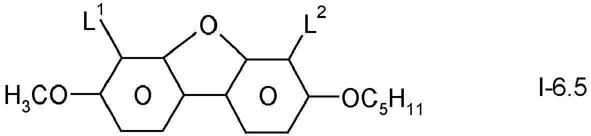 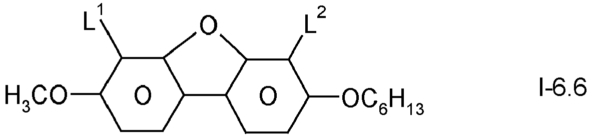 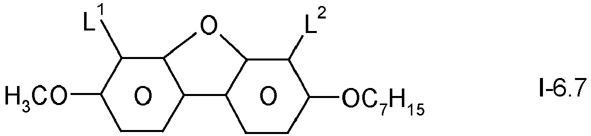 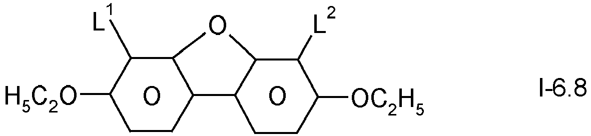 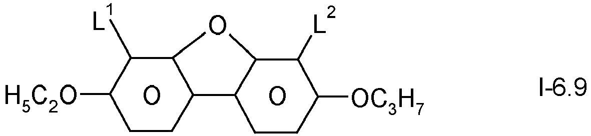 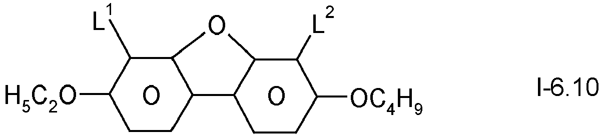 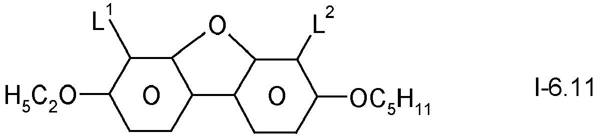 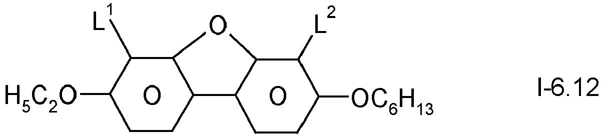 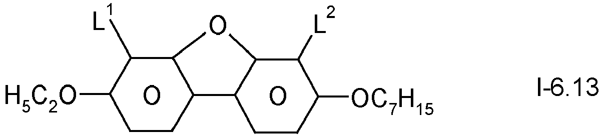 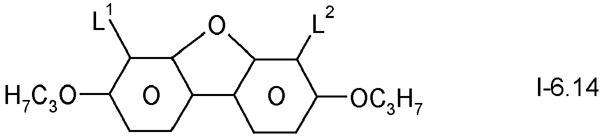 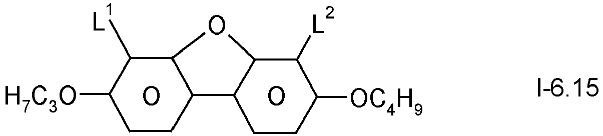 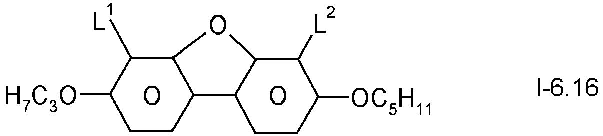 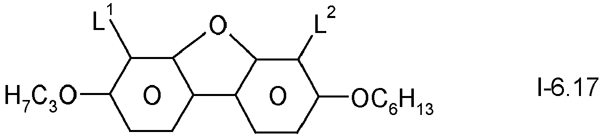 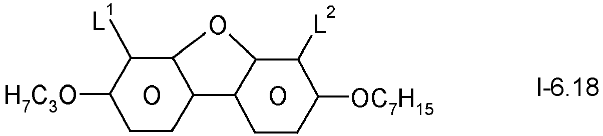 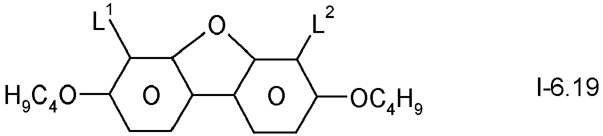 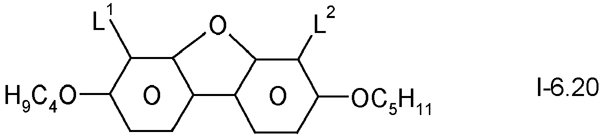 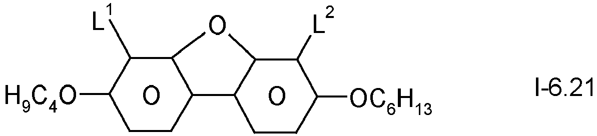 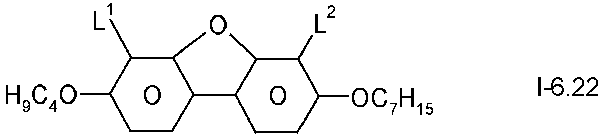 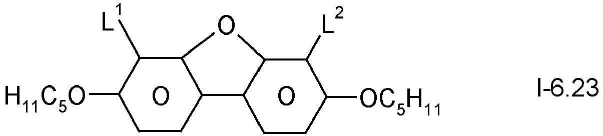 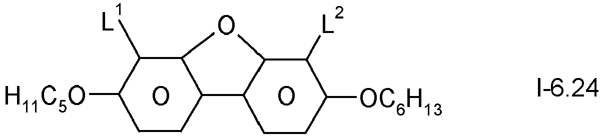 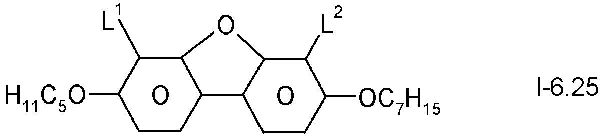 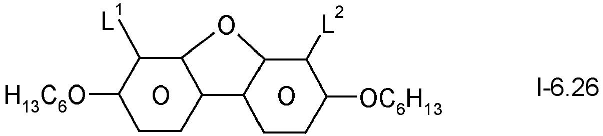 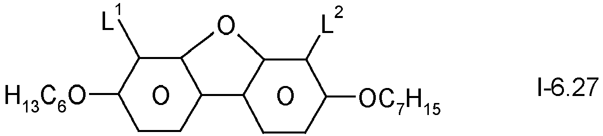 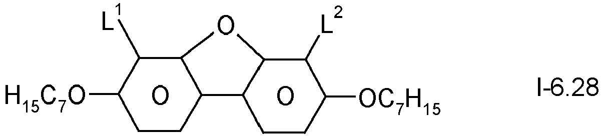 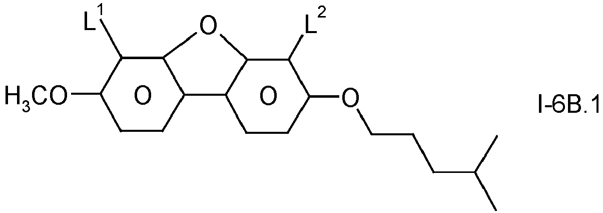 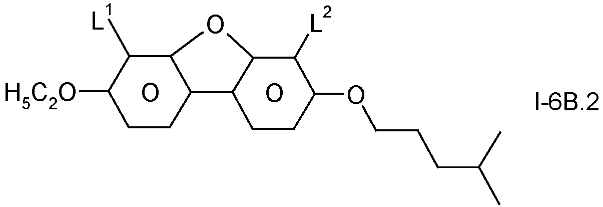 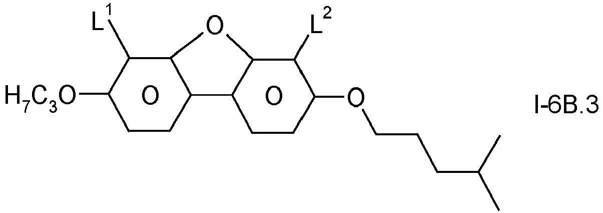 in which L¹ and L² have the meanings indicated in Claim 1.

4. Liquid-crystalline medium according to one or more of Claims 1 to 3, **characterised in that** L¹ and L² in formula I in each case denote F.

5. Liquid-crystalline medium according to one or more of Claims 1 to 4, **characterised in that** it additionally comprises one or more compounds selected from the group of the compounds of the formulae IIA, IIB and IIC, 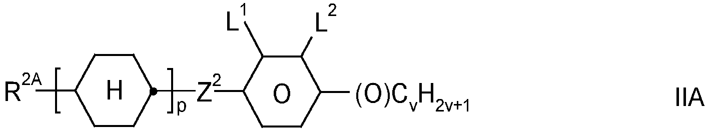 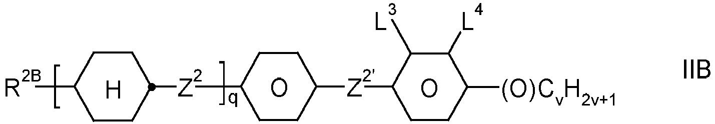 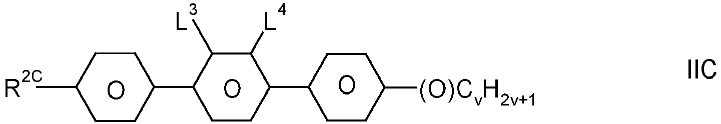 in which
R^{2A}, R^{2B} and R^{2C} in each case, independently of one another, denote H, an alkyl or alkenyl radical having up to 15 C atoms which is unsubstituted, monosubstituted by CN or CF₃ or at least monosubstituted by halogen, where, in addition, one or more CH₂ groups in these radicals may be replaced by -O-, -S-, 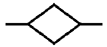 , -C≡C-, -CF₂O-, -OCF₂-, -OC-O- or -O-CO- in such a way that O atoms are not linked directly to one another,
L¹⁻⁴ in each case, independently of one another, denote F or Cl,
Z² and Z²' in each case, independently of one another, denote a single bond, -CH₂CH₂-, -CH=CH-, -CF₂O-, -OCF₂-, -CH₂O-, -OCH₂-, -COO-, -OCO-, -C₂F₄-, -CF=CF-, -CH=CHCH₂O-,
p denotes 0, 1 or 2,
q denotes 0 or 1, and
v denotes 1 to 6.

6. Liquid-crystalline medium according to one or more of Claims 1 to 5, **characterised in that** the medium additionally comprises one or more compounds of the formula III, 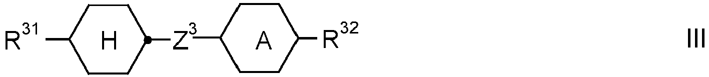 in which
R³¹ and R³² in each case, independently of one another, denote a straight-chain alkyl, alkoxyalkyl or alkoxy radical having up to 12 C atoms, and 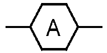
denotes 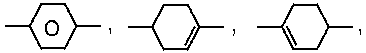 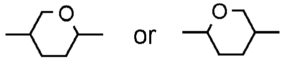
Z³ denotes a single bond, -CH₂CH₂-, -CH=CH-, -CF₂O-, -OCF₂-, -CH₂O-, -OCH₂-, -COO-, -OCO-, -C₂F₄-, -C₄H₉-, -CF=CF-.

7. Liquid-crystalline medium according to one or more of Claims 1 to 6, **characterised in that** the medium additionally comprises one or more compounds of the formulae L-1 to L-11, 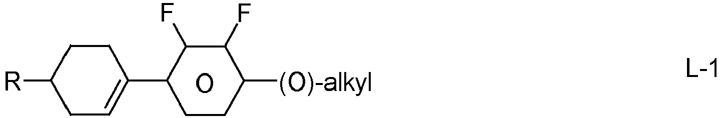 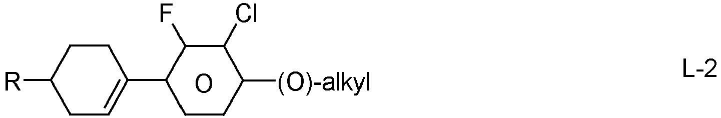 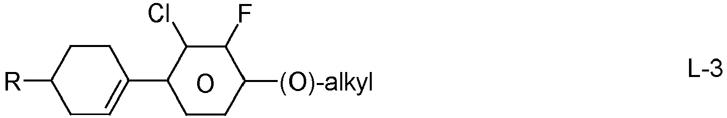 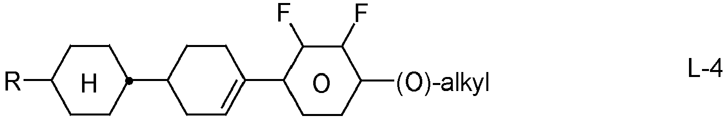 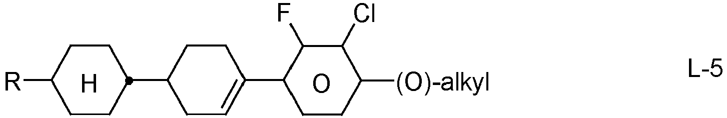 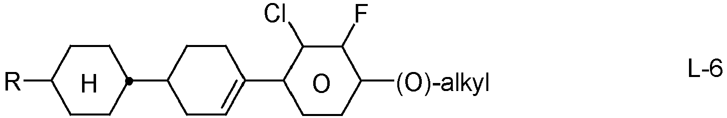 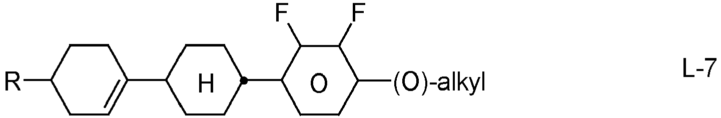 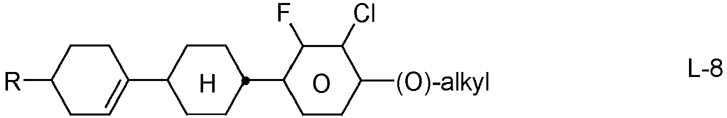 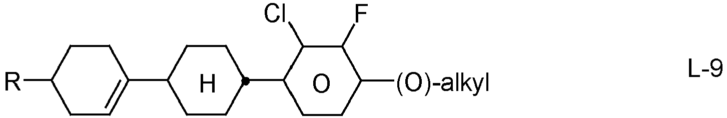 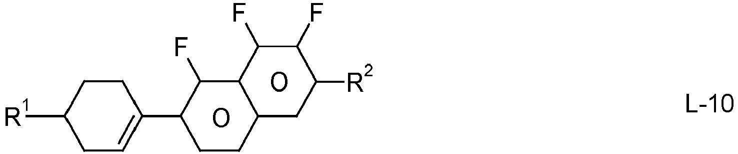 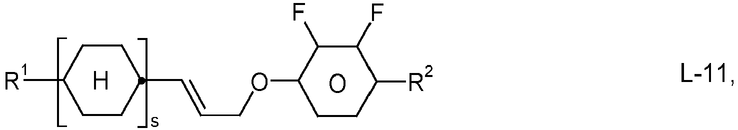 in which
R, R¹ and R² in each case, independently of one another, have the meanings indicated for R^{2A} in Claim 1, and alkyl denotes an alkyl radical having 1-6 C atoms, and
s denotes 1 or 2.

8. Liquid-crystalline medium according to one or more of Claims 1 to 7, **characterised in that** the medium additionally comprises one or more terphenyls of the formulae T-1 to T-21, 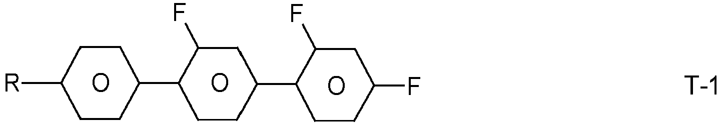 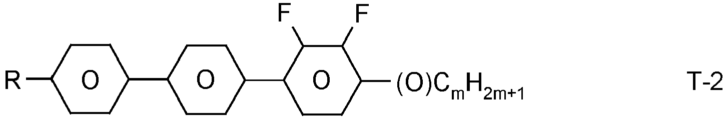 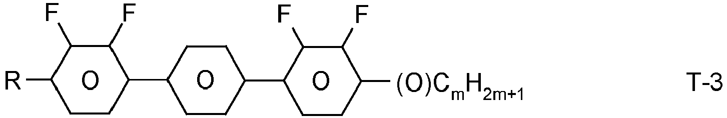 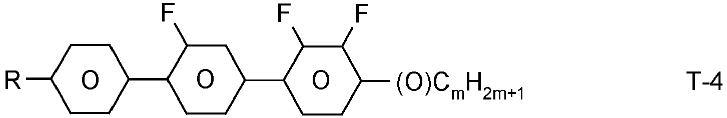 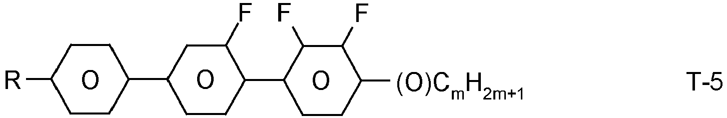 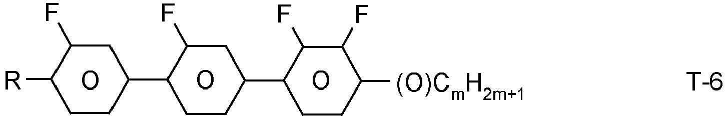 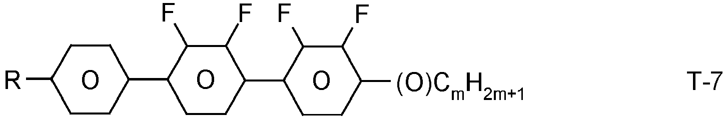 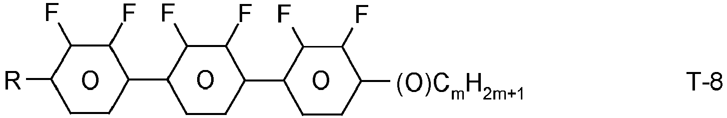 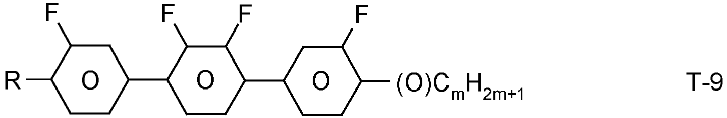 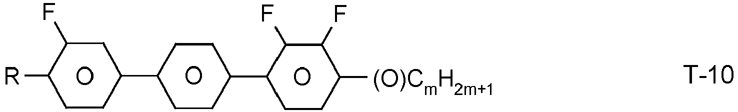 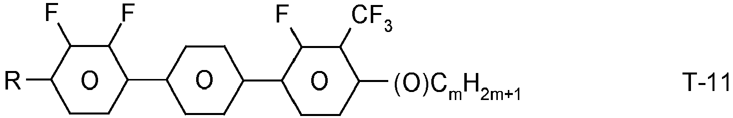 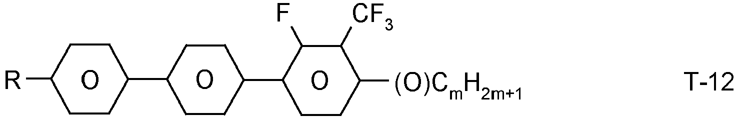 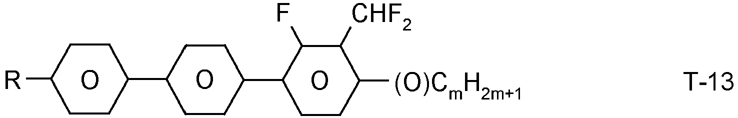 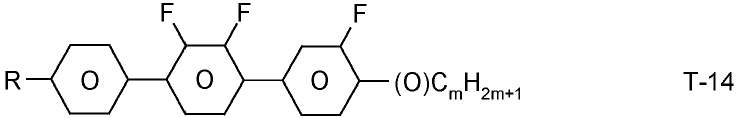 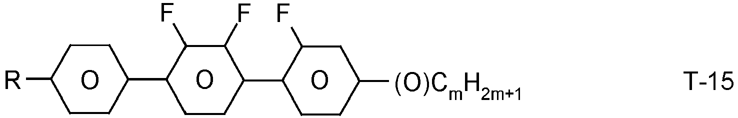 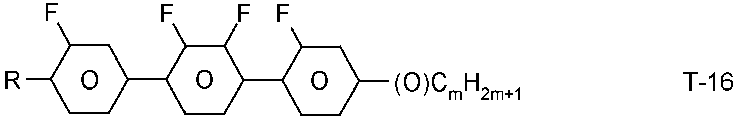 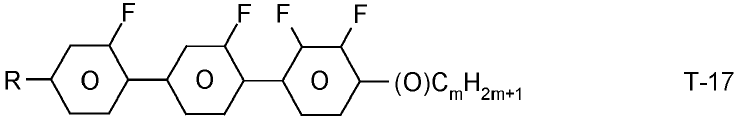 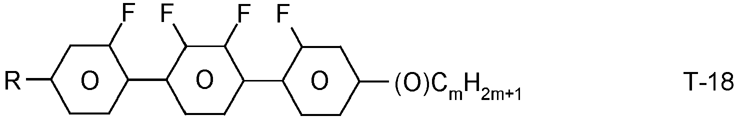 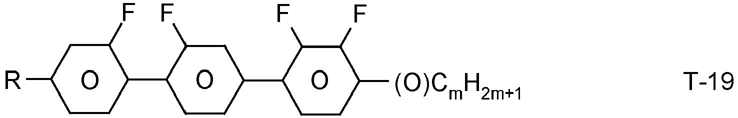 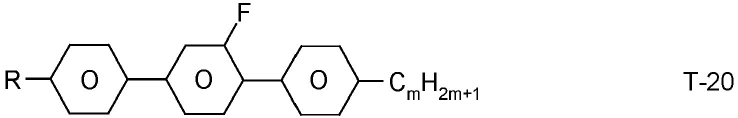 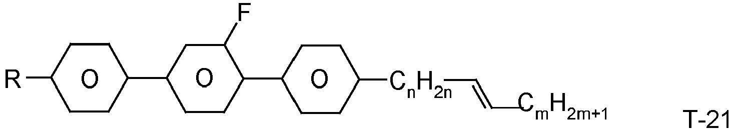 in which
R denotes a straight-chain alkyl or alkoxy radical having 1-7 C atoms, and
m denotes 1-6.

9. Liquid-crystalline medium according to one or more of Claims 1 to 8, **characterised in that** the medium additionally comprises one or more compounds selected from the group of the compounds of the formulae O-6, O-7 and 0-17, 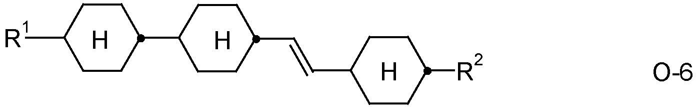 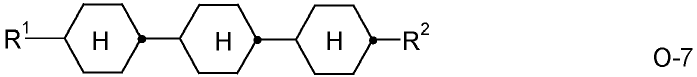 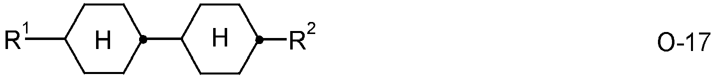 in which R¹ denotes alkyl or alkenyl having 1-6 or 2-6 C atoms respectively and R² denotes alkenyl having 2-6 C atoms.

10. Liquid-crystalline medium according to one or more of Claims 1 to 9, **characterised in that** the medium additionally comprises one or more indane compounds of the formula In, 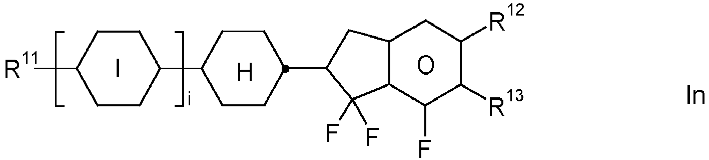 in which
R¹¹, R¹², R¹³ denote a straight-chain alkyl, alkoxy, alkoxyalkyl or alkenyl radical having 1-5 C atoms,
R¹² and R¹³ additionally also denote halogen, 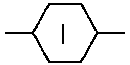 denotes 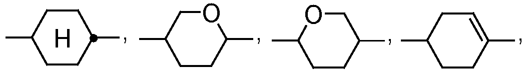 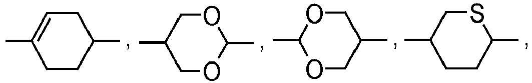 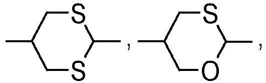
i denotes 0, 1 or 2.

11. Liquid-crystalline medium according to one or more of Claims 1 to 10, **characterised in that** the medium additionally comprises one or more compounds of the formulae BF-1 and BF-2, 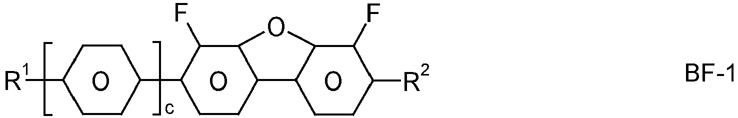 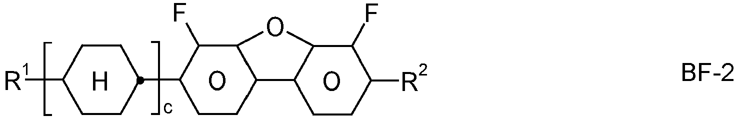 in which
R¹ and R² in each case, independently of one another, have the meaning of R^{2A}, and
c denotes 1 or 2.

12. Liquid-crystalline medium according to one or more of Claims 1 to 11, **characterised in that** the proportion of compounds of the formula I in the mixture as a whole is 1 - 40% by weight.

13. Liquid-crystalline medium according to one or more of Claims 1 to 12, **characterised in that** the medium comprises at least one polymerisable compound.

14. Liquid-crystalline medium according to one or more of Claims 1 to 13, **characterised in that** the medium comprises one or more additives.

15. Liquid-crystalline medium according to one or more of Claims 1 to 14, **characterised in that** the additive is selected from the group free-radical scavenger, antioxidant and/or UV stabiliser.

16. Process for the preparation of a liquid-crystalline medium according to one or more of Claims 1 to 15, **characterised in that** at least one compound of the formula I is mixed with at least one further liquid-crystalline compound, and optionally one or more additives and optionally at least one polymerisable compound are added.

17. Use of the liquid-crystalline medium according to one or more of Claims 1 to 15 in electro-optical displays.

18. Electro-optical display having active-matrix addressing, **characterised in that** it contains, as dielectric, a liquid-crystalline medium according to one or more of Claims 1 to 15.

19. Electro-optical display according to Claim 18, **characterised in that** it is a VA, PSA, PA-VA, PS-VA, PALC, IPS, PS-IPS, FFS or PS-FFS display.

## Revendications

1. Milieu cristallin liquide basé sur un mélange de composés polaires, **caractérisé en ce qu'**il comprend au moins un composé de la formule I, 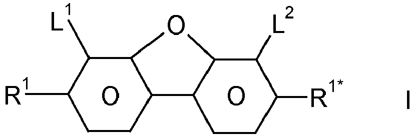 dans laquelle
R¹ et R^{1*} représentent dans chaque cas, de manière indépendante l'un de l'autre, un radical alkyle ou alcoxy qui comporte de 1 à 15 atome(s) de C, où, en outre, un ou plusieurs groupe(s) CH₂ dans ces radicaux peut/ peuvent dans chaque cas être remplacé(s), de manière indépendante les uns des autres, par -C≡C-, -CF₂O-, -OCF₂-, -CH=CH-, 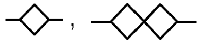 , -O-, -CO-O-, -O-CO- de telle sorte que des atomes de One soient pas liés directement les uns aux autres et où, en outre, un ou plusieurs atome(s) de H peut/peuvent être remplacé(s) par halogène,
L¹ et L² représentent dans chaque cas, de manière indépendante l'un de l'autre F, Cl, CF₃ ou CHF₂,
et
de façon additionnelle un ou plusieurs composé(s) des formules O-1 à O-18, 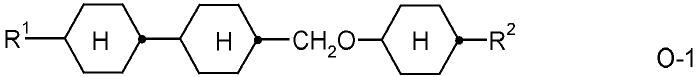 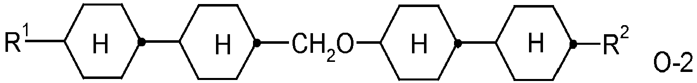 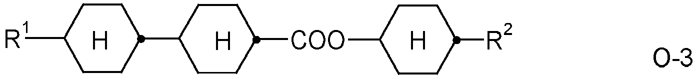 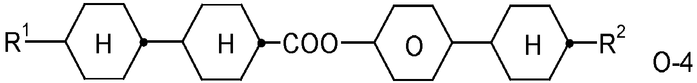 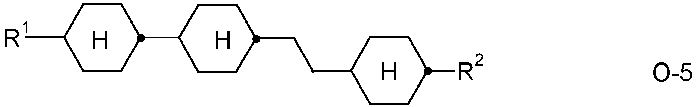 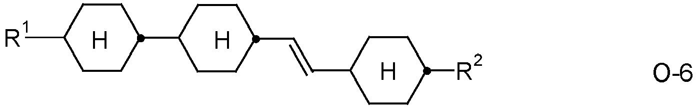 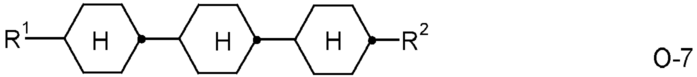 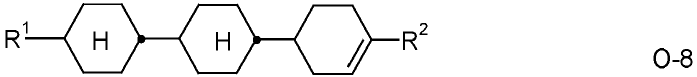 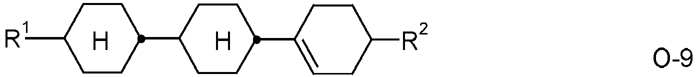 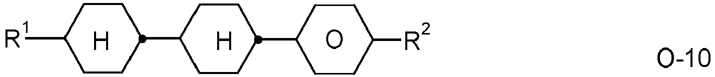 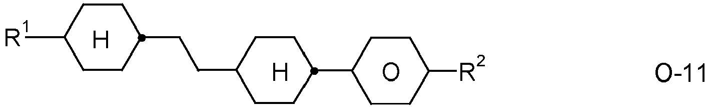 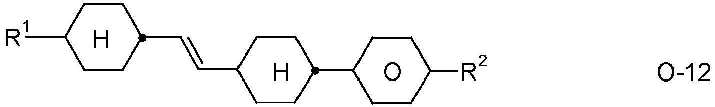 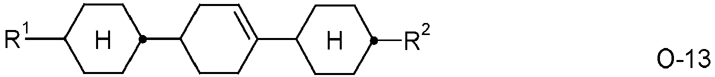 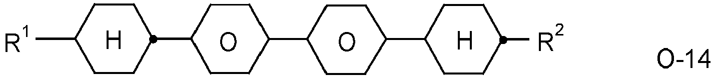 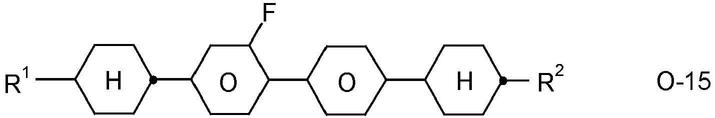 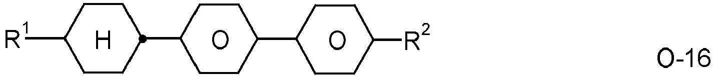 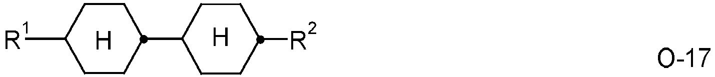 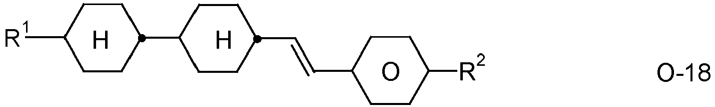 dans lesquelles
R¹ et R² représentent dans chaque cas, de manière indépendante les uns des autres, H, un radical alkyle ou alkényle qui comporte jusqu'à 15 atomes de C, lequel est non substitué, monosubstitué par CN ou par CF₃ ou au moins monosubstitué par halogène, où, en outre, un ou plusieurs groupe(s) CH₂ dans ces radicaux peut/peuvent être remplacé(s) par -O-, -S-, 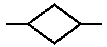 , -C≡C-, -CF₂O-, -OCF₂-, -OC-O- ou -O-CO- de telle sorte que des atomes de One soient pas liés directement les uns aux autres,

2. Milieu cristallin liquide selon la revendication 1, **caractérisé en ce que** le milieu comprend au moins un composé des formules I-1 à I-10, 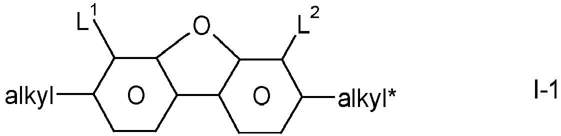 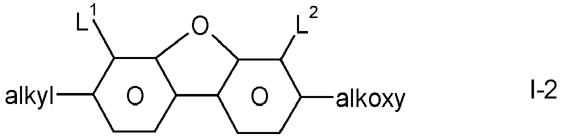 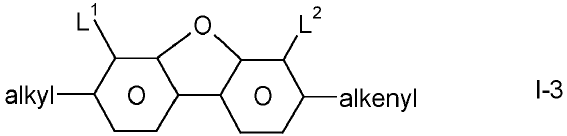 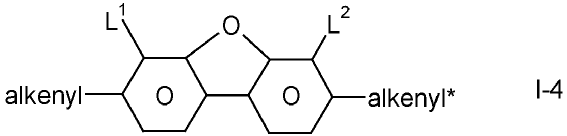 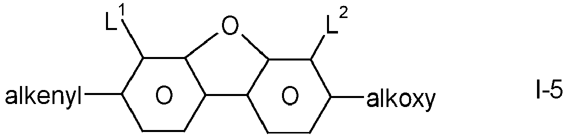 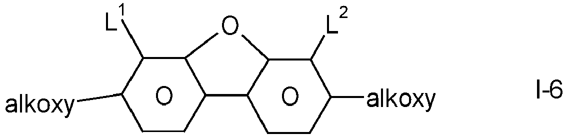 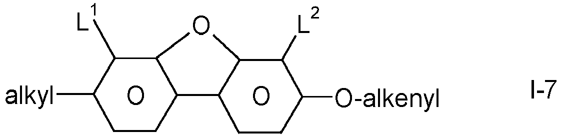 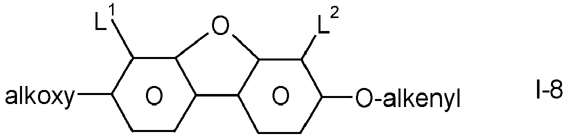 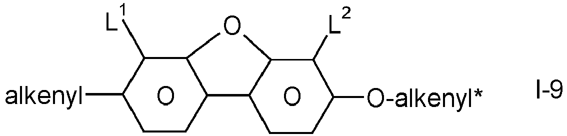 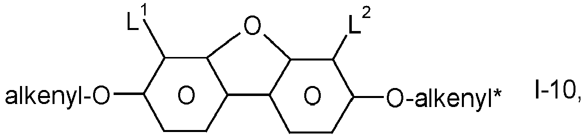 dans lesquelles
alkyl et alkyl* représentent dans chaque cas, de manière indépendante l'un de l'autre, un radical alkyle en chaîne droite qui comporte de 1 à 6 atome(s) de C,
alkenyl et alkenyl* représentent dans chaque cas, de manière indépendante l'un de l'autre, un radical alkényle en chaîne droite qui comporte de 2 à 6 atomes de C,
alkoxy et alkoxy* représentent dans chaque cas, de manière indépendante l'un de l'autre, un radical alcoxy en chaîne droite qui comporte de 1 à 6 atomes de C, et
L¹ et L² représentent dans chaque cas, de manière indépendante l'un de l'autre, F, Cl, CF₃ ou CHF₂.

3. Milieu cristallin liquide selon la revendication 1 ou 2, **caractérisé en ce que** le milieu comprend au moins un composé pris parmi le groupe des composés des formules I-2.1 à I-2.49, I-6.1 à I-6.28 et I-6B.1 à I-6B.3, 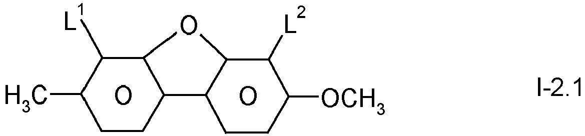 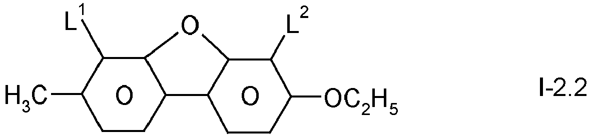 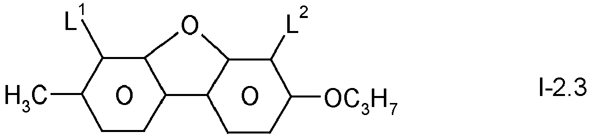 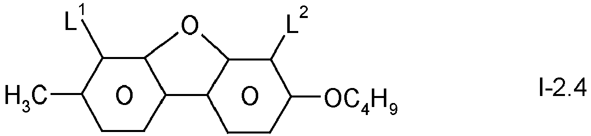 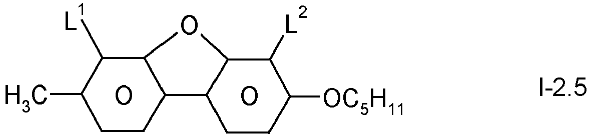 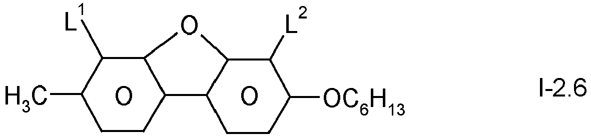 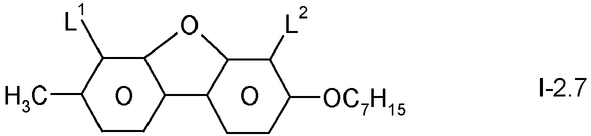 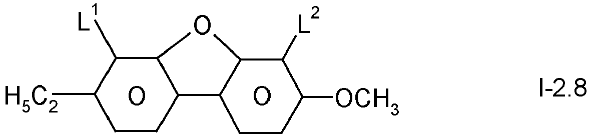 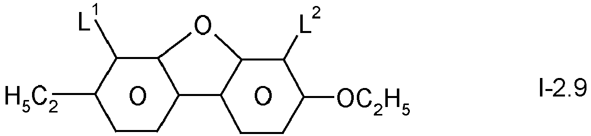 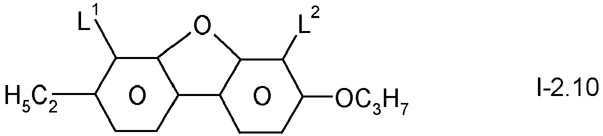 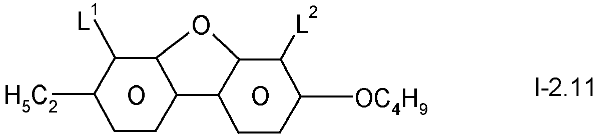 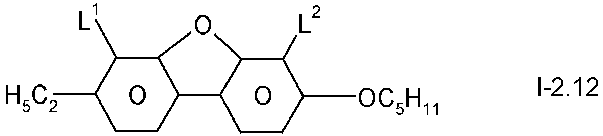 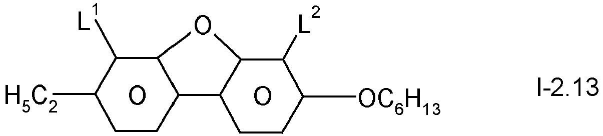 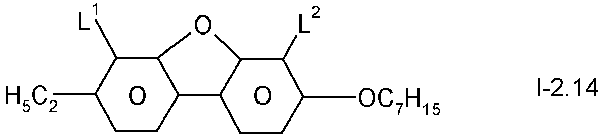 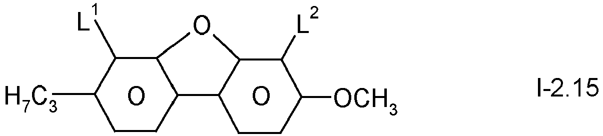 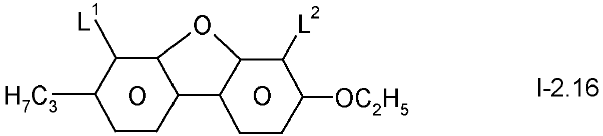 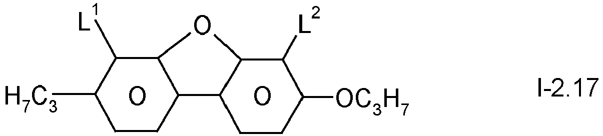 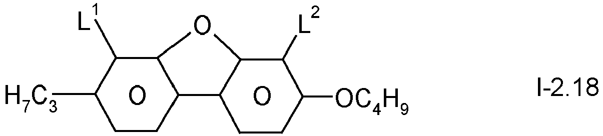 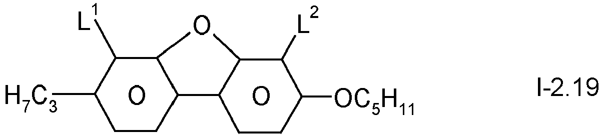 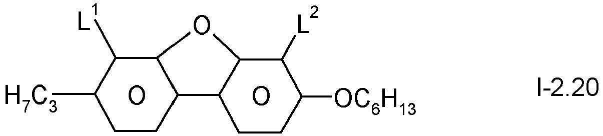 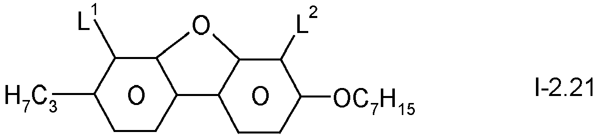 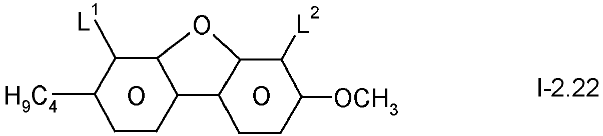 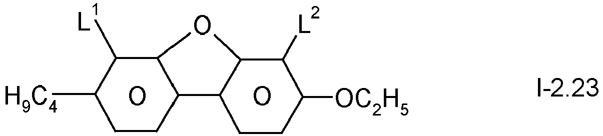 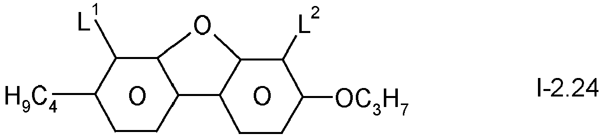 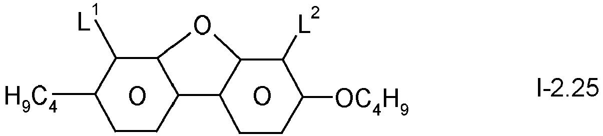 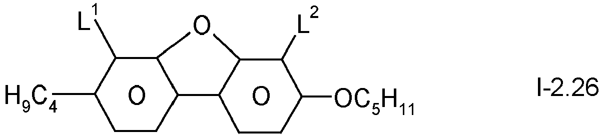 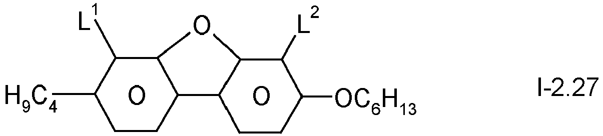 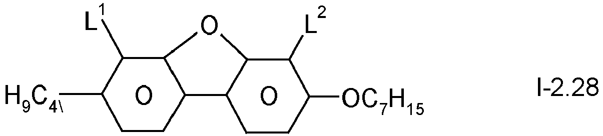 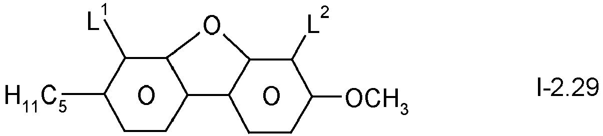 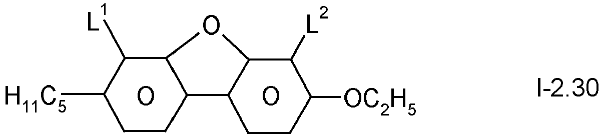 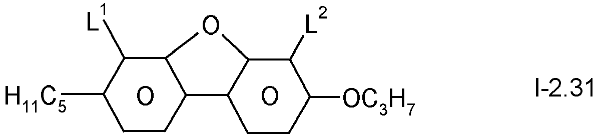 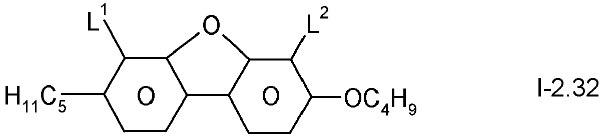 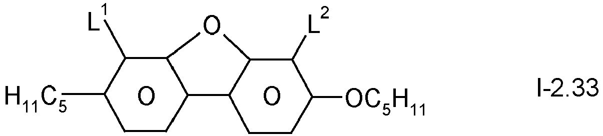 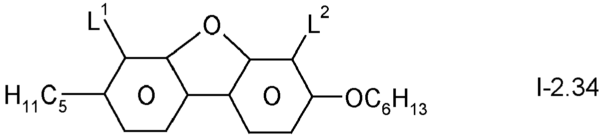 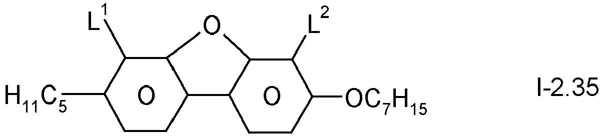 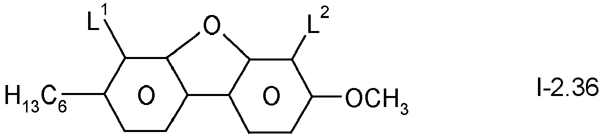 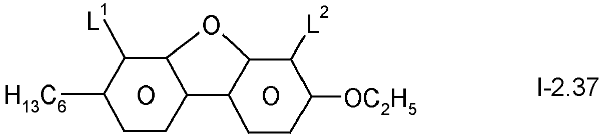 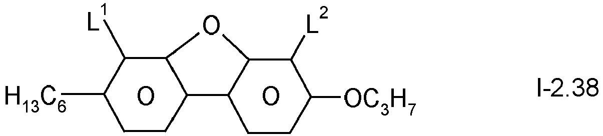 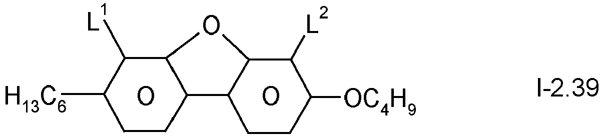 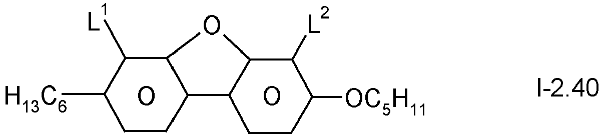 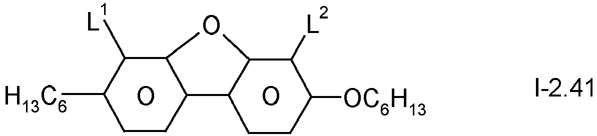 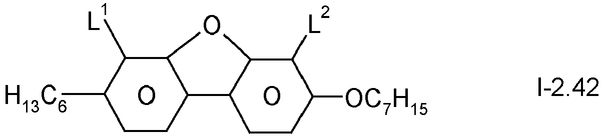 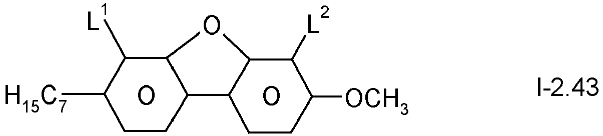 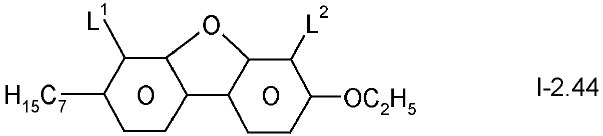 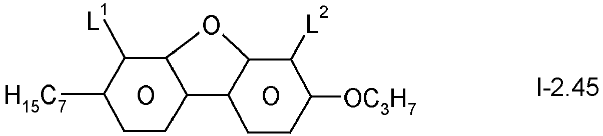 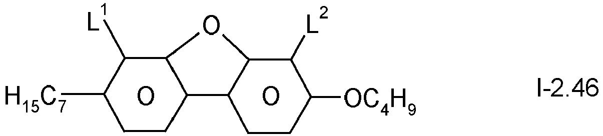 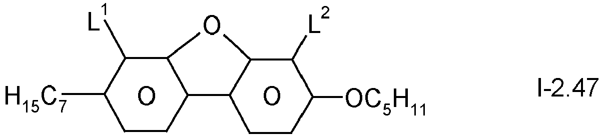 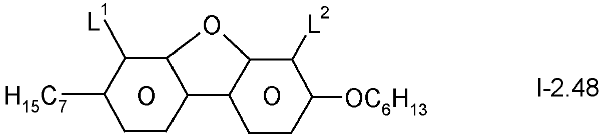 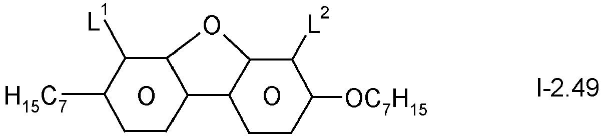 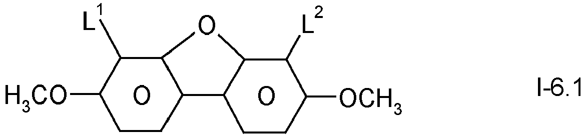 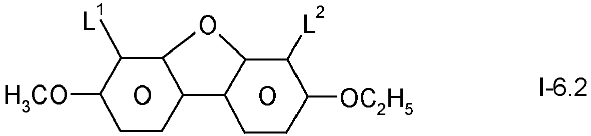 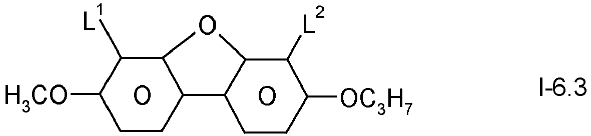 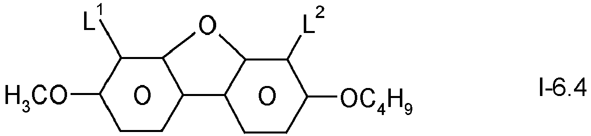 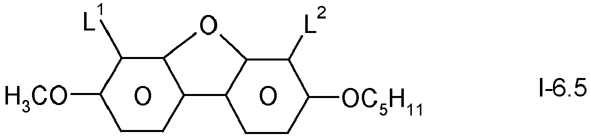 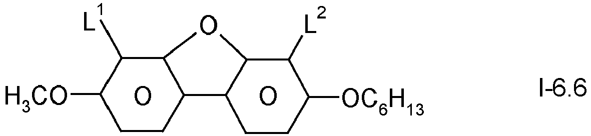 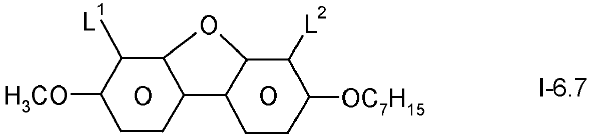 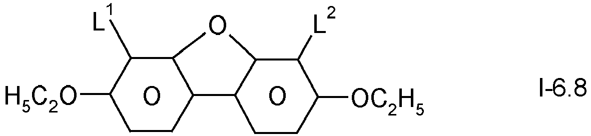 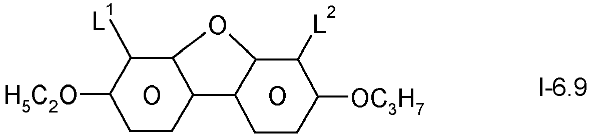 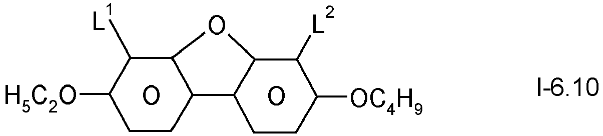 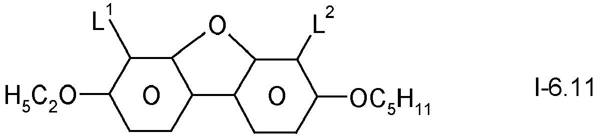 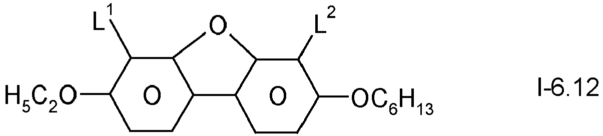 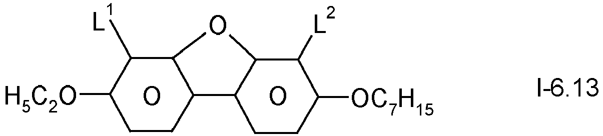 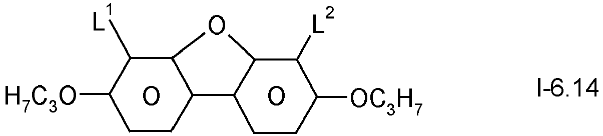 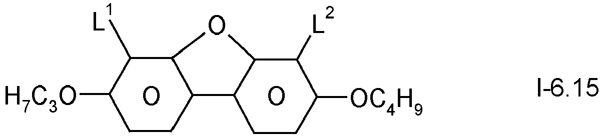 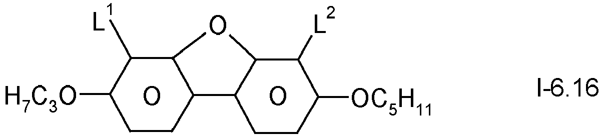 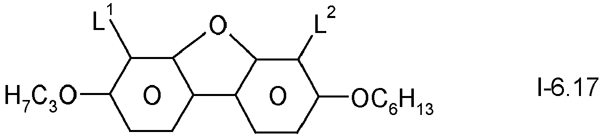 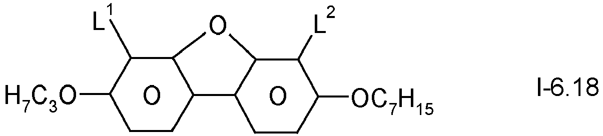 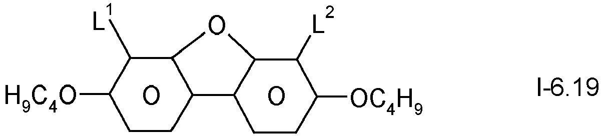 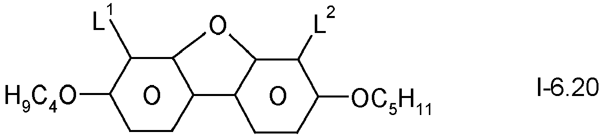 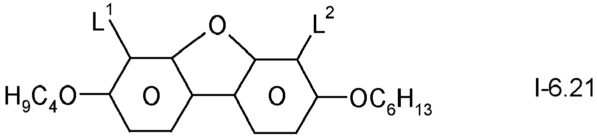 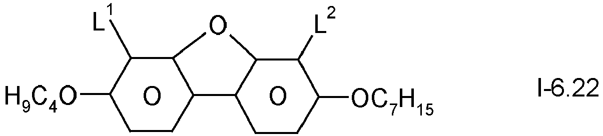 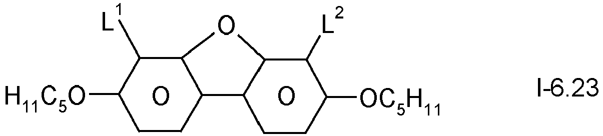 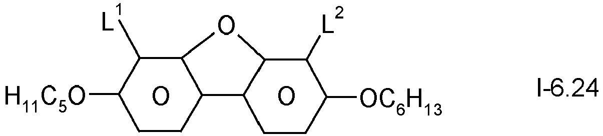 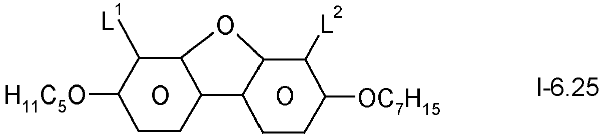 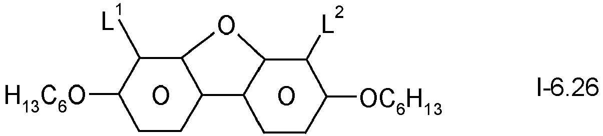 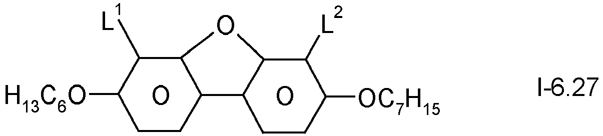 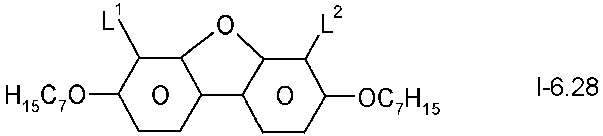 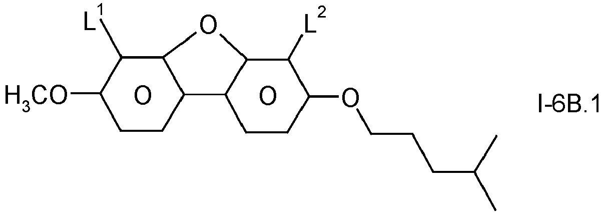 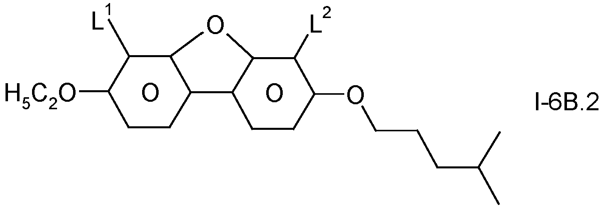 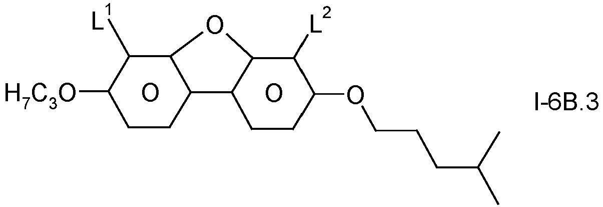 dans lesquelles L¹ et L² présentent les significations qui ont été indiquées selon la revendication 1.

4. Milieu cristallin liquide selon une ou plusieurs des revendications 1 à 3, **caractérisé en ce que** L¹ et L² dans la formule I représentent dans chaque cas F.

5. Milieu cristallin liquide selon une ou plusieurs des revendications 1 à 4, **caractérisé en ce qu'**il comprend de façon additionnelle un ou plusieurs composé(s) qui est/sont sélectionné(s) parmi le groupe des composés des formules IIA, IIB et IIC 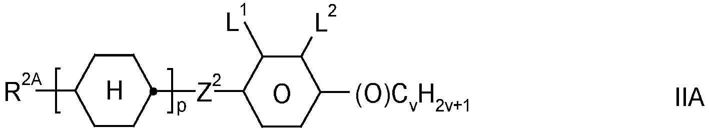 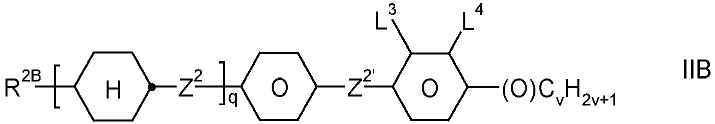 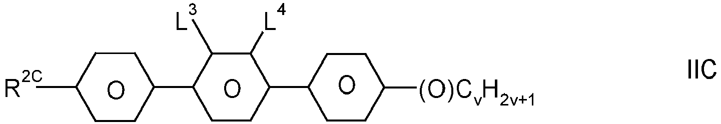 dans lesquelles
R^{2A}, R^{2B} et R^{2C} représentent dans chaque cas, de manière indépendante les uns des autres, H, un radical alkyle ou alkényle qui comporte jusqu'à 15 atomes de C, lequel est non substitué, monosubstitué par CN ou par CF₃ ou au moins monosubstitué par halogène, où, en outre, un ou plusieurs groupe(s) CH₂ dans ces radicaux peut/peuvent être remplacé(s) par -O-, -S-, 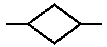 , -C≡C-, -CF₂O-, -OCF₂-, -OC-O- ou -O-CO- de telle sorte que des atomes de O ne soient pas liés directement les uns aux autres,
L¹⁻⁴ représentent dans chaque cas, de manière indépendante les uns des autres, F ou Cl,
Z² et Z^{2'} représentent dans chaque cas, de manière indépendante l'un de l'autre, une liaison simple, -CH₂CH₂-, -CH=CH-, -CF₂O-, -OCF₂-, -CH₂O-, -OCH₂-, -COO-, -OCO-, -C₂F₄-, -CF=CF-, -CH=CHCH₂O-,
p représente 0, 1 ou 2,
q représente 0 ou 1, et
v représente 1 à 6.

6. Milieu cristallin liquide selon une ou plusieurs des revendications 1 à 5, **caractérisé en ce que** le milieu comprend de façon additionnelle un ou plusieurs composé(s) de la formule III, 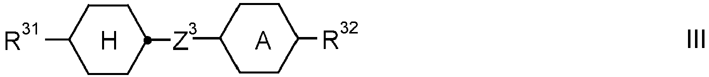 dans laquelle
R³¹ et R³² représentent dans chaque cas, de manière indépendante l'un de l'autre, un radical alkyle, alcoxyalkyle ou alcoxy en chaine droite qui comporte jusqu'à 12 atomes de C, et 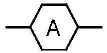
représente 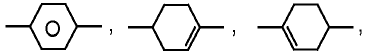 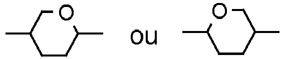
Z³ représente une liaison simple, -CH₂CH₂-, -CH=CH-, -CF₂O-, -OCF₂-, -CH₂O-, -OCH₂-, -COO-, -OCO-, -C₂F₄-, -C₄H₉-, -CF=CF-.

7. Milieu cristallin liquide selon une ou plusieurs des revendications 1 à 6, **caractérisé en ce que** le milieu comprend de façon additionnelle un ou plusieurs composé(s) des formules L-1 à L-11, 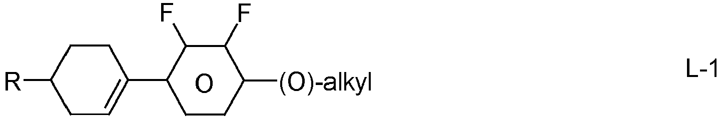 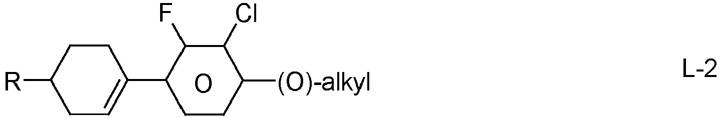 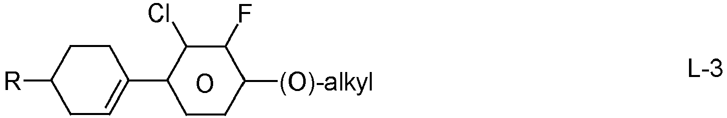 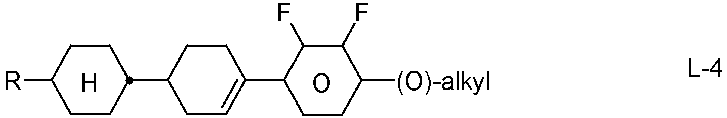 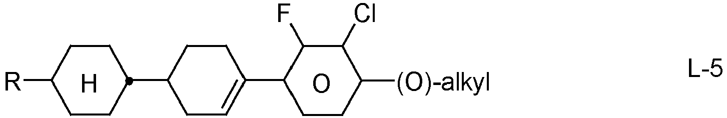 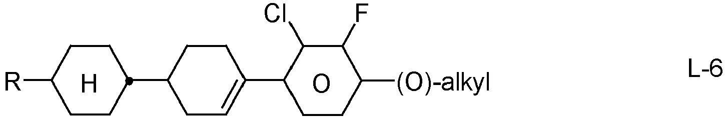 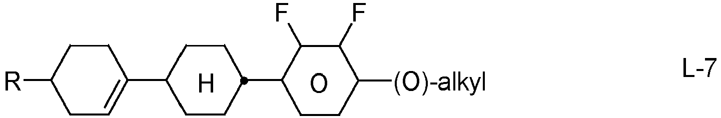 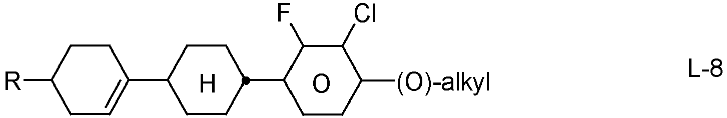 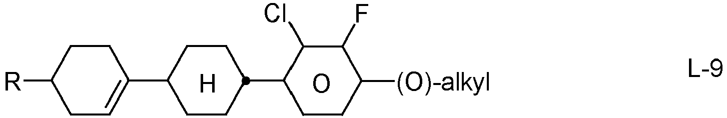 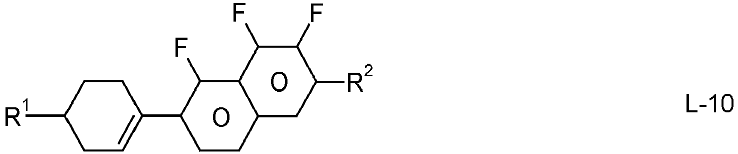 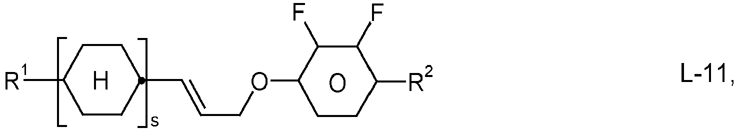 dans lesquelles
R, R¹ et R² présentent dans chaque cas, de manière indépendante les uns des autres, les significations qui ont été indiquées pour R^{2A} selon la revendication 1, et alkyl représente un radical alkyle qui comporte de 1 à 6 atome(s) de C, et
s représente 1 ou 2.

8. Milieu cristallin liquide selon une ou plusieurs des revendications 1 à 7, **caractérisé en ce que** le milieu comprend de façon additionnelle un ou plusieurs terphényle(s) des formules T-1 à T-21, 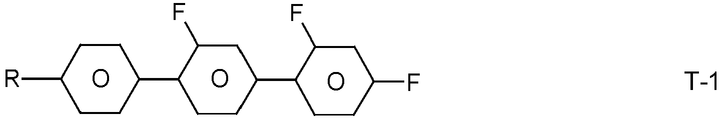 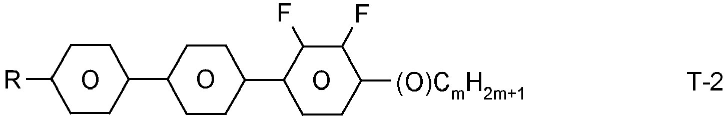 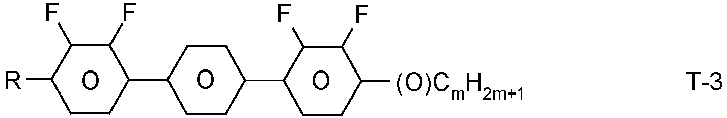 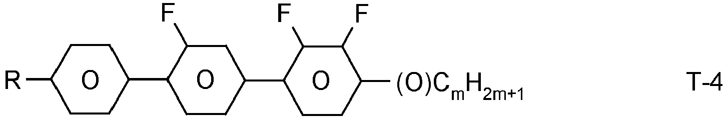 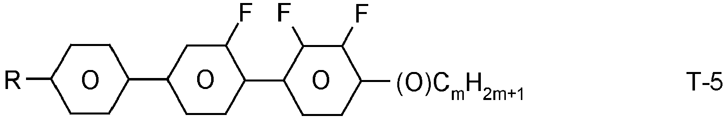 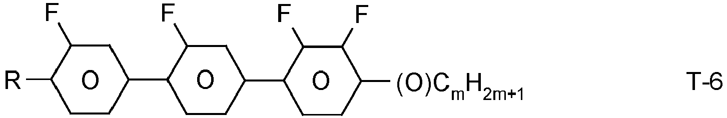 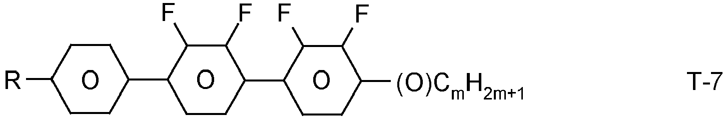 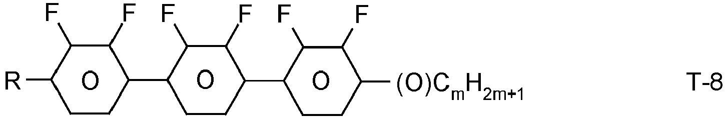 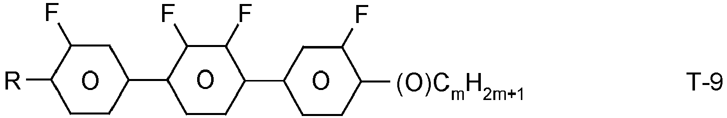 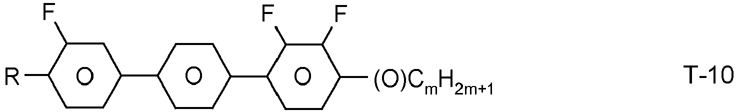 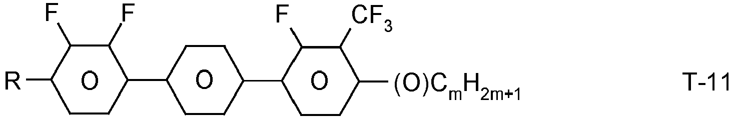 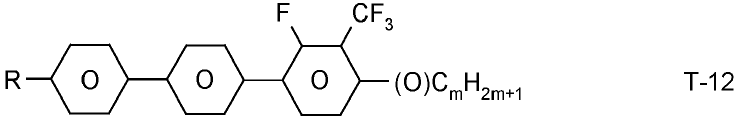 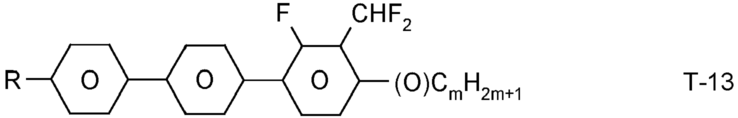 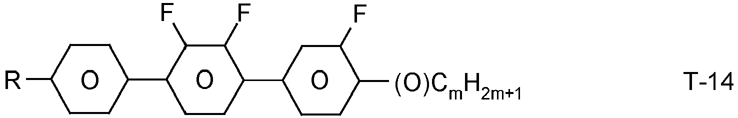 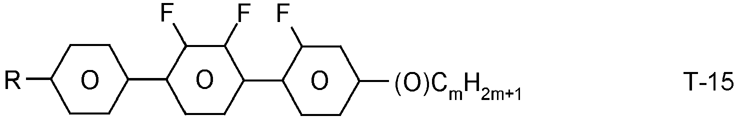 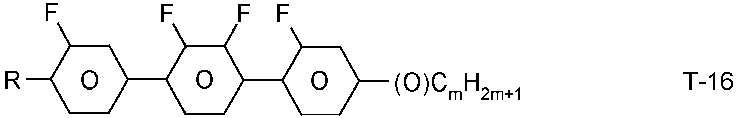 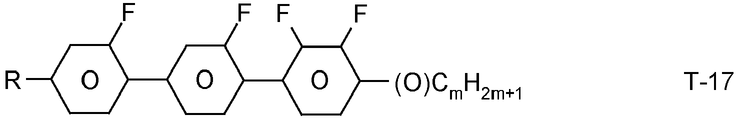 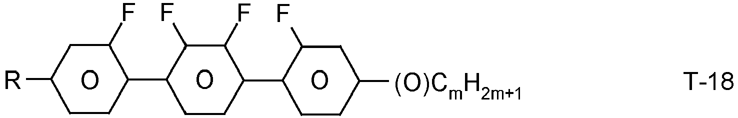 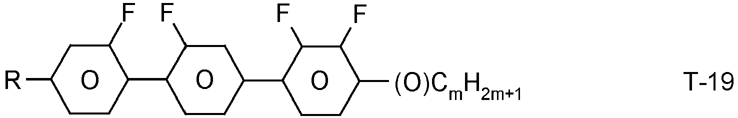 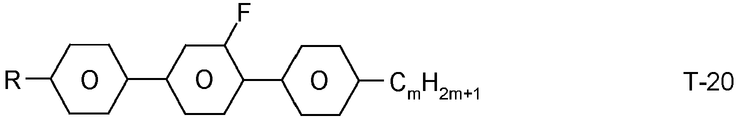 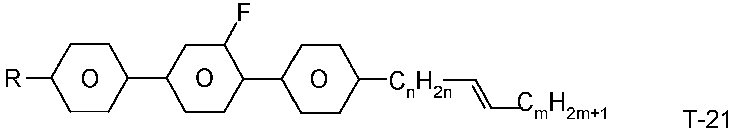 dans lesquelles
R représente un radical alkyle ou alcoxy en chaîne droite qui comporte de 1 à 7 atome(s) de C, et
m représente 1 à 6.

9. Milieu cristallin liquide selon une ou plusieurs des revendications 1 à 8, **caractérisé en ce que** le milieu comprend de façon additionnelle un ou plusieurs composé(s) qui est/sont sélectionné(s) parmi le groupe des composés des formules O-6, O-7 et O-17, 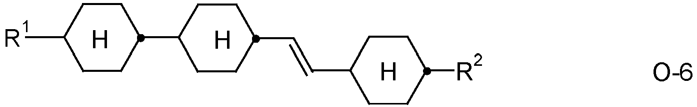 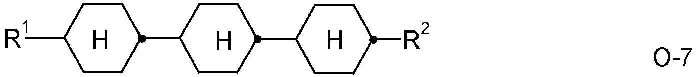 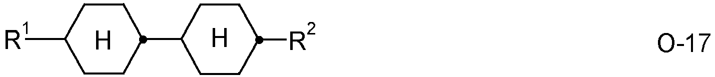 dans lesquelles
R¹ représente alkyle ou alkényle qui comporte respectivement de 1 à 6 ou de 2 à 6 atome(s) de C et R² représente alkényle qui comporte de 2 à 6 atomes de C.

10. Milieu cristallin liquide selon une ou plusieurs des revendications 1 à 9, **caractérisé en ce que** le milieu comprend de façon additionnelle un ou plusieurs composé(s) indane de la formule In, 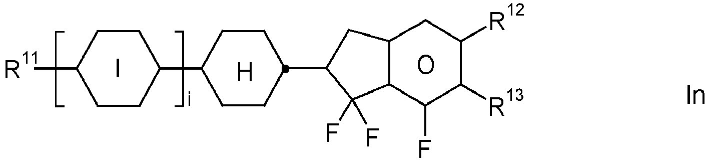 dans laquelle
R¹¹, R¹², R¹³ représentent un radical alkyle, alcoxy, alcoxyalkyle ou alkényle en chaîne droite qui comporte de 1 à 5 atome(s) de C,
R¹² et R¹³ représente de façon additionnelle également halogène, 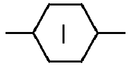 représente 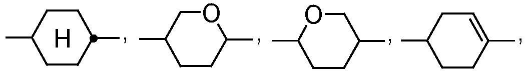 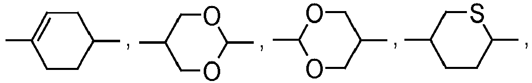 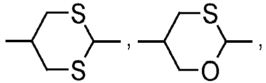
i représente 0, 1 ou 2.

11. Milieu cristallin liquide selon une ou plusieurs des revendications 1 à 10, **caractérisé en ce que** le milieu comprend de façon additionnelle un ou plusieurs composé(s) des formules BF-1 et BF-2, 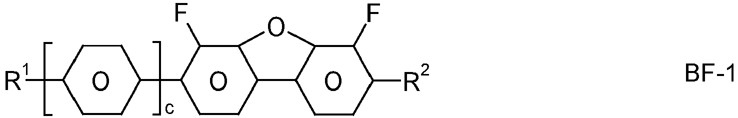 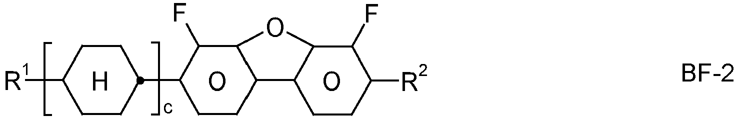 dans lesquelles
R¹ et R² présentent dans chaque cas, de manière indépendante l'un de l'autre, la signification de R^{2A}, et
c représente 1 ou 2.

12. Milieu cristallin liquide selon une ou plusieurs des revendications 1 à 11, **caractérisé en ce que** la proportion de composés de la formule I dans le mélange pris dans sa globalité est comprise entre 1% et 40% en poids.

13. Milieu cristallin liquide selon une ou plusieurs des revendications 1 à 12, **caractérisé en ce que** le milieu comprend au moins un composé polymérisable.

14. Milieu cristallin liquide selon une ou plusieurs des revendications 1 à 13, **caractérisé en ce que** le milieu comprend un ou plusieurs additif(s).

15. Milieu cristallin liquide selon une ou plusieurs des revendications 1 à 14, **caractérisé en ce que** l'additif est sélectionné parmi le groupe qui est constitué par les piégeurs de radicaux libres, les antioxydants et/ou les stabiliseurs UV.

16. Procédé pour la préparation d'un milieu cristallin liquide selon une ou plusieurs des revendications 1 à 15, **caractérisé en ce qu'**au moins un composé de la formule I est mélangé avec au moins un autre composé cristallin liquide, et en option, un ou plusieurs additif(s) et en option, au moins un composé polymérisable est/sont ajouté(s).

17. Utilisation du milieu cristallin liquide selon une ou plusieurs des revendications 1 à 15 dans les affichages électro-optiques.

18. Affichage électro-optique présentant un adressage par matrice active, **caractérisé en ce qu'**il contient, en tant que diélectrique, un milieu cristallin liquide selon une ou plusieurs des revendications 1 à 15.

19. Affichage électro-optique selon la revendication 18, **caractérisé en ce qu'**il s'agit d'un affichage VA, PSA, PA-VA, PS-VA, PALC, IPS, PS-IPS, FFS ou PS-FFS.
